# EUROPEAN PATENT SPECIFICATION

(11) **EP 3 362 075 B1**
(45) Date of publication and mention of the grant of the patent: **07.06.2023**
(21) Application number: 16856249.4
(22) Date of filing: 14.10.2016
(51) Int. Cl.: A61K 35/17, A61P 1/04, A61P 1/16, A61P 1/18, A61P 7/00, A61P 11/00, A61P 13/12, A61P 15/00, A61P 21/00, A61P 27/02, A61P 35/00, A61P 35/02, C12N 5/078, C12N 5/0783

(54) **NATURAL KILLER CELLS AND ILC3 CELLS AND USES THEREOF**
NATÜRLICHE KILLERZELLEN UND ILC3-ZELLEN UND VERWENDUNGEN DAVON
CELLULES TUEUSES NATURELLES ET CELLULES ILC3, ET LEURS UTILISATIONS

(30) Priority: 15.10.2015 US 201562242246 P; 30.12.2015 US 201562272984 P; 03.10.2016 US 201662403571 P
(43) Date of publication of application: 22.08.2018
(73) Proprietor: Celularity Inc., Florham Park, NJ 07932 (US)
(72) Inventor: ZHANG, Xiaokui, Livingston, NJ 07039 (US); DJURETIC, Ivana, Hoboken, NJ 07030 (US); KANG, Lin, Edison, NJ 08820 (US); VOSKINARIAN-BERSE, Vanessa, Millington, NJ 07946 (US); STOUT, Bhavani, Lebanon, NJ 08833 (US); HARIRI, Robert J., Bernardsville, NJ 07924 (US); HOFGARTNER, Wolfgang, Florham Park, NJ 07718 (US); EDINGER, James, Belford, NJ 07718 (US); LAW, Eric, East Brunswick, NJ 08816 (US); JANKOVIC, Vladimir, New York, NY 10044 (US)
(74) Representative: Mewburn Ellis LLP
(86) International application number: PCT/US2016/056988
(87) International publication number: WO 2017/066530

(56) References cited:
- WO-A1-2012/075412
- WO-A1-2012/128622
- WO-A1-2013/119118
- WO-A1-2017/046142
- WO-A2-2014/028453
- MIEKE W.H. ROEVEN ET AL: "The Aryl Hydrocarbon Receptor Antagonist StemRegenin1 Improves In Vitro Generation of Highly Functional Natural Killer Cells from CD34 + Hematopoietic Stem and Progenitor Cells", STEM CELLS AND DEVELOPMENT, vol. 24, no. 24, 28 September 2015 (2015-09-28), pages 2886-2898, XP055561683, ISSN: 1547-3287, DOI: 10.1089/scd.2014.0597
- TIFFANY HUGHES ET AL: "The Transcription Factor AHR Prevents the Differentiation of a Stage 3 Innate Lymphoid Cell Subset to Natural Killer Cells", CELL REPORTS, vol. 8, no. 1, 1 July 2014 (2014-07-01), pages 150-162, XP055561624, US ISSN: 2211-1247, DOI: 10.1016/j.celrep.2014.05.042
- MARTHA LUEVANO ET AL: "Generation of natural killer cells from hematopoietic stem cells in vitro for immunotherapy", CELLULAR & MOLECULAR IMMUNOLOGY, vol. 9, no. 4, 18 June 2012 (2012-06-18), pages 310-320, XP055561634, CH ISSN: 1672-7681, DOI: 10.1038/cmi.2012.17
- VIRGINIE M. RENOUX ET AL: "Identification of a Human Natural Killer Cell Lineage-Restricted Progenitor in Fetal and Adult Tissues", IMMUNITY., vol. 43, no. 2, 1 August 2015 (2015-08-01) , pages 394-407, XP055561573, US ISSN: 1074-7613, DOI: 10.1016/j.immuni.2015.07.011
- SPANHOLTZ ET AL.: 'High log-scale expansion of functional human natural killer cells from umbilical cord blood CD 34-positive cells for adoptive cancer immunotherapy.' PLOS ONE. vol. 5, no. 2, 15 February 2010, pages 1 - 13, XP055376087
- AHN ET AL.: 'Lineage relationships of human interleukin-22-producing CD 56+ RORyt+ innate lymphoid cells and conventional natural killer cells.' BLOOD. vol. 121, no. 12, 21 March 2013, pages 2234 - 43, XP055376089
- MONTALDO ET AL.: 'Human RORyt(+) CD 34(+) cells are lineage-specified progenitors of group 3 RORyt(+) innate lymphoid cells.' IMMUNITY. vol. 41, no. 6, 18 December 2014, pages 988 - 1000, XP055376090

## Description

### 1. FIELD

Provided herein are methods of producing populations of natural killer (NK) cells and/or ILC3 cells from a population of hematopoietic stem or progenitor cells in media comprising stem cell mobilizing factors, *e.g*., three-stage methods of producing NK cells and/or ILC3 cells in media comprising stem cell mobilizing factors starting with hematopoietic stem or progenitor cells from cells of the placenta, for example, from placental perfusate (*e.g*., human placental perfusate) or other tissues, for example, umbilical cord blood or peripheral blood.

Further provided herein are methods of using the placental perfusate, the NK cells and/or ILC3 cells and/or NK progenitor cells described herein, to, *e.g*., suppress the proliferation of tumor cells, or to inhibit pathogen infection, *e.g*., viral infection. In certain embodiments, the NK cells and/or ILC3 cells and/or NK progenitor cells produced by the three-stage methods described herein are used in combination with, and/or treated with, one or more immunomodulatory compounds.

### 2. BACKGROUND

Natural killer (NK) cells are cytotoxic lymphocytes that constitute a major component of the innate immune system.

NK cells are activated in response to interferons or macrophage-derived cytokines. The cytotoxic activity of NK cells is largely regulated by two types of surface receptors, which may be considered "activating receptors" or "inhibitory receptors," although some receptors, *e.g*., CD94 and 2B4 (CD244), can work either way depending on ligand interactions.

Among other activities, NK cells play a role in the host rejection of tumors and have been shown capable of killing virus-infected cells. Natural killer cells can become activated by cells lacking, or displaying reduced levels of, major histocompatibility complex (MHC) proteins. Cancer cells with altered or reduced level of self-class I MHC expression result in induction of NK cell sensitivity. Activated and expanded NK cells, and in some cases LAK cells, from peripheral blood have been used in both *ex vivo* therapy and *in vivo* treatment of patients having advanced cancer, with some success against bone marrow related diseases, such as leukemia; breast cancer; and certain types of lymphoma.

In spite of the advantageous properties of NK cells in killing tumor cells and virus-infected cells, there remains a need in the art to develop efficient methods to produce and expand natural killer cells that retain tumoricidal functions.

NK cells are innate lymphoid cells (ILCs). Innate lymphoid cells are related through their dependency on transcription factor ID2 for development. One type of ILC, known as the ILC3 cell, is described in the literature as expressing RORγt and producing IL-22, as well as playing a role in the immune response of adults, without manifesting cytotoxic effectors such as perforin, granzymes, and death receptors (Montaldo et al., 2014, Immunity 41:988-1000; Killig et al., 2014, Front. Immunol. 5:142; Withers et al., 2012, J. Immunol. 189(5):2094-2098). Roeven wt al., (2015) Stem Cells and Development, 24(24):2886-2898 relates to StemRegenin 1 use in *in vitro* generation of NK cells from CD34+ hematopoietic stem and progenitor cells. Hughes et al., (2014) Cell Reports, 8(1):150-162 relates to the role of aryl hydrocarbon receptor in ILC3 differentiation to NK cells. Luevano et al., (2012) Cellular & Molecular Immunology, 9(4):310-320 relates to the generation of NK cells from hematopoietic stem cells *in vitro* for immunotherapy. Renoux et al., (2015) Immunity, 43(2):394-407 identifies a human NK cell lineage-restricted progenitor in fetal and adult tissues. WO 2013/119118 relates to the ex vivo differentiation of NK cells from CD34+ hematopoietic stem cells. WO 2017/046142 relates to a method for the ex vivo production of a population of highly functional NK cells from CD34+ cells and to the use of such cells for adoptive cell therapy.

### 3. SUMMARY

The present invention is defined in the claims. Disclosed herein are methods of expanding and differentiating cells, for example, hematopoietic cells, such as hematopoietic stem cells, e.g., CD34⁺ hematopoietic stem cells, to produce natural killer (NK) cells and/or ILC3 cells.

In one aspect, provided herein are methods of producing NK cell populations and/or ILC3 cell populations according to the claims that comprise three stages as described herein (and referred to herein as the "three-stage method"). Natural killer cells and/or ILC3 cells produced by the three-stage methods provided herein are referred to herein as "NK cells produced by the three-stage method," "ILC3 cells produced by the three-stage method," or "NK cells and/or ILC3 cells produced by the three-stage method." In certain embodiments, said method comprises one or more further or intermediate steps. In certain embodiments, said method does not comprise any fourth or intermediate step in which the cells are contacted (or cultured).

In one aspect, provided herein is a method according to the claims of producing NK cells comprising culturing hematopoietic stem cells or progenitor cells, *e.g*., CD34⁺ stem cells or progenitor cells, in a first medium comprising a stem cell mobilizing agent and thrombopoietin (Tpo) to produce a first population of cells, subsequently culturing said first population of cells in a second medium comprising a stem cell mobilizing agent and interleukin-15 (IL-15), and lacking Tpo, to produce a second population of cells, and subsequently culturing said second population of cells in a third medium comprising IL-2 and IL-15, and lacking a stem cell mobilizing agent and low-molecular weight heparin (LMWH), to produce a third population of cells, wherein the third population of cells comprises natural killer cells that are CD56+, CD3-, and wherein at least 80%, of the natural killer cells are viable. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, *e.g*., low-molecular weight heparin. In certain embodiments, such natural killer cells comprise natural killer cells that are CD16-. In certain embodiments, such natural killer cells comprise natural killer cells that are CD94+. In certain embodiments, such natural killer cells comprise natural killer cells that are CD94+ or CD16+. In certain embodiments, such natural killer cells comprise natural killer cells that are CD94- or CD16-. In certain embodiments, such natural killer cells comprise natural killer cells that are CD94+ and CD16+. In certain embodiments, such natural killer cells comprise natural killer cells that are CD94- and CD16-. In certain examples, at least one, two, or all three of said first medium, second medium, and third medium are not the medium GBGM9. In certain examples, the third medium lacks added desulphated glycosaminoglycans. In certain embodiments, the third medium lacks desulphated glycosaminoglycans.

In one aspect, provided herein is a method of producing NK cells according to the claims comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising IL-2 and IL-15, and lacking LMWH, to produce a third population of cells; wherein the third population of cells comprises natural killer cells that are CD56+, CD3-, and CD11a+. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, *e.g.*, low-molecular weight heparin.

In one aspect, provided herein is a method according to the claims of producing NK cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising IL-2 and IL-15, and lacking each of stem cell factor (SCF) and LMWH, to produce a third population of cells; wherein the third population of cells comprises natural killer cells that are CD56+, CD3-, and CD11a+. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, e.g., low-molecular weight heparin.

In one aspect, provided herein is a method according to the claims of producing NK cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising IL-2 and IL-15, and lacking each of SCF, a stem cell mobilizing agent, and LMWH, to produce a third population of cells; wherein the third population of cells comprises natural killer cells that are CD56+, CD3-, and CD11a. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, *e.g*., low-molecular weight heparin.

In one aspect, provided herein is a method according to the claims of producing NK cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; (c) culturing the second population of cells in a third medium comprising IL-2 and IL-15, and lacking each of a stem cell mobilizing agent and LMWH, to produce a third population of cells; and (d) isolating CD11a+ cells from the third population of cells to produce a fourth population of cells; wherein the fourth population of cells comprises natural killer cells that are CD56+, CD3-, and CD11a+. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, e.g., low-molecular weight heparin.

In certain embodiments, said natural killer cells express perforin and eomesodermin (EOMES). In certain embodiments, said natural killer cells do not express either RAR-related orphan receptor gamma (RORyt) or interleukin-1 receptor 1 (IL1R1).

Disclosed herein is a method of producing II,C3 cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising IL-2 and IL-15, and lacking LMWH, to produce a third population of cells; wherein the third population of cells comprises ILC3 cells that are CD56+, CD3-, and CD11a-. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, *e.g*., low-molecular weight heparin.

Disclosed herein is a method of producing II,C3 cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising a stem cell mobilizing agent, IL-2 and IL-15, and lacking LMWH, to produce a third population of cells; wherein the third population of cells comprises ILC3 cells that are CD56+, CD3-, and CD11a-. In certain examples, said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, *e.g*., low-molecular weight heparin.

Disclosed herein is a method of producing II,C3 cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising SCF, IL-2 and IL-15, and lacking LMWH, to produce a third population of cells; wherein the third population of cells comprises ILC3 cells that are CD56+, CD3-, and CD11a-. In certain examples, said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, *e.g*., low-molecular weight heparin.

Disclosed herein is a method of producing II,C3 cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising a stem cell mobilizing agent, SCF, IL-2 and IL-15, and lacking LMWH, to produce a third population of cells; wherein the third population of cells comprises ILC3 cells that are CD56+, CD3-, and CD11a-. In certain examples, said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, e.g., low-molecular weight heparin.

In one aspect, provided herein is a method according to the claims of producing II,C3 cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; (c) culturing the second population of cells in a third medium comprising IL-2 and IL-15, and lacking each of a stem cell mobilizing agent and LMWH, to produce a third population of cells; and (d) isolating CD11a- cells from the third population of cells to produce a fourth population of cells; wherein the fourth population of cells comprises ILC3 cells that are CD56+, CD3-, and CD11a-. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, *e.g*., low-molecular weight heparin.

In certain embodiments, said ILC3 cells express RORγt and IL1R1. In certain embodiments, said ILC3 cells do not express either perforin or EOMES. In certain embodiments, said third medium lacks added desulphated glycosaminoglycans. In certain embodiments, said third medium lacks desulphated glycosaminoglycans.

In certain embodiments, said hematopoietic stem or progenitor cells are mammalian cells. In specific embodiments, said hematopoietic stem or progenitor cells are human cells. In specific embodiments, said hematopoietic stem or progenitor cells are primate cells. In specific embodiments, said hematopoietic stem or progenitor cells are canine cells. In specific embodiments, said hematopoietic stem or progenitor cells are rodent cells. In specific embodiments, said hematopoietic stem or progenitor cells are cells from a mammal other than a human, primate, canine or rodent.

In certain aspects, the hematopoietic stem cells or progenitor cells cultured in the first medium are CD34⁺ stem cells or progenitor cells. In certain aspects, the hematopoietic stem cells or progenitor cells are placental hematopoietic stem cells or progenitor cells. In certain aspects, the placental hematopoietic stem cells or progenitor cells are obtained from, or obtainable from placental perfusate (e.g. obtained from or obtainable from isolated nucleated cells from placental perfusate). In certain aspects, said hematopoietic stem or progenitor cells are obtained from, or obtainable from, umbilical cord blood. In certain aspects, said hematopoietic stem or progenitor cells are fetal liver cells. In certain aspects, said hematopoietic stem or progenitor cells are mobilized peripheral blood cells. In certain aspects, said hematopoietic stem or progenitor cells are bone marrow cells.

Said first medium used in the three-stage method comprises a stem cell mobilizing agent and thrombopoietin (Tpo). In certain aspects, the first medium used in the three-stage method comprises, in addition to a stem cell mobilizing agent and Tpo, one or more of Low Molecular Weight Heparin (LMWH), Flt-3 Ligand (Flt-3L), stem cell factor (SCF), IL-6, IL-7, granulocyte colony-stimulating factor (G-CSF), or granulocyte-macrophage-stimulating factor (GM-CSF). In certain aspects, said first medium does not comprise added LMWH. In certain aspects, said first medium does not comprise added desulphated glycosaminoglycans. In certain aspects, said first medium does not comprise LMWH. In certain aspects, said first medium does not comprise desulphated glycosaminoglycans. In certain aspects, the first medium used in the three-stage method comprises, in addition to a stem cell mobilizing agent and Tpo, each of LMWH, Flt-3L, SCF, IL-6, IL-7, G-CSF, and GM-CSF. In certain aspects, the first medium used in the three-stage method comprises, in addition to a stem cell mobilizing agent and Tpo, each of Flt-3L, SCF, IL-6, IL-7, G-CSF, and GM-CSF. In certain aspects, said Tpo is present in the first medium at a concentration of from 1 ng/mL to 100 ng/mL, from 1 ng/mL to 50 ng/mL, from 20 ng/mL to 30 ng/mL, or about 25 ng/mL. In certain aspects, in the first medium, the LMWH is present at a concentration of from 1U/mL to 10U/mL; the Flt-3L is present at a concentration of from 1 ng/mL to 50 ng/mL; the SCF is present at a concentration of from 1 ng/mL to 50 ng/mL; the IL-6 is present at a concentration of from 0.01 ng/mL to 0.1 ng/mL; the IL-7 is present at a concentration of from 1 ng/mL to 50 ng/mL; the G-CSF is present at a concentration of from 0.01 ng/mL to 0.50 ng/mL; and the GM-CSF is present at a concentration of from 0.005 ng/mL to 0.1 ng/mL. In certain aspects, in the first medium, the Flt-3L is present at a concentration of from 1 ng/mL to 50 ng/mL; the SCF is present at a concentration of from 1 ng/mL to 50 ng/mL; the IL-6 is present at a concentration of from 0.01 ng/mL to 0.1 ng/mL; the IL-7 is present at a concentration of from 1 ng/mL to 50 ng/mL; the G-CSF is present at a concentration of from 0.01 ng/mL to 0.50 ng/mL; and the GM-CSF is present at a concentration of from 0.005 ng/mL to 0.1 ng/mL. In certain aspects, in the first medium, the LMWH is present at a concentration of from 4U/mL to 5U/mL; the Flt-3L is present at a concentration of from 20 ng/mL to 30 ng/mL; the SCF is present at a concentration of from 20 ng/mL to 30 ng/mL; the IL-6 is present at a concentration of from 0.04 ng/mL to 0.06 ng/mL; the IL-7 is present at a concentration of from 20 ng/mL to 30 ng/mL; the G-CSF is present at a concentration of from 0.20 ng/mL to 0.30 ng/mL; and the GM-CSF is present at a concentration of from 0.005 ng/mL to 0.5 ng/mL. In certain aspects, in the first medium, the Flt-3L is present at a concentration of from 20 ng/mL to 30 ng/mL; the SCF is present at a concentration of from 20 ng/mL to 30 ng/mL; the IL-6 is present at a concentration of from 0.04 ng/mL to 0.06 ng/mL; the IL-7 is present at a concentration of from 20 ng/mL to 30 ng/mL; the G-CSF is present at a concentration of from 0.20 ng/mL to 0.30 ng/mL; and the GM-CSF is present at a concentration of from 0.005 ng/mL to 0.5 ng/mL. In certain aspects, in the first medium, the LMWH is present at a concentration of about 4.5U/mL; the Flt-3L is present at a concentration of about 25 ng/mL; the SCF is present at a concentration of about 27 ng/mL; the IL-6 is present at a concentration of about 0.05 ng/mL; the IL-7 is present at a concentration of about 25 ng/mL; the G-CSF is present at a concentration of about .25 ng/mL; and the GM-CSF is present at a concentration of about 0.01 ng/mL. In certain aspects, in the first medium, the Flt-3L is present at a concentration of about 25 ng/mL; the SCF is present at a concentration of about 27 ng/mL; the IL-6 is present at a concentration of about 0.05 ng/mL; the IL-7 is present at a concentration of about 25 ng/mL; the G-CSF is present at a concentration of about .25 ng/mL; and the GM-CSF is present at a concentration of about 0.01 ng/mL. In certain embodiments, said first medium is not GBGM^{®}.

Said second medium used in the three-stage method comprises a stem cell mobilizing agent and interleukin-15 (IL-15), and lacks Tpo. In certain aspects, the second medium used in the three-stage method comprises, in addition to a stem cell mobilizing agent and IL-15, one or more of LMWH, Flt-3, SCF, IL-6, IL-7, G-CSF, and GM-CSF. In certain aspects, the second medium does not comprise added LMWH. In certain aspects, the second medium does not comprise added desulphated glycosaminoglycans. In certain aspects, the second medium does not comprise LMWH. In certain aspects, the second medium does not comprise desulphated glycosaminoglycans. In certain aspects, the second medium used in the three-stage method comprises, in addition to a stem cell mobilizing agent and IL-15, each of LMWH, Flt-3, SCF, IL-6, IL-7, G-CSF, and GM-CSF. In certain aspects, the second medium used in the three-stage method comprises, in addition to a stem cell mobilizing agent and IL-15, each of Flt-3, SCF, IL-6, IL-7, G-CSF, and GM-CSF. In certain aspects, said IL-15 is present in said second medium at a concentration of from 1 ng/mL to 50 ng/mL, from 10 ng/mL to 30 ng/mL, or about 20 ng/mL. In certain aspects, in said second medium, the LMWH is present at a concentration of from 1U/mL to 10U/mL; the Flt-3L is present at a concentration of from 1 ng/mL to 50 ng/mL; the SCF is present at a concentration of from 1 ng/mL to 50 ng/mL; the IL-6 is present at a concentration of from 0.01 ng/mL to 0.1 ng/mL; the IL-7 is present at a concentration of from 1 ng/mL to 50 ng/mL; the G-CSF is present at a concentration of from 0.01 ng/mL to 0.50 ng/mL; and the GM-CSF is present at a concentration of from 0.005 ng/mL to 0.1 ng/mL. In certain aspects, in the second medium, the LMWH is present in the second medium at a concentration of from 4U/mL to 5U/mL; the Flt-3L is present at a concentration of from 20 ng/mL to 30 ng/mL; the SCF is present at a concentration of from 20 ng/mL to 30 ng/mL; the IL-6 is present at a concentration of from 0.04 ng/mL to 0.06 ng/mL; the IL-7 is present at a concentration of from 20 ng/mL to 30 ng/mL; the G-CSF is present at a concentration of from 0.20 ng/mL to 0.30 ng/mL; and the GM-CSF is present at a concentration of from 0.005 ng/mL to 0.5 ng/mL. In certain aspects, in the second medium, the Flt-3L is present at a concentration of from 20 ng/mL to 30 ng/mL; the SCF is present at a concentration of from 20 ng/mL to 30 ng/mL; the IL-6 is present at a concentration of from 0.04 ng/mL to 0.06 ng/mL; the IL-7 is present at a concentration of from 20 ng/mL to 30 ng/mL; the G-CSF is present at a concentration of from 0.20 ng/mL to 0.30 ng/mL; and the GM-CSF is present at a concentration of from 0.005 ng/mL to 0.5 ng/mL. In certain aspects, in the second medium, the LMWH is present in the second medium at a concentration of from 4U/mL to 5U/mL; the Flt-3L is present at a concentration of from 20 ng/mL to 30 ng/mL; the SCF is present at a concentration of from 20 ng/mL to 30 ng/mL; the IL-6 is present at a concentration of from 0.04 ng/mL to 0.06 ng/mL; the IL-7 is present at a concentration of from 20 ng/mL to 30 ng/mL; the G-CSF is present at a concentration of from 0.20 ng/mL to 0.30 ng/mL; and the GM-CSF is present at a concentration of from 0.005 ng/mL to 0.5 ng/mL. In certain aspects, in the second medium, the Flt-3L is present at a concentration of from 20 ng/mL to 30 ng/mL; the SCF is present at a concentration of from 20 ng/mL to 30 ng/mL; the IL-6 is present at a concentration of from 0.04 ng/mL to 0.06 ng/mL; the IL-7 is present at a concentration of from 20 ng/mL to 30 ng/mL; the G-CSF is present at a concentration of from 0.20 ng/mL to 0.30 ng/mL; and the GM-CSF is present at a concentration of from 0.005 ng/mL to 0.5 ng/mL. In certain aspects, in the second medium, the LMWH is present in the second medium at a concentration of about 4.5U/mL; the Flt-3L is present at a concentration of about 25 ng/mL; the SCF is present at a concentration of about 27 ng/mL; the IL-6 is present at a concentration of about 0.05 ng/mL; the IL-7 is present at a concentration of about 25 ng/mL; the G-CSF is present at a concentration of about 0.25 ng/mL; and the GM-CSF is present at a concentration of about 0.01 ng/mL. In certain aspects, in the second medium, the Flt-3L is present at a concentration of about 25 ng/mL; the SCF is present at a concentration of about 27 ng/mL; the IL-6 is present at a concentration of about 0.05 ng/mL; the IL-7 is present at a concentration of about 25 ng/mL; the G-CSF is present at a concentration of about 0.25 ng/mL; and the GM-CSF is present at a concentration of about 0.01 ng/mL. In certain embodiments, said second medium is not GBGM^{®}.

The stem cell mobilizing factor present in said first and second medium of the present invention is StemRegenin-1 (SR-1) (4-(2-(2-(benzo[b]thiophen-3-yl)-9-isopropyl-9H-purin-6-ylamino)ethyil)phenol), or the compound CH223191 (1-Methyl-N-[2-methyl-4-[2-(2-methylphenyl)diazenyl]phenyl-1H-pyrazole-5-carboxamide].

An aryl hydrocarbon receptor inhibitor may be resveratrol. An aryl hydrocarbon receptor inhibitor may be compound of the formula 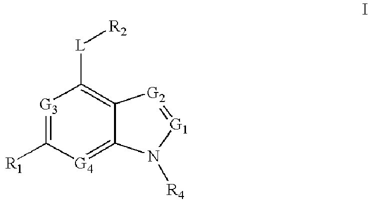 in which:
G₁ is selected from N and CR₃;
G₂, G₃ and G₄ are independently selected from CH and N; with the proviso that at least 1 of G₃ and G₄ is N; with the proviso that G₁ and G₂ are not both N;
L is selected from --NR₅ₐ(CH₂)₀₋₃--, --NR₅ₐCH(C(O)OCH₃)CH₂--, --NR₅ₐ(CH2)2NR_{5b}--,-NR₅ₐ(CH₂)₂S-, --NR₅ₐCH₂CH(CH₃)CH₂--, --NR₅ₐCH₂CH(OH)-- and-NR₅ₐCH(CH₃)CH₂--; wherein R₅ₐ and R_{5b} are independently selected from hydrogen and C₁₋₄alkyl;
R₁ is selected from hydrogen, phenyl, thiophenyl, furanyl, 1H-benzoimidazolyl, isoquinolinyl, 1H-imidazopyridinyl, benzothiophenyl, pyrimidinyl, 1H-pyrazolyl, pyridinyl, 1H-imidazolyl, pyrrolidinyl, pyrazinyl, pyridazinyl, 1H-pyrrolyl and thiazolyl; wherein said phenyl, thiophenyl, furanyl, 1H-benzoimidazolyl, isoquinolinyl, 1H-imidazopyridinyl, benzothiophenyl, pyrimidinyl, 1H-pyrazolyl, pyridinyl, 1H-imidazolyl, pyrrolidinyl, pyrazinyl, pyridazinyl, 1H-pyrrolyl or thiazolyl of R₁ can be optionally substituted by 1 to 3 radicals independently selected from cyano, hydroxy, C₁₋₄alkyl, C₁₋₄alkoxy, halo, halo-substituted-C₁₋₄alkyl, halo-substituted-C₁₋₄alkoxy, hydroxy, amino, --C(O)R₈ₐ, --S(O)₀₋₂R₈ₐ, --C(O)OR₈ₐ and --C(O)NR₈ₐR_{8b}; wherein R₈ₐ and R_{8b} are independently selected from hydrogen and C₁₋₄alkyl; with the proviso that R₁ and R₃ are not both hydrogen;
R₂ is selected from --S(O)₂NR₆ₐR_{6b}, --NR₉ₐC(O)R_{9b}, --NR₆ₐC(O)NR_{6b}R_{6c}, phenyl, 1H-pyrrolopyridin-3-yl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl and 1H-indazolyl; wherein R₆ₐ, R_{6b} and R_{6c} are independently selected from hydrogen and C₁₋₄alkyl; wherein said phenyl, 1H-pyrrolopyridin-3-yl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl or 1H-indazolyl of R₂ is optionally substituted with 1 to 3 radicals independently selected from hydroxy, halo, methyl, methoxy, amino,-O(CH₂)ₙNR₇ₐR_{7b}, -S(O)₂NR₇ₐR_{7b}, --OS(O)₂NR₇ₐR_{7b} and --NR₇ₐS(O)₂R_{7b}; wherein R₇ₐ and R_{7b} are independently selected from hydrogen and C₁₋₄alkyl;
R₃ is selected from hydrogen, C₁₋₄alkyl and biphenyl; and
R₄ is selected from C₁₋₁₀alkyl, prop-1-en-2-yl, cyclohexyl, cyclopropyl, 2-(2-oxopyrrolidin-1-yl)ethyl, oxetan-3-yl, benzhydryl, tetrahydro-2H-pyran-3-yl, tetrahydro-2H-pyran-4-yl, phenyl, tetrahydrofuran-3-yl, benzyl, (4-pentylphenyl)(phenyl)methyl and 1-(1-(2-oxo-6,9,12-trioxa-3-azatetradecan-14-yl)-1H-1,2,3-triazol-4-yl)ethyl; wherein said alkyl, cyclopropyl, cyclohexyl, 2-(2-oxopyrrolidin-1-yl)ethyl, oxetan-3-yl, oxetan-2-yl, benzhydryl, tetrahydro-2H-pyran-2-yl, tetrahydro-2H-pyran-3-yl, tetrahydro-2H-pyran-4-yl, phenyl, tetrahydrofuran-3-yl, tetrahydrofuran-2-yl, benzyl, (4-pentylphenyl)(phenyl)methyl or 1-(1-(2-oxo-6,9,12-trioxa-3-azatetradecan-14-yl)-1H-1,2,3-triazol-4-yl)ethyl can be optionally substituted with 1 to 3 radicals independently selected from hydroxy, C₁₋₄alkyl and halo-substituted-C₁₋₄alkyl; or a salt thereof.

In certain aspects, said aryl hydrocarbon receptor inhibitor is StemRegenin-1 (SR-1) (4-(2-(2-(benzo[b]thiophen-3-yl)-9-isopropyl-9H-purin- 6-ylamino)ethyl)phenol). In certain aspects, said aryl hydrocarbon receptor inhibitor is the compound CH223191 (1-Methyl-N-[2-methyl-4-[2-(2-methylphenyl)diazenyl]phenyl-1H-pyrazole-5-carboxamide].

In certain aspects, the stem cell mobilizing factor present in said first medium, said second medium, or said first and second mediums is a pyrimido(4,5-b)indole derivative. In certain aspects, said pyrimido(4,5-b)indole derivative is one or more of: 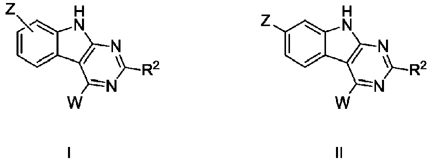 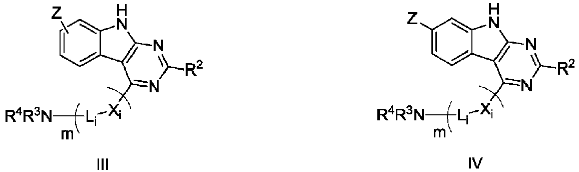 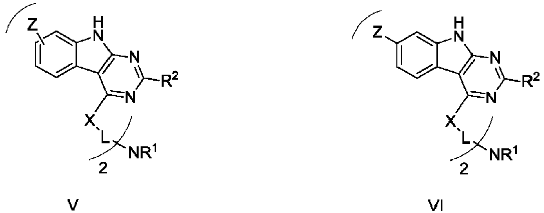 or a salt or a prodrug thereof, wherein:
Z is
   1) -P(O) (OR<1>) (OR<1>),
   2) -C(0)OR<1>,
   3) -C(0)NHR<1>,
   4) -C(0)N(R)R<1>,
   5) -C(0)R<1>,
   6) -CN,
   7) -SR,
   8) -S(0)2NH2,
   9) -S(0)2NHR<1>,
   10) -S(0)2N(R)R<1>,
   11) -S(0)R<1>,
   12) -S(0)2R<1>,
   13) -L,
   14) -benzyl optionally substituted with 1 , 2 or 3 R<A> or R<1> substituents,
   15) -L-heteroaryl optionally substituted with one or more R<A> or R<1> substituents attached on either or both the L and the heteroaryl groups,
   16) -L-heterocyclyl optionally substituted with one or more R<A> or R<1> substituents attached on either one or both the L and the heterocyclyl groups,
   17) -L-aryl optionally substituted with one or more R<A> or R<1> substituents attached on either or both the L and the heteroaryl groups,
   18) -heteroaryl optionally substituted with one or more R<A> or R<1> substituents, or
   19) -aryl optionally substituted with one or more R<A> or R<1> substituents,
and wherein each substituent is optionally attached to the L group if it is not already present, and wherein, when (R<1>) and R<1> are attached to a nitrogen atom, optionally they join together with the nitrogen atom to form a 3 to 7-membered ring which optionally includes one or more other heteroatom selected from N, 0 and S, optionally the is substituted with one or more R<1> or R<A>;
Wis
   1) -H,
   2) -halogen,
   3) -OR<1>,
   4) -L-OH,
   5) -L-OR<1>,
   6) -SR<1>,
   7) -CN,
   8) -P(0)(OR<1>)(OR<1>),
   9) -NHR<1>,
   10) -N(R<1>)R<1>,
   11) -L-NH2,
   12) -L-NHR<1>,
   13) -L-N(R<1>)R<1>,
   14) -L-SR<1>,
   15) -L-S(0)R<1>,
   16) -L-S(0)2R<1>,
   17) -L-P(0)(OR<1>)(OR<1>
   18) -C(0)OR<1>,
   19) -C(0)NH2,
   20) -C(0)NHR<1>,
   21) -C(0)N(R<1>)R<1>,
   22) -NHC(0)R<1>,
   23) -NR1C(0)R<1>, -NHC(0)0R<1>,

      -NR1C(0)0R<1>,

      -0C(0)NH2,

      -0C(0)NHR<1>,

      -0C(0)N(R)R<1>,

      -0C(0)R<1>,

      -C(0)R<1>,

      -NHC(0)NH2,

      -NHC(0)NHR<1>,

      -NHC(0)N(R)R<1>,

      -NR C(0)NH2,

      -NR C(0)NHR<1>,

      -NR C(0)N(R )R<1>,

      -NHS(0)2R<1>,

      -NR S(0)2R<1>,

      -S(0)2NH2,

      -S(0)2NHR<1>,

      -S(0)2N(R )R<1>,

      -S(0)R<1>,

      -S(0)2R<1>,

      -0S(0)2R1 ,

      -S(0)20R<1>,

      - benzyl optionally substituted with 1 , 2 or 3 R<A> or R<1> substituents,
      - L-heteroaryl optionally substituted with one or more R<A> or R<1> substituents attached on either or both the L and the heteroaryl groups,
      - L-heterocyclyl optionally substituted with one or more R<A> or R<1> substituents attached on either or both the L and the heterocyclyl goups,
      - L-aryl optionally substituted with one or more R<A> or R<1> substituents attached on either or both the L and aryl groups,
      - L-NR<1>(R<1>),
      - L-)2 NR<1>,
      - L-(N(R1)-L)n - N(R1)R1 , -L-(N(R<1>)-L)n - heteroaryl optionally substituted with one or more R<A> or R<1> substituents attached on either or both the L and heteroaryl groups,
      - L-(N(R<1>)-L)n - heterocyclyl optionally substituted with one or more R<A> or R<1> substituents attached on either or both the L and heterocyclyl groups,
      - L-(N(R<1>)-L)n - aryl optionally substituted with one or more R<A> or R<1> substituents attached on either or both the L and aryl groups,
      - 0-L-N(R )R<1>,
      - 0-L- heteroaryl optionally substituted with one or more R<A> or R<1> substituents attached on either or both the L and heteroaryl groups,
      - 0-L- heterocyclyl optionally substituted with one or more R<A> or R<1> substituents attached on either or both the L and heterocyclyl groups,
      - 0-L- aryl optionally substituted with one or more R<A> or R<1> substituents attached on either or both the L and aryl groups,
      - 0-L)2-NR<1>,
      - 0-L-(N(R )-L)n - N(R )R<1> ,
      - 0-L-(N(R<1>)-L)n - heteroaryl optionally substituted with one or more R<A> or R<1> substituents attached on either or both the L and heteroaryl groups,
      - 0-L-(N(R<1>)-L)n - heterocyclyl optionally substituted with one or more R<A> or R<1> substituents attached on either or both the L and heterocyclyl groups,
      - 0-L-(N(R<1>)-L)n- aryl optionally substituted with one or more R<A> or R<1> substituents,
      - S-L- heteroaryl optionally substituted with one or more R<A> or R<1> substituents,
      - S-L- heterocyclyl optionally substituted with one or more R<A> or R<1> substituents,
      - S-L- aryl optionally substituted with one or more R<A> or R<1> substituents attached on either or both the L and aryl groups,
      - S-L)2 NR1 ,
      - S-L-(N(R1)-L)"- N(R1)R1,
      - S-L-(N(R<1>)-L)n - heteroaryl optionally substituted with one or more R<A> substituents, -S-L-(N(R<1>)-L)n - heterocyclyl optionally substituted with one or more R<A> substituents, -S-L-(N(R<1>)-L)n - aryl optionally substituted with one or more R<A> substituents,
      - NR<1>(R<1>),
      - (N(R1)-L)n - N(R1)R1 ,
      - N(R1)L)2 -NR1 , 76) -(N(R1)-L)"- N(R1)RA,
   77) -(N(R<1>)-L)n - heteroaryl optionally substituted with one or more R<A> or R<1> substituents,
   78) -(N(R<1>)-L)n - heterocyclyl optionally substituted with one or more R<A> or R<1> substituents,
   79) -(N(R<1>)-L)n - aryl optionally substituted with one or more R<A> or R<1> substituents,
   80) -heteroaryl optionally substituted with one or more R<A> substituents, or
   81) -aryl optionally substituted with one or more R<A> substituents,
and wherein each substituent is optionally attached to the L group if it is not already present, and wherein when two R<1> substituents are present on the same nitrogen atom, then each R<1> substituent is independently selected from the list of R<1> values described thereafter,
and wherein n is an integer equal to either 0, 1 , 2, 3, 4, or 5,
and wherein, when (R<1>) and R<1> are attached to a nitrogen atom, optionally they join together with the nitrogen atom to form a 3 to 7-membered ring which optionally includes one or more other heteroatom selected from N, 0 and S, optionally the ring is substituted with one or more R<1> or R<A>;
L is
   1) -Ci-6 alkyl,
   2) -C2-6 alkenyl,
   3) -C2-6 alkynyl,
   4) -C3-7 cycloalkyi,
   5) -C3-7 cycloalkenyl,
   6) heterocyclyl,
   7) -Ci-6 alkyl-C3-7 cycloalkyi,
   8) -Ci-6 alkyl-heterocyclyl,
   9) aryl, or
   10) heteroaryl,
and wherein the alkyl, the alkenyl, the alkynyl, the cycloalkyi, the cycloalkenyl, the heterocyclyl, the aryl and the heteroaryl groups are each independently optionally substituted with one or two R<A> substituent;
Ri is
   1) -H,
   2) -C1-6 alkyl,
   3) -C2-6 alkenyl,
   4) -C2-6 alkynyl, 5) -C3-7 cycloalkyl,
   6) -C3-7 cycloalkenyl,
   7) -C1-5 perfluorinated,
   8) -heterocydyl,
   9) -aryl,
   10) -heteroaryl,
   11) -benzyl, or
   12) 5-[(3aS,4S,6aR)-2-oxohexahydro-1 H-thieno[3,4-d]imidazol-4-yl]pentanoyl,
and wherein the alkyi, the alkenyl, the alkynyl, the cycloalkenyl, the perfluorinated alkyi, the heterocydyl, the aryl, the heteroaryl and the benzyl groups are each independently optionally substituted with 1 , 2 or 3 R<A> or R<1> substituents;
R2 is
   1) -H,
   2) -C1-6 alkyi,
   3) -SR,
   4) -C(0)R1,
   5) -S(0)R1,
   6) -S(0)2R<1>,
   7) -benzyl optionally substituted with 1 , 2 or 3 R<A> or R<1> substituents,
   8) -L-heteroaryl optionally substituted with one or more R<A> or R<1> substituents attached on either one or both the L and the heteroaryl groups,
   9) -L-heterocyclyl optionally substituted with one or more R<A> or R<1> substituents attached on either one or both the L and the heterocydyl groups,
   10) -L-aryl optionally substituted with one or more R<A> or R<1> substituents attached on either one or both the L and the aryl groups,
   11) -heteroaryl optionally substituted with one or more R<A> or R<1> substituents, or
   12) -aryl optionally substituted with one or more R<A> or R<1> substituents,
and wherein each substituent is optionally attached to the L group if it is not already present;
R<A> is
   1) -halogen,
   2) -CFs, 3) -OH,
   4) -OR<1>,
   5) -L-OH,
   6) -L-OR<1>,
   7) -OCFs,
   8) -SH,
   9) -SR1 ,
   10) -CN,
   11 ) -NO2,
   12) -NH2,
   13) -NHR<1>,
   14) -NR<1>R<1>,
   15) -L-NH2,
   16) -L-NHR<1>,
   17) -L-NR<4>R<1>,
   18) -L-SR<1>,
   19) -L-S(0)R<1>,
   20) -L-S(0)2R<1>,
   21 ) -C(0)OH,
   22) -C(0)OR<1>,
   23) -C(0)NH2,
   24) -C(0)NHR<1>,
   25) -C(0)N(R<1>)R<1>,
   26) -NHC(0)R<1>,
   27) -NR1C(0)R<1>,
   28) -NHC(0)OR<1>,
   29) -NR1C(0)0R<1>,
   30) -OC(0)NH2,
   31) -OC(0)NHR<1>,
   32) -OC(0)N(R )R<1>,
   33) -OC(0)R<1>,
   34) -C(0)R1, 35) -NHC(0)NH2,
   36) -NHC(0)NHR1,
   37) -NHC(0)N(R )R<1>,
   38) -NR C(0)NH2,
   39) -NR C(0)NHR<1>
   40) -NR1C(0)N(R1)R1,
   41 ) -NHS(0)2R<1>,
   42) -NR S(0)2R<1>,
   43) -S(0)2NH2,
   44) -S(0)2NHR<1>,
   45) -S(0)2N(R )R<1>,
   46) -S(0)R<1>,
   47) -S(0)2R<1>,
   48) -0S(0)2R<1>,
   49) -S(0)20R<1>,
   50) -benzyl,
   51) -N3, or
   52) -C(-N=N-)(CF3),
and wherein the benzyl group is optionally substituted with 1 , 2 or 3 R<A> or R<1> substituents.

In certain aspects, said pyrimido(4,5-b)indole derivative has the chemical structure

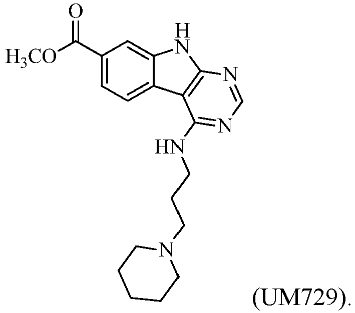

In cetain aspects, said pyrimido(4,5-b)indole derivative has the chemical structure

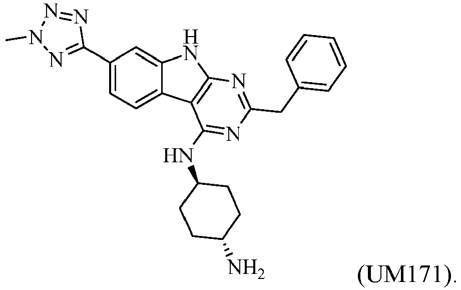

Said third medium used in the three-stage method comprises IL-2 and IL-15, and lacks a stem cell mobilizing agent and LMWH. In certain aspects, the third medium used in the three-stage method comprises, in addition to IL-2 and IL-15, one or more of SCF, IL-6, IL-7, G-CSF, or GM-CSF. In certain aspects, the third medium used in the three-stage method comprises, in addition to IL-2 and IL-15, each of SCF, IL-6, IL-7, G-CSF, and GM-CSF. In certain aspects, said IL-2 is present in said third medium at a concentration of from 10 U/mL to 10,000 U/mL and said IL-15 is present in said third medium at a concentration of from 1 ng/mL to 50 ng/mL. In certain aspects, said IL-2 is present in said third medium at a concentration of from 100 U/mL to 10,000 U/mL and said IL-15 is present in said third medium at a concentration of from 1 ng/mL to 50 ng/mL. In certain aspects, said IL-2 is present in said third medium at a concentration of from 300 U/mL to 3,000 U/mL and said IL-15 is present in said third medium at a concentration of from 10 ng/mL to 30 ng/mL. In certain aspects, said IL-2 is present in said third medium at a concentration of about 1,000 U/mL and said IL-15 is present in said third medium at a concentration of about 20 ng/mL. In certain aspects, in said third medium, the SCF is present at a concentration of from 1 ng/mL to 50 ng/mL; the IL-6 is present at a concentration of from 0.01 ng/mL to 0.1 ng/mL; the IL-7 is present at a concentration of from 1 ng/mL to 50 ng/mL; the G-CSF is present at a concentration of from 0.01 ng/mL to 0.50 ng/mL; and the GM-CSF is present at a concentration of from 0.005 ng/mL to 0.1 ng/mL. In certain aspects, in said third medium, the SCF is present at a concentration of from 20 ng/mL to 30 ng/mL; the IL-6 is present at a concentration of from 0.04 ng/mL to 0.06 ng/mL; the IL-7 is present at a concentration of from 20 ng/mL to 30 ng/mL; the G-CSF is present at a concentration of from 0.20 ng/mL to 0.30 ng/mL; and the GM-CSF is present at a concentration of from 0.005 ng/mL to 0.5 ng/mL. In certain aspects, in said third medium, the SCF is present at a concentration of about 22 ng/mL; the IL-6 is present at a concentration of about 0.05 ng/mL; the IL-7 is present at a concentration of about 20 ng/mL; the G-CSF is present at a concentration of about 0.25 ng/mL; and the GM-CSF is present at a concentration of about 0.01 ng/mL. In certain embodiments, said third medium is not GBGM^{®}.

In certain aspects, the third medium comprises 100 ng/mL IL-7, 22 ng/mL SCF, 1000 ng/mL IL-2, and 20 ng/mL IL-15 and lacks SR1. In certain aspects, the third medium comprises 22 ng/mL SCF, 1000 ng/mL IL-2, and 20 ng/mL IL-15 and lacks SR1. In certain aspects, the third medium comprises 20 ng/mL IL-7, 22 ng/mL SCF, 1000 ng/mL IL-2, and 20 ng/mL IL-15 and lacks SR1. In certain aspects, the third medium comprises 20 ng/mL IL-7, 22 ng/mL SCF, and 1000 ng/mL IL-2 and lacks SR1.

In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, *e.g*., low-molecular weight heparin.

Generally, the particularly recited medium components do not refer to possible constituents in an undefined component of said medium, e.g., serum. For example, said Tpo, IL-2, and IL-15 are not comprised within an undefined component of the first medium, second medium or third medium, *e.g*., said Tpo, IL-2, and IL-15 are not comprised within serum. Further, said LMWH, Flt-3, SCF, IL-6, IL-7, G-CSF, and/or GM-CSF are not comprised within an undefined component of the first medium, second medium or third medium, *e.g*., said LMWH, Flt-3, SCF, IL-6, IL-7, G-CSF, and/or GM-CSF are not comprised within serum.

In certain aspects, said first medium, second medium or third medium comprises human serum-AB. In certain aspects, any of said first medium, second medium or third medium comprises 1% to 20% human serum-AB, 5% to 15% human serum-AB, or about 2, 5, or 10% human serum-AB.

In certain aspects, any of said first medium, second medium or third medium comprises 2-mercaptoethanol. In certain aspects, any of said first medium, second medium or third medium comprises gentamycin.

In certain embodiments, in the three-stage methods described herein, said hematopoietic stem or progenitor cells are cultured in said first medium for 7, 8, 9, 10, 11, 12, or 13 days before said culturing in said second medium. In certain embodiments, cells are cultured in said second medium for 2, 3, 4, 5, or 6 days before said culturing in said third medium. In certain embodiments, cells are cultured in said third medium for 10, 11, 12, 13, 14, 15, 16, 17, 18, 19, 20, 21, 22, 23, 24, 25, 26, 27, 28, 29, or 30 days,.

Said hematopoietic stem or progenitor cells are cultured in said first medium for 7-13 days to produce a first population of cells; said first population of cells are cultured in said second medium for 2-6 days to produce a second population of cells; and said second population of cells are cultured in said third medium for 10-30 days, *i.e.,* the cells are cultured a total of 19-49 days.

In one embodiment, in the three-stage methods described herein, said hematopoietic stem or progenitor cells are cultured in said first medium for 8-12 days to produce a first population of cells; said first population of cells are cultured in said second medium for 3-5 days to produce a second population of cells; and said second population of cells are cultured in said third medium for 15-25 days, *i.e.,* the cells are cultured a total of 26-42 days.

In a specific embodiment, in the three-stage methods described herein, said hematopoietic stem or progenitor cells are cultured in said first medium for about 10 days to produce a first population of cells; said first population of cells are cultured in said second medium for about 4 days to produce a second population of cells; and said second population of cells are cultured in said third medium for about 21 days, *i.e.,* the cells are cultured a total of about 35 days.

In certain aspects, said culturing in said first medium, second medium and third medium are all performed under static culture conditions, e.g., in a culture dish or culture flask. In certain aspects, said culturing in at least one of said first medium, second medium or third medium are performed in a spinner flask. In certain aspects, said culturing in said first medium and said second medium is performed under static culture conditions, and said culturing in said third medium is performed in a spinner flask.

In certain aspects, said culturing is performed in a spinner flask. In other aspects, said culturing is performed in a G-Rex device. In yet other aspects, said culturing is performed in a WAVE bioreactor.

In certain aspects, said hematopoietic stem or progenitor cells are initially inoculated into said first medium from 1 × 10⁴ to 1 × 10⁵ cells/mL. In a specific aspect, said hematopoietic stem or progenitor cells are initially inoculated into said first medium at about 3 × 10⁴ cells/mL.

In certain aspects, said first population of cells are initially inoculated into said second medium from 5 × 10⁴ to 5 × 10⁵ cells/mL. In a specific aspect, said first population of cells is initially inoculated into said second medium at about 1 × 10⁵ cells/mL.

In certain aspects said second population of cells is initially inoculated into said third medium from 1 × 10⁵ to 5 × 10⁶ cells/mL. In certain aspects, said second population of cells is initially inoculated into said third medium from 1 × 10⁵ to 1 × 10⁶ cells/mL. In a specific aspect, said second population of cells is initially inoculated into said third medium at about 5 × 10⁵ cells/mL. In a more specific aspect, said second population of cells is initially inoculated into said third medium at about 5 × 10⁵ cells/mL in a spinner flask. In a specific aspect, said second population of cells is initially inoculated into said third medium at about 3 × 10⁵ cells/mL. In a more specific aspect, said second population of cells is initially inoculated into said third medium at about 3 × 10⁵ cells/mL in a static culture.

In certain aspects, the three-stage method disclosed herein produces at least 5000-fold more natural killer cells as compared to the number of hematopoietic stem cells initially inoculated into said first medium. In certain aspects, said three-stage method produces at least 10,000-fold more natural killer cells as compared to the number of hematopoietic stem cells initially inoculated into said first medium. In certain aspects, said three-stage method produces at least 50,000-fold more natural killer cells as compared to the number of hematopoietic stem cells initially inoculated into said first medium. In certain aspects, said three-stage method produces at least 75,000-fold more natural killer cells as compared to the number of hematopoietic stem cells initially inoculated into said first medium. In certain aspects, the viability of said natural killer cells is determined by 7-aminoactinomycin D (7AAD) staining. In certain aspects, the viability of said natural killer cells is determined by annexin-V staining. In specific aspects, the viability of said natural killer cells is determined by both 7-AAD staining and annexin-V staining. In certain aspects, the viability of said natural killer cells is determined by trypan blue staining.

In certain aspects, the three-stage method disclosed herein produces at least 5000-fold more ILC3 cells as compared to the number of hematopoietic stem cells initially inoculated into said first medium. In certain aspects, said three-stage method produces at least 10,000-fold more ILC3 cells as compared to the number of hematopoietic stem cells initially inoculated into said first medium. In certain aspects, said three-stage method produces at least 50,000-fold more ILC3 cells as compared to the number of hematopoietic stem cells initially inoculated into said first medium. In certain aspects, said three-stage method produces at least 75,000-fold more ILC3 cells as compared to the number of hematopoietic stem cells initially inoculated into said first medium.

In certain aspects, the three-stage method disclosed herein produces natural killer cells that comprise at least 20% CD56+CD3- natural killer cells. In certain aspects, the three-stage method produces natural killer cells that comprise at least 40% CD56+CD3- natural killer cells. In certain aspects, the three-stage method produces natural killer cells that comprise at least 60% CD56+CD3- natural killer cells. In certain aspects, the three-stage method produces natural killer cells that comprise at least 70% CD56+CD3- natural killer cells. In certain aspects, the three-stage method produces natural killer cells that comprise at least 75% CD56+CD3- natural killer cells. In certain aspects, the three-stage method produces natural killer cells that comprise at least 80% CD56+CD3- natural killer cells.

In certain aspects, the three-stage method disclosed herein produces natural killer cells that comprise at least 20% CD56+CD3-CD11a+ natural killer cells. In certain aspects, the three-stage method disclosed herein produces natural killer cells that comprise at least 40% CD56+CD3- CD11a+ natural killer cells. In certain aspects, the three-stage method disclosed herein produces natural killer cells that comprise at least 60% CD56+CD3- CD11a+ natural killer cells. In certain aspects, the three-stage method disclosed herein produces natural killer cells that comprise at least 80% CD56+CD3- CD11a+ natural killer cells.

In certain aspects, the three-stage method disclosed herein produces ILC3 cells that comprise at least 20% CD56+CD3- CD11a- ILC3 cells. In certain aspects, the three-stage method disclosed herein produces ILC3 cells that comprise at least 40% CD56+CD3- CD11a-ILC3 cells. In certain aspects, the three-stage method disclosed herein produces ILC3 cells that comprise at least 60% CD56+CD3- CD11a- ILC3 cells. In certain aspects, the three-stage method disclosed herein produces natural killer cells that comprise at least 80% CD56+CD3-CD1 1a-ILC3 cells.

In certain aspects, the three-stage method disclosed herein, produces natural killer cells that exhibit at least 20% cytotoxicity against K562 cells when said natural killer cells and said K562 cells are co-cultured *in vitro* at a ratio of 10:1. In certain aspects, the three-stage method produces natural killer cells that exhibit at least 35% cytotoxicity against the K562 cells when said natural killer cells and said K562 cells are co-cultured *in vitro* at a ratio of 10:1. In certain aspects, the three-stage method produces natural killer cells that exhibit at least 45% cytotoxicity against the K562 cells when said natural killer cells and said K562 cells are co-cultured *in vitro* at a ratio of 10:1. In certain aspects, the three-stage method produces natural killer cells that exhibit at least 60% cytotoxicity against the K562 cells when said natural killer cells and said K562 cells are co-cultured *in vitro* at a ratio of 10:1. In certain aspects, the three-stage method produces natural killer cells that exhibit at least 75% cytotoxicity against the K562 cells when said natural killer cells and said K562 cells are co-cultured *in vitro* at a ratio of 10:1.

In certain aspects, the three-stage method disclosed herein, produces ILC3 cells that exhibit at least 20% cytotoxicity against K562 cells when said ILC3 cells and said K562 cells are co-cultured *in vitro* at a ratio of 10:1. In certain aspects, the three-stage method produces ILC3 cells that exhibit at least 35% cytotoxicity against the K562 cells when said ILC3 cells and said K562 cells are co-cultured *in vitro* at a ratio of 10:1. In certain aspects, the three-stage method produces ILC3 cells that exhibit at least 45% cytotoxicity against the K562 cells when said ILC3 cells and said K562 cells are co-cultured *in vitro* at a ratio of 10:1. In certain aspects, the three-stage method produces ILC3 cells that exhibit at least 60% cytotoxicity against the K562 cells when said ILC3 cells and said K562 cells are co-cultured *in vitro* at a ratio of 10:1. In certain aspects, the three-stage method produces ILC3 cells that exhibit at least 75% cytotoxicity against the K562 cells when said ILC3 cells and said K562 cells are co-cultured *in vitro* at a ratio of 10:1.

In certain aspects, after said third culturing step, said third population of cells, *e.g.*, said population of natural killer cells, is cryopreserved. In certain aspects, after said fourth culturing step, said fourth population of cells, *e.g*., said population of natural killer cells, is cryopreserved.

Disclosed herein are populations of cells comprising natural killer cells, *i.e*., natural killers cells produced by a three-stage method described herein. Accordingly, disclosed herein is an isolated natural killer cell population produced by a three-stage method described herein. In a specific example, said natural killer cell population comprises at least 20% CD56+CD3- natural killer cells. In a specific example, said natural killer cell population comprises at least 40% CD56+CD3- natural killer cells. In a specific example, said natural killer cell population comprises at least 60% CD56+CD3- natural killer cells. In a specific example, said natural killer cell population comprises at least 80% CD56+CD3- natural killer cells. In specific examples, the natural killer cell population is formulated into a pharmaceutical composition suitable for use *in vivo,* for example, suitable for human use *in vivo.*

Disclosed herein are populations of cells comprising ILC3 cells, *i.e.,* natural killer cells produced by a three-stage method described herein. In specific examples, the population of cells comprising ILC3 cells is formulated into a pharmaceutical composition suitable for use *in vivo,* for example, suitable for human use *in vivo.*

Disclosed herein is an isolated NK progenitor cell population, wherein said NK progenitor cells are produced according to the three-stage method described herein. In specific examples, the NK progenitor cell population is formulated into a pharmaceutical composition suitable for use *in vivo,* for example, suitable for human use *in vivo.*

Disclosed herein is an isolated mature NK cell population, wherein said mature NK cells are produced according to the three-stage method described herein. In specific examples, the mature NK cell population is formulated into a pharmaceutical composition suitable for use *in vivo,* for example, suitable for human use *in vivo.*

Disclosed herein is an isolated ILC3 population, wherein said ILC3 cells are produced according to the three-stage method described herein. In specific examples, the isolated ILC3 population is formulated into a pharmaceutical composition suitable for use *in vivo,* for example, suitable for human use *in vivo.*

Disclosed herein is a cell population, wherein said cell population is the third population of cells produced by a method described herein. Disclosed herein is a cell population, wherein said cell population is the fourth population of cells produced by a method described herein.

Disclosed herein is an isolated NK cell population, wherein said NK cells are activated, wherein said activated NK cells are produced according to the three-stage method described herein. In specific examples, the isolated NK population is formulated into a pharmaceutical composition suitable for use *in vivo,* for example, suitable for human use *in vivo.*

Disclosed herein is the use of NK cell populations produced using the three-stage methods described herein to suppress tumor cell proliferation, treat viral infection, or treat cancer, *e.g.,* blood cancers and solid tumors. In certain examples, the NK cell populations are contacted with, or used in combination with, an immunomodulatory compound, *e.g*., an immunomodulatory compound described herein, or thalidomide. In certain examples, the NK cell populations are treated with, or used in combination with, an immunomodulatory compound, *e.g*., an immunomodulatory compound described herein, or thalidomide.

In a specific example, said cancer is a solid tumor. In another said cancer is a blood cancer. In specific examples, the cancer is glioblastoma, primary ductal carcinoma, leukemia, acute T cell leukemia, chronic myeloid lymphoma (CML), acute myelogenous leukemia (AML), chronic myelogenous leukemia (CML), lung carcinoma, colon adenocarcinoma, histiocytic lymphoma, colorectal carcinoma, colorectal adenocarcinoma, prostate cancer, multiple myeloma, or retinoblastoma. In more specific examples, the cancer is AML. In more specific examples, the cancer is multiple myeloma.

In another specific embodiment, the hematopoietic cells, *e.g*., hematopoietic stem cells or progenitor cells, from which the NK cell populations are produced, are obtained from placental perfusate, umbilical cord blood or peripheral blood. In one embodiment, the hematopoietic cells, *e.g*., hematopoietic stem cells or progenitor cells, from which NK cell populations are produced, are obtained from placenta, *e.g*., from placental perfusate. In one embodiment, the hematopoietic cells, *e.g*., hematopoietic stem cells or progenitor cells, from which the NK cell populations are produced, are not obtained from umbilical cord blood. In one embodiment, the hematopoietic cells, *e.g*., hematopoietic stem cells or progenitor cells, from which the NK cell populations are produced, are not obtained from peripheral blood. In another specific embodiment, the hematopoietic cells, *e.g*., hematopoietic stem cells or progenitor cells, from which the NK cell populations are produced, are combined cells from placental perfusate and cord blood, *e.g*., cord blood from the same placenta as the perfusate. In another specific embodiment, said umbilical cord blood is isolated from a placenta other than the placenta from which said placental perfusate is obtained. In certain embodiments, the combined cells can be obtained by pooling or combining the cord blood and placental perfusate. In certain embodiments, the cord blood and placental perfusate are combined at a ratio of 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like by volume to obtain the combined cells. In a specific embodiment, the cord blood and placental perfusate are combined at a ratio of from 10:1 to 1:10, from 5:1 to 1:5, or from 3:1 to 1:3. In another specific embodiment, the cord blood and placental perfusate are combined at a ratio of 10:1, 5:1, 3:1, 1:1, 1:3, 1:5 or 1:10. In a more specific embodiment, the cord blood and placental perfusate are combined at a ratio of 8.5:1.5 (85%:15%).

In certain embodiments, the cord blood and placental perfusate are combined at a ratio of 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like, as determined by total nucleated cells (TNC) content to obtain the combined cells. In a specific embodiment, the cord blood and placental perfusate are combined at a ratio of from 10:1 to 10:1, from 5:1 to 1:5, or from 3:1 to 1: 3. In another specific embodiment, the cord blood and placental perfusate are combined at a ratio of 10:1, 5:1, 3:1, 1:1, 1:3, 1:5 or 1:10.

Disclosed herein is a method of treating an individual having cancer or a viral infection, comprising administering to said individual an effective amount of cells from an isolated NK cell population produced using the three-stage methods described herein. In certain examples, the cancer is a solid tumor. In certain examples, the cancer is a hematological cancer. In a specific example, the hematological cancer is leukemia. In another specific example, the hematological cancer is lymphoma. In another specific example, the hematological cancer is acute myeloid leukemia. In another specific example, the hematological cancer is chronic lymphocytic leukemia. In another specific example, the hematological cancer is chronic myelogenous leukemia. In certain aspects, said natural killer cells have been cryopreserved prior to said contacting or said administering. In other aspects, said natural killer cells have not been cryopreserved prior to said contacting or said administering.

In a specific example, the NK cell populations produced using the three-stage methods described herein have been treated with an immunomodulatory compound, *e.g*. an immunomodulatory compound described herein, or thalidomide, prior to said administration. In a specific example, the NK cell populations produced using the three-stage methods described herein have been treated with IL2 and IL12 and IL18, IL12 and IL15, IL12 and IL18, IL2 and IL12 and IL15 and IL18, or IL2 and IL15 and IL18 prior to said administration. In another specific example, the isolated NK cell population produced using the three-stage methods described herein has been pretreated with one or more of IL2, IL12, IL18, or IL15 prior to said administration. In another specific example, the method comprises administering to the individual (1) an effective amount of an isolated NK cell population produced using a three-stage method described herein; and (2) an effective amount of an immunomodulatory compound or thalidomide. An "effective amount" in this context means an amount of cells in an NK cell population, and optionally immunomodulatory compound or thalidomide, that results in a detectable improvement in one or more symptoms of said cancer or said infection, compared to an individual having said cancer or said infection who has not been administered said NK cell population and, optionally, an immunomodulatory compound or thalidomide. In a specific example, said immunomodulatory compound is lenalidomide or pomalidomide. In another example, the method additionally comprises administering an anticancer compound to the individual, *e.g*., one or more of the anticancer compounds described below.

Disclosed herein is a method of suppressing the proliferation of tumor cells comprising bringing a therapeutically effective amount of an NK cell population into proximity with the tumor cells, *e.g*., contacting the tumor cells with the cells in an NK cell population. Hereinafter, unless noted otherwise, the term "proximity" refers to sufficient proximity to elicit the desired result; *e.g*., in certain embodiments, the term proximity refers to contact. In certain examples, said contacting takes place *in vitro.* In certain examples, said contacting takes place *ex vivo.* In other examples, said contacting takes place *in vivo.* A plurality of NK cells can be used in the method of suppressing the proliferation of the tumor cells comprising bringing a therapeutically effective amount of the NK cell population into proximity with the tumor cells, *e.g*., contacting the tumor cells with the cells in the NK cell population. In certain examples, said tumor cells are breast cancer cells, head and neck cancer cells, or sarcoma cells. In certain examples, said tumor cells are primary ductal carcinoma cells, leukemia cells, acute T cell leukemia cells, chronic myeloid lymphoma (CML) cells, chronic myelogenous leukemia (CML) cells, lung carcinoma cells, colon adenocarcinoma cells, histiocytic lymphoma cells, colorectal carcinoma cells, colorectal adenocarcinoma cells, or retinoblastoma cells.

Disclosed herein are a plurality of natural killer cells for use in a method of suppressing the proliferation of tumor cells comprising contacting the tumor cells with the plurality of natural killer cells, wherein the natural killer cells are produced by the methods described herein. In certain examples, said contacting takes place in a human individual. In certain examples, said method comprises administering said natural killer cells to said individual. In certain examples, said tumor cells are multiple myeloma cells. In certain examples, said tumor cells are acute myeloid leukemia (AML) cells. In certain examples, Said individual has relapsed/refractory AML. In certain examples, said individual has AML that has failed at least one non-innate lymphoid cell (ILC) therapeutic against AML. In certain examples, said individual is 65 years old or greater, and is in first remission. In certain example, said individual has been conditioned with fludarabine, cytarabine, or both prior to administering said NK cells. In certain examples, said tumor cells are breast cancer cells, head and neck cancer cells, or sarcoma cells. In certain examples, said tumor cells are primary ductal carcinoma cells, leukemia cells, acute T cell leukemia cells, chronic myeloid lymphoma (CML) cells, chronic myelogenous leukemia (CML) cells, lung carcinoma cells, colon adenocarcinoma cells, histiocytic lymphoma cells, colorectal carcinoma cells, colorectal adenocarcinoma cells, or retinoblastoma cells. In certain examples, said tumor cells are solid tumor cells, liver tumor cells, lung tumor cells, pancreatic tumor cells, renal tumor cells or glioblastoma multiforme (GBM) cells. In certain examples, said natural killer cells are administered with an anti-CD33 antibody, an anti-CD20 antibody, an anti-CD138 antibody or an anti-CD32 antibody. In certain examples, said NK cells have or have not been cryopreserved prior to said contacting or said administering.

Administration of an isolated population of NK cells or a pharmaceutical composition thereof may be systemic or local. In specific examples, administration is parenteral. In specific examples, administration of an isolated population of NK cells or a pharmaceutical composition thereof to a subject is by injection, infusion, intravenous (IV) administration, intrafemoral administration, or intratumor administration. In specific examples, administration of an isolated population of NK cells or a pharmaceutical composition thereof to a subject is performed with a device, a matrix, or a scaffold. In specific examples, administration an isolated population of NK cells or a pharmaceutical composition thereof to a subject is by injection. In specific examples, administration an isolated population of NK cells or a pharmaceutical composition thereof to a subject is via a catheter. In specific examples, the injection of NK cells is local injection. In more specific examples, the local injection is directly into a solid tumor (*e.g*., a sarcoma). In specific examples, administration of an isolated population of NK cells or a pharmaceutical composition thereof to a subject is by injection by syringe. In specific examples, administration of an isolated population of NK cells or a pharmaceutical composition thereof to a subject is via guided delivery. In specific examples, administration of an isolated population of NK cells or a pharmaceutical composition thereof to a subject by injection is aided by laparoscopy, endoscopy, ultrasound, computed tomography, magnetic resonance, or radiology.

In a specific examples, the isolated NK cell population produced using the three-stage methods described herein has been treated with an immunomodulatory compound, *e.g.* an immunomodulatory compound described herein, below, or thalidomide, and/or IL2 and IL12 and IL18, IL12 and IL15, IL12 and IL18, IL2 and IL12 and IL15 and IL18, or IL2 and IL15 and IL18, prior to said contacting or bringing into proximity. In another specific example, the isolated NK cell population produced using the three-stage methods described herein has been treated with one or more of IL2, IL12, IL18, or IL15 prior to said contacting or bringing into proximity. In another specific example, an effective amount of an immunomodulatory compound, *e.g*. an immunomodulatory compound described herein, below, or thalidomide is additionally brought into proximity with the tumor cells *e.g.,* the tumor cells are contacted with the immunomodulatory compound or thalidomide. An "effective amount" in this context means an amount of cells in an NK cell population, and optionally an immunomodulatory compound or thalidomide, that results in a detectable suppression of said tumor cells compared to an equivalent number of tumor cells not contacted or brought into proximity with cells in an NK cell population, and optionally an immunomodulatory compound or thalidomide. In another specific example, the method further comprises bringing an effective amount of an anticancer compound, *e.g*., an anticancer compound described below, into proximity with the tumor cells, *e.g*., contacting the tumor cells with the anticancer compound.

In a specific example, of this method, the tumor cells are blood cancer cells. In another specific example, the tumor cells are solid tumor cells. In another example, the tumor cells are primary ductal carcinoma cells, leukemia cells, acute T cell leukemia cells, chronic myeloid lymphoma (CML) cells, acute myelogenous leukemia cells (AML), chronic myelogenous leukemia (CML) cells, glioblastoma cells, lung carcinoma cells, colon adenocarcinoma cells, histiocytic lymphoma cells, multiple myeloma cells, retinoblastoma cell, colorectal carcinoma cells, prostate cancer cells, or colorectal adenocarcinoma cells. In more specific examples, the tumor cells are AML cells. In more specific examples, the tumor cells are multiple myeloma cells. In another specific example, said contacting or bringing into proximity takes place *in vitro.* In another specific example, said contacting or bringing into proximity takes place *ex vivo.* In another specific example, said contacting or bringing into proximity takes place *in vivo.* In a more specific example, said *in vivo* contacting or bringing into proximity takes place in a human. In a specific example, said tumor cells are solid tumor cells. In a specific example, said tumor cells are liver tumor cells. In a specific example, said tumor cells are lung tumor cells. In a specific example, said tumor cells are pancreatic tumor cells. In a specific example, said tumor cells are renal tumor cells. In a specific example, said tumor cells are glioblastoma multiforme (GBM) cells. In a specific example, said natural killer cells are administered with an antibody. In a specific example, said natural killer cells are administered with an anti-CD33 antibody. In a specific example, said natural killer cells are administered with an anti-CD20 antibody. In a specific example, said natural killer cells are administered with an anti-CD138 antibody. In a specific example, said natural killer cells are administered with an anti-CD32 antibody.

Disclosed herein is a method of treating an individual having multiple myeloma, comprising administering to the individual (1) lenalidomide; (2) melphalan; and (3) NK cells, wherein said NK cells are effective to treat multiple myeloma in said individual. In a specific example, said NK cells are cord blood NK cells, or NK cells produced from cord blood hematopoietic cells, *e.g*., hematopoietic stem cells. In another example, embodiment, said NK cells have been produced by any of the methods described herein for producing NK cells, *e.g*., for producing NK cell populations using a three-stage method. In another example, said NK cells have been expanded prior to said administering. In another example, said lenalidomide, melphalan, and/or NK cells are administered separately from each other. In certain specific example, of the method of treating an individual with multiple myeloma, said NK cell populations are produced by a three-stage method, as described herein.

Disclosed herein is a method of treating an individual having acute myelogenous leukemia (AML), comprising administering to the individual NK cells (optionally activated by pretreatment with IL2 and IL12 and IL18, IL12 and IL15, IL12 and IL18, IL2 and IL12 and IL15 and IL18, or IL2 and IL15 and IL18), wherein said NK cells are effective to treat AML in said individual. In a specific example, the isolated NK cell population produced using the three-stage methods described herein has been pretreated with one or more of IL2, IL12, IL18, or IL15 prior to said administering. In a specific example, said NK cells are cord blood NK cells, or NK cells produced from cord blood hematopoietic cells, *e.g*., hematopoietic stem cells. In another example, said NK cells have been produced by any of the methods described herein for producing NK cells, *e.g*., for producing NK cell populations using a three-stage method as set forth herein. In certain specific examples, of the method of treating an individual with AML, said NK cell populations are produced by a three-stage method, as described herein. In a particular example, the AML to be treated by the foregoing methods comprises refractory AML, poor-prognosis AML, or childhood AML. In certain examples, said individual has AML that has failed at least one non-natural killer or non-innate lymphoid cell therapeutic against AML. In specific examples, said individual is 65 years old or greater, and is in first remission. In specific example, said individual has been conditioned with fludarabine, cytarabine, or both prior to administering said natural killer cells. Disclosed herein is a method of treating an individual having chronic lymphocytic leukemia (CLL), comprising administering to the individual a therapeutically effective dose of (1) lenalidomide; (2) melphalan; (3) fludarabine; and (4) NK cells, *e.g*., a NK cell population produced using a three-stage method described herein, wherein said NK cells are effective to treat said CLL in said individual. In a specific example, said NK cells are cord blood NK cells, or NK cells produced from cord blood hematopoietic cells, *e.g*., hematopoietic stem cells. In another example, said NK cells have been produced by any of the methods described herein for producing NK cells, *e.g.,* for producing NK cell populations using a three-stage method described herein. In a specific example, of any of the above methods, said lenalidomide, melphalan, fludarabine, and expanded NK cells are administered to said individual separately. In certain specific example, of the method of treating an individual with CLL, said NK cell populations are produced by a three-stage method, as described herein.

Disclosed herein is a method of suppressing the proliferation of tumor cells comprising bringing a therapeutically effective amount of an ILC3 cell population into proximity with the tumor cells, *e.g*., contacting the tumor cells with the cells in an ILC3 cell population. Hereinafter, unless noted otherwise, the term "proximity" refers to sufficient proximity to elicit the desired result; *e.g.,* in certain embodiments, the term proximity refers to contact. In certain examples, said contacting takes place *in vitro.* In certain examples, said contacting takes place *ex vivo.* In other examples, said contacting takes place *in vivo.* A plurality of ILC3 cells can be used in the method of suppressing the proliferation of the tumor cells comprising bringing a therapeutically effective amount of the ILC3 cell population into proximity with the tumor cells, *e.g*., contacting the tumor cells with the cells in the ILC3 cell population. In certain example, said tumor cells are breast cancer cells, head and neck cancer cells, or sarcoma cells. In certain examples, said tumor cells are primary ductal carcinoma cells, leukemia cells, acute T cell leukemia cells, chronic myeloid lymphoma (CML) cells, chronic myelogenous leukemia (CML) cells, lung carcinoma cells, colon adenocarcinoma cells, histiocytic lymphoma cells, colorectal carcinoma cells, colorectal adenocarcinoma cells, or retinoblastoma cells.

Disclosed herein are a plurality of ILC3 cells for use in a method of suppressing the proliferation of tumor cells comprising contacting the tumor cells with the plurality of ILC3 cells, wherein the ILC3 cells are produced by the methods described herein. In certain examples, said contacting takes place in a human individual. In certain examples, said method comprises administering said ILC3 cells to said individual. In certain examples, said tumor cells are multiple myeloma cells. In certain examples, said tumor cells are acute myeloid leukemia (AML) cells. In certain example, said individual has relapsed/refractory AML. In certain examples, said individual has AML that has failed at least one non-innate lymphoid cell (ILC) therapeutic against AML. In certain examples, said individual is 65 years old or greater, and is in first remission. In certain examples, said individual has been conditioned with fludarabine, cytarabine, or both prior to administering said ILC3 cells. In certain examples, said tumor cells are breast cancer cells, head and neck cancer cells, or sarcoma cells. In certain examples, said tumor cells are primary ductal carcinoma cells, leukemia cells, acute T cell leukemia cells, chronic myeloid lymphoma (CML) cells, chronic myelogenous leukemia (CML) cells, lung carcinoma cells, colon adenocarcinoma cells, histiocytic lymphoma cells, colorectal carcinoma cells, colorectal adenocarcinoma cells, or retinoblastoma cells. In certain examples, said tumor cells are solid tumor cells, liver tumor cells, lung tumor cells, pancreatic tumor cells, renal tumor cells or glioblastoma multiforme (GBM) cells. In certain examples, said ILC3 cells are administered with an anti-CD33 antibody, an anti-CD20 antibody, an anti-CD138 antibody or an anti-CD32 antibody. In certain examples, said ILC3 cells have or have not been cryopreserved prior to said contacting or said administering.

Administration of an isolated population of ILC3 cells or a pharmaceutical composition thereof may be systemic or local. In specific examples, administration is parenteral. In specific examples, administration of an isolated population of ILC3 cells or a pharmaceutical composition thereof to a subject is by injection, infusion, intravenous (IV) administration, intrafemoral administration, or intratumor administration. In specific examples, administration of an isolated population of ILC3 cells or a pharmaceutical composition thereof to a subject is performed with a device, a matrix, or a scaffold. In specific examples, administration an isolated population of ILC3 cells or a pharmaceutical composition thereof to a subject is by injection. In specific examples, administration an isolated population of ILC3 cells or a pharmaceutical composition thereof to a subject is via a catheter. In specific examples, the injection of ILC3 cells is local injection. In more specific examples, the local injection is directly into a solid tumor (*e.g.,* a sarcoma). In specific examples, administration of an isolated population of ILC3 cells or a pharmaceutical composition thereof to a subject is by injection by syringe. In specific examples administration of an isolated population of ILC3 cells or a pharmaceutical composition thereof to a subject is via guided delivery. In specific examples, administration of an isolated population of ILC3 cells or a pharmaceutical composition thereof to a subject by injection is aided by laparoscopy, endoscopy, ultrasound, computed tomography, magnetic resonance, or radiology.

In a specific example, the isolated ILC3 cell population produced using the three-stage methods described herein has been treated with an immunomodulatory compound, *e.g.* an immunomodulatory compound described herein, below, or thalidomide, and/or IL2 and IL12 and IL18, IL12 and IL15, IL12 and IL18, IL2 and IL12 and IL15 and IL18, or IL2 and IL15 and IL18, prior to said contacting or bringing into proximity. In a specific example, the isolated NK cell population produced using the three-stage methods described herein has been treated with one or more of IL2, IL12, IL18, or IL15 prior to said contacting or bringing into proximity. In another specific example, an effective amount of an immunomodulatory compound, e.g. an immunomodulatory compound described herein, below, or thalidomide is additionally brought into proximity with the tumor cells *e.g.,* the tumor cells are contacted with the immunomodulatory compound or thalidomide. An "effective amount" in this context means an amount of cells in an ILC3 cell population, and optionally an immunomodulatory compound or thalidomide, that results in a detectable suppression of said tumor cells compared to an equivalent number of tumor cells not contacted or brought into proximity with cells in an ILC3 cell population, and optionally an immunomodulatory compound or thalidomide. In another specific example, the method further comprises bringing an effective amount of an anticancer compound, *e.g*., an anticancer compound described below, into proximity with the tumor cells, *e.g*., contacting the tumor cells with the anticancer compound.

In a specific example, of this method, the tumor cells are blood cancer cells. In another specific embodiment, the tumor cells are solid tumor cells. In another example, the tumor cells are primary ductal carcinoma cells, leukemia cells, acute T cell leukemia cells, chronic myeloid lymphoma (CML) cells, acute myelogenous leukemia cells (AML), chronic myelogenous leukemia (CML) cells, glioblastoma cells, lung carcinoma cells, colon adenocarcinoma cells, histiocytic lymphoma cells, multiple myeloma cells, retinoblastoma cell, colorectal carcinoma cells, prostate cancer cells, or colorectal adenocarcinoma cells. In another specific example, said contacting or bringing into proximity takes place *in vitro.* In another specific example, said contacting or bringing into proximity takes place *ex vivo.* In another specific example, said contacting or bringing into proximity takes place *in vivo.* In a more specific example, said *in vivo* contacting or bringing into proximity takes place in a human. In a specific example, said tumor cells are solid tumor cells. In a specific example, said tumor cells are liver tumor cells. In a specific example, said tumor cells are lung tumor cells. In a specific example, said tumor cells are pancreatic tumor cells. In a specific example, said tumor cells are renal tumor cells. In a specific example, said tumor cells are glioblastoma multiforme (GBM) cells. In a specific example, said ILC3 cells are administered with an antibody. In a specific example, said ILC3 cells are administered with an anti-CD33 antibody. In a specific example, said ILC3 cells are administered with an anti-CD20 antibody. In a specific example, said ILC3 cells are administered with an anti-CD138 antibody. In a specific example, said ILC3 cells are administered with an anti-CD32 antibody.

Disclosed herein is a method of treating an individual having multiple myeloma, comprising administering to the individual (1) lenalidomide; (2) melphalan; and (3) II,C3 cells, wherein said ILC3 cells are effective to treat multiple myeloma in said individual. In a specific example, said ILC3 cells are cord blood ILC3 cells, or ILC3 cells produced from cord blood hematopoietic cells, *e.g*., hematopoietic stem cells. In another example, said ILC3 cells have been produced by any of the methods described herein for producing ILC3 cells, *e.g*., for producing ILC3 cell populations using a three-stage method. In another example, said ILC3 cells have been expanded prior to said administering. In another example, said lenalidomide, melphalan, and/or ILC3 cells are administered separately from each other. In certain specific examples, of the method of treating an individual with multiple myeloma, said ILC3 cell populations are produced by a three-stage method, as described herein.

Disclosed herein is a method of treating an individual having acute myelogenous leukemia (AML), comprising administering to the individual ILC3 cells (optionally activated by pretreatment with IL2 and IL12 and IL18, IL12 and IL15, IL12 and IL18, IL2 and IL12 and IL15 and IL18, or IL2 and IL15 and IL18), wherein said ILC3 cells are effective to treat AML in said individual. In a specific example, the isolated NK cell population produced using the three-stage methods described herein has been pretreated with one or more of IL2, IL12, II,18, or II,15 prior to said administering. In a specific example, said ILC3 cells are cord blood ILC3 cells, or ILC3 cells produced from cord blood hematopoietic cells, *e.g*., hematopoietic stem cells. In another example, said ILC3 cells have been produced by any of the methods described herein for producing ILC3 cells, *e.g.,* for producing ILC3 cell populations using a three-stage method as set forth herein. In certain specific examples, of the method of treating an individual with AML, said ILC3 cell populations are produced by a three-stage method, as described herein. In a particular example, the AML to be treated by the foregoing methods comprises refractory AML, poor-prognosis AML, or childhood AML. In certain examples, said individual has AML that has failed at least one non-ILC3 or non-innate lymphoid cell therapeutic against AML. In specific examples, said individual is 65 years old or greater, and is in first remission. In specific example, said individual has been conditioned with fludarabine, cytarabine, or both prior to administering said ILC3 cells. Disclosed herein is a method of treating an individual having chronic lymphocytic leukemia (CLL), comprising administering to the individual a therapeutically effective dose of (1) lenalidomide; (2) melphalan; (3) fludarabine; and (4) ILC3 cells, *e.g.,* a ILC3 cell population produced using a three-stage method described herein, wherein said ILC3 cells are effective to treat said CLL in said individual. In a specific example, said ILC3 cells are cord blood ILC3 cells, or ILC3 cells produced from cord blood hematopoietic cells, *e.g*., hematopoietic stem cells. In another example, said ILC3 cells have been produced by any of the methods described herein for producing ILC3 cells, *e.g.,* for producing ILC3 cell populations using a three-stage method described herein. In a specific example of any of the above methods, said lenalidomide, melphalan, fludarabine, and expanded II,C3 cells are administered to said individual separately. In certain specific examples, of the method of treating an individual with CLL, said ILC3 cell populations are produced by a three-stage method, as described herein.

In certain examples, the NK cell populations produced using a three-stage method described herein are cryopreserved, *e.g*., cryopreserved using a method described herein. In a certain examples, the NK cell populations produced using a three-stage method described herein are cryopreserved in a cryopreservation medium, *e.g*., a cryopreservation medium described herein. In a specific example, cryopreservation of the NK progenitor cell populations and/or NK cell populations produced using a three-stage method described herein comprises (1) preparing a cell suspension solution comprising an NK progenitor cell population and/or an NK cell population produced using a three-stage method described herein; (2) adding cryopreservation medium to the cell suspension solution from step (1) to obtain a cryopreserved cell suspension; (3) cooling the cryopreserved cell suspension from step (3) to obtain a cryopreserved sample; and (4) storing the cryopreserved sample below -80 °C.

In certain examples, of the methods of treatment or tumor suppression above, NK cell populations produced by a three-stage method described herein are combined with other natural killer cells, *e.g*., natural killer cells isolated from placental perfusate, umbilical cord blood or peripheral blood, or produced from hematopoietic cells by a different method. In specific examples, the natural killer cell populations are combined with natural killer cells from another source, or made by a different method, in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

In certain examples, the ILC3 cell populations produced using a three-stage method described herein are cryopreserved, e.g., cryopreserved using a method described herein. In a certain examples, the ILC3 cell populations produced using a three-stage method described herein are cryopreserved in a cryopreservation medium, *e.g*., a cryopreservation medium described herein. In a specific example, cryopreservation of the ILC3 progenitor cell populations and/or ILC3 cell populations produced using a three-stage method described herein comprises (1) preparing a cell suspension solution comprising an ILC3 progenitor cell population and/or an ILC3 cell population produced using a three-stage method described herein; (2) adding cryopreservation medium to the cell suspension solution from step (1) to obtain a cryopreserved cell suspension; (3) cooling the cryopreserved cell suspension from step (3) to obtain a cryopreserved sample; and (4) storing the cryopreserved sample below -80 °C.

In certain examples, of the methods of treatment or tumor suppression above, ILC3 cell populations produced by a three-stage method described herein are combined with other ILC3 cells, *e.g*., ILC3 cells isolated from placental perfusate, umbilical cord blood or peripheral blood, or produced from hematopoietic cells by a different method. In specific examples, the ILC3 cell populations are combined with ILC3 cells from another source, or made by a different method, in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

Disclosed herein is a method of repairing the gastrointestinal tract after chemotherapy comprising administering to an individual a plurality of ILC3 cells, wherein the ILC3 cells are produced by a three-stage method described herein. A plurality of ILC3 cells can be used in the method of repairing the gastrointestinal tract after chemotherapy comprising administering to an individual a plurality of the ILC3 cells, wherein the ILC3 cells are produced by a three-stage method described herein.

Disclosed herein is a method of protecting an individual against radiation comprising administering to an individual a plurality of ILC3 cells, wherein the ILC3 cells are produced by a three-stage method described herein. A plurality of ILC3 cells can be used in the method of protecting an individual against radiation comprising administering to an individual a plurality of the ILC3 cells, wherein the ILC3 cells are produced by a three-stage method described herein. In certain aspects of the method, said ILC3 cells are used as an adjunct to bone marrow transplantation.

Disclosed herein is a method of reconstituting the thymus of an individual comprising administering to an individual a plurality of ILC3 cells, wherein the ILC3 cells are produced by a three-stage method described herein. A plurality of ILC3 cells can be used in the method of reconstituting the thymus of an individual comprising administering to an individual a plurality of the ILC3 cells, wherein the ILC3 cells are produced by a three-stage method described herein.

Disclosed herein is a composition comprising isolated NK cells produced by a three-stage method described herein. Said NK cells are produced from hematopoietic cells, e.g., hematopoietic stem or progenitor cells isolated from placental perfusate, umbilical cord blood, and/or peripheral blood. In another specific example, said NK cells comprise at least 70% of cells in the composition. In another specific example, said NK cells comprise at least 80%, 85%, 90%, 95%, 98% or 99% of cells in the composition. In certain examples, at least 80%, 82%, 84%, 86%, 88% or 90% of NK cells in Said composition are CD3⁻ and CD56⁺. In certain examples, at least 65%, 70%, 75%, 80%, 82%, 84%, 86%, 88% or 90% of NK cells in said composition are CD16-. In certain examples, at least 5%, 10%, 15%, 20%, 25%, 30%, 35%, 40%, 45%, 50%, 55% or 60% of NK cells in said composition are CD94+.

Disclosed herein is a population of natural killer cells that is CD56+CD3- CD117+CD11a+, wherein said natural killer cells express perforin and/or EOMES, and do not express one or more of RORγt, aryl hydrocarbon receptor, and IL1R1. In certain aspects, said natural killer cells express perforin and EOMES, and do not express any of RORγt, aryl hydrocarbon receptor, or IL1R1. In certain aspects, said natural killer cells additionally express T-bet, GZMB, NKp46, NKp30, and NKG2D. In certain aspects, said natural killer cells express CD94. In certain aspects, said natural killer cells do not express CD94.

Disclosed herein is a population of ILC3 cells that is CD56+CD3- CD117+CD11a-, wherein said ILC3 cells express one or more of RORγt, aryl hydrocarbon receptor, and IL1R1, and do not express one or more of CD94, perforin, and EOMES. In certain aspects, said ILC3 cells express RORγt, aryl hydrocarbon receptor, and IL1R1, and do not express any of CD94, perforin, or EOMES. In certain aspects, said ILC3 cells additionally express CD226 and/or 2B4. In certain aspects, said ILC3 cells additionally express one or more of IL-22, TNFα, and DNAM-1. In certain aspects, said ILC3 cells express CD226, 2B4, IL-22, TNFα, and DNAM-1.

In certain aspects, provided herein is a method according to the claims of producing a cell population comprising natural killer cells and ILC3 cells, comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; (c) culturing the second population of cells in a third medium comprising IL-2 and IL-15, and lacking each of a stem cell mobilizing agent and LMWH, to produce a third population of cells; and (d) separating CD11a+ cells and CD11a-cells from the third population of cells; and (e) combining the CD11a+ cells with the CD11a-cells in a ratio of 50:1, 40:1, 30:1, 20:1, 10:1, 5:1, 4:1, 3:1, 2:1, 1:1, 1:2, 1:3, 1:4, 1:5, 1:10, 1:20, 1:30, 1:40, or 1:50 to produce a fourth population of cells. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, *e.g*., low-molecular weight heparin. In certain aspects, in the fourth population of cells, the CD11a+ cells and CD11a- cells are combined in a ratio of 50:1. In certain aspects, in the fourth population of cells, the CD11a+ cells and CD11a- cells are combined in a ratio of 20:1. In certain aspects, in the fourth population of cells, the CD1 1a+ cells and CD11a- cells are combined in a ratio of 10:1. In certain aspects, in the fourth population of cells, the CD11a+ cells and CD11a- cells are combined in a ratio of 5:1. In certain aspects, in the fourth population of cells, the CD11a+ cells and CD11a- cells are combined in a ratio of 1:1. In certain aspects, in the fourth population of cells, the CD11a+ cells and CD11a-cells are combined in a ratio of 1:5. In certain aspects, in the fourth population of cells, the CD11a+ cells and CD11a- cells are combined in a ratio of 1:10. In certain aspects, in the fourth population of cells, the CD11a+ cells and CD11a- cells are combined in a ratio of 1:20. In certain aspects, in the fourth population of cells, the CD11a+ cells and CD11a- cells are combined in a ratio of 1:50.

In certain aspects, a plurality of the NK cells in said population expresses one or more of the microRNAS dme-miR-7, hsa-let-7a, hsa-let-7c, hsa-let-7e, hsa-let-7g, hsa-miR-103, hsa-miR-106a, hsa-miR-10b, hsa-miR-1183, hsa-miR-124, hsa-miR-1247, hsa-miR-1248, hsa-miR-1255A, hsa-miR-126, hsa-miR-140-3p, hsa-miR-144, hsa-miR-151-3p, hsa-miR-155, hsa-miR-15a, hsa-miR-16, hsa-miR-17, hsa-miR-181a, hsa-miR-182, hsa-miR-192, hsa-miR-199a-3p, hsa-miR-200a, hsa-miR-20a, hsa-miR-214, hsa-miR-221, hsa-miR-29a, hsa-miR-29b, hsa-miR-30b, hsa-miR-30c, hsa-miR-31, hsa-miR-335, hsa-miR-374b, hsa-miR-454, hsa-miR-484, hsa-miR-513C, hsa-miR-516-3p, hsa-miR-520h, hsa-miR-548K, hsa-miR-548P, hsa-miR-600, hsa-miR-641, hsa-miR-643, hsa-miR-874, hsa-miR-875-5p, and hsa-miR-92a-2 at a detectably higher level as peripheral blood natural killer cells. In certain aspects, a plurality of the NK cells in said population expresses one or more of the microRNAS miR188-5p, miR-339-5p, miR-19a, miR-34c, miR-18a, miR-500, miR-22, miR-222, miR-7a, miR-532-3p, miR-223, miR-26b, miR-26a, miR-191, miR-181d, miR-322, and miR342-3p at a detectably lower level than peripheral blood natural killer cells. In certain aspects, a plurality of the NK cells in said population expresses one or more of the microRNAS miR-181a, miR-30b, and miR30c at an equivalent level to peripheral blood natural killer cells.

In a specific embodiment, said NK cells are from a single individual, that is, said hemtopoietic stem and progenitor cells are from a single individual. In a more specific embodiment, said NK cells comprise natural killer cells from at least two different individuals, that is, said hemtopoietic stem and progenitor cells are from at least two different individuals. In another specific embodiment, said NK cells are from a different individual than the individual for whom treatment with the NK cells is intended, that is, said hemtopoietic stem and progenitor cells are from a different individual than the individual for whom treatment with the NK cells is intended. In another specific embodiment, said NK cells have been contacted or brought into proximity with an immunomodulatory compound or thalidomide in an amount and for a time sufficient for said NK cells to express detectably more granzyme B or perforin than an equivalent number of natural killer cells, *i.e.* NK cells, not contacted or brought into proximity with said immunomodulatory compound or thalidomide. In another specific embodiment, a composition comprising said NK cells additionally comprises an immunomodulatory compound or thalidomide. In certain embodiments, the immunomodulatory compound is a compound described below, *e.g*., an amino-substituted isoindoline compound. In certain embodiments, the immunomodulatory compound is lenalidomide. In certain embodiments, the immunomodulatory compound is pomalidomide.

In another specific embodiment, a composition comprising said NK cells additionally comprises one or more anticancer compounds, *e.g.*, one or more of the anticancer compounds described below.

In a more specific embodiment, the composition comprises NK cells produced by a three-stage method described herein and natural killer cells from another source, or made by another method. In a specific embodiment, said other source is placental blood and/or umbilical cord blood. In another specific embodiment, said other source is peripheral blood. In more specific embodiments, the NK cells are combined with natural killer cells from another source, or made by another method in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

In another specific embodiment, the composition comprises NK cells produced using a three-stage method described herein and either isolated placental perfusate or isolated placental perfusate cells. In a more specific embodiment, said placental perfusate is from the same individual as said NK cells. In another more specific embodiment, said placental perfusate comprises placental perfusate from a different individual than said NK cells. In another specific embodiment, all, or substantially all (*e.g*., greater than 90%, 95%, 98% or 99%) of cells in said placental perfusate are fetal cells. In another specific embodiment, the placental perfusate or placental perfusate cells, comprise fetal and maternal cells. In a more specific embodiment, the fetal cells in said placental perfusate comprise less than about 90%, 80%, 70%, 60% or 50% of the cells in said perfusate. In another specific embodiment, said perfusate is obtained by passage of a 0.9% NaCl solution through the placental vasculature. In another specific embodiment, said perfusate comprises a culture medium. In another specific embodiment, said perfusate has been treated to remove erythrocytes. In another specific embodiment, said composition comprises an immunomodulatory compound, *e.g*., an immunomodulatory compound described below, *e.g.,* an amino-substituted isoindoline compound. In another specific embodiment, the composition additionally comprises one or more anticancer compounds, *e.g*., one or more of the anticancer compounds described below.

In another specific embodiment, the composition comprises NK cells produced using a three-stage method described herein and placental perfusate cells. In a more specific embodiment, said placental perfusate cells are from the same individual as said NK cells. In another more specific embodiment, said placental perfusate cells are from a different individual than said NK cells. In another specific embodiment, the composition comprises isolated placental perfusate and isolated placental perfusate cells, wherein said isolated perfusate and said isolated placental perfusate cells are from different individuals. In another more specific embodiment of any of the above embodiments comprising placental perfusate, said placental perfusate comprises placental perfusate from at least two individuals. In another more specific embodiment of any of the above embodiments comprising placental perfusate cells, said isolated placental perfusate cells are from at least two individuals. In another specific embodiment, said composition comprises an immunomodulatory compound. In another specific embodiment, the composition additionally comprises one or more anticancer compounds, *e.g*., one or more of the anticancer compounds described below.

Disclosed herein is a composition, *e.g*., a pharmaceutical composition, comprising an isolated NK cell population, *e.g*., produced by any embodiment of the three-stage method described herein. Said isolated NK cell population is produced from hematopoietic cells, *e.g*., hematopoietic stem or progenitor cells isolated from placenta, *e.g*., from placental perfusate, umbilical cord blood, and/or peripheral blood. In another specific example, said isolated NK cell population comprises at least 70% of cells in the composition. In another specific example, said isolated NK cell population comprises at least 80%, 85%, 90%, 95%, 98% or 99% of cells in the composition. In another specific example, said NK cells comprise at least 70% of cells in the composition. In certain example, at least 80%, 82%, 84%, 86%, 88% or 90% of NK cells in said composition are CD3⁻ and CD56⁺. In certain examples, at least 65%, 70%, 75%, 80%, 82%, 84%, 86%, 88% or 90% of NK cells in said composition are CD16-. In certain examples, at least 5%, 10%, 15%, 20%, 25%, 30%, 35%, 40%, 45%, 50%, 55% or 60% of NK cells in said composition are CD94+.

In another specific example, said isolated NK cells in said composition are from a single individual, that is, said hemtopoietic stem and progenitor cells are from a single individual. In a more specific example, said isolated NK cells comprise NK cells from at least two different individuals, that is, said hemtopoietic stem and progenitor cells are from at least two different individuals. In another specific example, said isolated NK cells in said composition are from a different individual than the individual for whom treatment with the NK cells is intended, that is, said hemtopoietic stem and progenitor cells are from a different individual than the individual for whom treatment with the NK cells is intended. In another specific example, said NK cells have been contacted or brought into proximity with an immunomodulatory compound or thalidomide in an amount and for a time sufficient for said NK cells to express detectably more granzyme B or perforin than an equivalent number of natural killer cells, *i.e.* NK cells not contacted or brought into proximity with said immunomodulatory compound or thalidomide. In another specific example, said composition additionally comprises an immunomodulatory compound or thalidomide. In certain examples, the immunomodulatory compound is a compound described below.

In another specific example, the composition additionally comprises one or more anticancer compounds, *e.g*., one or more of the anticancer compounds described below.

In a more specific example, the composition comprises NK cells from another source, or made by another method. In a specific example, said other source is placental blood and/or umbilical cord blood. In another specific example, said other source is peripheral blood. In more specific examples, the NK cell population in said composition is combined with NK cells from another source, or made by another method in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

In another specific example, the composition comprises an NK cell population and either isolated placental perfusate or isolated placental perfusate cells. In a more specific example, said placental perfusate is from the same individual as said NK cell population. In another more specific example, said placental perfusate comprises placental perfusate from a different individual than said NK cell population. In another specific example, all, or substantially all (*e.g*., greater than 90%, 95%, 98% or 99%), of cells in said placental perfusate are fetal cells. In another specific example, the placental perfusate or placental perfusate cells, comprise fetal and maternal cells. In a more specific example, the fetal cells comprise less than about 90%, 80%, 70%, 60% or 50% of the cells in said placental perfusate. In another specific example, said perfusate is obtained by passage of a 0.9% NaCl solution through the placental vasculature. In another specific example, said perfusate comprises a culture medium. In another specific example, said perfusate has been treated to remove erythrocytes. In another specific example, said composition comprises an immunomodulatory compound, *e.g*., an immunomodulatory compound described below, *e.g.,* an amino-substituted isoindoline compound. In another specific example, the composition additionally comprises one or more anticancer compounds, *e.g*., one or more of the anticancer compounds described below.

In another specific example, the composition comprises an NK cell population and placental perfusate cells. In a more specific example, said placental perfusate cells are from the same individual as said NK cell population. In another more specific example, said placental perfusate cells are from a different individual than said NK cell population. In another specific example, the composition comprises isolated placental perfusate and isolated placental perfusate cells, wherein said isolated perfusate and said isolated placental perfusate cells are from different individuals. In another more specific example of any of the above examples comprising placental perfusate, said placental perfusate comprises placental perfusate from at least two individuals. In another more specific example of any of the above examples comprising placental perfusate cells, said isolated placental perfusate cells are from at least two individuals. In another specific example, said composition comprises an immunomodulatory compound. In another specific example, the composition additionally comprises one or more anticancer compounds, *e.g*., one or more of the anticancer compounds described below.

### 3.1. Terminology

As used herein, the terms "immunomodulatory compound" and "IMiD^{™}" do not encompass thalidomide.

As used herein, "lenalidomide" means 3-(4'aminoisoindoline-1'-one)-1-piperidine-2,6-dione (Chemical Abstracts Service name) or 2,6-Piperidinedione,3-(4-amino-1,3-dihydro-1-oxo-2H-isoindol-2-yl)- (International Union of Pure and Applied Chemistry (IUPAC) name). As used herein, "pomalidomide" means 4-amino-2-(2,6-dioxopiperidin-3-yl)isoindole-1,3-dione.

As used herein, "multipotent," when referring to a cell, means that the cell has the capacity to differentiate into a cell of another cell type. In certain examples, "a multipotent cell" is a cell that has the capacity to grow into a subset of the mammalian body's approximately 260 cell types. Unlike a pluripotent cell, a multipotent cell does not have the capacity to form all of the cell types.

As used herein, "feeder cells" refers to cells of one type that are co-cultured with cells of a second type, to provide an environment in which the cells of the second type can be maintained, and perhaps proliferate. Without being bound by any theory, feeder cells can provide, for example, peptides, polypeptides, electrical signals, organic molecules (*e.g*., steroids), nucleic acid molecules, growth factors (*e.g*., bFGF), other factors (*e.g.,* cytokines), and metabolic nutrients to target cells. In certain examples, feeder cells grow in a mono-layer.

As used herein, the "natural killer cells" or "NK cells" produced using the methods described herein, without further modification, include natural killer cells from any tissue source.

As used herein, the "ILC3 cells" produced using the methods described herein, without further modification, include ILC3 cells from any tissue source.

As used herein, "placental perfusate" means perfusion solution that has been passed through at least part of a placenta, *e.g.,* a human placenta, *e.g.,* through the placental vasculature, and includes a plurality of cells collected by the perfusion solution during passage through the placenta.

As used herein, "placental perfusate cells" means nucleated cells, *e.g.,* total nucleated cells, isolated from, or isolatable from, placental perfusate.

As used herein, "tumor cell suppression," "suppression of tumor cell proliferation," and the like, includes slowing the growth of a population of tumor cells, *e.g.,* by killing one or more of the tumor cells in said population of tumor cells, for example, by contacting or bringing, *e.g.,* NK cells or an NK cell population produced using a three-stage method described herein into proximity with the population of tumor cells, *e.g*., contacting the population of tumor cells with NK cells or an NK cell population produced using a three-stage method described herein. In certain examples, said contacting takes place *in vitro* or *ex vivo.* In other examples, said contacting takes place *in vivo.*

As used herein, the term "hematopoietic cells" includes hematopoietic stem cells and hematopoietic progenitor cells.

As used herein, the "undefined component" is a term of art in the culture medium field that refers to components whose constituents are not generally provided or quantified. Examples of an "undefined component" include, without limitation, serum, for example, human serum (*e.g*., human serum AB) and fetal serum (*e.g*., fetal bovine serum or fetal calf serum).

As used herein, "+", when used to indicate the presence of a particular cellular marker, means that the cellular marker is detectably present in fluorescence activated cell sorting over an isotype control; or is detectable above background in quantitative or semi-quantitative RT-PCR.

As used herein, "-", when used to indicate the presence of a particular cellular marker, means that the cellular marker is not detectably present in fluorescence activated cell sorting over an isotype control; or is not detectable above background in quantitative or semi-quantitative RT-PCR.

### 4. BRIEF DESCRIPTION OF THE FIGURES

FIG. 1A-C: Effects on (A) fold expansion, (B) cell purity (CD56+CD3-), and (C) cytotoxicity of NK cells against K562 cells at a 10: 1 (E:T) ratio for the three-stage method using StemRegenin-1 (SR-1) or CH223191 at 1 µM, 10 µM, and 30 µM, as compared to previous NK cell expansion media ("NK cell exp") or dimethyl sulfoxide (DMSO).
FIG. 2: Multi-color flow cytometry of CD3-CD56+ gated cells produced by the three-stage method, showing the expression of CD11a and the natural cytotoxicity receptor NKp30, the c-lectin receptor NKG2D, DNAM-1, 2B4, the cytolytic mediators perforin and granzyme B, and EOMES, the regulator of NK cell maturation and cytolytic function.
FIG. 3: Cytotoxicity of 35-day three-stage NK cells (n=10) against tumor cell lines K562 (CML), HL-60 (AML), and RPMI8226 (multiple myeloma). Lysis was measured at a 10:1 effector-to-target ratio.
FIG. 4: Multi-color flow cytometry comparing FITC isotype control cells to three-stage NK cells in expression of perforin (top), a cytolytic mediator, and CD 107 (bottom), a marker of degranulation.
FIG. 5: Production of cytokines by three-stage NK cells (n=11) when co-cultured with K562 (CML) cells at a 1:1 ratio for 24 hours.

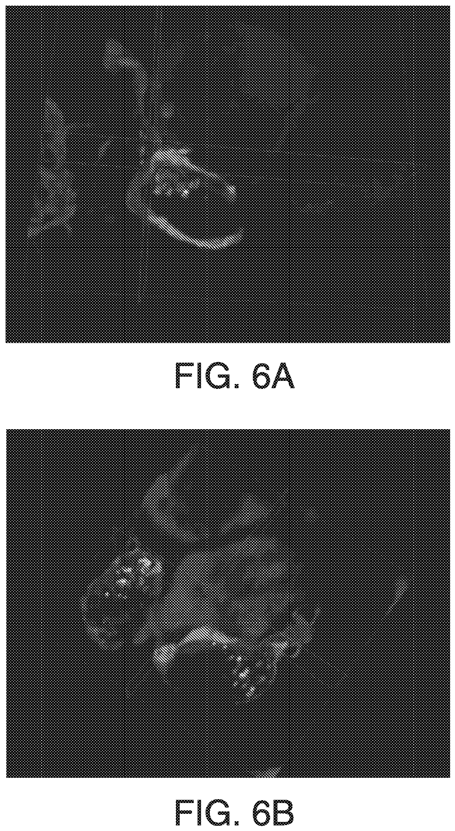

FIG. 6A-B: The formation of an F-actin immunological synapse with polarization of perforin captured by confocal imaging of three-stage NK cells and K562 (CML) (A) and RPMI8226 (multiple myeloma) (B) cells at an effector-to-target of 1:1, 15 minutes post-incubation at 63x magnification. Cells were fixed with formaldehyde and F-Actin was stained with Alexa-488 conjugated phalloidin, and co-staining was performed with perforin antibodies followed by Alexa Fluor 555 dye conjugated goat anti-rabbit secondary antibodies. Tumor target cells were also stained with cell tracker violet dye. Arrows indicate the NK cells, perforin, and target cells.
FIG. 7: Cytotoxicity of three-stage NK cells (n=3) against tumor cell lines K562 (CML), HL-60 (AML), and RPMI8226 (multiple myeloma). Lysis was measured at various effector-to-target ratios.
FIG. 8: CD107a expression in three-stage NK cells (n=4) upon stimulation with tumor cells (K562 or HL-60) or phorbol 12-myristate 13-acetate plus ionomycin (PMA). CD107a expression is a marker for degranulation. The results from four donors are shown.
FIG. 9: IFNy secretion in three-stage NK cells (n=3) upon stimulation with tumor cells (K562, HL-60, or RPMI8226) or phorbol 12-myristate 13-acetate plus ionomycin (PMA). The results from three donors are shown.
FIG. 10: Cytolytic activity of three-stage NK cells (n = 2) against primary AML target cells (A1, A2, and KG1a) at an effector-to-target ratio of 3:1. Results are shown for 24 hours of incubation. The results from two donors are shown.
FIG. 11: IFNy secretion in three-stage NK cells (n=5) upon stimulation with primary AML target cells (AML1-4, and KG1a), compared with stimulation with HL-60 (AML) tumor cell line. The results from five donors are shown. The boxes are added for ease of comparison.
FIG. 12: Multi-color flow cytometry of CD3-CD56+ gated cells produced by the three-stage method, showing the expression of CD1 1a and CD117. Two subsets of cells, CD11a+ and CD11a-, are shown in boxes.
FIG. 13: Multi-color flow cytometry of the CD11a+ (right) and CD11a- (left) cells from Figure 12, showing the expression of CD56 and CD94.
FIG. 14: Multi-color flow cytometry of the CD11a+ (top) and CD11a- (bottom) cells from Figure 12, showing the levels of expression of perforin, EOMES, RORyt, and IL1R1 for each subset.
FIG. 15: Quantification of the percentage of CD11a+ and CD11a- three-stage cells expressing perforin, granzyme B, EOMES, T-bet, IL1R1, RORyt, and AHR, as determined by flow cytometry. The results are shown across four donors.
FIG. 16: Quantification of the percentage of CD11a+ and CD11a- three-stage cells expressing IFNy, GM-CSF, IL-22, IL-8, and TNFα, upon stimulation with cytokines, as determined by flow cytometry. The results are shown across four donors. The bars on the graph indicate expression as follows, from left to right: i) no stimulation, ii) stimulation with PMA and ionomycin (PMAi), (iii) stimulation with PMAi and IL23, (iv) stimulation with IL12 and IL18, and (v) stimulation with IL1b and IL23.
FIG. 17: Multi-color flow cytometry of CD3-CD56+ gated cells produced by the three-stage method, showing the expression of CD11a compared with NKp46, NKp30, and NKG2D.
FIG. 18: Multi-color flow cytometry of CD3-CD56+ gated cells produced by the three-stage method, showing the expression of CD11a compared with CD226 (DNAM-1) and CD244 (2B4).
FIG. 19: Multi-color flow cytometry of CD3-CD56+ gated cells produced by the three-stage method, showing the expression of CD11a compared with CD16, NKG2A, and KIRs.
FIG. 20A-D: Percentage of three-stage cells expressing (A) CD56 and (B) CD14/15, and percentage of CD56+ three-stage cells expressing (C) RORyt and (D) perforin, using the indicated third medium conditions from Table 6, as measured by flow cytometry. Results from two donors are shown as adjacent bars for each condition.
FIG. 21A-C: CD107a expression using FACS in (A) CD11a+ and (B) CD11a-cell subpopulations in the presence of K562 cells. The graph in (C) shows the results for four populations of CD56+CD3- three-stage NK cells.
FIG. 22: Cytotoxicity of CD11a+ (positive) and CD11a- (negative) subpopulations of a CD56+CD3- population of three-stage NK cells against K562 cells. Effector to target cell ratio is indicated on the x-axis.
FIG. 23: Cytokine secretion analysis performed in the presence and absence of co-culture of K562 target cells and CD1 1a positive and negative effector cells (1: 1 ratio of effector: target). The left hand bar for positive and negative cell subpopulations indicates the secretion in the absence of K562 cells, whereas the right hand bar for positive and negative cell subpopulations indicates secretion in the presence of K562 cells.

### 5. DETAILED DESCRIPTION

Provided herein are novel methods according to the claims of producing and expanding NK cells and/or ILC3 cells from hematopoietic stem cells or progenitor cells. Also provided herein are methods according to the claims, *e.g*., three-stage methods, of producing NK cell populations and/or ILC3 cell populations from hematopoietic stem cells or progenitor cells. The hematopoietic cells (*e.g*., CD34+ hematopoietic stem cells) used to produce the NK cells and/or ILC3 cells, and NK cell populations and/or ILC3 cell populations, may be obtained from any source, for example, without limitation, placenta, umbilical cord blood, placental blood, peripheral blood, spleen or liver. The NK cells and/or ILC3 cells or NK cell populations and/or ILC3 cell populations are produced from expanded hematopoietic stem cells and/or hematopoietic progenitor cells. In one embodiment, hematopoietic cells are collected from a source of such cells, *e.g.,* placenta, for example from placental perfusate, umbilical cord blood, placental blood, peripheral blood, spleen, liver (*e.g*., fetal liver) and/or bone marrow.

The hematopoietic cells used to produce the NK cells and/or ILC3 cells, and NK cell populations and/or ILC3 cell populations, may be obtained from any animal species. In certain embodiments, the hematopoietic stem or progenitor cells are mammalian cells. In specific embodiments, said hematopoietic stem or progenitor cells are human cells. In specific embodiments, said hematopoietic stem or progenitor cells are primate cells. In specific embodiments, said hematopoietic stem or progenitor cells are canine cells. In specific embodiments, said hematopoietic stem or progenitor cells are rodent cells.

### 5.1. Hematopoietic Cells

Hematopoietic cells useful in the methods disclosed herein can be any hematopoietic cells able to differentiate into NK cells and/or ILC3 cells, *e.g.*, precursor cells, hematopoietic progenitor cells, hematopoietic stem cells, or the like. Hematopoietic cells can be obtained from tissue sources such as, *e.g.,* bone marrow, cord blood, placental blood, peripheral blood, liver or the like, or combinations thereof. Hematopoietic cells can be obtained from placenta. In a specific embodiment, the hematopoietic cells are obtained from placental perfusate. In one embodiment, the hematopoietic cells are not obtained from umbilical cord blood. In one embodiment, the hematopoietic cells are not obtained from peripheral blood. Hematopoietic cells from placental perfusate can comprise a mixture of fetal and maternal hematopoietic cells, *e.g*., a mixture in which maternal cells comprise greater than 5% of the total number of hematopoietic cells. In certain embodiments, hematopoietic cells from placental perfusate comprise at least about 90%, 95%, 98%, 99% or 99.5% fetal cells.

In another specific embodiment, the hematopoietic stem cells or progenitor cells, from which the NK cell populations and/or ILC3 cell populations produced using a three-stage method described herein are produced, are obtained from placental perfusate, umbilical cord blood, fetal liver, mobilized peripheral blood, or bone marrow. In another specific embodiment, the hematopoietic stem cells or progenitor cells, from which the NK cell populations and/or ILC3 cell populations produced using a three-stage method described herein are produced, are combined cells from placental perfusate and cord blood, *e.g.,* cord blood from the same placenta as the perfusate. In another specific embodiment, said umbilical cord blood is isolated from a placenta other than the placenta from which said placental perfusate is obtained. In certain embodiments, the combined cells can be obtained by pooling or combining the cord blood and placental perfusate. In certain embodiments, the cord blood and placental perfusate are combined at a ratio of 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like by volume to obtain the combined cells. In a specific embodiment, the cord blood and placental perfusate are combined at a ratio of from 10:1 to 1:10, from 5:1 to 1:5, or from 3:1 to 1:3. In another specific embodiment, the cord blood and placental perfusate are combined at a ratio of 10:1, 5:1, 3:1, 1:1, 1:3, 1:5 or 1:10. In a more specific embodiment, the cord blood and placental perfusate are combined at a ratio of 8.5:1.5 (85%: 15%).

In certain embodiments, the cord blood and placental perfusate are combined at a ratio of 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like by total nucleated cells (TNC) content to obtain the combined cells. In a specific embodiment, the cord blood and placental perfusate are combined at a ratio of from 10:1 to 10:1, from 5:1 to 1:5, or from 3:1 to 1: 3. In another specific embodiment, the cord blood and placental perfusate are combined at a ratio of 10:1, 5:1, 3:1, 1:1, 1:3, 1:5 or 1:10.

In another specific embodiment, the hematopoietic stem cells or progenitor cells from which said NK cell populations and/or ILC3 cell populations produced using a three-stage method described herein are produced, are from both umbilical cord blood and placental perfusate, but wherein said umbilical cord blood is isolated from a placenta other than the placenta from which said placental perfusate is obtained.

In certain embodiments, the hematopoietic cells are CD34⁺ cells. In specific embodiments, the hematopoietic cells useful in the methods disclosed herein are CD34⁺CD38⁺ or CD34⁺CD38⁻. In a more specific embodiment, the hematopoietic cells are CD34⁺CD38⁻Lin⁻. In another specific embodiment, the hematopoietic cells are one or more of CD2⁻, CD3⁻, CD11b⁻, CD11c⁻, CD14⁻, CD16⁻, CD19⁻, CD24⁻, CD56⁻, CD66b⁻ and/or glycophorin A⁻. In another specific embodiment, the hematopoietic cells are CD2⁻, CD3⁻, CD11b⁻, CD11c⁻, CD14⁻, CD16⁻, CD19⁻, CD24⁻, CD56⁻, CD66b⁻ and glycophorin A⁻. In another more specific embodiment, the hematopoietic cells are CD34⁺CD38⁻CD33⁻CD117⁻. In another more specific embodiment, the hematopoietic cells are CD34⁺CD38⁻CD33⁻CD117⁻ CD235⁻CD36⁻.

In another embodiment, the hematopoietic cells are CD45⁺. In another specific embodiment, the hematopoietic cells are CD34⁺CD45⁺. In another embodiment, the hematopoietic cell is Thy-1⁺. In a specific embodiment, the hematopoietic cell is CD34⁺Thy-1⁺. In another embodiment, the hematopoietic cells are CD133⁺. In specific embodiments, the hematopoietic cells are CD34⁺CD133⁺ or CD133⁺Thy-1⁺. In another specific embodiment, the CD34⁺ hematopoietic cells are CXCR4⁺. In another specific embodiment, the CD34⁺ hematopoietic cells are CXCR4⁻. In another embodiment, the hematopoietic cells are positive for KDR (vascular growth factor receptor 2). In specific embodiments, the hematopoietic cells are CD34⁺KDR⁺, CD133⁺KDR⁺ or Thy-1⁺KDR⁺. In certain other embodiments, the hematopoietic cells are positive for aldehyde dehydrogenase (ALDH⁺), e.g., the cells are CD34⁺ALDH⁺.

In certain other embodiments, the CD34⁺ cells are CD45⁻. In specific embodiments, the CD34⁺ cells, *e.g*., CD34⁺, CD45⁻ cells express one or more, or all, of the miRNAs hsa-miR-380, hsa-miR-512, hsa-miR-517, hsa-miR-518c, hsa-miR-519b, hsa-miR-520a, hsa-miR-337, hsa-miR-422a, hsa-miR-549, and/or hsa-miR-618.

In certain embodiments, the hematopoietic cells are CD34⁻.

The hematopoietic cells can also lack certain markers that indicate lineage commitment, or a lack of developmental naiveté. For example, in another embodiment, the hematopoietic cells are HLA-DR-. In specific embodiments, the hematopoietic cells are CD34⁺HLA-DR⁻, CD133⁺HLA-DR⁻, Thy-1⁺HLA-DR⁻ or ALDH⁺HLA-DR⁻ In another embodiment, the hematopoietic cells are negative for one or more, or all, of lineage markers CD2, CD3, CD11b, CD11c, CD14, CD16, CD19, CD24, CD56, CD66b and glycophorin A.

Thus, hematopoietic cells can be selected for use in the methods disclosed herein on the basis of the presence of markers that indicate an undifferentiated state, or on the basis of the absence of lineage markers indicating that at least some lineage differentiation has taken place. Methods of isolating cells, including hematopoietic cells, on the basis of the presence or absence of specific markers is discussed in detail below.

Hematopoietic cells used in the methods provided herein can be a substantially homogeneous population, *e.g.*, a population comprising at least about 95%, at least about 98% or at least about 99% hematopoietic cells from a single tissue source, or a population comprising hematopoietic cells exhibiting the same hematopoietic cell-associated cellular markers. For example, in various embodiments, the hematopoietic cells can comprise at least about 95%, 98% or 99% hematopoietic cells from bone marrow, cord blood, placental blood, peripheral blood, or placenta, *e.g*., placenta perfusate.

Hematopoietic cells used in the methods provided herein can be obtained from a single individual, *e.g*., from a single placenta, or from a plurality of individuals, *e.g*., can be pooled. Where the hematopoietic cells are obtained from a plurality of individuals and pooled, the hematopoietic cells may be obtained from the same tissue source. Thus, in various embodiments, the pooled hematopoietic cells are all from placenta, *e.g*., placental perfusate, all from placental blood, all from umbilical cord blood, all from peripheral blood, and the like.

Hematopoietic cells used in the methods disclosed herein can, in certain embodiments, comprise hematopoietic cells from two or more tissue sources. For example, in certain embodiments, when hematopoietic cells from two or more sources are combined for use in the methods herein, a plurality of the hematopoietic cells used to produce natural killer cells using a three-stage method described herein comprise hematopoietic cells from placenta, *e.g*., placenta perfusate. In various embodiments, the hematopoietic cells used to produce NK cell populations and/or ILC3 cell populations produced using a three-stage method described herein, comprise hematopoietic cells from placenta and from cord blood; from placenta and peripheral blood; from placenta and placental blood, or placenta and bone marrow. In one embodiment, the hematopoietic cells comprise hematopoietic cells from placental perfusate in combination with hematopoietic cells from cord blood, wherein the cord blood and placenta are from the same individual, i.e., wherein the perfusate and cord blood are matched. In embodiments in which the hematopoietic cells comprise hematopoietic cells from two tissue sources, the hematopoietic cells from the sources can be combined in a ratio of, for example, 1:10, 2:9, 3:8, 4:7:, 5:6, 6:5, 7:4, 8:3, 9:2, 1:10, 1:9, 1:8, 1:7, 1:6, 1:5, 1:4, 1:3, 1:2, 1:1, 2:1, 3:1, 4:1, 5:1, 6:1, 7:1, 8:1 or 9:1.

### 5.1.1. Placental Hematopoietic Stem Cells

In certain embodiments, the hematopoietic cells used in the methods provided herein are placental hematopoietic cells. In one embodiment, placental hematopoietic cells are CD34⁺. In a specific embodiment, the placental hematopoietic cells are predominantly (*e.g*., at least about 50%, 55%, 60%, 65%, 70%, 75%, 80%, 85%, 90%, 95% or 98%) CD34⁺CD38⁻ cells. In another specific embodiment, the placental hematopoietic cells are predominantly (*e.g.*, at least about 50%, 55%, 60%, 65%, 70%, 75%, 80%, 85%, 90%, 95% or 98%) CD34⁺CD38⁺ cells. Placental hematopoietic cells can be obtained from a post-partum mammalian (*e.g*., human) placenta by any means known to those of skill in the art, *e.g.,* by perfusion.

In another embodiment, the placental hematopoietic cell is CD45⁻. In a specific embodiment, the hematopoietic cell is CD34⁺CD45⁻. In another specific embodiment, the placental hematopoietic cells are CD34⁺CD45⁺.

### 5.2. Production of Natural Killer and/or ILC3 Cells and Natural Killer Cell and/or ILC3 Cell Populations

Production of NK cells and/or ILC3 cells and NK cell and/or ILC3 cell populations by the present methods comprises expanding a population of hematopoietic cells. During cell expansion, a plurality of hematopoietic cells within the hematopoietic cell population differentiate into NK cells and/or ILC3 cells. In one aspect, provided herein is a method according to the claims of producing NK cells comprising culturing hematopoietic stem cells or progenitor cells, *e.g.,* CD34⁺ stem cells or progenitor cells, in a first medium comprising a stem cell mobilizing agent and thrombopoietin (Tpo) to produce a first population of cells, subsequently culturing said first population of cells in a second medium comprising a stem cell mobilizing agent and interleukin-15 (IL-15), and lacking Tpo, to produce a second population of cells, and subsequently culturing said second population of cells in a third medium comprising IL-2 and IL-15, and lacking a stem cell mobilizing agent and LMWH, to produce a third population of cells, wherein the third population of cells comprises natural killer cells that are CD56+, CD3-, and wherein at least 80%, of the natural killer cells are viable. In certain embodiments, such natural killer cells comprise natural killer cells that are CD16-. In certain embodiments, such natural killer cells comprise natural killer cells that are CD94+. In certain embodiments, such natural killer cells comprise natural killer cells that are CD94+ or CD16+. In certain embodiments, such natural killer cells comprise natural killer cells that are CD94- or CD16-. In certain embodiments, such natural killer cells comprise natural killer cells that are CD94+ and CD16+. In certain embodiments, such natural killer cells comprise natural killer cells that are CD94- and CD16-. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, *e.g*., low-molecular weight heparin.

Disclosed herein is a method of producing NK cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising IL-2 and IL-15, and lacking LMWH, to produce a third population of cells; wherein the third population of cells comprises natural killer cells that are CD56+, CD3-, and CD11a+. In certain examples said first medium and/or said second medium lack leukemia inhibiting factor (LIF) and/or macrophage inflammatory protein-1 alpha (MIP-1α). In certain examples said third medium lacks LIF, MIP-1α, and FMS-like tyrosine kinase-3 ligand (Flt-3L). In specific examples said first medium and said second medium lack LIF and MIP-1α, and said third medium lacks LIF, MIP-1α, and Flt3L. In certain examples none of the first medium, second medium or third medium comprises heparin, *e.g*., low-molecular weight heparin.

In one aspect, provided herein is a method according to the claims of producing according to the claims NK cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising IL-2 and IL-15, and lacking each of stem cell factor (SCF) and LMWH, to produce a third population of cells; wherein the third population of cells comprises natural killer cells that are CD56+, CD3-, and CD11a+. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin *e.g*., low-molecular weight heparin.

In one aspect, provided herein is a method according to the claims of producing NK cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising IL-2 and IL-15, and lacking each of SCF, a stem cell mobilizing agent, and LMWH, to produce a third population of cells; wherein the third population of cells comprises natural killer cells that are CD56+, CD3-, and CD11a+. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, *e.g*., low-molecular weight heparin.

In one aspect, provided herein is a method according to the claims of producing NK cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; (c) culturing the second population of cells in a third medium comprising IL-2 and IL-15, and lacking each of a stem cell mobilizing agent and LMWH, to produce a third population of cells; and (d) isolating CD11a+ cells from the third population of cells to produce a fourth population of cells; wherein the fourth population of cells comprises natural killer cells that are CD56+, CD3-, and CD11a+. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, *e.g*., low-molecular weight heparin.

In certain embodiments, of any of the above embodiments, said natural killer cells express perforin and EOMES. In certain embodiments, said natural killer cells do not express either RORyt or IL1R1. Disclosed

Disclosed herein is a method of producing II,C3 cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising IL-2 and IL-15, and lacking LMWH, to produce a third population of cells; wherein the third population of cells comprises ILC3 cells that are CD56+, CD3-, and CD11a-. In certain examples said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, *e.g.*, low-molecular weight heparin.

Disclosed herein is a method of producing II,C3 cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising a stem cell mobilizing agent, IL-2 and IL-15, and lacking LMWH, to produce a third population of cells; wherein the third population of cells comprises ILC3 cells that are CD56+, CD3-, and CD11a-. In certain examples, said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, *e.g.,* low-molecular weight heparin.

Disclosed herein is a method of producing II,C3 cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising SCF, IL-2 and IL-15, and lacking LMWH, to produce a third population of cells; wherein the third population of cells comprises ILC3 cells that are CD56+, CD3-, and CD11a-. In certain examples, said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, *e.g*., low-molecular weight heparin.

Disclosed herein is a method of producing II,C3 cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising a stem cell mobilizing agent, SCF, IL-2 and IL-15, and lacking LMWH, to produce a third population of cells; wherein the third population of cells comprises ILC3 cells that are CD56+, CD3-, and CD11a-. In certain examples, said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, *e.g*., low-molecular weight heparin.

In one aspect, provided herein is a method according to the claims of producing II,C3 cells comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; (c) culturing the second population of cells in a third medium comprising IL-2 and IL-15, and lacking each of a stem cell mobilizing agent and LMWH, to produce a third population of cells; and (d) isolating CD11a- cells, or removing CD11a+ cells, from the third population of cells to produce a fourth population of cells; wherein the fourth population of cells comprises ILC3 cells that are CD56+, CD3-, and CD11a-. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, *e.g*., low-molecular weight heparin.

In certain embodiments, said ILC3 cells express RORyt and IL1R1. In certain embodiments, said ILC3 cells do not express either perforin or EOMES.

### 5.2.1. Production of NK Cell and/or ILC3 Cell Populations Using a Three-Stage Method

Provided herein is a three-stage method according to the claims of producing NK cell and/or ILC3 cell populations. In certain embodiments, the method of expansion and differentiation of the hematopoietic cells, as described herein, to produce NK cell and/or ILC3 cell populations according to a three-stage method described herein comprises maintaining the cell population comprising said hematopoietic cells at between about 2 × 10⁴ and about 6 × 10⁶ cells per milliliter. In certain aspects, said hematopoietic stem or progenitor cells are initially inoculated into said first medium from 1 × 10⁴ to 1 × 10⁵ cells/mL. In a specific aspect, said hematopoietic stem or progenitor cells are initially inoculated into said first medium at about 3 × 10⁴ cells/mL.

In certain aspects, said first population of cells are initially inoculated into said second medium from 5 × 10⁴ to 5 × 10⁵ cells/mL. In a specific aspect, said first population of cells is initially inoculated into said second medium at about 1 × 10⁵ cells/mL.

In certain aspects said second population of cells is initially inoculated into said third medium from 1 × 10⁵ to 5 × 10⁶ cells/mL. In certain aspects, said second population of cells is initially inoculated into said third medium from 1 × 10⁵ to 1 × 10⁶ cells/mL. In a specific aspect, said second population of cells is initially inoculated into said third medium at about 5 × 10⁵ cells/mL. In a more specific aspect, said second population of cells is initially inoculated into said third medium at about 5 × 10⁵ cells/mL in a spinner flask. In a specific aspect, said second population of cells is initially inoculated into said third medium at about 3 × 10⁵ cells/mL. In a more specific aspect, said second population of cells is initially inoculated into said third medium at about 3 × 10⁵ cells/mL in a static culture.

The method comprises a first stage ("stage 1") comprising culturing hematopoietic stem cells or progenitor cells, *e.g.*, CD34⁺ stem cells or progenitor cells, in a first medium for a specified time period, *e.g*., as described herein, to produce a first population of cells. The first medium comprises a stem cell mobilizing agent and thrombopoietin (Tpo). In certain embodiments, the first medium comprises in addition to a stem cell mobilizing agent and Tpo, one or more of LMWH, Flt-3L, SCF, IL-6, IL-7, G-CSF, and GM-CSF. In a specific embodiment, the first medium comprises each of the first medium comprises in addition to a stem cell mobilizing agent and Tpo, each of LMWH, Flt-3L, SCF, IL-6, IL-7, G-CSF, and GM-CSF. In a specific embodiment, the first medium lacks added LMWH. In a specific embodiment, the first medium lacks added desulphated glycosaminoglycans. In a specific embodiment, the first medium lacks LMWH. In a specific embodiment, the first medium lacks desulphated glycosaminoglycans. In a specific embodiment, the first medium comprises each of the first medium comprises in addition to a stem cell mobilizing agent and Tpo, each of Flt-3L, SCF, IL-6, IL-7, G-CSF, and GM-CSF. In specific embodiments, the first medium lacks leukemia inhibiting factor (LIF), macrophage inhibitory protein-1alpha (MIP-1α) or both.

Subsequently in "stage 2" said cells are cultured in a second medium for a specified time period, *e.g.,* as described herein, to produce a second population of cells. The second medium comprises a stem cell mobilizing agent and interleukin-15 (IL-15), and lacks Tpo. In certain embodiments, the second medium comprises, in addition to a stem cell mobilizing agent and IL-15, one or more of LMWH, Flt-3, SCF, IL-6, IL-7, G-CSF, and GM-CSF. In certain embodiments, the second medium comprises, in addition to a stem cell mobilizing agent and IL-15, each of LMWH, Flt-3, SCF, IL-6, IL-7, G-CSF, and GM-CSF. In a specific embodiment, the second medium lacks added LMWH. In a specific embodiment, the second medium lacks added desulphated glycosaminoglycans. In a specific embodiment, the second medium lacks heparin, *e.g*., LMWH. In a specific embodiment, the second medium lacks desulphated glycosaminoglycans. In certain embodiments, the second medium comprises, in addition to a stem cell mobilizing agent and IL-15, each of Flt-3, SCF, IL-6, IL-7, G-CSF, and GM-CSF. In specific embodiments, the second medium lacks leukemia inhibiting factor (LIF), macrophage inhibitory protein-1alpha (MIP-1α) or both.

Subsequently, in "stage 3" said cells are cultured in a third medium for a specified time period, *e.g.,* as described herein, to produce a third population of cell, *e.g.,* natural killer cells. The third medium comprises IL-2 and IL-15, and lacks a stem cell mobilizing agent and LMWH. In certain embodiments, the third medium comprises in addition to IL-2 and IL-15, one or more of SCF, IL-6, IL-7, G-CSF, and GM-CSF. In certain embodiments, the third medium comprises, in addition to IL-2 and IL-15, each of SCF, IL-6, IL-7, G-CSF, and GM-CSF. In specific embodiments, the first medium lacks one, two, or all three of LIF, MIP-1α, and Flt3L. In specific embodiments, the third medium lacks added desulphated glycosaminoglycans. In specific embodiments, the third medium lacks desulphated glycosaminoglycans. In specific embodiments, the third medium lacks heparin, *e.g*., LMWH.

In a specific embodiment, the three-stage method according to the claims is used to produce NK cell and/or ILC3 cell populations. In certain embodiments, the three-stage method is conducted in the absence of stromal feeder cell support. In certain embodiments, the three-stage method is conducted in the absence of exogenously added steroids (*e.g*., cortisone, hydrocortisone, or derivatives thereof).

Said first medium used in the three-stage method comprises a stem cell mobilizing agent and thrombopoietin (Tpo). In certain aspects, the first medium used in the three-stage method comprises, in addition to a stem cell mobilizing agent and Tpo, one or more of Low Molecular Weight Heparin (LMWH), Flt-3 Ligand (Flt-3L), stem cell factor (SCF), IL-6, IL-7, granulocyte colony-stimulating factor (G-CSF), or granulocyte-macrophage-stimulating factor (GM-CSF). In certain aspects, the first medium used in the three-stage method comprises, in addition to a stem cell mobilizing agent and Tpo, each of LMWH, Flt-3L, SCF, IL-6, IL-7, G-CSF, and GM-CSF. In certain aspects, the first medium used in the three-stage method comprises, in addition to a stem cell mobilizing agent and Tpo, each of Flt-3L, SCF, IL-6, IL-7, G-CSF, and GM-CSF. In a specific aspect, the first medium lacks added LMWH. In a specific aspect, the first medium lacks added desulphated glycosaminoglycans. In a specific aspect, the first medium lacks LMWH. In a specific aspect, the first medium lacks desulphated glycosaminoglycans. In certain aspects, said Tpo is present in the first medium at a concentration of from 1 ng/mL to 100 ng/mL, from 1 ng/mL to 50 ng/mL, from 20 ng/mL to 30 ng/mL, or about 25 ng/mL. In certain aspects, in the first medium, the LMWH is present at a concentration of from 1U/mL to 10U/mL; the Flt-3L is present at a concentration of from 1 ng/mL to 50 ng/mL; the SCF is present at a concentration of from 1 ng/mL to 50 ng/mL; the IL-6 is present at a concentration of from 0.01 ng/mL to 0.1 ng/mL; the IL-7 is present at a concentration of from 1 ng/mL to 50 ng/mL; the G-CSF is present at a concentration of from 0.01 ng/mL to 0.50 ng/mL; and the GM-CSF is present at a concentration of from 0.005 ng/mL to 0.1 ng/mL. In certain aspects, in the first medium, the Flt-3L is present at a concentration of from 1 ng/mL to 50 ng/mL; the SCF is present at a concentration of from 1 ng/mL to 50 ng/mL; the IL-6 is present at a concentration of from 0.01 ng/mL to 0.1 ng/mL; the IL-7 is present at a concentration of from 1 ng/mL to 50 ng/mL; the G-CSF is present at a concentration of from 0.01 ng/mL to 0.50 ng/mL; and the GM-CSF is present at a concentration of from 0.005 ng/mL to 0.1 ng/mL. In certain aspects, in the first medium, the LMWH is present at a concentration of from 4U/mL to 5U/mL; the Flt-3L is present at a concentration of from 20 ng/mL to 30 ng/mL; the SCF is present at a concentration of from 20 ng/mL to 30 ng/mL; the IL-6 is present at a concentration of from 0.04 ng/mL to 0.06 ng/mL; the IL-7 is present at a concentration of from 20 ng/mL to 30 ng/mL; the G-CSF is present at a concentration of from 0.20 ng/mL to 0.30 ng/mL; and the GM-CSF is present at a concentration of from 0.005 ng/mL to 0.5 ng/mL. In certain aspects, in the first medium, the Flt-3L is present at a concentration of from 20 ng/mL to 30 ng/mL; the SCF is present at a concentration of from 20 ng/mL to 30 ng/mL; the IL-6 is present at a concentration of from 0.04 ng/mL to 0.06 ng/mL; the IL-7 is present at a concentration of from 20 ng/mL to 30 ng/mL; the G-CSF is present at a concentration of from 0.20 ng/mL to 0.30 ng/mL; and the GM-CSF is present at a concentration of from 0.005 ng/mL to 0.5 ng/mL. In certain aspects, in the first medium, the LMWH is present at a concentration of about 4.5U/mL; the Flt-3L is present at a concentration of about 25 ng/mL; the SCF is present at a concentration of about 27 ng/mL; the IL-6 is present at a concentration of about 0.05 ng/mL; the IL-7 is present at a concentration of about 25 ng/mL; the G-CSF is present at a concentration of about .25 ng/mL; and the GM-CSF is present at a concentration of about 0.01 ng/mL. In certain aspects, in the first medium, the Flt-3L is present at a concentration of about 25 ng/mL; the SCF is present at a concentration of about 27 ng/mL; the IL-6 is present at a concentration of about 0.05 ng/mL; the IL-7 is present at a concentration of about 25 ng/mL; the G-CSF is present at a concentration of about .25 ng/mL; and the GM-CSF is present at a concentration of about 0.01 ng/mL. In certain embodiments, said first medium additionally comprises one or more of the following: antibiotics such as gentamycin; antioxidants such as transferrin, insulin, and/or beta-mercaptoethanol; sodium selenite; ascorbic acid; ethanolamine; and glutathione. In certain embodiments, the medium that provides the base for the first medium is a cell/tissue culture medium known to those of skill in the art, *e.g*., a commercially available cell/tissue culture medium such as SCGM^{™}, STEMMACS^{™}, GBGM^{®}, AIM-V^{®}, X-VIVO^{™} 10, X-VIVO^{™} 15, OPTMIZER, STEMSPAN^{®} H3000, CELLGRO COMPLETE^{™}, DMEM:Ham's F12 ("F12") (*e.g*., 2:1 ratio, or high glucose or low glucose DMEM), Advanced DMEM (Gibco), EL08-1D2, Myelocult^{™} H5100, IMDM, and/or RPMI-1640; or is a medium that comprises components generally included in known cell/tissue culture media, such as the components included in GBGM^{®}, AIM-V^{®}, X-VIVO^{™} 10, X-VIVO^{™} 15, OPTMIZER, STEMSPAN^{®} H3000, CELLGRO COMPLETE^{™}, DMEM:Ham's F12 ("F12") (*e.g*., 2:1 ratio, or high glucose or low glucose DMEM), Advanced DMEM (Gibco), EL08-1D2, Myelocult^{™} H5100, IMDM, and/or RPMI-1640. In certain embodiments, said first medium is not GBGM^{®}. In specific embodiments of any of the above embodiments, the first medium lacks LIF, MIP-1α, or both.

Said second medium used in the three-stage method comprises a stem cell mobilizing agent and interleukin-15 (IL-15), and lacks Tpo. In certain aspects, the second medium used in the three-stage method comprises, in addition to a stem cell mobilizing agent and IL-15, one or more of LMWH, Flt-3, SCF, IL-6, IL-7, G-CSF, and GM-CSF. In certain aspects, the second medium used in the three-stage method comprises, in addition to a stem cell mobilizing agent and IL-15, each of LMWH, Flt-3, SCF, IL-6, IL-7, G-CSF, and GM-CSF. In certain aspects, the second medium used in the three-stage method comprises, in addition to a stem cell mobilizing agent and IL-15, each of Flt-3, SCF, IL-6, IL-7, G-CSF, and GM-CSF. In a specific aspect, the second medium lacks added LMWH. In a specific aspect, the second medium lacks added desulphated glycosaminoglycans. In a specific aspect, the second medium lacks LMWH. In a specific aspect, the second medium lacks desulphated glycosaminoglycans. In certain aspects, said IL-15 is present in said second medium at a concentration of from 1 ng/mL to 50 ng/mL, from 10 ng/mL to 30 ng/mL, or about 20 ng/mL. In certain aspects, in said second medium, the LMWH is present at a concentration of from 1U/mL to 10U/mL; the Flt-3L is present at a concentration of from 1 ng/mL to 50 ng/mL; the SCF is present at a concentration of from 1 ng/mL to 50 ng/mL; the IL-6 is present at a concentration of from 0.01 ng/mL to 0.1 ng/mL; the IL-7 is present at a concentration of from 1 ng/mL to 50 ng/mL; the G-CSF is present at a concentration of from 0.01 ng/mL to 0.50 ng/mL; and the GM-CSF is present at a concentration of from 0.005 ng/mL to 0.1 ng/mL. In certain aspects, in said second medium, the Flt-3L is present at a concentration of from 1 ng/mL to 50 ng/mL; the SCF is present at a concentration of from 1 ng/mL to 50 ng/mL; the IL-6 is present at a concentration of from 0.01 ng/mL to 0.1 ng/mL; the IL-7 is present at a concentration of from 1 ng/mL to 50 ng/mL; the G-CSF is present at a concentration of from 0.01 ng/mL to 0.50 ng/mL; and the GM-CSF is present at a concentration of from 0.005 ng/mL to 0.1 ng/mL. In certain aspects, in the second medium, the LMWH is present in the second medium at a concentration of from 4U/mL to 5U/mL; the Flt-3L is present at a concentration of from 20 ng/mL to 30 ng/mL; the SCF is present at a concentration of from 20 ng/mL to 30 ng/mL; the IL-6 is present at a concentration of from 0.04 ng/mL to 0.06 ng/mL; the IL-7 is present at a concentration of from 20 ng/mL to 30 ng/mL; the G-CSF is present at a concentration of from 0.20 ng/mL to 0.30 ng/mL; and the GM-CSF is present at a concentration of from 0.005 ng/mL to 0.5 ng/mL. In certain aspects, in the second medium, the Flt-3L is present at a concentration of from 20 ng/mL to 30 ng/mL; the SCF is present at a concentration of from 20 ng/mL to 30 ng/mL; the IL-6 is present at a concentration of from 0.04 ng/mL to 0.06 ng/mL; the IL-7 is present at a concentration of from 20 ng/mL to 30 ng/mL; the G-CSF is present at a concentration of from 0.20 ng/mL to 0.30 ng/mL; and the GM-CSF is present at a concentration of from 0.005 ng/mL to 0.5 ng/mL. In certain aspects, in the second medium, the LMWH is present in the second medium at a concentration of from 4U/mL to 5U/mL; the Flt-3L is present at a concentration of from 20 ng/mL to 30 ng/mL; the SCF is present at a concentration of from 20 ng/mL to 30 ng/mL; the IL-6 is present at a concentration of from 0.04 ng/mL to 0.06 ng/mL; the IL-7 is present at a concentration of from 20 ng/mL to 30 ng/mL; the G-CSF is present at a concentration of from 0.20 ng/mL to 0.30 ng/mL; and the GM-CSF is present at a concentration of from 0.005 ng/mL to 0.5 ng/mL. In certain aspects, in the second medium, the Flt-3L is present at a concentration of from 20 ng/mL to 30 ng/mL; the SCF is present at a concentration of from 20 ng/mL to 30 ng/mL; the IL-6 is present at a concentration of from 0.04 ng/mL to 0.06 ng/mL; the IL-7 is present at a concentration of from 20 ng/mL to 30 ng/mL; the G-CSF is present at a concentration of from 0.20 ng/mL to 0.30 ng/mL; and the GM-CSF is present at a concentration of from 0.005 ng/mL to 0.5 ng/mL. In certain aspects, in the second medium, the LMWH is present in the second medium at a concentration of about 4.5U/mL; the Flt-3L is present at a concentration of about 25 ng/mL; the SCF is present at a concentration of about 27 ng/mL; the IL-6 is present at a concentration of about 0.05 ng/mL; the IL-7 is present at a concentration of about 25 ng/mL; the G-CSF is present at a concentration of about 0.25 ng/mL; and the GM-CSF is present at a concentration of about 0.01 ng/mL. In certain aspects, in the second medium, the Flt-3L is present at a concentration of about 25 ng/mL; the SCF is present at a concentration of about 27 ng/mL; the IL-6 is present at a concentration of about 0.05 ng/mL; the IL-7 is present at a concentration of about 25 ng/mL; the G-CSF is present at a concentration of about 0.25 ng/mL; and the GM-CSF is present at a concentration of about 0.01 ng/mL. In certain embodiments, said second medium additionally comprises one or more of the following: antibiotics such as gentamycin; antioxidants such as transferrin, insulin, and/or beta-mercaptoethanol; sodium selenite; ascorbic acid; ethanolamine; and glutathione. In certain embodiments, the medium that provides the base for the second medium is a cell/tissue culture medium known to those of skill in the art, *e.g*., a commercially available cell/tissue culture medium such as SCGM^{™}, STEMMACS^{™}, GBGM^{®}, AIM-V^{®}, X-VIVO^{™} 10, X-VIVO^{™} 15, OPTMIZER, STEMSPAN^{®} H3000, CELLGRO COMPLETE^{™}, DMEM:Ham's F12 ("F12") (*e.g*., 2:1 ratio, or high glucose or low glucose DMEM), Advanced DMEM (Gibco), EL08-1D2, Myelocult^{™} H5100, IMDM, and/or RPMI-1640; or is a medium that comprises components generally included in known cell/tissue culture media, such as the components included in GBGM^{®}, AIM-V^{®}, X-VIVO^{™} 10, X-VIVO^{™} 15, OPTMIZER, STEMSPAN^{®} H3000, CELLGRO COMPLETE^{™}, DMEM:Ham's F12 ("F12") (e.g., 2:1 ratio, or high glucose or low glucose DMEM), Advanced DMEM (Gibco), EL08-1D2, Myelocult^{™} H5100, IMDM, and/or RPMI-1640. In certain embodiments, said second medium is not GBGM^{®}. In specific embodiments of any of the above embodiments, the first medium lacks LIF, MIP-1α, or both.

Said third medium used in the three-stage method comprises IL-2 and IL-15, and lacks a stem cell mobilizing agent and LMWH. Said medium used in the three-stage method comprises IL-2 and IL-15, and lacks LMWH. In certain aspects, said third medium used in the three-stage method comprises IL-2 and IL-15, and lacks SCF and LMWH. In certain aspects, said third medium used in the three-stage method comprises IL-2 and IL-15, and lacks SCF, a stem cell mobilizing agent and LMWH. In certain aspects, said third medium used in the three-stage method comprises SCF, IL-2 and IL-15, and lacks LMWH. Said third medium used in the three-stage method comprises IL-2 and IL-15, and lacks a stem cell mobilizing agent and LMWH. In certain aspects, the third medium used in the three-stage method comprises, in addition to IL-2 and IL-15, one or more of SCF, IL-6, IL-7, G-CSF, or GM-CSF. In certain aspects, the third medium used in the three-stage method comprises, in addition to IL-2 and IL-15, each of SCF, IL-6, IL-7, G-CSF, and GM-CSF. In certain aspects, said IL-2 is present in said third medium at a concentration of from 10 U/mL to 10,000 U/mL and said IL-15 is present in said third medium at a concentration of from 1 ng/mL to 50 ng/mL. In certain aspects, said IL-2 is present in said third medium at a concentration of from 100 U/mL to 10,000 U/mL and said IL-15 is present in said third medium at a concentration of from 1 ng/mL to 50 ng/mL. In certain aspects, said IL-2 is present in said third medium at a concentration of from 300 U/mL to 3,000 U/mL and said IL-15 is present in said third medium at a concentration of from 10 ng/mL to 30 ng/mL. In certain aspects, said IL-2 is present in said third medium at a concentration of about 1,000 U/mL and said IL-15 is present in said third medium at a concentration of about 20 ng/mL. In certain aspects, in said third medium, the SCF is present at a concentration of from 1 ng/mL to 50 ng/mL; the IL-6 is present at a concentration of from 0.01 ng/mL to 0.1 ng/mL; the IL-7 is present at a concentration of from 1 ng/mL to 50 ng/mL; the G-CSF is present at a concentration of from 0.01 ng/mL to 0.50 ng/mL; and the GM-CSF is present at a concentration of from 0.005 ng/mL to 0.1 ng/mL. In certain aspects, in said third medium, the SCF is present at a concentration of from 20 ng/mL to 30 ng/mL; the IL-6 is present at a concentration of from 0.04 ng/mL to 0.06 ng/mL; the IL-7 is present at a concentration of from 20 ng/mL to 30 ng/mL; the G-CSF is present at a concentration of from 0.20 ng/mL to 0.30 ng/mL; and the GM-CSF is present at a concentration of from 0.005 ng/mL to 0.5 ng/mL. In certain aspects, in said third medium, the SCF is present at a concentration of about 22 ng/mL; the IL-6 is present at a concentration of about 0.05 ng/mL; the IL-7 is present at a concentration of about 20 ng/mL; the G-CSF is present at a concentration of about 0.25 ng/mL; and the GM-CSF is present at a concentration of about 0.01 ng/mL. In certain aspects, the third medium comprises 100 ng/mL IL-7, 22 ng/mL SCF, 1000 ng/mL IL-2, and 20 ng/mL IL-15 and lacks SR1. In certain aspects, the third medium comprises 22 ng/mL SCF, 1000 ng/mL IL-2, and 20 ng/mL IL-15 and lacks SR1. In certain aspects, the third medium comprises 20 ng/mL IL-7, 22 ng/mL SCF, 1000 ng/mL IL-2, and 20 ng/mL IL-15 and lacks SR1. In certain aspects, the third medium comprises 20 ng/mL IL-7, 22 ng/mL SCF, and 1000 ng/mL IL-2 and lacks SR1. In specific embodiments of any of the above embodiments, the first medium lacks one, two, or all three of LIF, MIP-1α, Flt-3L.

In certain embodiments, said third medium additionally comprises one or more of the following: antibiotics such as gentamycin; antioxidants such as transferrin, insulin, and/or beta-mercaptoethanol; sodium selenite; ascorbic acid; ethanolamine; and glutathione. In certain embodiments, the medium that provides the base for the third medium is a cell/tissue culture medium known to those of skill in the art, e.g., a commercially available cell/tissue culture medium such as SCGM^{™}, STEMMACS^{™}, GBGM^{®}, AIM-V^{®}, X-VIVO^{™} 10, X-VIVO^{™} 15, OPTMIZER, STEMSPAN^{®} H3000, CELLGRO COMPLETE^{™}, DMEM:Ham's F12 ("F12") (*e.g*., 2:1 ratio, or high glucose or low glucose DMEM), Advanced DMEM (Gibco), EL08-1D2, Myelocult^{™} H5100, IMDM, and/or RPMI-1640; or is a medium that comprises components generally included in known cell/tissue culture media, such as the components included in GBGM^{®}, AIM-V^{®}, X-VIVO^{™} 10, X-VIVO^{™} 15, OPTMIZER, STEMSPAN^{®} H3000, CELLGRO COMPLETE^{™}, DMEM:Ham's F12 ("F12") (*e.g*., 2:1 ratio, or high glucose or low glucose DMEM), Advanced DMEM (Gibco), EL08-1D2, Myelocult^{™} H5100, IMDM, and/or RPMI-1640. In certain embodiments, said third medium is not GBGM^{®}.

Generally, the particularly recited medium components do not refer to possible constituents in an undefined component of said medium. For example, said Tpo, IL-2, and IL-15 are not comprised within an undefined component of the first medium, second medium or third medium, *e.g*., said Tpo, IL-2, and IL-15 are not comprised within serum. Further, said LMWH, Flt-3, SCF, IL-6, IL-7, G-CSF, and/or GM-CSF are not comprised within an undefined component of the first medium, second medium or third medium, *e.g*., said LMWH, Flt-3, SCF, IL-6, IL-7, G-CSF, and/or GM-CSF are not comprised within serum.

In certain aspects, said first medium, second medium or third medium comprises human serum-AB. In certain aspects, any of said first medium, second medium or third medium comprises 1% to 20% human serum-AB, 5% to 15% human serum-AB, or about 2, 5, or 10% human serum-AB.

In certain embodiments, in the three-stage methods described herein, said hematopoietic stem or progenitor cells are cultured in said first medium for 7, 8, 9, 10, 11, 12, or 13, days. In certain embodiments, in the three-stage methods described herein, cells are cultured in said second medium for 2, 3, 4, 5, or 6, days. In certain embodiments, in the three-stage methods described herein, cells are cultured in said third medium for 10, 11, 12, 13, 14, 15, 16, 17, 18, 19, 20, 21, 22, 23, 24, 25, 26, 27, 28, 29, or 30 days,

Said hematopoietic stem or progenitor cells are cultured in said first medium for 7-13 days to produce a first population of cells, before said culturing in said second medium; said first population of cells are cultured in said second medium for 2-6 days to produce a second population of cells before said culturing in said third medium; and said second population of cells are cultured in said third medium for 10-30 days, *i.e.,* the cells are cultured a total of 19-49 days.

In a specific embodiment, in the three-stage methods described herein, in the three-stage methods described herein, said hematopoietic stem or progenitor cells are cultured in said first medium for 8-12 days to produce a first population of cells, before said culturing in said second medium; said first population of cells are cultured in said second medium for 3-5 days to produce a second population of cells before said culturing in said third medium; and said second population of cells are cultured in said third medium for 15-25 days, *i.e.,* the cells are cultured a total of 26-42 days.

In a specific embodiment, in the three-stage methods described herein, said hematopoietic stem or progenitor cells are cultured in said first medium for about 10 days to produce a first population of cells, before said culturing in said second medium; said first population of cells are cultured in said second medium for about 4 days to produce a second population of cells before said culturing in said third medium; and said second population of cells are cultured in said third medium for about 21 days, *i.e.,* the cells are cultured a total of about 35 days.

In certain aspects, the three-stage method disclosed herein produces at least 5000-fold more natural killer cells as compared to the number of hematopoietic stem cells initially inoculated into said first medium. In certain aspects, said three-stage method produces at least 10,000-fold more natural killer cells as compared to the number of hematopoietic stem cells initially inoculated into said first medium. In certain aspects, said three-stage method produces at least 50,000-fold more natural killer cells as compared to the number of hematopoietic stem cells initially inoculated into said first medium. In certain aspects, said three-stage method produces at least 75,000-fold more natural killer cells as compared to the number of hematopoietic stem cells initially inoculated into said first medium. In certain aspects, the viability of said natural killer cells is determined by 7-aminoactinomycin D (7AAD) staining. In certain aspects, the viability of said natural killer cells is determined by annexin-V staining. In specific aspects, the viability of said natural killer cells is determined by both 7-AAD staining and annexin-V staining. In certain aspects, the viability of said natural killer cells is determined by trypan blue staining.

In certain aspects, the three-stage method disclosed herein produces at least 5000-fold more ILC3 cells as compared to the number of hematopoietic stem cells initially inoculated into said first medium. In certain aspects, said three-stage method produces at least 10,000-fold more ILC3 cells as compared to the number of hematopoietic stem cells initially inoculated into said first medium. In certain aspects, said three-stage method produces at least 50,000-fold more ILC3 cells as compared to the number of hematopoietic stem cells initially inoculated into said first medium. In certain aspects, said three-stage method produces at least 75,000-fold more ILC3 cells as compared to the number of hematopoietic stem cells initially inoculated into said first medium.

In certain aspects, the three-stage method produces natural killer cells that comprise at least 20% CD56+CD3- natural killer cells. In certain aspects, the three-stage method produces natural killer cells that comprise at least 40% CD56+CD3- natural killer cells. In certain aspects, the three-stage method produces natural killer cells that comprise at least 60% CD56+CD3- natural killer cells. In certain aspects, the three-stage method produces natural killer cells that comprise at least 70% CD56+CD3- natural killer cells. In certain aspects, the three-stage method produces natural killer cells that comprise at least 80% CD56+CD3- natural killer cells.

In certain aspects, the three-stage method disclosed herein produces natural killer cells that comprise at least 20% CD56+CD3-CD11a+ natural killer cells. In certain aspects, the three-stage method disclosed herein produces natural killer cells that comprise at least 40% CD56+CD3- CD11a+ natural killer cells. In certain aspects, the three-stage method disclosed herein produces natural killer cells that comprise at least 60% CD56+CD3- CD11a+ natural killer cells. In certain aspects, the three-stage method disclosed herein produces natural killer cells that comprise at least 80% CD56+CD3- CD11a+ natural killer cells.

In certain aspects, the three-stage method disclosed herein produces ILC3 cells that comprise at least 20% CD56+CD3- CD11a- ILC3 cells. In certain aspects, the three-stage method disclosed herein produces ILC3 cells that comprise at least 40% CD56+CD3- CD1 1a-ILC3 cells. In certain aspects, the three-stage method disclosed herein produces ILC3 cells that comprise at least 60% CD56+CD3- CD11a- ILC3 cells. In certain aspects, the three-stage method disclosed herein produces natural killer cells that comprise at least 80% CD56+CD3-CD11a- ILC3 cells.

In certain aspects, the three-stage method produces natural killer cells that exhibit at least 20% cytotoxicity against K562 cells when said natural killer cells and said K562 cells are co-cultured *in vitro* or *ex vivo* at a ratio of 10:1. In certain aspects, the three-stage method produces natural killer cells that exhibit at least 35% cytotoxicity against the K562 cells when said natural killer cells and said K562 cells are co-cultured *in vitro* or *ex vivo* at a ratio of 10:1. In certain aspects, the three-stage method produces natural killer cells that exhibit at least 45% cytotoxicity against the K562 cells when said natural killer cells and said K562 cells are co-cultured *in vitro* or *ex vivo* at a ratio of 10:1. In certain aspects, the three-stage method produces natural killer cells that exhibit at least 60% cytotoxicity against the K562 cells when said natural killer cells and said K562 cells are co-cultured *in vitro* or *ex vivo* at a ratio of 10:1. In certain aspects, the three-stage method produces natural killer cells that exhibit at least 75% cytotoxicity against the K562 cells when said natural killer cells and said K562 cells are co-cultured *in vitro* or *ex vivo* at a ratio of 10:1.

In certain aspects, the three-stage method produces ILC3 cells that exhibit at least 20% cytotoxicity against K562 cells when said ILC3 cells and said K562 cells are co-cultured *in vitro* or *ex vivo* at a ratio of 10:1. In certain aspects, the three-stage method produces ILC3 cells that exhibit at least 35% cytotoxicity against the K562 cells when said ILC3 cells and said K562 cells are co-cultured *in vitro* or *ex vivo* at a ratio of 10:1. In certain aspects, the three-stage method produces ILC3 cells that exhibit at least 45% cytotoxicity against the K562 cells when said ILC3 cells and said K562 cells are co-cultured *in vitro* or *ex vivo* at a ratio of 10:1. In certain aspects, the three-stage method produces ILC3 cells that exhibit at least 60% cytotoxicity against the K562 cells when said ILC3 cells and said K562 cells are co-cultured *in vitro* or *ex vivo* at a ratio of 10:1. In certain aspects, the three-stage method produces ILC3 cells that exhibit at least 75% cytotoxicity against the K562 cells when said ILC3 cells and said K562 cells are co-cultured *in vitro* or *ex vivo* at a ratio of 10:1.

In certain aspects, after said third culturing step, said third population of cells, *e.g*., said population of natural killer cells and/or ILC3 cells, is cryopreserved. In certain aspects, after said fourth step, said fourth population of cells, *e.g*., said population of natural killer cells and/or ILC3 cells, is cryopreserved.

Disclosed herein are populations of cells comprising natural killer cells, *i.e*., natural killers cells produced by a three-stage method described herein. Accordingly, disclosed herein is an isolated natural killer cell population produced by a three-stage method described herein. In a specific example, said natural killer cell population comprises at least 20% CD56+CD3- natural killer cells. In a specific example, said natural killer cell population comprises at least 40% CD56+CD3- natural killer cells. In a specific example, said natural killer cell population comprises at least 60% CD56+CD3- natural killer cells. In a specific examples, said natural killer cell population comprises at least 80% CD56+CD3- natural killer cells. In a specific example, said natural killer cell population comprises at least 60% CD16- cells. In a specific example, said natural killer cell population comprises at least 80% CD16- cells. In a specific example, said natural killer cell population comprises at least 20% CD94+ cells. In a specific example, said natural killer cell population comprises at least 40% CD94+ cells.

Disclosed herein is a population of natural killer cells that is CD56+CD3- CD117+CD11a+, wherein said natural killer cells express perforin and/or EOMES, and do not express one or more of RORyt, aryl hydrocarbon receptor (AHR), and IL1R1. In certain aspects, said natural killer cells express perforin and EOMES, and do not express any of RORyt, aryl hydrocarbon receptor, or IL1R1. In certain aspects, said natural killer cells additionally express T-bet, GZMB, NKp46, NKp30, and NKG2D. In certain aspects, said natural killer cells express CD94. In certain aspects, said natural killer cells do not express CD94.

Disclosed herein is a population of ILC3 cells that is CD56+CD3- CD117+CD11a-, wherein said ILC3 cells express one or more of RORyt, aryl hydrocarbon receptor, and IL1R1, and do not express one or more of CD94, perforin, and EOMES. In certain aspects, said ILC3 cells express RORyt, aryl hydrocarbon receptor, and IL1R1, and do not express any of CD94, perforin, or EOMES. In certain aspects, said ILC3 cells additionally express CD226 and/or 2B4. In certain aspects, said ILC3 cells additionally express one or more of IL-22, TNFα, and DNAM-1. In certain aspects, said ILC3 cells express CD226, 2B4, IL-22, TNFα, and DNAM-1.

In certain aspects, provided herein is a method according to the claims of producing a cell population comprising natural killer cells and ILC3 cells, comprising (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; (c) culturing the second population of cells in a third medium comprising IL-2 and IL-15, and lacking each of a stem cell mobilizing agent and LMWH, to produce a third population of cells; and (d) separating CD11a+ cells and CD11a-cells from the third population of cells; and (e) combining the CD11a+ cells with the CD11a-cells in a ratio of 50:1, 40:1, 30:1, 20:1, 10:1, 5:1, 4:1, 3:1, 2:1, 1:1, 1:2, 1:3, 1:4, 1:5, 1:10, 1:20, 1:30, 1:40, or 1:50 to produce a fourth population of cells. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, e.g., low-molecular weight heparin. In certain aspects, in the fourth population of cells, the CD11a+ cells and CD11a- cells are combined in a ratio of 50:1. In certain aspects, in the fourth population of cells, the CD11a+ cells and CD11a- cells are combined in a ratio of 20:1. In certain aspects, in the fourth population of cells, the CD11a+ cells and CD11a- cells are combined in a ratio of 10:1. In certain aspects, in the fourth population of cells, the CD11a+ cells and CD11a- cells are combined in a ratio of 5:1. In certain aspects, in the fourth population of cells, the CD11a+ cells and CD11a- cells are combined in a ratio of 1:1. In certain aspects, in the fourth population of cells, the CD11a+ cells and CD11a-cells are combined in a ratio of 1:5. In certain aspects, in the fourth population of cells, the CD11a+ cells and CD11a- cells are combined in a ratio of 1:10. In certain aspects, in the fourth population of cells, the CD11a+ cells and CD11a- cells are combined in a ratio of 1:20. In certain aspects, in the fourth population of cells, the CD11a+ cells and CD11a- cells are combined in a ratio of 1:50.

### 5.3. Stem Cell Mobilizing Factors

### 5.3.1. Chemistry definitions

To facilitate understanding of the disclosure of stem cell mobilizing factors set forth herein, a number of terms are defined below.

Generally, the nomenclature used herein and the laboratory procedures in biology, cellular biology, biochemistry, organic chemistry, medicinal chemistry, and pharmacology described herein are those well known and commonly employed in the art. Unless defined otherwise, all technical and scientific terms used herein generally have the same meaning as commonly understood by one of ordinary skill in the art to which this disclosure belongs.

The term "about" or "approximately" means an acceptable error for a particular value as determined by one of ordinary skill in the art, which depends in part on how the value is measured or determined. In certain examples, the term "about" or "approximately" means within 1, 2, 3, or 4 standard deviations. In certain examples, the term "about" or "approximately" means within 50%, 20%, 15%, 10%, 9%, 8%, 7%, 6%, 5%, 4%, 3%, 2%, 1%, 0.5%, or 0.05% of a given value or range.

The term "aryl hydrocarbon receptor" or "AHR" refers to a protein encoded by the AHR gene in humans, or a variant thereof (for example, *see* GenBank Accession Nos. P35869.2 and AAH70080.1) .

The term "aryl hydrocarbon receptor antagonist," "AHR antagonist," "aryl hydrocarbon receptor inhibitor," or "AHR inhibitor" refers to a compound that downregulates or reduces the activity of an aryl hydrocarbon receptor.

The term "alkyl" refers to a linear or branched saturated monovalent hydrocarbon radical, wherein the alkyl is optionally substituted with one or more substituents Q as described herein. The term "alkyl" also encompasses both linear and branched alkyl, unless otherwise specified. In certain embodiments, the alkyl is a linear saturated monovalent hydrocarbon radical that has 1 to 20 (C₁₋₂₀), 1 to 15 (C₁₋₁₅), 1 to 10 (C₁₋₁₀), or 1 to 6 (C₁₋₆) carbon atoms, or branched saturated monovalent hydrocarbon radical of 3 to 20 (C₃₋₂₀), 3 to 15 (C₃₋₁₅), 3 to 10 (C₃₋₁₀), or 3 to 6 (C₃₋₆) carbon atoms. As used herein, linear C₁₋₆ and branched C₃₋₆ alkyl groups are also referred as "lower alkyl." Examples of alkyl groups include, but are not limited to, methyl, ethyl, propyl (including all isomeric forms), n-propyl, isopropyl, butyl (including all isomeric forms), *n*-butyl, isobutyl, *sec*-butyl, *t*-butyl, pentyl (including all isomeric forms), and hexyl (including all isomeric forms). For example, C₁₋₆ alkyl refers to a linear saturated monovalent hydrocarbon radical of 1 to 6 carbon atoms or a branched saturated monovalent hydrocarbon radical of 3 to 6 carbon atoms.

The term "alkylene" refers to a linear or branched saturated divalent hydrocarbon radical, wherein the alkylene is optionally substituted with one or more substituents Q as described herein. For example, C₁₋₆ alkylene refers to a linear saturated divalent hydrocarbon radical of 1 to 6 carbon atoms or a branched saturated divalent hydrocarbon radical of 3 to 6 carbon atoms. In certain embodiments, the alkylene is a linear saturated divalent hydrocarbon radical that has 1 to 20 (C₁₋₂₀), 1 to 15 (C₁₋₁₅), 1 to 10 (C₁₋₁₀), or 1 to 6 (C₁₋₆) carbon atoms, or branched saturated divalent hydrocarbon radical of 3 to 20 (C₃₋₂₀), 3 to 15 (C₃₋₁₅), 3 to 10 (C₃₋₁₀), or 3 to 6 (C₃₋₆) carbon atoms. As used herein, linear C₁₋₆ and branched C₃₋₆ alkylene groups are also referred as "lower alkylene." Examples of alkylene groups include, but are not limited to, methylene, ethylene, propylene (including all isomeric forms), *n*-propylene, isopropylene, butylene (including all isomeric forms), *n*-butylene, isobutylene, *t*-butylene, pentylene (including all isomeric forms), and hexylene (including all isomeric forms).

The term "alkenyl" refers to a linear or branched monovalent hydrocarbon radical, which contains one or more, in one examples, one, two, three, four, or five, in another embodiment, one, carbon-carbon double bond(s). The alkenyl is optionally substituted with one or more substituents Q as described herein. The term "alkenyl" also embraces radicals having *"cis"* and *"trans"* configurations, or alternatively, "Z" and "E" configurations, as appreciated by those of ordinary skill in the art. As used herein, the term "alkenyl" encompasses both linear and branched alkenyl, unless otherwise specified. For example, C₂₋₆ alkenyl refers to a linear unsaturated monovalent hydrocarbon radical of 2 to 6 carbon atoms or a branched unsaturated monovalent hydrocarbon radical of 3 to 6 carbon atoms. In certain examples, the alkenyl is a linear monovalent hydrocarbon radical of 2 to 20 (C₂₋₂₀), 2 to 15 (C₂₋₁₅, 2 to 10 (C₂₋₁₀), or 2 to 6 (C₂₋₆) carbon atoms, or a branched monovalent hydrocarbon radical of 3 to 20 (C₃₋₂₀), 3 to 15 (C₃₋₁₅), 3 to 10 (C₃₋₁₀), or 3 to 6 (C₃₋₆) carbon atoms. Examples of alkenyl groups include, but are not limited to, ethenyl, propen-1-yl, propen-2-yl, allyl, butenyl, and 4-methylbutenyl.

The term "alkenylene" refers to a linear or branched divalent hydrocarbon radical, which contains one or more, in one examples, one to five, in another examples, one, carbon-carbon double bond(s). The alkenylene is optionally substituted with one or more substituents Q as described herein. The term "alkenylene" embraces radicals having a *"cis"* or *"trans"* configuration or a mixture thereof, or alternatively, a "Z" or "E" configuration or a mixture thereof, as appreciated by those of ordinary skill in the art. For example, C₂₋₆ alkenylene refers to a linear unsaturated divalent hydrocarbon radical of 2 to 6 carbon atoms or a branched unsaturated divalent hydrocarbon radical of 3 to 6 carbon atoms. In certain examples, the alkenylene is a linear divalent hydrocarbon radical of 2 to 20 (C₂₋₂₀), 2 to 15 (C₂₋₁₅), 2 to 10 (C₂₋₁₀), or 2 to 6 (C₂₋₆) carbon atoms, or a branched divalent hydrocarbon radical of 3 to 20 (C₃₋₂₀), 3 to 15 (C₃₋₁₅), 3 to 10 (C₃₋₁₀), or 3 to 6 (C₃₋₆) carbon atoms. Examples of alkenylene groups include, but are not limited to, ethenylene, allylene, propenylene, butenylene, and 4-methylbutenylene.

The term "alkynyl" refers to a linear or branched monovalent hydrocarbon radical, which contains one or more, in one example, one, two, three, four, or five, in another example, one, carbon-carbon triple bond(s). The alkynyl is optionally substituted with one or more substituents Q as described herein. The term "alkynyl" also encompasses both linear and branched alkynyl, unless otherwise specified. In certain examples, the alkynyl is a linear monovalent hydrocarbon radical of 2 to 20 (C₂₋₂₀), 2 to 15 (C₂₋₁₅, 2 to 10 (C₂₋₁₀), or 2 to 6 (C₂₋₆) carbon atoms, or a branched monovalent hydrocarbon radical of 3 to 20 (C₃₋₂₀), 3 to 15 (C₃₋₁₅), 3 to 10 (C₃₋₁₀), or 3 to 6 (C₃₋₆) carbon atoms. Examples of alkynyl groups include, but are not limited to, ethynyl (-C=CH) and propargyl (-CH₂C≡CH). For example, C₂₋₆ alkynyl refers to a linear unsaturated monovalent hydrocarbon radical of 2 to 6 carbon atoms or a branched unsaturated monovalent hydrocarbon radical of 3 to 6 carbon atoms.

The term "alkynylene" refers to a linear or branched divalent hydrocarbon radical, which contains one or more, in one example, one to five, in another example, one, carbon-carbon triple bond(s). The alkynylene is optionally substituted with one or more substituents Q as described herein. For example, C₂₋₆ alkynylene refers to a linear unsaturated divalent hydrocarbon radical of 2 to 6 carbon atoms or a branched unsaturated divalent hydrocarbon radical of 3 to 6 carbon atoms. In certain examples, the alkynylene is a linear divalent hydrocarbon radical of 2 to 20 (C₂₋₂₀), 2 to 15 (C₂₋₁₅, 2 to 10 (C₂₋₁₀), or 2 to 6 (C₂₋₆) carbon atoms, or a branched divalent hydrocarbon radical of 3 to 20 (C₃₋₂₀), 3 to 15 (C₃₋₁₅), 3 to 10 (C₃₋₁₀), or 3 to 6 (C₃₋₆) carbon atoms. Examples of alkynylene groups include, but are not limited to, ethynylene, propynylene (including all isomeric forms, *e.g*., 1-propynylene and propargylene), butynylene (including all isomeric forms, *e.g*., 1-butyn-1-ylene and 2-butyn-1-ylene), pentynylene (including all isomeric forms, *e.g*., 1-pentyn-1-ylene and 1-methyl-2-butyn-1-ylene), and hexynylene (including all isomeric forms, *e.g*., 1-hexyn-1-ylene).

The term "cycloalkyl" refers to a cyclic saturated or non-aromatic unsaturated, bridged or non-bridged monovalent hydrocarbon radical, which is optionally substituted with one or more substituents Q as described herein. In certain examples, the cycloalkyl is a cyclic saturated bridged or non-bridged monovalent hydrocarbon radical. In certain examples, the cycloalkyl has from 3 to 20 (C₃₋₂₀), from 3 to 15 (C₃₋₁₅), from 3 to 10 (C₃₋₁₀), or from 3 to 7 (C₃₋₇) carbon atoms. Examples of cycloalkyl groups include, but are not limited to, cyclopropyl, cyclobutyl, cyclopentyl, cyclohexyl, cycloheptyl, bicyclo[2.1.1]hexyl, bicyclo[2.2.1]heptyl, decalinyl, and adamantyl.

The term "cycloalkylene" refers to a cyclic divalent hydrocarbon radical, which is optionally substituted with one or more substituents Q as described herein. In one example, cycloalkyl groups is saturated or unsaturated but non-aromatic, and/or bridged, and/or non-bridged, and/or fused bicyclic groups. In certain examples, the cycloalkylene has from 3 to 20 (C₃₋₂₀), from 3 to 15 (C₃₋₁₅), from 3 to 10 (C₃₋₁₀), or from 3 to 7 (C₃₋₇) carbon atoms. Examples of cycloalkylene groups include, but are not limited to, cyclopropylene (*e.g*., 1,1-cyclopropylene and 1,2-cyclopropylene), cyclobutylene (*e.g*., 1,1-cyclobutylene, 1,2-cyclobutylene, or 1,3-cyclobutylene), cyclopentylene (*e.g*., 1,1-cyclopentylene, 1,2-cyclopentylene, or 1,3-cyclopentylene), cyclohexylene (*e.g*., 1,1-cyclohexylene, 1,2-cyclohexylene, 1,3-cyclohexylene, or 1,4-cyclohexylene), cycloheptylene (*e.g*., 1,1-cycloheptylene, 1,2-cycloheptylene, 1,3-cycloheptylene, or 1,4-cycloheptylene), decalinylene, and adamantylene.

The term "aryl" refers to a monocyclic aromatic carbocyclic group and/or multicyclic monovalent aromatic carbocyclic group that contain at least one aromatic hydrocarbon ring. In certain examples, the aryl has from 6 to 20 (C₆₋₂₀), from 6 to 15 (C₆₋₁₅), or from 6 to 10 (C₆₋₁₀) ring atoms. Examples of aryl groups include, but are not limited to, phenyl, naphthyl, fluorenyl, azulenyl, anthryl, phenanthryl, pyrenyl, biphenyl, and terphenyl. In certain examples, the term "aryl" refers to a bicyclic or tricyclic carbon ring, where one of the rings is aromatic and the others of which can be saturated, partially unsaturated, or aromatic, for example, dihydronaphthyl, indenyl, indanyl, or tetrahydronaphthyl (tetralinyl). The aryl is optionally substituted with one or more substituents Q as described herein.

The term "arylene" refers to a divalent monocyclic aromatic group and/or divalent polycyclic aromatic group that contain at least one aromatic carbon ring. In certain examples, the arylene has from 6 to 20 (C₆₋₂₀), from 6 to 15 (C₆₋₁₅), or from 6 to 10 (C₆₋₁₀) ring atoms. Examples of arylene groups include, but are not limited to, phenylene, naphthylene, fluorenylene, azulenylene, anthrylene, phenanthrylene, pyrenylene, biphenylene, and terphenylene. Arylene also refers to bicyclic or tricyclic carbon rings, where one of the rings is aromatic and the others of which can be saturated, partially unsaturated, or aromatic, for example, dihydronaphthylene, indenylene, indanylene, or tetrahydronaphthylene (tetralinylene). The arylene is optionally substituted with one or more substituents Q as described herein.

The term "aralkyl" or "arylalkyl" refers to a monovalent alkyl group substituted with one or more aryl groups. In certain examples, the aralkyl has from 7 to 30 (C₇₋₃₀), from 7 to 20 (C₇₋₂₀), or from 7 to 16 (C₇₋₁₆) carbon atoms. Examples of aralkyl groups include, but are not limited to, benzyl, 1-phenylethyl, 2-phenylethyl, and 3-phenylpropyl. The aralkyl is optionally substituted with one or more substituents Q as described herein.

The term "heteroaryl" refers to a monovalent monocyclic aromatic group or monovalent polycyclic aromatic group that contain at least one aromatic ring, wherein at least one aromatic ring contains one or more heteroatoms, each of which is independently selected from O, S, N, and P, in the ring. For clarity, the terms "aryl" and "heteroaryl" as used herein are mutually exclusive, *i.e.*, "aryl" groups do not include "heteroaryl" groups, and vice versa. A heteroaryl group is bonded to the rest of a molecule through its aromatic ring. Each ring of a heteroaryl group can contain one or two O atoms, one or two S atoms, one to four N atoms, and/or one or two P atoms, provided that the total number of heteroatoms in each ring is four or less and each ring contains at least one carbon atom. In certain examples, the heteroaryl has from 5 to 20, from 5 to 15, or from 5 to 10 ring atoms. Examples of monocyclic heteroaryl groups include, but are not limited to, furanyl, imidazolyl, isothiazolyl, isoxazolyl, oxadiazolyl, oxadiazolyl, oxazolyl, pyrazinyl, pyrazolyl, pyridazinyl, pyridyl, pyrimidinyl, pyrrolyl, thiadiazolyl, thiazolyl, thienyl, tetrazolyl, triazinyl, and triazolyl. Examples of bicyclic heteroaryl groups include, but are not limited to, benzofuranyl, benzimidazolyl, benzoisoxazolyl, benzopyranyl, benzothiadiazolyl, benzothiazolyl, benzothienyl, benzotriazolyl, benzoxazolyl, furopyridyl, imidazopyridinyl, imidazothiazolyl, indolizinyl, indolyl, indazolyl, isobenzofuranyl, isobenzothienyl, isoindolyl, isoquinolinyl, isothiazolyl, naphthyridinyl, oxazolopyridinyl, phthalazinyl, pteridinyl, purinyl, pyridopyridyl, pyrrolopyridyl, quinolinyl, quinoxalinyl, quinazolinyl, thiadiazolopyrimidyl, and thienopyridyl. Examples of tricyclic heteroaryl groups include, but are not limited to, acridinyl, benzindolyl, carbazolyl, dibenzofuranyl, perimidinyl, phenanthrolinyl, phenanthridinyl, phenarsazinyl, phenazinyl, phenothiazinyl, phenoxazinyl, and xanthenyl. The heteroaryl is optionally substituted with one or more substituents Q as described herein.

The term "heteroarylene" refers to a divalent monocyclic aromatic group or divalent polycyclic aromatic group that contain at least one aromatic ring, wherein at least one aromatic ring contains one or more heteroatoms independently selected from O, S, and N in the ring. For clarity, the terms "arylene" and "heteroarylene" as used herein are mutually exclusive, i.e., "arylene" groups do not include "heteroarylene" groups, and vice versa. A heteroarylene group is bonded to the rest of a molecule through its aromatic ring. Each ring of a heteroarylene group can contain one or two O atoms, one or two S atoms, and/or one to four N atoms, provided that the total number of heteroatoms in each ring is four or less and each ring contains at least one carbon atom. In certain examples, the heteroarylene has from 5 to 20, from 5 to 15, or from 5 to 10 ring atoms. Examples of monocyclic heteroarylene groups include, but are not limited to, furanylene, imidazolylene, isothiazolylene, isoxazolylene, oxadiazolylene, oxadiazolylene, oxazolylene, pyrazinylene, pyrazolylene, pyridazinylene, pyridylene, pyrimidinylene, pyrrolylene, thiadiazolylene, thiazolylene, thienylene, tetrazolylene, triazinylene, and triazolylene. Examples of bicyclic heteroarylene groups include, but are not limited to, benzofuranylene, benzimidazolylene, benzoisoxazolylene, benzopyranylene, benzothiadiazolylene, benzothiazolylene, benzothienylene, benzotriazolylene, benzoxazolylene, furopyridylene, imidazopyridinylene, imidazothiazolylene, indolizinylene, indolylene, indazolylene, isobenzofuranylene, isobenzothienylene, isoindolylene, isoquinolinylene, isothiazolylene, naphthyridinylene, oxazolopyridinylene, phthalazinylene, pteridinylene, purinylene, pyridopyridylene, pyrrolopyridylene, quinolinylene, quinoxalinylene, quinazolinylene, thiadiazolopyrimidylene, and thienopyridylene. Examples of tricyclic heteroarylene groups include, but are not limited to, acridinylene, benzindolylene, carbazolylene, dibenzofuranylene, perimidinylene, phenanthrolinylene, phenanthridinylene, phenarsazinylene, phenazinylene, phenothiazinylene, phenoxazinylene, and xanthenylene. The heteroarylene is optionally substituted with one or more substituents Q as described herein.

The term "heterocyclyl" or "heterocyclic" refers to a monovalent monocyclic non-aromatic ring system or monovalent polycyclic ring system that contains at least one non-aromatic ring, wherein one or more of the non-aromatic ring atoms are heteroatoms, each of which is independently selected from O, S, N, and P; and the remaining ring atoms are carbon atoms. In certain examples, the heterocyclyl or heterocyclic group has from 3 to 20, from 3 to 15, from 3 to 10, from 3 to 8, from 4 to 7, or from 5 to 6 ring atoms. A heterocyclyl group is bonded to the rest of a molecule through its non-aromatic ring. In certain examples, the heterocyclyl is a monocyclic, bicyclic, tricyclic, or tetracyclic ring system, which can be spiro, fused, or bridged, and in which nitrogen or sulfur atoms can be optionally oxidized, nitrogen atoms can be optionally quaternized, and some rings can be partially or fully saturated, or aromatic. The heterocyclyl can be attached to the main structure at any heteroatom or carbon atom which results in the creation of a stable compound. Examples of heterocyclic groups include, but are not limited to, azepinyl, benzodioxanyl, benzodioxolyl, benzofuranonyl, benzopyranonyl, benzopyranyl, benzotetrahydrofuranyl, benzotetrahydrothienyl, benzothiopyranyl, benzoxazinyl, β-carbolinyl, chromanyl, chromonyl, cinnolinyl, coumarinyl, decahydroisoquinolinyl, dihydrobenzisothiazinyl, dihydrobenzisoxazinyl, dihydrofuryl, dihydroisoindolyl, dihydropyranyl, dihydropyrazolyl, dihydropyrazinyl, dihydropyridinyl, dihydropyrimidinyl, dihydropyrrolyl, dioxolanyl, 1,4-dithianyl, furanonyl, imidazolidinyl, imidazolinyl, indolinyl, isobenzotetrahydrofuranyl, isobenzotetrahydrothienyl, isochromanyl, isocoumarinyl, isoindolinyl, isothiazolidinyl, isoxazolidinyl, morpholinyl, octahydroindolyl, octahydroisoindolyl, oxazolidinonyl, oxazolidinyl, oxiranyl, piperazinyl, piperidinyl, 4-piperidonyl, pyrazolidinyl, pyrazolinyl, pyrrolidinyl, pyrrolinyl, quinuclidinyl, tetrahydrofuryl, tetrahydroisoquinolinyl, tetrahydropyranyl, tetrahydrothienyl, thiamorpholinyl, thiazolidinyl, tetrahydroquinolinyl, and 1,3,5-trithianyl. The heterocyclyl is optionally substituted with one or more substituents Q as described herein.

The term "heterocyclylene" refers to a divalent monocyclic non-aromatic ring system or divalent polycyclic ring system that contains at least one non-aromatic ring, wherein one or more of the non-aromatic ring atoms are heteroatoms independently selected from O, S, and N; and the remaining ring atoms are carbon atoms. In certain examples, the heterocyclylene group has from 3 to 20, from 3 to 15, from 3 to 10, from 3 to 8, from 4 to 7, or from 5 to 6 ring atoms. In certain examples, the heterocyclylene is a monocyclic, bicyclic, tricyclic, or tetracyclic ring system, which can be fused or bridged, and in which nitrogen or sulfur atoms can be optionally oxidized, nitrogen atoms can be optionally quaternized, and some rings can be partially or fully saturated, or aromatic. The heterocyclylene can be attached to the main structure at any heteroatom or carbon atom which results in the creation of a stable compound. Examples of such heterocyclylene groups include, but are not limited to, azepinylene, benzodioxanylene, benzodioxolylene, benzofuranonylene, benzopyranonylene, benzopyranylene, benzotetrahydrofuranylene, benzotetrahydrothienylene, benzothiopyranylene, benzoxazinylene, β-carbolinylene, chromanylene, chromonylene, cinnolinylene, coumarinylene, decahydroisoquinolinylene, dihydrobenzisothiazinylene, dihydrobenzisoxazinylene, dihydrofurylene, dihydroisoindolylene, dihydropyranylene, dihydropyrazolylene, dihydropyrazinylene, dihydropyridinylene, dihydropyrimidinylene, dihydropyrrolylene, dioxolanylene, 1,4-dithianylene, furanonylene, imidazolidinylene, imidazolinylene, indolinylene, isobenzotetrahydrofuranylene, isobenzotetrahydrothienylene, isochromanylene, isocoumarinylene, isoindolinylene, isothiazolidinylene, isoxazolidinylene, morpholinylene, octahydroindolylene, octahydroisoindolylene, oxazolidinonylene, oxazolidinylene, oxiranylene, piperazinylene, piperidinylene, 4-piperidonylene, pyrazolidinylene, pyrazolinylene, pyrrolidinylene, pyrrolinylene, quinuclidinylene, tetrahydrofurylene, tetrahydroisoquinolinylene, tetrahydropyranylene, tetrahydrothienylene, thiamorpholinylene, thiazolidinylene, tetrahydroquinolinylene, and 1,3,5-trithianylene. The heterocyclylene is optionally substituted with one or more substituents Q as described herein.

The term "halogen", "halide" or "halo" refers to fluorine, chlorine, bromine, and/or iodine.

The term "haloalkyl" refers to an alkyl group substituted with one or more, in one embodiment, one, two, or three, halo groups, where the alkyl is as defined herein. The haloalkyl is optionally substituted with one or more substituents Q as described herein.

The term "alkoxy" refers to -O-alkyl, where the alkyl is as defined herein.

The term "haloalkoxy" refers to -O-haloalkyl, where the haloalkyl is as defined herein.

The term "optionally substituted" is intended to mean that a group or substituent, such as an alkyl, alkylene, alkenyl, alkenylene, alkynyl, alkynylene, cycloalkyl, cycloalkylene, aryl, arylene, aralkyl (*e.g*., benzyl), heteroaryl, heteroarylene, heterocyclyl, and heterocyclylene group, may be substituted with one or more substituents Q, each of which is independently selected from, *e.g*., (a) oxo (=O), cyano (-CN), halo, and nitro (-NO₂); (b) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, and heterocyclyl, each of which is further optionally substituted with one or more, in one embodiment, one, two, three, four, or five, substituents Q^{a}; and (c) -C(O)R^{a}, -C(O)OR^{a}, -C(O)NR^{b}R^{c}, -C(NR^{a})NR^{b}R^{c}, -OR^{a}, -OC(O)R^{a}, -OC(O)OR^{a}, -OC(O)NR^{b}R^{c}, -OC(=NR^{a})NR^{b}R^{c}, -OS(O)R^{a}, -OS(O)₂R^{a}, -OS(O)NR^{b}R^{c}, -OS(O)₂NR^{b}R^{c}, -NR^{b}R^{c}, -NR^{a}C(O)R^{d}, -NR^{a}C(O)OR^{d}, -NR^{a}C(O)NR^{b}R^{c}, -NR^{a}C(=NR^{d})NR^{b}R^{c}, -NR^{a}S(O)R^{d}, -NR^{a}S(O)₂R^{d}, -NR^{a}S(O)NR^{b}R^{c}, -NR^{a}S(O)₂NR^{b}R^{c}, -P(O)R^{a}R^{d}, -P(O)(OR^{a})R^{d}, -P(O)(OR^{a})(OR^{d}), -SR^{a}, -S(O)R^{a}, -S(O)₂R^{a}, -S(O)NR^{b}R^{c}, and -S(O)₂NR^{b}R^{c}, wherein each R^{a}, R^{b}, R^{c}, and R^{d} is independently (i) hydrogen; (ii) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, or heterocyclyl, each of which is optionally substituted with one or more, in one embodiment, one, two, three, or four, substituents Q^{a}; or (iii) R^{b} and R^{c} together with the N atom to which they are attached form heteroaryl or heterocyclyl, each of which is optionally substituted with one or more, in one embodiment, one, two, three, or four, substituents Q^{a}. As used herein, all groups described herein that can be substituted are "optionally substituted," unless otherwise specified.

In one example, each substituent Q^{a} is independently selected from the group consisting of (a) oxo, cyano, halo, and nitro; and (b) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, and heterocyclyl; and (c) -C(O)R^{e}, -C(O)OR^{e}, -C(O)NR^{f}R^{g}, -C(NR^{e})NR^{f}R^{g}, -OR^{e}, -OC(O)R^{e}, -OC(O)OR^{e}, -OC(O)NR^{f}R^{g}, -OC(=NR^{e})NR^{f}R^{g}, -OS(O)R^{e}, -OS(O)₂R^{e}, -OS(O)NR^{f}R^{g}, -OS(O)₂NR^{f}R^{g}, -NR^{f}R^{g}, -NR^{e}C(O)R^{h}, -NR^{e}C(O)OR^{h}, -NR^{e}C(O)NR^{f}R^{g}, -NR^{e}C(=NR^{h})NR^{f}R^{g}, -NR^{e}S(O)R^{h}, -NR^{e}S(O)₂R^{h}, -NR^{e}S(O)NR^{f}R^{g}, -NR^{e}S(O)₂NR^{f}R^{g}, -P(O)R^{e}R^{b}, -P(O)(OR^{e})R^{h}, -P(O)(OR^{e})(OR^{h}), -SR^{e}, -S(O)R^{e}, -S(O)₂R^{e}, -S(O)NR^{f}R^{g}, and -S(O)₂NR^{f}R^{g}; wherein each R^{e}, R^{f}, R^{g}, and R^{h} is independently (i) hydrogen, C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, or heterocyclyl; or (ii) R^{f} and R^{g} together with the N atom to which they are attached form heteroaryl or heterocyclyl.

In certain examples, "optically active" and "enantiomerically active" refer to a collection of molecules, which has an enantiomeric excess of no less than about 50%, no less than about 70%, no less than about 80%, no less than about 90%, no less than about 91%, no less than about 92%, no less than about 93%, no less than about 94%, no less than about 95%, no less than about 96%, no less than about 97%, no less than about 98%, no less than about 99%, no less than about 99.5%, or no less than about 99.8%. In certain examples, the compound comprises about 95% or more of the desired enantiomer and about 5% or less of the less preferred enantiomer based on the total weight of the two enantiomers in question.

In describing an optically active compound, the prefixes R and S are used to denote the absolute configuration of the optically active compound about its chiral center(s). The (+) and (-) are used to denote the optical rotation of an optically active compound, that is, the direction in which a plane of polarized light is rotated by the optically active compound. The (-) prefix indicates that an optically active compound is levorotatory, that is, the compound rotates the plane of polarized light to the left or counterclockwise. The (+) prefix indicates that an optically active compound is dextrorotatory, that is, the compound rotates the plane of polarized light to the right or clockwise. However, the sign of optical rotation, (+) and (-), is not related to the absolute configuration of a compound, R and S.

The term "isotopic variant" refers to a compound that contains an unnatural proportion of an isotope at one or more of the atoms that constitute such a compound. In certain examples, an "isotopic variant" of a compound contains unnatural proportions of one or more isotopes, including, but not limited to, hydrogen (¹H), deuterium (²H), tritium (³H), carbon-11 (¹¹C), carbon-12 (¹²C), carbon-13 (¹³C), carbon-14 (¹⁴C), nitrogen-13 (¹³N), nitrogen-14 (¹⁴N), nitrogen-15 (¹⁵N), oxygen-14 (¹⁴O), oxygen-15 (¹⁵O), oxygen-16 (¹⁶O), oxygen-17 (¹⁷O), oxygen-18 (¹⁸O), fluorine-17 (¹⁷F), fluorine-18 (¹⁸F), phosphorus-31 (³¹P), phosphorus-32 (³²P), phosphorus-33 (³³P), sulfur-32 (³²S), sulfur-33 (³³S), sulfur-34 (³⁴S), sulfur-35 (³⁵S), sulfur-36 (³⁶S), chlorine-35 (³⁵Cl), chlorine-36 (³⁶Cl), chlorine-37 (³⁷Cl), bromine-79 (⁷⁹Br), bromine-81 (⁸¹Br), iodine-123 (¹²³I), iodine-125 (¹²⁵I), iodine-127 (¹²⁷I), iodine-129 (¹²⁹I), and iodine-131 (¹³¹I). In certain examples, an "isotopic variant" of a compound is in a stable form, that is, non-radioactive. In certain examples, an "isotopic variant" of a compound contains unnatural proportions of one or more isotopes, including, but not limited to, hydrogen (¹H), deuterium (²H), carbon-12 (¹²C), carbon-13 (¹³C), nitrogen-14 (¹⁴N), nitrogen-15 (¹⁵N), oxygen-16 (¹⁶O), oxygen-17 (¹⁷O), oxygen-18 (¹⁸O), fluorine-17 (¹⁷F), phosphorus-31 (³¹P), sulfur-32 (³²S), sulfur-33 (³³S), sulfur-34 (³⁴S), sulfur-36 (³⁶S), chlorine-35 (³⁵Cl), chlorine-37 (³⁷Cl), bromine-79 (⁷⁹Br), bromine-81 (⁸¹Br), and iodine-127 (¹²⁷I). In certain examples, an "isotopic variant" of a compound is in an unstable form, that is, radioactive. In certain examples, an "isotopic variant" of a compound contains unnatural proportions of one or more isotopes, including, but not limited to, tritium (³H), carbon-11 (¹¹C), carbon-14 (¹⁴C), nitrogen-13 (¹³N), oxygen-14 (¹⁴O), oxygen-15 (¹⁵O), fluorine-18 (¹⁸F), phosphorus-32 (³²P), phosphorus-33 (³³P), sulfur-35 (³⁵S), chlorine-36 (³⁶Cl), iodine-123 (¹²³I), iodine-125 (¹²⁵I), iodine-129 (¹²⁹I), and iodine-131 (¹³¹I). It will be understood that, in a compound as provided herein, any hydrogen can be ²H, for example, or any carbon can be ¹³C, for example, or any nitrogen can be ¹⁵N, for example, or any oxygen can be ¹⁸O, for example, where feasible according to the judgment of one of skill. In certain examples, an "isotopic variant" of a compound contains unnatural proportions of deuterium (D).

The term "solvate" refers to a complex or aggregate formed by one or more molecules of a solute, *e.g.*, a compound provided herein, and one or more molecules of a solvent, which present in a stoichiometric or non-stoichiometric amount. Suitable solvents include, but are not limited to, water, methanol, ethanol, *n*-propanol, isopropanol, and acetic acid. In certain embodiments, the solvent is pharmaceutically acceptable. In one example, the complex or aggregate is in a crystalline form. In another example, the complex or aggregate is in a noncrystalline form. Where the solvent is water, the solvate is a hydrate. Examples of hydrates include, but are not limited to, a hemihydrate, monohydrate, dihydrate, trihydrate, tetrahydrate, and pentahydrate.

The phrase "an enantiomer, a mixture of enantiomers, a mixture of two or more diastereomers, or an isotopic variant thereof; or a pharmaceutically acceptable salt, solvate, hydrate, or prodrug thereof' has the same meaning as the phrase "(i) an enantiomer, a mixture of enantiomers, a mixture of two or more diastereomers, or an isotopic variant of the compound referenced therein; (ii) a pharmaceutically acceptable salt, solvate, hydrate, or prodrug of the compound referenced therein; or (iii) a pharmaceutically acceptable salt, solvate, hydrate, or prodrug of an enantiomer, a mixture of enantiomers, a mixture of two or more diastereomers, or an isotopic variant of the compound referenced therein. "

### 5.3.2. Stem cell mobilizing compounds

The stem cell mobilizing agent of the claimed method is StemRegenin-1 (SR-1) (4-(2-(2-(benzo[b]thiophen-3-yl)-9-isopropyl-9H-purin-6-ylamino)ethyl)phenol),or the compound CH223191 (1-Methyl-N-[2-methyl-4-[2-(2-methylphenyl)diazenyl]phenyl-1H-pyrazole-5-carboxamide]. The stem cell mobilizing compound is an aryl hydrocarbon receptor inhibitor, *e.g.*, an aryl hydrocarbon receptor antagonist.

In another example, the stem cell mobilizing compound is a 5,6-fused heteroaryl compound, including, but not limited to, those described in U.S. Pat. App. Pub. Nos. 2010/0183564, 2014/0023626, and 2014/0114070,

In yet another example, the stem cell mobilizing compound is a compound of Formula I: 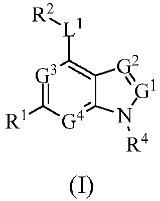 or an enantiomer, a mixture of enantiomers, a mixture of two or more diastereomers, or an isotopic variant thereof; or a pharmaceutically acceptable salt, solvate, hydrate, or prodrug thereof; wherein:
G¹ is N and CR³;
G², G³, and G⁴ are each independently CH and N; with the proviso that at least one of G³ and G⁴ is N, and at least one of G¹ and G² is not N;
L¹ is -NR^{1a}-, -NR^{1a}(CH₂)₁₋₃-, -NR^{1a}CH(C(O)OCH₃)CH₂-, -NR^{1a}(CH₂)₂NR^{1c}-, -NR^{1a}(CH₂)₂S-, -NR^{1a}CH₂CH(CH₃)CH₂-, -NR^{1a}CH₂CH(OH)-, or -NR^{1a}CH(CH₃)CH₂-;
R¹ is (i) hydrogen; or (ii) phenyl, furanyl, pyrrolyl, imidazolyl, pyrazolyl, thienyl, thiazolyl, pyridinyl, pyrimidinyl, pyrrolidinyl, pyrazinyl, pyridazinyl, benzoimidazolyl, isoquinolinyl, imidazopyridinyl, or benzothienyl, each of which is optionally substituted by one, two, or three substituents, where each substituent is independently cyano, halo, C₁₋₄ alkyl, C₁₋₄ alkoxy, C₁₋₄ haloalkyl, C₁₋₄ haloalkoxy, hydroxyl, amino, -C(O)R^{1a}, -C(O)OR^{1a}, -C(O)NR^{1a}R^{1b}, -SR^{1a}, -S(O)R^{1a}, or -S(O)₂R^{1a};
R² is (i) -NR^{1a}C(O)R^{1c}, -NR^{1c}C(O)NR^{1a}R^{1b}, or -S(O)₂NR^{1a}R^{1b}; or (ii) phenyl, pyrrolopyridin-3-yl, indolyl, thienyl, pyridinyl, 1,2,4-triazolyl, 2-oxoimidazolidinyl, pyrazolyl, 2-oxo-2,3-dihydro-1*H*-benzoimidazolyl, or indazolyl, each of which is optionally substituted with one, two, or three substituents, where each substituent is independently hydroxyl, halo, methyl, methoxy, amino, -O(CH₂)₁₋₃NR^{1a}R^{1b}, -OS(O)₂NR^{1a}R^{1b}, -NR^{1a}S(O)₂R^{1b}, or -S(O)₂NR^{1a}R^{1b};
R³ is hydrogen, C₁₋₄ alkyl, or biphenyl; with the proviso that at least one of R¹ and R³ is not hydrogen;
R⁴ is C₁₋₁₀ alkyl, prop-1-en-2-yl, cyclohexyl, cyclopropyl, 2-(2-oxopyrrolidin-1-yl)ethyl, oxetan-3-yl, benzhydryl, tetrahydro-2*H*-pyran-3-yl, tetrahydro-2*H*-pyran-4-yl, phenyl, tetrahydrofuran-3-yl, benzyl, (4-pentylphenyl)(phenyl)methyl, or 1-(1-(2-oxo-6,9,12-trioxa-3-azatetradecan-14-yl)-1*H*-1,2,3-triazol-4-yl)ethyl, each of which is optionally substituted with one, two, or three substituents, where each substituent is independently hydroxyl, C₁₋₄ alkyl, or C₁₋₄ haloalkyl; and
each R^{1a}, R^{1b}, and R^{1c} is independently hydrogen or C₁₋₄ alkyl; or R^{1a} and R^{1b} together with the N atom to which they are attached form heterocyclyl.

In one example, in Formula I, G¹ is CR³, in one embodiment, CH; G², G³, and G⁴ are each N; and R¹, R², R³, R⁴, and L¹ are each as defined herein.

In another example, in Formula I, G¹, G³, and G⁴ are each N; G² is CH; and R¹, R², R⁴, and L¹ are each as defined herein.

In yet another example, in Formula I, G¹ is CR³, in one embodiment, CH; G² and G³ are each N; G⁴ is CH; and R¹, R², R³, R⁴, and L¹ are each as defined herein.

In yet another example, in Formula I, G¹ is CR³, in one embodiment, CH; G² and G⁴ are each N; G³ is CH; and R¹, R², R³, R⁴, and L¹ are each as defined herein.

In yet another example, in Formula I, G¹ is CR³, in one embodiment, CH; G² is CH; G³ and G⁴ are each N; and R¹, R², R³, R⁴, and L¹ are each as defined herein.

In still example, in Formula I,
G¹ is CH;
G², G³, and G⁴ are each N;
R¹ is benzothienyl, optionally substituted by one, two, or three substituents, each of which is independently cyano, halo, C₁₋₄ alkyl, C₁₋₄ alkoxy, C₁₋₄ haloalkyl, C₁₋₄ haloalkoxy, hydroxyl, amino, -C(O)R^{1a}, -C(O)OR^{1a}, -C(O)NR^{1a}R^{1b}, -SR^{1a}, -S(O)R^{1a}, or -S(O)₂R^{1a};
R² is phenyl, optionally substituted with one, two, or three substituents, each of which is independently hydroxyl, halo, methyl, methoxy, amino, -O(CH₂)₁₋₃NR^{1a}R^{1b}, -OS(O)₂NR^{1a}R^{1b}, -NR^{1a}S(O)₂R^{1b}, or -S(O)₂NR^{1a}R^{1b};
R⁴ is C₁₋₁₀ alkyl, optionally substituted with one, two, or three substituents, each of which is independently hydroxyl, C₁₋₄ alkyl, or C₁₋₄ haloalkyl;
L¹ is -NR^{1a}(CH₂)₂-; and
R^{1a} and R^{1b} are each as defined herein.

In yet another example, the stem cell mobilizing compound is a compound of Formula II: 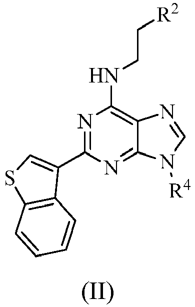 or an enantiomer, a mixture of enantiomers, a mixture of two or more diastereomers, or an isotopic variant thereof; or a pharmaceutically acceptable salt, solvate, hydrate, or prodrug thereof; wherein R² and R⁴ are each as defined herein.

In yet another example, the stem cell mobilizing compound is a compound of Formula III: 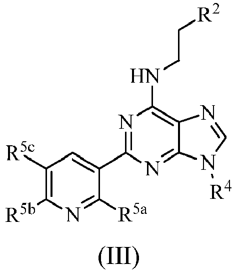 or an enantiomer, a mixture of enantiomers, a mixture of two or more diastereomers, or an isotopic variant thereof; or a pharmaceutically acceptable salt, solvate, hydrate, or prodrug thereof; wherein R² and R⁴ are each as defined herein; and R^{5a}, R^{5b}, and R^{5c} are each independently hydrogen, cyano, methyl, halo, trifluoromethyl, or -SO₂CH₃.

In yet another embodiment, the stem cell mobilizing compound is 4-(2-(2-(benzo[*b*]thien-3-yl)-9-isopropyl-9*H*-purin-6-ylamino)ethyl)phenol. In certain embodiments, the stem cell mobilizing compound is StemRegenin-1 (SR-1), having the structure of:

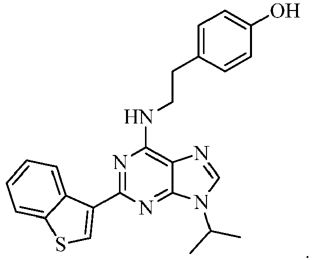

In yet another embodiment, the stem cell mobilizing compound is 1-methyl-*N*-(2-methyl-4-(2-(2-methylphenyl)diazenyl)phenyl)-1*H*-pyrazole-5-carboxamide. In certain embodiments, the stem cell mobilizing compound is CH223191, which has the structure of:

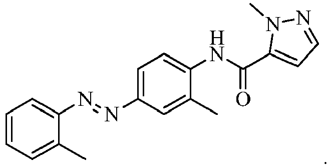

In yet another example, the stem cell mobilizing compound is a pyrimido(4,5-*b*)indole.

In yet another example, the stem cell mobilizing compound is a compound of Formula IV: 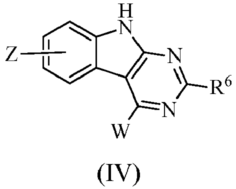 or an enantiomer, a mixture of enantiomers, a mixture of two or more diastereomers, or an isotopic variant thereof; or a pharmaceutically acceptable salt, solvate, hydrate, or prodrug thereof; wherein:
Z is cyano, C₁₋₆ alkyl, C₂₋₆ alkenyl, C₂₋₆ alkynyl, C₃₋₁₀ cycloalkyl, C₆₋₁₄ aryl, C₇₋₁₅ aralkyl, benzyl, heteroaryl, heterocyclyl, -L- C₆₋₁₄ aryl, -L-heteroaryl, -L-heterocyclyl, -C(O)R^{1a}, -C(O)OR^{1a}, -C(O)NHR^{1a}, -C(O)N(R^{1a})R^{1b}, -P(O)(OR^{1a})(OR^{1c}), -SR^{1a}, -S(O)R^{1a}, -S(O)₂R^{1a}, -S(O)₂NH₂, -S(O)₂NHR^{1a}, or -S(O)₂N(R^{1a})R^{1b};
W is hydrogen, halo, cyano, C₆₋₁₄ aryl, benzyl, heteroaryl, heterocyclyl, -L-C₆₋₁₄ aryl, -L-heteroaryl, -L-heterocyclyl, -L-OH, -L-OR^{1a}, -L-NH₂, -L-NHR^{1a}, -L-N(R^{1a})R^{1b}, -L-SR^{1a}, -L-S(O)R^{1a}, -L-S(O)₂R^{1a}, -L-P(O)(OR^{1a})(OR^{1c}), -L-(N(R^{1c})-L)ₙ-N(R^{1a})R^{1b}, -L-(N(R^{1c})-L)ₙ-C₆₋₁₄ aryl, -L-(N(R^{1c})-L)ₙ-heteroaryl, -L-(N(R^{1c})-L)ₙ-heterocyclyl, -O-L-N(R^{1a})R^{1b}, -O-L-C₆₋₁₄ aryl, -O-L-heteroaryl, -O-L-heterocyclyl, -O-L-(N(R^{1c})-L)ₙ-N(R^{1a})R^{1b}, -O-L-(N(R^{1c})-L)ₙ₋C₆₋₁₄ aryl, -O-L-(N(R^{1c})-L)ₙ-heteroaryl, -O-L-(N(R^{1c})-L)ₙ-heterocyclyl, -S-L-N(R^{1a})R^{1b}, -S-L-C₆₋₁₄ aryl, -S-L-heteroaryl, -S-L-heterocyclyl, -S-L-(N(R^{1c})-L)ₙ-N(R^{1a})R^{1b}, -S-L-(N(R^{1c})-L)ₙ-C₆₋₁₄ aryl, -S-L-(N(R^{1c})-L)ₙ-heteroaryl, -S-L-(N(R^{1c})-L)ₙ-heterocyclyl, -(N(R^{1c})-L)ₙ-N(R^{1a})R^{1b}, -(N(R^{1c})-L)ₙ₋C₆₋₁₄ aryl, -(N(R^{1c})-L)ₙ-heteroaryl, -(N(R^{1c})-L)ₙ-heterocyclyl, -C(O)R^{1a}, -C(O)OR^{1a}, -C(O)NH₂, -C(O)NHR^{1a}, -C(O)N(R^{1a})R^{1b}, -NHR^{1a}, -N(R^{1a})R^{1b}, -NHC(O)R^{1a}, -NR^{1a}C(O)R^{1c}, -NHC(O)OR^{1a}, -NR^{1a}C(O)OR^{1c}, -NHC(O)NH₂, -NHC(O)NHR^{1a}, -NHC(O)N(R^{1a})R^{1b}, -NR^{1a}C(O)NH₂, -NR^{1c}C(O)NHR^{1a}, -NR^{1c}C(O)N(R^{1a})R^{1b}, -NHS(O)₂R^{1a}, -NR^{1c}S(O)₂R^{1a}, -OR^{1a}, -OC(O)R^{1a}, -OC(O)OR^{1a}, -OC(O)NH₂, -OC(O)NHR^{1a}, -OC(O)N(R^{1a})R^{1b}, -OS(O)₂R^{1a} , -P(O)(OR^{1a})(OR^{1c}), -SR^{1a}, -S(O)R^{1a}, -S(O)₂R^{1a}, -S(O)₂NH₂, -S(O)₂NHR^{1a}, -S(O)₂N(R^{1a})R^{1b}, or -S(O)₂OR^{1a};
each L is independently C₁₋₆ alkylene, C₂₋₆ alkenylene, C₂₋₆ alkynylene, C₃₋₇ cycloalkylene, C₆₋₁₄ arylene, heteroarylene, heterocyclylene, C₁₋₆ alkylene-C₃₋₇ cycloalkylene, or C₁₋₆ alkylene-heterocyclylene;
R⁶ is hydrogen, C₁₋₆ alkyl, C₆₋₁₄ aryl, benzyl, heteroaryl, -C(O)R^{1a}, -SR^{1a},-S(O)R^{1a}, -S(O)₂R^{1a}, -L-C₆₋₁₄ aryl, -L-heteroaryl, or -L-heterocyclyl;
each n is independently an integer of 1, 2, 3, 4, or 5; and
each R^{1a}, R^{1b}, and R^{1c} is independently (i) hydrogen; (ii) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, or heterocyclyl; or (iii) R^{1a} and R^{1b} together with the N atom to which they are attached form heterocyclyl;
wherein each alkyl, alkylene, alkenyl, alkenylene, alkynyl, alkynylene, cycloalkyl, cycloalkylene, aryl, benzyl, arylene, heteroaryl, heteroarylene, heterocyclyl, and heterocyclylene is optionally substituted with one or more, in one embodiment, one, two, three, or four, substituents Q, wherein each substituent Q is independently selected from (a) oxo, cyano, halo, and nitro; (b) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, and heterocyclyl, each of which is further optionally substituted with one or more, in one example, one, two, three, or four, substituents Q^{a}; and (c) -C(O)R^{a}, -C(O)OR^{a}, -C(O)NR^{b}R^{c}, -C(NR^{a})NR^{b}R^{c}, -OR^{a}, -OC(O)R^{a}, -OC(O)OR^{a}, -OC(O)NR^{b}R^{c}, -OC(=NR^{a})NR^{b}R^{c}, -OS(O)R^{a}, -OS(O)₂R^{a}, -OS(O)NR^{b}R^{c}, -OS(O)₂NR^{b}R^{c}, -NR^{b}R^{c}, -NR^{a}C(O)R^{d}, -NR^{a}C(O)OR^{d}, -NR^{a}C(O)NR^{b}R^{c}, -NR^{a}C(=NR^{d})NR^{b}R^{c}, -NR^{a}S(O)R^{d}, -NR^{a}S(O)₂R^{d}, -NR^{a}S(O)NR^{b}R^{c}, -NR^{a}S(O)₂NR^{b}R^{c}, -SR^{a}, -S(O)R^{a}, -S(O)₂R^{a}, -S(O)NR^{b}R^{c}, and -S(O)₂NR^{b}R^{c}, wherein each R^{a}, R^{b}, R^{c}, and R^{d} is independently (i) hydrogen; (ii) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, or heterocyclyl, each of which is further optionally substituted with one or more, in one example, one, two, three, or four, substituents Q^{a}; or (iii) R^{b} and R^{c} together with the N atom to which they are attached form heterocyclyl, which is further optionally substituted with one or more, in one example, one, two, three, or four, substituents Q^{a};
wherein each Q^{a} is independently selected from the group consisting of (a) oxo, cyano, halo, and nitro; (b) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, and heterocyclyl; and (c) -C(O)R^{e}, -C(O)OR^{e}, -C(O)NR^{f}R^{g}, -C(NR^{e})NR^{f}R^{g}, -OR^{e}, -OC(O)R^{e}, -OC(O)OR^{e}, -OC(O)NR^{f}R^{g}, -OC(=NR^{e})NR^{f}R^{g}, -OS(O)R^{e}, -OS(O)₂R^{e}, -OS(O)NR^{f}R^{g}, -OS(O)₂NR^{f}R^{g}, -NR^{f}R^{g}, -NR^{e}C(O)R^{h}, -NR^{e}C(O)OR^{h}, -NR^{e}C(O)NR^{f}R^{g}, -NR^{e}C(=NR^{h})NR^{f}R^{g}, -NR^{e}S(O)R^{h}, -NR^{e}S(O)₂R^{h}, -NR^{e}S(O)NR^{f}R^{g}, -NR^{e}S(O)₂NR^{f}R^{g}, -SR^{e}, -S(O)R^{e}, -S(O)₂R^{e}, -S(O)NR^{f}R^{g}, and -S(O)₂NR^{f}R^{g}; wherein each R^{e}, R^{f}, R^{g}, and R^{h} is independently (i) hydrogen; (ii) C₁₋₆ alkyl, C₂₋₆ alkenyl, C₂₋₆ alkynyl, C₃₋₁₀ cycloalkyl, C₆₋₁₄ aryl, C₇₋₁₅ aralkyl, heteroaryl, or heterocyclyl; or (iii) R^{f} and R^{g} together with the N atom to which they are attached form heterocyclyl.

In yet another example, the stem cell mobilizing compound is a compound of Formula V: 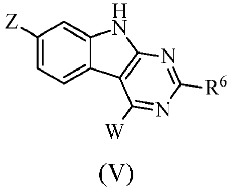 or an enantiomer, a mixture of enantiomers, a mixture of two or more diastereomers, or an isotopic variant thereof; or a pharmaceutically acceptable salt, solvate, hydrate, or prodrug thereof; wherein R⁶, W, and Z are each as defined herein.

In one example, in Formula IV or V,
Z is cyano, heteroaryl, or -C(O)OR^{1a};
W is heterocyclyl, -L-heterocyclyl, -O-L-heterocyclyl, -(N(R^{1c})-L)ₙ-N(R^{1a})R^{1b}, -(N(R^{1c})-L)ₙ-heterocyclyl, -NHR^{1a}, or -N(R^{1a})R^{1b};
each L is independently C₁₋₆ alkylene or C₃₋₇ cycloalkylene;
R⁶ is hydrogen, C₁₋₆ alkyl, benzyl, -C(O)R^{1a}, -L-C₆₋₁₄ aryl, or -L-heteroaryl;
each n is independently an integer of 1; and
R^{1a}, R^{1b}, and R^{1c} are each as defined herein;
wherein each alkyl, alkylene, cycloalkylene, aryl, benzyl, heteroaryl, and heterocyclyl is optionally substituted with one or more substituents Q as defined herein.

In another example, in Formula IV or V,
Z is cyano, 5-membered heteroaryl, or -C(O)O-C₁₋₆ alkyl;
W is heterocyclyl, -L-heterocyclyl, -O-L-heterocyclyl, -(N(R^{1c})-L)ₙ-N(R^{1a})R^{1b}, -(N(R^{1c})-L)ₙ-heterocyclyl, -NHR^{1a}, or -N(R^{1a})R^{1b};
each L is independently C₁₋₆ alkylene or C₃₋₇ cycloalkylene;
R⁶ is hydrogen, methyl, benzyl, -L-C₆₋₁₄ aryl, or -L-heteroaryl;
each n is independently an integer of 1; and
R^{1a}, R^{1b}, and R^{1c} are each as defined herein;
wherein each alkylene, cycloalkylene, aryl, benzyl, heteroaryl, and heterocyclyl is optionally substituted with one or more substituents Q as defined herein.

In one example, in Formula IV or V, W is -L-N(R^{1a})R^{1b}, -L-(N(R^{1c})-L)ₙ-N(R^{1a})R^{1b}, -O-L-N(R^{1a})R^{1b}, -O-L-(N(R^{1c})-L)ₙ-N(R^{1a})R^{1b}, -S-L-N(R^{1a})R^{1b}, -S-L-(N(R^{1c})-L)ₙ-N(R^{1a})R^{1b}, or -(N(R^{1c})-L)ₙ-N(R^{1a})R^{1b}; and R⁶, R^{1a}, R^{1b}, R^{1c}, L, and Z are each as defined herein.

In yet another example, the stem cell mobilizing compound is a compound of Formula VI: 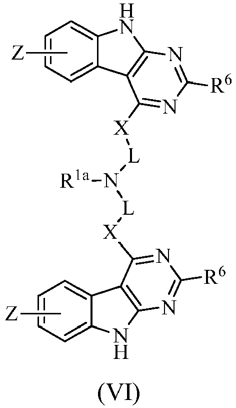 or an enantiomer, a mixture of enantiomers, a mixture of two or more diastereomers, or an isotopic variant thereof; or a pharmaceutically acceptable salt, solvate, hydrate, or prodrug thereof; wherein X is a bond, O, S, or NR^{1c}; and R^{1a}, R^{1c}, R⁶, L, and Z are each as defined herein.

In still another example, the stem cell mobilizing compound is a compound of Formula VII: 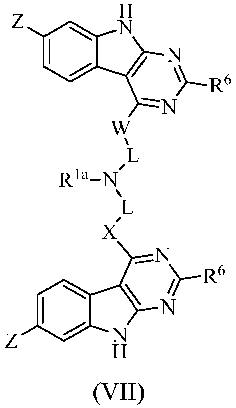 or an enantiomer, a mixture of enantiomers, a mixture of two or more diastereomers, or an isotopic variant thereof; or a pharmaceutically acceptable salt, solvate, hydrate, or prodrug thereof; wherein R^{1a}, R⁶, L, X, and Z are each as defined herein.

In yet another example, the stem cell mobilizing compound is a compound having the structure of:

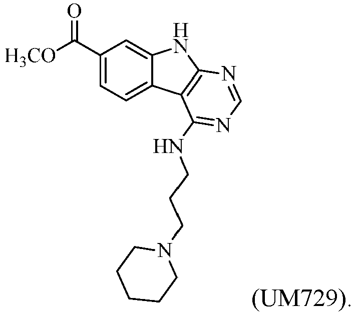

In yet another example, the stem cell mobilizing compound is a compound having the structure of:

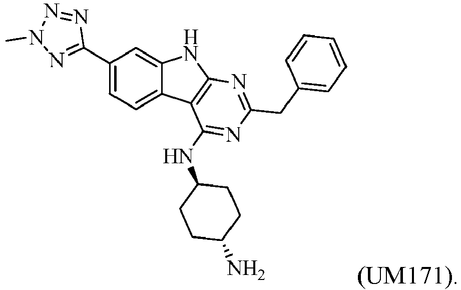

In yet another example, the stem cell mobilizing compound is resveratrol, tetraethylenepentamine (TEPA), alpha naphthoflavone, 3'-methoxy-4'-nitroflavone, 3,4-dimethoxyflavone, 4',5,7-trihydroxyflavone (apigenin), 6-methyl-1,3,8-trichlorodibenzofuran, epigallocatechin, or epigallocatechingallate.

In yet another example, the stem cell mobilizing compound is resveratrol. In certain examples, the stem cell mobilizing compound is (Z)-resveratrol. In certain examples, the stem cell mobilizing compound is (E)-resveratrol.

In still another example, the stem cell mobilizing compound is tetraethylenepentamine (TEPA).

All of the compounds described herein are either commercially available or can be prepared according to the methods described in the patents or patent publications disclosed herein. Further, optically pure compounds can be asymmetrically synthesized or resolved using known resolving agents or chiral columns as well as other standard synthetic organic chemistry techniques. Additional information on stem cell mobilizing compounds, their preparation, and use can be found, for example, in U.S. Pat. App. Pub. Nos. 2010/0183564, 2014/0023626, and 2014/0114070; and Kim et al., Mol. Pharmacol., 2006, 69, 1871-1878

The groups or variables, G¹, G², G³, G⁴, R¹ R², R³, R⁴, R^{5a}, R^{5b}, R^{5c}, R⁶, X, L, L¹, X, W, Z, and n, in Formulae provided herein, *e.g*., Formulae I to VII, are further defined in the embodiments described herein. All combinations of the examples provided herein for such groups and/or variables are within the scope of this disclosure.

In certain examples, G¹ is N. In certain examples, G¹ is CR³, wherein R³ is as defined herein. In certain examples, G¹ is CH.

In certain examples, G² is N. In certain examples, G² is CH.

In certain examples, G³ is N. In certain examples, G³ is CH.

In certain examples, G⁴ is N. In certain examples, G⁴ is CH.

In certain examples, R¹ is hydrogen. In certain examples, R¹ is phenyl optionally substituted as described herein. In certain examples, R¹ is furanyl optionally substituted as described herein. In certain examples, R¹ is pyrrolyl optionally substituted as described herein. In certain examples, R¹ is imidazolyl optionally substituted as described herein. In certain examples, R¹ is pyrazolyl optionally substituted as described herein. In certain examples, R¹ is thienyl optionally substituted as described herein. In certain examples, R¹ is thiazolyl optionally substituted as described herein. In certain examples, R¹ is pyridinyl optionally substituted as described herein. In certain examples, R¹ is pyrimidinyl optionally substituted as described herein. In certain examples, R¹ is pyrrolidinyl optionally substituted as described herein. In certain examples, R¹ is pyrazinyl optionally substituted as described herein. In certain examples, R¹ is pyridazinyl optionally substituted as described herein. In certain examples, R¹ is benzoimidazolyl optionally substituted as described herein. In certain examples, R¹ is isoquinolinyl optionally substituted as described herein. In certain examples, R¹ is imidazopyridinyl optionally substituted as described herein. In certain examples, R¹ is benzothienyl optionally substituted as described herein.

In certain examples, R² is -NR^{1a}C(O)R^{1c}, wherein R^{1a} and R^{1c} are each as defined herein. In certain examples, R² is -NR^{1c}C(O)NR^{1a}R^{1b}, wherein R^{1a}, R^{1b}, and R^{1c} are each as defined herein. In certain examples, R² is -S(O)₂NR^{1a}R^{1b}, wherein R^{1a} and R^{1b} are each as defined herein. In certain examples, R² is phenyl optionally substituted as described herein. In certain examples, R² is pyrrolopyridin-3-yl optionally substituted as described herein. In certain examples, R² is indolyl optionally substituted as described herein. In certain examples, R² is thienyl optionally substituted as described herein. In certain examples, R² is pyridinyl optionally substituted as described herein. In certain examples, R² is 1,2,4-triazolyl optionally substituted as described herein. In certain examples, R² is 2-oxoimidazolidinyl optionally substituted as described herein. In certain examples, R² is pyrazolyl optionally substituted as described herein. In certain examples, R² is 2-oxo-2,3-dihydro-1*H*-benzoimidazolyl optionally substituted as described herein. In certain examples, R² is indazolyl optionally substituted as described herein.

In certain examples, R³ is hydrogen. In certain examples, R³ is C₁₋₄ alkyl, optionally substituted with one or more substituents Q as described herein. In certain examples, R³ is biphenyl, optionally substituted with one or more substituents Q as described herein.

In certain examples, R⁴ is C₁₋₁₀ alkyl optionally substituted as described herein. In certain examples, R⁴ is prop-1-en-2-yl optionally substituted as described herein. In certain examples, R⁴ is cyclohexyl optionally substituted as described herein. In certain examples, R⁴ is cyclopropyl optionally substituted as described herein. In certain examples, R⁴ is 2-(2-oxopyrrolidin-1-yl)ethyl optionally substituted as described herein. In certain examples, R⁴ is oxetan-3-yl optionally substituted as described herein. In certain examples, R⁴ is benzhydryl optionally substituted as described herein. In certain examples, R⁴ is tetrahydro-2*H*-pyran-3-yl optionally substituted as described herein. In certain examples, R⁴ is tetrahydro-2*H*-pyran-4-yl optionally substituted as described herein. In certain examples, R⁴ is phenyl optionally substituted as described herein. In certain examples, R⁴ is tetrahydrofuran-3-yl optionally substituted as described herein. In certain examples, R⁴ is benzyl optionally substituted as described herein. In certain examples, R⁴ is (4-pentylphenyl)(phenyl)methyl optionally substituted as described herein. In certain examples, R⁴ is 1-(1-(2-oxo-6,9,12-trioxa-3-azatetradecan-14-yl)-1*H*-1,2,3-triazol-4-yl)ethyl optionally substituted as described herein.

In certain examples, L¹ is -NR^{1a}-, wherein R^{1a} is as defined herein. In certain examples, L¹ is -NR^{1a}(CH₂)₁₋₃-, wherein R is as defined herein. In certain examples, L¹ is -NR^{1a}CH(C(O)OCH₃)CH₂-, wherein R^{1a} is as defined herein. In certain examples, L¹ is -NR^{1a}(CH₂)₂NR^{1c}-, wherein R^{1a} and R^{1c} are each as defined herein. In certain examples, L¹ is -NR^{1a}(CH₂)₂S-, wherein R^{1a} is as defined herein. In certain examples, L¹ is -NR^{1a}CH₂CH(CH₃)CH₂-, wherein R^{1a} is as defined herein. In certain examples, L¹ is -NR^{1a}CH₂CH(OH)-, wherein R^{1a} is as defined herein. In certain examples, L¹ is -NR^{1a}CH(CH₃)CH₂-, wherein R^{1a} is as defined herein.

In certain examples, R^{5a} is hydrogen. In certain examples, R^{5a} is cyano. In certain examples, R^{5a} is methyl. In certain embodiments, R^{5a} is halo. In certain examples, R^{5a} is fluoro, chloro, or bromo. In certain examples, R^{5a} is trifluoromethyl. In certain examples, R^{5a} is -SO₂CH₃.

In certain examples, R^{5b} is hydrogen. In certain examples, R^{5b} is cyano. In certain examples, R^{5b} is methyl. In certain examples, R^{5b} is halo. In certain examples, R^{5b} is fluoro, chloro, or bromo. In certain embodiments, R^{5b} is trifluoromethyl. In certain examples, R^{5b} is -SO₂CH₃.

In certain examples, R^{5c} is hydrogen. In certain examples, R^{5c} is cyano. In certain embodiments, R^{5c} is methyl. In certain examples, R^{5c} is halo. In certain examples, R^{5c} is fluoro, chloro, or bromo. In certain examples, R^{5c} is trifluoromethyl. In certain examples, R^{5c} is -SO₂CH₃.

In certain examples, L is C₁₋₆ alkylene, optionally substituted with one or more substituents Q as described herein. In certain examples, L is ethylene, propylene, or butylenes, each optionally substituted with one or more substituents Q as described herein. In certain examples, L is C₂₋₆ alkenylene, optionally substituted with one or more substituents Q as described herein. In certain examples, L is C₂₋₆ alkynylene, optionally substituted with one or more substituents Q as described herein. In certain examples, L is C₃₋₇ cycloalkylene, optionally substituted with one or more substituents Q as described herein. In certain examples, L is cyclohexylene, optionally substituted with one or more substituents Q as described herein. In certain examples, L is C₆₋₁₄ arylene, optionally substituted with one or more substituents Q as described herein. In certain examples, L is heteroarylene, optionally substituted with one or more substituents Q as described herein. In certain examples, L is heterocyclylene, optionally substituted with one or more substituents Q as described herein. In certain examples, L is C₁₋₆ alkylene-C₃₋₇ cycloalkylene, optionally substituted with one or more substituents Q as described herein. In certain examples, L is C₁₋₆ alkylene-heterocyclylene, optionally substituted with one or more substituents Q as described herein.

In certain examples, R⁶ is hydrogen. In certain examples, R⁶ is C₁₋₆ alkyl, optionally substituted with one or more substituents Q as described herein. In certain examples, R⁶ is methyl, optionally substituted with one or more substituents Q as described herein. In certain examples, R⁶ is C₆₋₁₄ aryl, optionally substituted with one or more substituents Q as described herein. In certain examples, R⁶ is benzyl, optionally substituted with one or more substituents Q as described herein. In certain examples, R⁶ is heteroaryl, optionally substituted with one or more substituents Q as described herein. In certain examples, R⁶ is -C(O)R^{1a}, where R^{1a} is as defined herein. In certain examples, R⁶ is -SR^{1a}, where R^{1a} is as defined herein. In certain examples, R⁶ is -S(O)R^{1a}, where R^{1a} is as defined herein. In certain examples, R⁶ is -S(O)₂R^{1a}, where R^{1a} is as defined herein. In certain examples, R⁶ is -L-C₆₋₁₄ aryl, where L is as defined herein. In certain examples, R⁶ is -L-heteroaryl, where L is as defined herein. In certain examples, R⁶ is or -L-heterocyclyl, where L is as defined herein.

In certain examples, W is hydrogen. In certain examples, W is halo. In certain examples, W is cyano. In certain examples, W is C₆₋₁₄ aryl, optionally substituted with one or more substituents Q as described herein. In certain examples, W is benzyl, optionally substituted with one or more substituents Q as described herein. In certain examples, W is heteroaryl, optionally substituted with one or more substituents Q as described herein. In certain examples, W is heterocyclyl, optionally substituted with one or more substituents Q as described herein.

In certain examples, W is -L-C₆₋₁₄ aryl, optionally substituted with one or more substituents Q as described herein, where L is as defined herein. In certain examples, W is -L-heteroaryl, optionally substituted with one or more substituents Q as described herein, where L is as defined herein. In certain examples, W is -L-heterocyclyl, optionally substituted with one or more substituents Q as described herein, where L is as defined herein. In certain examples, W is -L-OH, where L is as defined herein. In certain examples, W is -L-OR^{1a}, where R^{1a} and L are each as defined herein. In certain examples, W is -L-NH₂, where L is as defined herein. In certain examples, W is -L-NHR^{1a}, where R^{1a} and L are each as defined herein. In certain example, W is -L-N(R^{1a})R^{1b}, where R^{1a}, R^{1b}, and L are each as defined herein. In certain examples, W is -L-SR^{1a}, where R^{1a} and L are each as defined herein. In certain examples, W is -L-S(O)R^{1a}, where R^{1a} and L are each as defined herein. In certain examples, W is -L-S(O)₂R^{1a}, where R^{1a} and L are each as defined herein. In certain examples, W is -L-P(O)(OR^{1a})(OR^{1c}), where R^{1a}, R^{1c}, and L are each as defined herein.

In certain examples, W is -L-(N(R^{1c})-L)ₙ-N(R^{1a})R^{1b} , where R^{1a}, R^{1b}, R^{1c}, L and n are each as defined herein. In certain examples, W is -L-(N(R^{1c})-L)ₙ-C₆₋₁₄ aryl, optionally substituted with one or more substituents Q as described herein, where R^{1c}, L, and n are each as defined herein. In certain examples, W is -L-(N(R^{1c})-L)ₙ-heteroaryl, optionally substituted with one or more substituents Q as described herein, where R^{1c}, L, and n are each as defined herein. In certain examples, W is -L-(N(R^{1c})-L)ₙ-heterocyclyl, optionally substituted with one or more substituents Q as described herein, where R^{1c}, L, and n are each as defined herein.

In certain examples, W is -O-L-N(R^{1a})R^{1b}, where R^{1a}, R^{1b}, and L are each as defined herein. In certain examples, W is -O-L-C₆₋₁₄ aryl, optionally substituted with one or more substituents Q as described herein, where L is as defined herein. In certain examples, W is -O-L-heteroaryl, optionally substituted with one or more substituents Q as described herein, where L is as defined herein. In certain examples, W is -O-L-heterocyclyl, optionally substituted with one or more substituents Q as described herein, where L is as defined herein.

In certain examples, W is -O-L-(N(R^{1c})-L)ₙ-N(R^{1a})R^{1b}, where R^{1a}, R^{1b}, R^{1c}, L, and n are each as defined herein. In certain examples, W is -O-L-(N(R^{1c})-L)ₙ-C₆₋₁₄ aryl, optionally substituted with one or more substituents Q as described herein, where R^{1c}, L, and n are each as defined herein. In certain examples, W is -O-L-(N(R^{1c})-L)ₙ- heteroaryl, optionally substituted with one or more substituents Q as described herein, where R^{1c}, L, and n are each as defined herein. In certain examples, W is -O-L-(N(R^{1c})-L)ₙ-heterocyclyl, optionally substituted with one or more substituents Q as described herein, where R^{1c}, L, and n are each as defined herein.

In certain examples, W is -S-L-N(R^{1a})R^{1b}, where R^{1a}, R^{1b}, and L are each as defined herein. In certain examples, W is -S-L-C₆₋₁₄ aryl, optionally substituted with one or more substituents Q as described herein, where L is as defined herein. In certain examples, W is -S-L-heteroaryl, optionally substituted with one or more substituents Q as described herein, where L is as defined herein. In certain examples, W is -S-L-heterocyclyl, optionally substituted with one or more substituents Q as described herein, where L is as defined herein.

In certain examples, W is -S-L-(N(R^{1c})-L)ₙ-N(R^{1a})R^{1b}, where R^{1a}, R^{1b}, R^{1c}, L, and n are each as defined herein. In certain examples, W is -S-L-(N(R^{1c})-L)ₙ-C₆₋₁₄ aryl, optionally substituted with one or more substituents Q as described herein, where R^{1c}, L, and n are each as defined herein. In certain examples, W is -S-L-(N(R^{1c})-L)ₙ-heteroaryl, optionally substituted with one or more substituents Q as described herein, where R^{1c}, L, and n are each as defined herein. In certain examples, W is -S-L-(N(R^{1c})-_{L})ₙ-heterocyclyl, optionally substituted with one or more substituents Q as described herein, where R^{1c}, L, and n are each as defined herein.

In certain examples, W is -(N(R^{1c})-L)ₙ-N(R^{1a})R^{1b}, where R^{1a}, R^{1b}, R^{1c}, L, and n are each as defined herein. In certain examples, W is -(N(R^{1c})-L)ₙ-C₆₋₁₄ aryl, optionally substituted with one or more substituents Q as described herein, where R^{1c}, L, and n are each as defined herein. In certain examples, W is -(N(R^{1c})-L)ₙ-heteroaryl, optionally substituted with one or more substituents Q as described herein, where R^{1c}, L, and n are each as defined herein. In certain examples, W is -(N(R^{1c})-L)ₙ-heterocyclyl, optionally substituted with one or more substituents Q as described herein, where R^{1c}, L, and n are each as defined herein.

In certain examples, W is -C(O)R^{1a}, where R^{1a} is as defined herein. In certain examples, W is -C(O)OR^{1a}, where R^{1a} is as defined herein. In certain examples, W is -C(O)NH₂. In certain examples, W is -C(O)NHR^{1a}, where R^{1a} is as defined herein. In certain examples, W is -C(O)N(R^{1a})R^{1b}, where R^{1a} and R^{1b} are each as defined herein. In certain examples, W is -NHR^{1a}, where R^{1a} is as defined herein. In certain examples, W is -N(R^{1a})R^{1b}, where R^{1a} and R^{1b} are each as defined herein. In certain examples, W is -NHC(O)R^{1a}, where R^{1a} is as defined herein. In certain examples, W is -NR^{1a}C(O)R^{1c}, where R^{1a} and R^{1c} are each as defined herein. In certain examples, W is -NHC(O)OR^{1a}, where R^{1a} is as defined herein. In certain examples, W is -NR^{1a}C(O)OR^{1c}, where R^{1a} and R^{1c} are each as defined herein. In certain examples, W is -NHC(O)NH₂. In certain examples, W is -NHC(O)NHR^{1a}, where R^{1a} is as defined herein. In certain examples, W is -NHC(O)N(R^{1a})R^{1b}, where R^{1a} and R^{1b} are each as defined herein. In certain examples, W is -NR^{1a}C(O)NH₂, where R^{1a} is as defined herein. In certain examples, W is -NR^{1c}C(O)NHR^{1a}, where R^{1a} and R^{1c} are each as defined herein. In certain examples, W is -NR^{1c}C(O)N(R^{1a})R^{1b}, where R^{1a}, R^{1b}, and R^{1c} are each as defined herein. In certain examples, W is -NHS(O)₂R^{1a}, where R^{1a} is as defined herein. In certain examples, W is -NR^{1c}S(O)₂R^{1a}, where R^{1a} and R^{1c} are each as defined herein. In certain examples, W is -OR^{1a}, where R^{1a} is as defined herein. In certain examples, W is -OC(O)R^{1a}, where R^{1a} is as defined herein. In certain examples, W is -OC(O)OR^{1a}, where R^{1a} is as defined herein. In certain examples, W is -OC(O)NH₂. In certain examples, W is -OC(O)NHR^{1a}, where R^{1a} is as defined herein. In certain examples, W is -OC(O)N(R^{1a})R^{1b}, where R^{1a} and R^{1b} are each as defined herein. In certain examples, W is -OS(O)₂R^{1a}, where R^{1a} is as defined herein. In certain examples, W is -P(O)(OR^{1a})(OR^{1c}), where R^{1a} and R^{1c} are each as defined herein. In certain examples, W is -SR^{1a}, where R^{1a} is as defined herein. In certain examples, W is -S(O)R^{1a}, where R^{1a} is as defined herein. In certain examples, W is -S(O)₂R^{1a}, where R^{1a} is as defined herein. In certain examples, W is -S(O)₂NH₂. In certain examples, W is -S(O)₂NHR^{1a}, where R^{1a} is as defined herein. In certain examples, W is -S(O)₂N(R^{1a})R^{1b}, where R^{1a} and R^{1b} are each as defined herein. In certain examples, W is -S(O)₂OR^{1a}, where R^{1a} is as defined herein.

In certain examples, Z is cyano. In certain examples, Z is C₁₋₆ alkyl, optionally substituted with one or more substituents Q as described herein. In certain examples, Z is C₂₋₆ alkenyl, optionally substituted with one or more substituents Q as described herein. In certain examples, Z is C₂₋₆ alkynyl, optionally substituted with one or more substituents Q as described herein. In certain examples, Z is C₃₋₁₀ cycloalkyl, optionally substituted with one or more substituents Q as described herein. In certain examples, Z is C₆₋₁₄ aryl, optionally substituted with one or more substituents Q as described herein. In certain examples, Z is C₇₋₁₅ aralkyl, optionally substituted with one or more substituents Q as described herein. In certain examples, Z is benzyl, optionally substituted with one or more substituents Q as described herein. In certain examples, Z is heteroaryl, optionally substituted with one or more substituents Q as described herein. In certain examples, Z is 5-membered heteroaryl, optionally substituted with one or more substituents Q as described herein. In certain examples, Z is tetrazolyl, optionally substituted with one or more substituents Q as described herein. In certain examples, Z is 1,2,4-oxadiazolyl, optionally substituted with one or more substituents Q as described herein. In certain examples, Z is heterocyclyl, optionally substituted with one or more substituents Q as described herein. In certain examples, Z is -L- C₆₋₁₄ aryl, optionally substituted with one or more substituents Q as described herein, where L is as defined herein. In certain examples, Z is -L-heteroaryl, optionally substituted with one or more substituents Q as described herein, where L is as defined herein. In certain examples, Z is -L-heterocyclyl, optionally substituted with one or more substituents Q as described herein, where L is as defined herein.

In certain examples, Z is -C(O)R^{1a}, wherein R^{1a} is as defined herein. In certain examples, Z is -C(O)OR^{1a}, wherein R^{1a} is as defined herein. In certain examples, Z is -C(O)OC₁₋₆ alkyl, wherein the alkyl is optionally substituted with one or more substituents Q as defined herein. In certain examples, Z is -C(O)OCH₃. In certain examples, Z is -C(O)NHR^{1a}, wherein R^{1a} is as defined herein. In certain examples, Z is -C(O)N(R^{1a})R^{1b}, wherein R^{1a} and R^{1b} are each as defined herein. In certain examples, Z is -P(O)(OR^{1a})(OR^{1c}), wherein R^{1a} and R^{1c} are each as defined herein. In certain examples, Z is -SR^{1a}, wherein R^{1a} is as defined herein. In certain examples, Z is -S(O)R^{1a}, wherein R^{1a} is as defined herein. In certain examples, Z is -S(O)₂R^{1a}, wherein R^{1a} is as defined herein. In certain examples, Z is -S(O)₂NH₂. In certain examples, Z is -S(O)₂NHR^{1a}, wherein R^{1a} is as defined herein. In certain examples, Z is -S(O)₂N(R^{1a})R^{1b}, wherein R^{1a} and R^{1b} are each as defined herein.

In certain examples, X is a bond. In certain examples, X is O. In certain examples, X is S. In certain examples, X is NR^{1c}, where R^{1c} is as defined herein.

In certain examples, n is 1. In certain examples, n is 2. In certain examples, n is 3. In certain examples, n is 4. In certain examples, n is 5.

In certain examples, the compounds provided herein show activity as antagonists of an AHR.

The compounds provided herein may be enantiomerically pure, such as a single enantiomer or a single diastereomer, or be stereoisomeric mixtures, such as a mixture of enantiomers, *e*.*g*., a racemic mixture of two enantiomers; or a mixture of two or more diastereomers. As such, one of skill in the art will recognize that administration of a compound in its (*R*) form is equivalent, for compounds that undergo epimerization *in vivo*, to administration of the compound in its (*S*) form. Conventional techniques for the preparation/isolation of individual enantiomers include synthesis from a suitable optically pure precursor, asymmetric synthesis from achiral starting materials, or resolution of an enantiomeric mixture, for example, chiral chromatography, recrystallization, resolution, diastereomeric salt formation, or derivatization into diastereomeric adducts followed by separation.

### 5.4. Isolation of NK Cells

Methods of isolating natural killer cells are known in the art and can be used to isolate the natural killer cells, *e.g.,* NK cells produced using the three-stage method, described herein. For example, NK cells can be isolated or enriched, for example, by staining cells, in one embodiment, with antibodies to CD56 and CD3, and selecting for CD56⁺CD3⁻ cells. In certain examples, the NK cells are enriched for CD56⁺CD3⁻ cells in comparison with total cells produced using the three-stage method, described herein. NK cells, *e.g.,* cells produced using the three-stage method, described herein, can be isolated using a commercially available kit, for example, the NK Cell Isolation Kit (Miltenyi Biotec). NK cells, *e.g.,* cells produced using the three-stage method, described herein, can also be isolated or enriched by removal of cells other than NK cells in a population of cells that comprise the NK cells, *e.g.,* cells produced using the three-stage method, described herein. For example, NK cells, *e.g.,* cells produced using the three-stage method, described herein, may be isolated or enriched by depletion of cells displaying non-NK cell markers using, *e*.*g*., antibodies to one or more of CD3, CD4, CD14, CD19, CD20, CD36, CD66b, CD123, HLA DR and/or CD235a (glycophorin A). Negative isolation can be carried out using a commercially available kit, *e.g.,* the NK Cell Negative Isolation Kit (Dynal Biotech). Cells isolated by these methods may be additionally sorted, *e.g.,* to separate CD11a+ and CD11a- cells, and/or CD117+ and CD117- cells, and/or CD16⁺ and CD16⁻ cells, and/or CD94⁺ and CD94⁻. In certain embodiments, cells, *e.g.,* cells produced by the three-step methods described herein, are sorted to separate CD11a+ and CD11a- cells. In specific examples, CD11a+ cells are isolated. In certain examples, the cells are enriched for CD11a⁺ cells in comparison with total cells produced using the three-stage method, described herein. In specific embodiments, CD11a- cells are isolated. In certain examples, the cells are enriched for CD11a- cells in comparison with total cells produced using the three-stage method, described herein. In certain examples, cells are sorted to separate CD117+ and CD117- cells. In specific examples, CD117+ cells are isolated. In certain examples, the cells are enriched for CD117⁺ cells in comparison with total cells produced using the three-stage method, described herein. In specific examples, CD117- cells are isolated. In certain examples, the cells are enriched for CD117- cells in comparison with total cells produced using the three-stage method, described herein. In certain examples, cells are sorted to separate CD16⁺ and CD16⁻ cells. In specific examples, CD16⁺ cells are isolated. In certain examples, the cells are enriched for CD16⁺ cells in comparison with total cells produced using the three-stage method, described herein. In specific examples, CD16⁻ cells are isolated. In certain examples, the cells are enriched for CD16- cells in comparison with total cells produced using the three-stage method, described herein. In certain examples, cells are sorted to separate CD94⁺ and CD94⁻ cells. In specific examples, CD94⁺ cells are isolated. In certain examples, the cells are enriched for CD94⁺ cells in comparison with total cells produced using the three-stage method, described herein. In specific examples, CD94⁻ cells are isolated. In certain examples, the cells are enriched for CD94- cells in comparison with total cells produced using the three-stage method, described herein. In certain examples, isolation is performed using magnetic separation. In certain examples, isolation is performed using flow cytometry.

Methods of isolating II,C3 cells are known in the art and can be used to isolate the ILC3 cells, *e*.*g*., ILC3 cells produced using the three-stage method, described herein. For example, ILC3 cells can be isolated or enriched, for example, by staining cells, in one embodiment, with antibodies to CD56, CD3, and CD11a, and selecting for CD56⁺CD3⁻CD11a⁻ cells. ILC3 cells, *e*.*g*., cells produced using the three-stage method, described herein, can also be isolated or enriched by removal of cells other than ILC3 cells in a population of cells that comprise the ILC3 cells, *e*.*g*., cells produced using the three-stage method, described herein. For example, ILC3 cells, *e*.*g*., cells produced using the three-stage method, described herein, may be isolated or enriched by depletion of cells displaying non-ILC3 cell markers using, *e*.*g*., antibodies to one or more of CD3, CD4, CD11a, CD14, CD19, CD20, CD36, CD66b, CD94, CD123, HLA DR and/or CD235a (glycophorin A). Cells isolated by these methods may be additionally sorted, *e*.*g*., to separate CD117⁺ and CD117⁻ cells. NK cells can be isolated or enriched, for example, by staining cells, in one example, with antibodies to CD56, CD3, CD94, and CD11a, and selecting for CD56⁺CD3⁺CD94⁺CD11a⁺ cells. NK cells, *e.g.,* cells produced using the three-stage method, described herein, can also be isolated or enriched by removal of cells other than NK cells in a population of cells that comprise the NK cells, *e.g.,* cells produced using the three-stage method, described herein. In certain examples, the NK cells are enriched for CD56⁺CD3⁻CD94⁺CD11a⁺ cells in comparison with total cells produced using the three-stage method described herein.

In one example, ILC3 cells are isolated or enriched by selecting for CD56⁺CD3⁻CD11a⁻ cells. In certain examples, the ILC3 cells are enriched for CD56⁺CD3⁻ CD11a⁻ cells in comparison with total cells produced using the three-stage method, described herein. In one example, ILC3 cells are isolated or enriched by selecting for CD56⁺CD3⁻ CD1a⁻CD117+ cells. In certain examples, the ILC3 cells are enriched for CD56⁺CD3⁻ CD11a⁻CD117+ cells in comparison with total cells produced using the three-stage method, described herein. In one example, ILC3 cells are isolated or enriched by selecting for CD56⁺CD3⁻CD11a⁻CD117⁺CDIL1R1⁺ cells. In certain examples, the ILC3 cells are enriched for CD56⁺CD3⁺CD11a⁻CD117⁺CDIL1R1⁺ cells in comparison with total cells produced using the three-stage method, described herein.

In one example, NK cells are isolated or enriched by selecting for CD56⁺CD3⁻CD94⁺CD11a⁺ cells. In certain examples, the NK cells are enriched for CD56⁺CD3⁻CD94⁺CD11a⁺ cells in comparison with total cells produced using the three-stage method, described herein. In one example, NK cells are isolated or enriched by selecting for CD56⁺CD3⁻CD94⁺CD11a⁺CD117⁻ cells. In certain examples, the NK cells are enriched for CD56⁺CD3⁻CD94⁺CD11a⁺CD117⁻ cells in comparison with total cells produced using the three-stage method, described herein.

Cell separation can be accomplished by, *e.g.,* flow cytometry, fluorescence-activated cell sorting (FACS), or, in one embodiment, magnetic cell sorting using microbeads conjugated with specific antibodies. The cells may be isolated, *e*.*g*., using a magnetic activated cell sorting (MACS) technique, a method for separating particles based on their ability to bind magnetic beads (*e*.*g*., about 0.5-100 µm diameter) that comprise one or more specific antibodies, *e.g.,* anti-CD56 antibodies. Magnetic cell separation can be performed and automated using, *e.g.,* an AUTOMACS^{™} Separator (Miltenyi). A variety of useful modifications can be performed on the magnetic microspheres, including covalent addition of antibody that specifically recognizes a particular cell surface molecule or hapten. The beads are then mixed with the cells to allow binding. Cells are then passed through a magnetic field to separate out cells having the specific cell surface marker. In one example, these cells can then isolated and re-mixed with magnetic beads coupled to an antibody against additional cell surface markers. The cells are again passed through a magnetic field, isolating cells that bound both the antibodies. Such cells can then be diluted into separate dishes, such as microtiter dishes for clonal isolation.

### 5.5. Placental Perfusate

NK cells and/or ILC3 cells, *e*.*g*., NK cell and/or ILC3 cell populations produced according to the three-stage method described herein may be produced from hematopoietic stem or progenitors from any source, *e*.*g*., placental tissue, placental perfusate, umbilical cord blood, placental blood, peripheral blood, spleen, liver, or the like. In certain embodiments, the hematopoietic stem cells are combined hematopoietic stem cells from placental perfusate and from cord blood from the same placenta used to generate the placental perfusate. Placental perfusate comprising placental perfusate cells that can be obtained, for example, by the methods disclosed in U.S. Patent Nos. 7,045,148 and 7,468,276 and U.S. Patent Application Publication No. 2009/0104164.

### 5.5.1. Cell Collection Composition

The placental perfusate and perfusate cells, from which hematopoietic stem or progenitors may be isolated, or useful in tumor suppression or the treatment of an individual having tumor cells, cancer or a viral infection, *e*.*g*., in combination with the NK cells and/or ILC3 cells, *e*.*g*., NK cell and/or ILC3 cell populations produced according to the three-stage method provided herein, can be collected by perfusion of a mammalian, *e*.*g*., human post-partum placenta using a placental cell collection composition. Perfusate can be collected from the placenta by perfusion of the placenta with any physiologically-acceptable solution, *e*.*g*., a saline solution, culture medium, or a more complex cell collection composition. A cell collection composition suitable for perfusing a placenta, and for the collection and preservation of perfusate cells is described in detail in related U.S. Application Publication No. 2007/0190042.

The cell collection composition can comprise any physiologically-acceptable solution suitable for the collection and/or culture of stem cells, for example, a saline solution (*e*.*g*., phosphate-buffered saline, Kreb's solution, modified Kreb's solution, Eagle's solution, 0.9% NaCl. *etc*.), a culture medium (*e*.*g*., DMEM, H.DMEM, *etc*.), and the like.

The cell collection composition can comprise one or more components that tend to preserve placental cells, that is, prevent the placental cells from dying, or delay the death of the placental cells, reduce the number of placental cells in a population of cells that die, or the like, from the time of collection to the time of culturing. Such components can be, *e.g.,* an apoptosis inhibitor (*e*.*g*., a caspase inhibitor or JNK inhibitor); a vasodilator *(e.g.,* magnesium sulfate, an antihypertensive drug, atrial natriuretic peptide (ANP), adrenocorticotropin, corticotropin-releasing hormone, sodium nitroprusside, hydralazine, adenosine triphosphate, adenosine, indomethacin or magnesium sulfate, a phosphodiesterase inhibitor, *etc*.); a necrosis inhibitor (*e*.*g*., 2-(1H-Indol-3-yl)-3-pentylamino-maleimide, pyrrolidine dithiocarbamate, or clonazepam); a TNF-α inhibitor; and/or an oxygen-carrying perfluorocarbon (*e*.*g*., perfluorooctyl bromide, perfluorodecyl bromide, *etc*.).

The cell collection composition can comprise one or more tissue-degrading enzymes, e.g., a metalloprotease, a serine protease, a neutral protease, a hyaluronidase, an RNase, or a DNase, or the like. Such enzymes include, but are not limited to, collagenases (*e*.*g*., collagenase I, II, III or IV, a collagenase from *Clostridium histolyticum*, *etc*.); dispase, thermolysin, elastase, trypsin, LIBERASE, hyaluronidase, and the like.

The cell collection composition can comprise a bacteriocidally or bacteriostatically effective amount of an antibiotic. In certain non-limiting examples, the antibiotic is a macrolide *(e.g.,* tobramycin), a cephalosporin *(e.g.,* cephalexin, cephradine, cefuroxime, cefprozil, cefaclor, cefixime or cefadroxil), a clarithromycin, an erythromycin, a penicillin *(e.g.,* penicillin V) or a quinolone *(e.g.,* ofloxacin, ciprofloxacin or norfloxacin), a tetracycline, a streptomycin, *etc.* In a particular example, the antibiotic is active against Gram(+) and/or Gram(-) bacteria, *e.g*., *Pseudomonas aeruginosa*, *Staphylococcus aureus*, and the like.

The cell collection composition can also comprise one or more of the following compounds: adenosine (about 1 mM to about 50 mM); D-glucose (about 20 mM to about 100 mM); magnesium ions (about 1 mM to about 50 mM); a macromolecule of molecular weight greater than 20,000 daltons, in one example, present in an amount sufficient to maintain endothelial integrity and cellular viability (*e*.*g*., a synthetic or naturally occurring colloid, a polysaccharide such as dextran or a polyethylene glycol present at about 25 g/l to about 100 g/l, or about 40 g/l to about 60 g/l); an antioxidant (*e*.*g*., butylated hydroxyanisole, butylated hydroxytoluene, glutathione, vitamin C or vitamin E present at about 25 µM to about 100 µM); a reducing agent (*e*.*g*., N-acetylcysteine present at about 0.1 mM to about 5 mM); an agent that prevents calcium entry into cells (*e*.*g*., verapamil present at about 2 µM to about 25 µM); nitroglycerin (*e*.*g*., about 0.05 g/L to about 0.2 g/L); an anticoagulant, in one example, present in an amount sufficient to help prevent clotting of residual blood (*e*.*g*., heparin or hirudin present at a concentration of about 1000 units/l to about 100,000 units/l); or an amiloride containing compound (*e*.*g*., amiloride, ethyl isopropyl amiloride, hexamethylene amiloride, dimethyl amiloride or isobutyl amiloride present at about 1.0 µM to about 5 µM).

### 5.5.2. Collection and Handling of Placenta

Generally, a human placenta is recovered shortly after its expulsion after birth. In one example, the placenta is recovered from a patient after informed consent and after a complete medical history of the patient is taken and is associated with the placenta. In one the medical history continues after delivery.

Prior to recovery of perfusate, the umbilical cord blood and placental blood are removed. In certain examples, after delivery, the cord blood in the placenta is recovered. The placenta can be subjected to a conventional cord blood recovery process. Typically a needle or cannula is used, with the aid of gravity, to exsanguinate the placenta (*see*, *e.g*., Anderson, U.S. Patent No. 5,372,581; Hessel et al., U.S. Patent No. 5,415,665). The needle or cannula is usually placed in the umbilical vein and the placenta can be gently massaged to aid in draining cord blood from the placenta. Such cord blood recovery may be performed commercially, *e*.*g*., LifeBank Inc., Cedar Knolls, N.J., ViaCord, Cord Blood Registry and CryoCell. In one example, the placenta is gravity drained without further manipulation so as to minimize tissue disruption during cord blood recovery.

Typically, a placenta is transported from the delivery or birthing room to another location, *e*.*g*., a laboratory, for recovery of cord blood and collection of perfusate. The placenta can be transported in a sterile, thermally insulated transport device (maintaining the temperature of the placenta between 20-28 °C), for example, by placing the placenta, with clamped proximal umbilical cord, in a sterile zip-lock plastic bag, which is then placed in an insulated container. In another example, the placenta is transported in a cord blood collection kit substantially as described in U.S. Patent No. 7,147,626. In one example, the placenta is delivered to the laboratory four to twenty-four hours following delivery. In certain examples, the proximal umbilical cord is clamped, for example within 4-5 cm (centimeter) of the insertion into the placental disc prior to cord blood recovery. In other examples, the proximal umbilical cord is clamped after cord blood recovery but prior to further processing of the placenta.

The placenta, prior to collection of the perfusate, can be stored under sterile conditions and at either room temperature or at a temperature of 5 to 25 °C (centigrade). The placenta may be stored for a period of longer than forty eight hours, or for a period of four to twenty-four hours prior to perfusing the placenta to remove any residual cord blood. The placenta can be stored in an anticoagulant solution at a temperature of 5 °C to 25 °C (centigrade). Suitable anticoagulant solutions are well known in the art. For example, a solution of heparin or warfarin sodium can be used. In one example, the anticoagulant solution comprises a solution of heparin (*e*.*g*., 1% w/w in 1:1000 solution). In some examples, the exsanguinated placenta is stored for no more than 36 hours before placental perfusate is collected.

### 5.5.3. Placental Perfusion

Methods of perfusing mammalian placentae and obtaining placental perfusate are disclosed, *e*.*g*., in Hariri, U.S. Patent Nos. 7,045,148 and 7,255,879, and in U.S. Application Publication Nos. 2009/0104164, 2007/0190042 and 20070275362, issued as U.S. Pat No. 8,057,788.

Perfusate can be obtained by passage of perfusion solution, *e*.*g*., saline solution, culture medium or cell collection compositions described above, through the placental vasculature. In one example, a mammalian placenta is perfused by passage of perfusion solution through either or both of the umbilical artery and umbilical vein. The flow of perfusion solution through the placenta may be accomplished using, *e*.*g*., gravity flow into the placenta. For example, the perfusion solution is forced through the placenta using a pump, *e*.*g*., a peristaltic pump. The umbilical vein can be, *e*.*g*., cannulated with a cannula, *e.g.,* a TEFLON^{®} or plastic cannula, that is connected to a sterile connection apparatus, such as sterile tubing. The sterile connection apparatus is connected to a perfusion manifold.

In preparation for perfusion, the placenta can be oriented in such a manner that the umbilical artery and umbilical vein are located at the highest point of the placenta. The placenta can be perfused by passage of a perfusion solution through the placental vasculature, or through the placental vasculature and surrounding tissue. In one example, the umbilical artery and the umbilical vein are connected simultaneously to a pipette that is connected via a flexible connector to a reservoir of the perfusion solution. The perfusion solution is passed into the umbilical vein and artery. The perfusion solution exudes from and/or passes through the walls of the blood vessels into the surrounding tissues of the placenta, and is collected in a suitable open vessel from the surface of the placenta that was attached to the uterus of the mother during gestation. The perfusion solution may also be introduced through the umbilical cord opening and allowed to flow or percolate out of openings in the wall of the placenta which interfaced with the maternal uterine wall. In another example, the perfusion solution is passed through the umbilical veins and collected from the umbilical artery, or is passed through the umbilical artery and collected from the umbilical veins, that is, is passed through only the placental vasculature (fetal tissue).

In one example, for example, the umbilical artery and the umbilical vein are connected simultaneously, *e*.*g*., to a pipette that is connected via a flexible connector to a reservoir of the perfusion solution. The perfusion solution is passed into the umbilical vein and artery. The perfusion solution exudes from and/or passes through the walls of the blood vessels into the surrounding tissues of the placenta, and is collected in a suitable open vessel from the surface of the placenta that was attached to the uterus of the mother during gestation. The perfusion solution may also be introduced through the umbilical cord opening and allowed to flow or percolate out of openings in the wall of the placenta which interfaced with the maternal uterine wall. Placental cells that are collected by this method, which can be referred to as a "pan" method, are typically a mixture of fetal and maternal cells.

In another example, the perfusion solution is passed through the umbilical veins and collected from the umbilical artery, or is passed through the umbilical artery and collected from the umbilical veins. Placental cells collected by this method, which can be referred to as a "closed circuit" method, are typically almost exclusively fetal.

The closed circuit perfusion method can, in one example, be performed as follows. A post-partum placenta is obtained within about 48 hours after birth. The umbilical cord is clamped and cut above the clamp. The umbilical cord can be discarded, or can processed to recover, *e*.*g*., umbilical cord stem cells, and/or to process the umbilical cord membrane for the production of a biomaterial. The amniotic membrane can be retained during perfusion, or can be separated from the chorion, *e*.*g*., using blunt dissection with the fingers. If the amniotic membrane is separated from the chorion prior to perfusion, it can be, *e*.*g*., discarded, or processed, *e.g.,* to obtain stem cells by enzymatic digestion, or to produce, *e.g.,* an amniotic membrane biomaterial, *e*.*g*., the biomaterial described in U.S. Application Publication No. 2004/0048796. After cleaning the placenta of all visible blood clots and residual blood, *e*.*g*., using sterile gauze, the umbilical cord vessels are exposed, *e*.*g*., by partially cutting the umbilical cord membrane to expose a cross-section of the cord. The vessels are identified, and opened, *e*.*g*., by advancing a closed alligator clamp through the cut end of each vessel. The apparatus, *e*.*g*., plastic tubing connected to a perfusion device or peristaltic pump, is then inserted into each of the placental arteries. The pump can be any pump suitable for the purpose, *e*.*g*., a peristaltic pump. Plastic tubing, connected to a sterile collection reservoir, *e*.*g*., a blood bag such as a 250 mL collection bag, is then inserted into the placental vein. Alternatively, the tubing connected to the pump is inserted into the placental vein, and tubes to a collection reservoir(s) are inserted into one or both of the placental arteries. The placenta is then perfused with a volume of perfusion solution, *e*.*g*., about 750 ml of perfusion solution. Cells in the perfusate are then collected, *e.g.,* by centrifugation.

In one example, the proximal umbilical cord is clamped during perfusion, and, more specifically, can be clamped within 4-5 cm (centimeter) of the cord's insertion into the placental disc.

The first collection of perfusion fluid from a mammalian placenta during the exsanguination process is generally colored with residual red blood cells of the cord blood and/or placental blood. The perfusion fluid becomes more colorless as perfusion proceeds and the residual cord blood cells are washed out of the placenta. Generally from 30 to 100 mL of perfusion fluid is adequate to initially flush blood from the placenta, but more or less perfusion fluid may be used depending on the observed results.

In certain examples, cord blood is removed from the placenta prior to perfusion (*e*.*g*., by gravity drainage), but the placenta is not flushed *(e.g.,* perfused) with solution to remove residual blood. In certain examples, cord blood is removed from the placenta prior to perfusion *(e.g.,* by gravity drainage), and the placenta is flushed *(e.g.,* perfused) with solution to remove residual blood.

The volume of perfusion liquid used to perfuse the placenta may vary depending upon the number of placental cells to be collected, the size of the placenta, the number of collections to be made from a single placenta, *etc.* In various examples, the volume of perfusion liquid may be from 50 mL to 5000 mL, 50 mL to 4000 mL, 50 mL to 3000 mL, 100 mL to 2000 mL, 250 mL to 2000 mL, 500 mL to 2000 mL, or 750 mL to 2000 mL. Typically, the placenta is perfused with 700-800 mL of perfusion liquid following exsanguination.

The placenta can be perfused a plurality of times over the course of several hours or several days. Where the placenta is to be perfused a plurality of times, it may be maintained or cultured under aseptic conditions in a container or other suitable vessel, and perfused with a cell collection composition, or a standard perfusion solution (*e.g*., a normal saline solution such as phosphate buffered saline ("PBS") with or without an anticoagulant (*e*.*g*., heparin, warfarin sodium, coumarin, bishydroxycoumarin), and/or with or without an antimicrobial agent (*e.g*., β-mercaptoethanol (0.1 mM); antibiotics such as streptomycin (*e*.*g*., at 40-100 µg/ml), penicillin *(e.g.,* at 40 U/ml), amphotericin B *(e.g.,* at 0.5 µg/ml). In one example, an isolated placenta is maintained or cultured for a period of time without collecting the perfusate, such that the placenta is maintained or cultured for 1, 2, 3, 4, 5, 6, 7, 8, 9, 10, 11, 12, 13, 14, 15, 16, 17, 18, 19, 20, 21, 22, 23, or 24 hours, or 2 or 3 or more days before perfusion and collection of perfusate. The perfused placenta can be maintained for one or more additional time(s), *e*.*g*., 1, 2, 3, 4, 5, 6, 7, 8, 9, 10, 11, 12, 13, 14, 15, 16, 17, 18, 19, 20, 21, 22, 23, 24 or more hours, and perfused a second time with, *e.g.,* 700-800 mL perfusion fluid. The placenta can be perfused 1, 2, 3, 4, 5 or more times, for example, once every 1, 2, 3, 4, 5 or 6 hours. In one example, perfusion of the placenta and collection of perfusion solution, *e*.*g*., placental cell collection composition, is repeated until the number of recovered nucleated cells falls below 100 cells/ml. The perfusates at different time points can be further processed individually to recover time-dependent populations of cells, e.g., total nucleated cells. Perfusates from different time points can also be pooled.

### 5.5.4. Placental Perfusate and Placental Perfusate Cells

Typically, placental perfusate from a single placental perfusion comprises about 100 million to about 500 million nucleated cells, including hematopoietic cells from which NK cells and/or ILC3 cells, *e.g.,* NK cells and/or ILC3 cells produced according to the three-stage method described herein, may be produced by the method disclosed herein. In certain examples, the placental perfusate or perfusate cells comprise CD34⁺ cells, *e.g.,* hematopoietic stem or progenitor cells. Such cells can, in a more specific example, comprise CD34⁺CD45⁻ stem or progenitor cells, CD34⁺CD45⁺ stem or progenitor cells, or the like. In certain examples, the perfusate or perfusate cells are cryopreserved prior to isolation of hematopoietic cells therefrom. In certain other examples, the placental perfusate comprises, or the perfusate cells comprise, only fetal cells, or a combination of fetal cells and maternal cells.

### 5.6. NK Cells

### 5.6.1. NK Cells Produced by Three-Stage Method

Disclosed herein is an isolated NK cell population, wherein said NK cells are produced according to the three-stage method described above.

Disclosed herein is an isolated NK cell population produced by a three-stage method described herein, wherein said NK cell population comprises a greater percentage of CD3-CD56+ cells than an NK progenitor cell population produced by a three-stage method described herein, *e.g.,* an NK progenitor cell population produced by the same three-stage method with the exception that the third culture step used to produce the NK progenitor cell population was of shorter duration than the third culture step used to produce the NK cell population. In a specific example, said NK cell population comprises about 70% or more, in some examples, 75%, 80%, 85%, 90%, 95%, 98%, or 99% CD3-CD56+ cells. In another specific examples, said NK cell population comprises no less than 80%, 85%, 90%, 95%, 98%, or 99% CD3-CD56+ cells. In another specific example, said NK cell population comprises between 70%-75%, 75%-80%, 80%-85%, 85%-90%, 90%-95%, or 95%-99% CD3-CD56+ cells.

In certain examples, said CD3⁻CD56⁺ cells in said NK cell population comprises CD3⁻CD56⁺ cells that are additionally NKp46⁺. In certain examples, said CD3⁻ CD56⁺ cells in said NK cell population comprises CD3⁻CD56⁺ cells that are additionally CD16-. In certain examples, said CD3⁺CD56⁺ cells in said NK cell population comprises CD3⁻ CD56⁺ cells that are additionally CD16+. In certain examples, said CD3⁺CD56⁺ cells in said NK cell population comprises CD3⁻CD56⁺ cells that are additionally CD94-. In certain examples, said CD3⁻CD56⁺ cells in said NK cell population comprises CD3⁻CD56⁺ cells that are additionally CD94+. In certain examples, said CD3⁻CD56⁺ cells in said NK cell population comprises CD3⁻CD56⁺ cells that are additionally CD11a⁺. In certain examples, said CD3⁻CD56⁺ cells in said NK cell population comprises CD3⁻CD56⁺ cells that are additionally NKp30⁺. In certain examples, said CD3⁺CD56⁺ cells in said NK cell population comprises CD3⁺CD56⁺ cells that are additionally CD161⁺. In certain examples, said CD3⁻CD56⁺ cells in said NK cell population comprises CD3⁻CD56⁺ cells that are additionally DNAM-1⁺. In certain examples, said CD3⁺CD56⁺ cells in said NK cell population comprises CD3⁻CD56⁺ cells that are additionally T-bet⁺.

In one example, an NK cell population produced by a three-stage method described herein comprises cells which are CD117+. In one example, an NK cell population produced by a three-stage method described herein comprises cells which are NKG2D+. In one example, an NK cell population produced by a three-stage method described herein comprises cells which are NKp44+. In one example, an NK cell population produced by a three-stage method described herein comprises cells which are CD244+. In one example, an NK cell population produced by a three-stage method described herein comprises cells which express perforin. In one example, an NK cell population produced by a three-stage method described herein comprises cells which express EOMES. In one example, an NK cell population produced by a three-stage method described herein comprises cells which express granzyme B. In one example, an NK cell population produced by a three-stage method described herein comprises cells which secrete IFNy, GM-CSF and/or TNFα.

### 5.7. ILC3 Cells

### 5.7.1. ILC3 Cells Produced by Three-Stage Method

Disclosed herein is an isolated II,C3 cell population, wherein said ILC3 cells are produced according to the three-stage method described above.

Disclosed herein is an isolated ILC3 cell population produced by a three-stage method described herein, wherein said ILC3 cell population comprises a greater percentage of CD3-CD56+ cells than an ILC3 progenitor cell population produced by a three-stage method described herein, *e*.*g*., an ILC3 progenitor cell population produced by the same three-stage method with the exception that the third culture step used to produce the ILC3 progenitor cell population was of shorter duration than the third culture step used to produce the ILC3 cell population. In a specific example, said ILC3 cell population comprises about 70% or more, in some examples, 75%, 80%, 85%, 90%, 95%, 98%, or 99% CD3-CD56+ cells. In another specific example, said ILC3 cell population comprises no less than 80%, 85%, 90%, 95%, 98%, or 99% CD3-CD56+ cells. In another specific example, said ILC3 cell population comprises between 70%-75%, 75%-80%, 80%-85%, 85%-90%, 90%-95%, or 95%-99% CD3-CD56+ cells.

In certain examples, said CD3⁻CD56⁺ cells in said ILC3 cell population comprises CD3⁻CD56⁺ cells that are additionally NKp46⁻. In certain examples, said CD3⁻ CD56⁺ cells in said ILC3 cell population comprises CD3⁻CD56⁺ cells that are additionally CD16-. In certain examples, said CD3⁻CD56⁺ cells in said ILC3 cell population comprises CD3⁻CD56⁺ cells that are additionally IL1R1+. In certain examples, said CD3⁻CD56⁺ cells in said ILC3 cell population comprises CD3⁻CD56⁺ cells that are additionally CD94-. In certain examples, said CD3⁺CD56⁺ cells in said ILC3 cell population comprises CD3⁻CD56⁺ cells that are additionally RORyt+. In certain examples, said CD3⁺CD56⁺ cells in said ILC3 cell population comprises CD3⁻CD56⁺ cells that are additionally CD11a⁻. In certain examples, said CD3⁻CD56⁺ cells in said ILC3 cell population comprises CD3⁻CD56⁺ cells that are additionally T-bet+.

In one example, an ILC3 cell population produced by a three-stage method described herein comprises cells which are CD117+. In one example, an ILC3 cell population produced by a three-stage method described herein comprises cells which are NKG2D⁻. In one example, an ILC3 cell population produced by a three-stage method described herein comprises cells which are NKp30⁻. In one example, an ILC3 cell population produced by a three-stage method described herein comprises cells which are CD244+. In one example, an ILC3 cell population produced by a three-stage method described herein comprises cells which are DNAM-1+. In one example, an ILC3 cell population produced by a three-stage method described herein comprises cells which express AHR. In one example, an ILC3 cell population produced by a three-stage method described herein comprises cells which do not express perforin. In one example, an ILC3 cell population produced by a three-stage method described herein comprises cells which do not express EOMES. In one example, an ILC3 cell population produced by a three-stage method described herein comprises cells which do not express granzyme B. In one example, an ILC3 cell population produced by a three-stage method described herein comprises cells which secrete IL-22 and/or IL-8.

In certain aspects, cell populations produced by the three-stage method described herein comprise CD11a+ cells and CD11a- cells in a ratio of 50:1, 40:1, 30:1, 20:1, 10:1, 5:1, 4:1, 3:1, 2:1, 1:1, 1:2, 1:3, 1:4, 1:5, 1:10, 1:20, 1:30, 1:40, or 1:50. In certain aspects, a population of cells described herein comprises CD11a+ cells and CD11a- cells in a ratio of 50:1. In certain aspects, a population of cells described herein comprises CD11a+ cells and CD11a-cells in a ratio of 20:1. In certain aspects, a population of cells described herein comprises CD11a+ cells and CD11a- cells in a ratio of 10:1. In certain aspects, a population of cells described herein comprises CD11a+ cells and CD11a- cells in a ratio of 5:1. In certain aspects, a population of cells described herein comprises CD11a+ cells and CD11a- cells in a ratio of 1:1. In certain aspects, a population of cells described herein comprises CD11a+ cells and CD11a-cells in a ratio of 1:5. In certain aspects, a population of cells described herein comprises CD11a+ cells and CD11a- cells in a ratio of 1:10. In certain aspects, a population of cells described herein comprises CD11a+ cells and CD11a- cells in a ratio of 1:20. In certain aspects, a population of cells described herein comprises CD11a+ cells and CD11a- cells in a ratio of 1:50.

In certain aspects, cell populations described herein are produced by combining the CD11a+ cells with the CD11a- cells in a ratio of 50:1, 40:1, 30:1, 20:1, 10:1, 5:1, 4:1, 3:1, 2:1, 1:1, 1:2, 1:3, 1:4, 1:5, 1:10, 1:20, 1:30, 1:40, or 1:50 to produce a combined population of cells. In certain aspects, a combined population of cells described herein comprises CD11a+ cells and CD11a- cells combined in a ratio of 50:1. In certain aspects, a combined population of cells described herein comprises CD11a+ cells and CD11a- cells combined in a ratio of 20:1. In certain aspects, a combined population of cells described herein comprises CD11a+ cells and CD11a- cells combined in a ratio of 10:1. In certain aspects, a combined population of cells described herein comprises CD11a+ cells and CD11a- cells combined in a ratio of 5:1. In certain aspects, a combined population of cells described herein comprises CD11a+ cells and CD11a-cells combined in a ratio of 1:1. In certain aspects, a combined population of cells described herein comprises CD11a+ cells and CD11a- cells combined in a ratio of 1:5. In certain aspects, a combined population of cells described herein comprises CD11a+ cells and CD11a- cells combined in a ratio of 1:10. In certain aspects, a combined population of cells described herein comprises CD11a+ cells and CD11a- cells combined in a ratio of 1:20. In certain aspects, a combined population of cells described herein comprises CD11a+ cells and CD11a- cells combined in a ratio of 1:50.

In certain aspects, cell populations produced by the three-stage method described herein comprise NK cells and II,C3 cells in a ratio of 50:1, 40:1, 30:1, 20:1, 10:1, 5:1, 4:1, 3:1, 2:1, 1:1, 1:2, 1:3, 1:4, 1:5, 1:10, 1:20, 1:30, 1:40, or 1:50. In certain aspects, a population of cells described herein comprises NK cells and ILC3 cells in a ratio of 50:1. In certain aspects, a population of cells described herein comprises NK cells and ILC3 cells in a ratio of 20:1. In certain aspects, a population of cells described herein comprises NK cells and ILC3 cells in a ratio of 10:1. In certain aspects, a population of cells described herein comprises NK cells and ILC3 cells in a ratio of 5:1. In certain aspects, a population of cells described herein comprises NK cells and ILC3 cells in a ratio of 1:1. In certain aspects, a population of cells described herein comprises NK cells and ILC3 cells in a ratio of 1:5. In certain aspects, a population of cells described herein comprises NK cells and ILC3 cells in a ratio of 1:10. In certain aspects, a population of cells described herein comprises NK cells and ILC3 cells in a ratio of 1:20. In certain aspects, a population of cells described herein comprises NK cells and ILC3 cells in a ratio of 1:50.

In certain aspects, cell populations described herein are produced by combining the NK cells with the ILC3 cells in a ratio of 50:1, 40:1, 30:1, 20:1, 10:1, 5:1, 4:1, 3:1, 2:1, 1:1, 1:2, 1:3, 1:4, 1:5, 1:10, 1:20, 1:30, 1:40, or 1:50 to produce a combined population of cells. In certain aspects, a combined population of cells described herein comprises NK cells and ILC3 cells combined in a ratio of 50:1. In certain aspects, a combined population of cells described herein comprises NK cells and ILC3 cells combined in a ratio of 20:1. In certain aspects, a combined population of cells described herein comprises NK cells and ILC3 cells combined in a ratio of 10:1. In certain aspects, a combined population of cells described herein comprises NK cells and ILC3 cells combined in a ratio of 5:1. In certain aspects, a combined population of cells described herein comprises NK cells and ILC3 cells combined in a ratio of 1:1. In certain aspects, a combined population of cells described herein comprises NK cells and ILC3 cells combined in a ratio of 1:5. In certain aspects, a combined population of cells described herein comprises NK cells and ILC3 cells combined in a ratio of 1:10. In certain aspects, a combined population of cells described herein comprises NK cells and ILC3 cells combined in a ratio of 1:20. In certain aspects, a combined population of cells described herein comprises NK cells and ILC3 cells combined in a ratio of 1:50.

### 5.8. NK Cells and/or ILC3 Cells In Combination With Placental Perfusate

Disclosed herein are compositions comprising NK cells and/or ILC3 cells according to the three-stage method described herein, in combination with placental perfusate, placental perfusate cells and/or adherent placental cells, e.g., for use in suppressing the proliferation of a tumor cell or plurality of tumor cells.

### 5.8.1. Combinations of NK Cells and/or ILC3 Cells and Perfusate or Perfusate Cells

Disclosed herein are compositions comprising combinations of NK cell and/or ILC3 cell populations produced according to the three-stage method described herein, and placental perfusate and/or placental perfusate cells. In one example, for example, provided herein is a volume of placental perfusate supplemented with NK cells and/or ILC3 cells produced using the methods described herein. In specific examples of a volume of placental perfusate supplemented with NK cells and II,C3 cells, the NK cells and II,C3 cells are present in ratios as described herein. In specific examples, for example, each milliliter of placental perfusate is supplemented with about 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸ or more NK cells and/or ILC3 cells produced using the methods described herein. In another example, placental perfusate cells are supplemented with NK cells and/or ILC3 cells produced using the methods described herein. In certain other examples, when placental perfusate cells are combined with NK cells and/or ILC3 cells produced using the methods described herein, the placental perfusate cells generally comprise about, greater than about, or fewer than about, 50%, 45%, 40%, 35%, 30%, 25%, 20%, 15%, 10%, 8%, 6%, 4%, 2% or 1% of the total number of cells. In certain other examples, when NK cells and/or ILC3 cells produced using the methods described herein are combined with a plurality of placental perfusate cells and/or combined natural killer cells, the NK cells and/or ILC3 cells or NK cell populations generally comprise about, greater than about, or fewer than about, 50%, 45%, 40%, 35%, 30%, 25%, 20%, 15%, 10%, 8%, 6%, 4%, 2% or 1% of the total number of cells. In certain other examples, when NK cells and/or ILC3 cells produced using the methods described herein are used to supplement placental perfusate, the volume of solution (e.g., saline solution, culture medium or the like) in which the cells are suspended comprises about, greater than about, or less than about, 50%, 45%, 40%, 35%, 30%, 25%, 20%, 15%, 10%, 8%, 6%, 4%, 2% or 1% of the total volume of perfusate plus cells, where the NK cells and/or ILC3 cells are suspended to about 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸ or more cells per milliliter prior to supplementation.

In other examples, any of the above combinations of cells is, in turn, combined with umbilical cord blood or nucleated cells from umbilical cord blood.

Disclosed herein is pooled placental perfusate that is obtained from two or more sources, *e.g.,* two or more placentas, and combined, *e.g.,* pooled. Such pooled perfusate can comprise approximately equal volumes of perfusate from each source, or can comprise different volumes from each source. The relative volumes from each source can be randomly selected, or can be based upon, *e.g.,* a concentration or amount of one or more cellular factors, *e*.*g*., cytokines, growth factors, hormones, or the like; the number of placental cells in perfusate from each source; or other characteristics of the perfusate from each source. Perfusate from multiple perfusions of the same placenta can similarly be pooled.

Similarly, disclosed herein are placental perfusate cells, and placenta-derived intermediate natural killer cells, that are obtained from two or more sources, *e*.*g*., two or more placentas, and pooled. Such pooled cells can comprise approximately equal numbers of cells from the two or more sources, or different numbers of cells from one or more of the pooled sources. The relative numbers of cells from each source can be selected based on, *e.g.,* the number of one or more specific cell types in the cells to be pooled, *e.g.,* the number of CD34⁺ cells, *etc.*

Disclosed herein are NK cells and/or ILC3 cells produced using the methods described herein, and combinations of such cells with placental perfusate and/or placental perfusate cells, that have been assayed to determine the degree or amount of tumor suppression (that is, the potency) to be expected from, *e*.*g*., a given number of NK cells and/or ILC3 cells or NK cell and/or ILC3 cell populations or a given volume of perfusate. For example, an aliquot or sample number of cells is contacted or brought into proximity with a known number of tumor cells under conditions in which the tumor cells would otherwise proliferate, and the rate of proliferation of the tumor cells in the presence of placental perfusate, perfusate cells, placental natural killer cells, or combinations thereof, over time (*e*.*g*., 1, 2, 3, 4, 5, 6, 7, 8, 9, or 10 weeks, or longer) is compared to the proliferation of an equivalent number of the tumor cells in the absence of perfusate, perfusate cells, placental natural killer cells, or combinations thereof. The potency of the cells can be expressed, *e*.*g*., as the number of cells or volume of solution required to suppress tumor cell growth, *e*.*g*., by about 10%, 15%, 20%, 25%, 30%, 35%, 40%, 45%, 50%, or the like.

In certain examples, NK cells and/or ILC3 cells produced using the methods described herein, are provided as pharmaceutical grade administrable units. Such units can be provided in discrete volumes, *e.g.,* 15 mL, 20 mL, 25 mL, 30 nL. 35 mL, 40 mL, 45 mL, 50 mL, 55 mL, 60 mL, 65 mL, 70 mL, 75 mL, 80 mL, 85 mL, 90 mL, 95 mL, 100 mL, 150 mL, 200 mL, 250 mL, 300 mL, 350 mL, 400 mL, 450 mL, 500 mL, or the like. Such units can be provided so as to contain a specified number of cells, *e.g.,* NK cells and/or ILC3 cells or NK cell and/or ILC3 cell populations in combination with other NK cells and/or ILC3 cells or perfusate cells, *e.g.,* 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸ or more cells per milliliter, or 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸, 1 × 10⁹, 5 × 10⁹, 1 × 10 ¹⁰, 5 × 10¹⁰, 1 × 10 ¹¹ or more cells per unit. In specific examples, the units can comprise about, at least about, or at most about 1 × 10⁴, 5 × 10⁴, 1 × 10⁵, 5 × 10⁵, 1 × 10⁶, 5 × 10⁶ or more NK cells per milliliter, or 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸, 1 × 10⁹, 5 × 10⁹, 1 × 10¹⁰, 5 × 10¹⁰, 1 × 10¹¹ or more cells per unit. Such units can be provided to contain specified numbers of NK cells and/or ILC3 cells or NK cell and/or ILC3 cell populations and/or any of the other cells.

In the above examples, the NK cells and/or ILC3 cells or NK cell and/or ILC3 cell populations or combinations of NK cells and/or ILC3 cells or NK cell and/or ILC3 cell populations with other NK cells and/or ILC3 cells, perfusate cells or perfusate can be autologous to a recipient (that is, obtained from the recipient), or allogeneic to a recipient (that is, obtained from at last one other individual from said recipient).

In certain examples, each unit of cells is labeled to specify one or more of volume, number of cells, type of cells, whether the unit has been enriched for a particular type of cell, and/or potency of a given number of cells in the unit, or a given number of milliliters of the unit, that is, whether the cells in the unit cause a measurable suppression of proliferation of a particular type or types of tumor cell.

### 5.8.2. Combinations of NK Cells and/or ILC3 Cells With Adherent Placental Stem Cells

In other examples, the NK cells and/or ILC3 cells produced using the methods described herein, *e*.*g*., NK cell and/or ILC3 cell populations produced using the three-stage method described herein, either alone or in combination with placental perfusate or placental perfusate cells, are supplemented with isolated adherent placental cells, *e*.*g*., placental stem cells and placental multipotent cells as described, *e*.*g*., in Hariri U.S. Patent Nos. 7,045,148 and 7,255,879, and in U.S. Patent Application Publication No. 2007/0275362.

In specific examples, NK cells and II,C3 cells, the NK cells and II,C3 cells are present in ratios as described herein. "Adherent placental cells" means that the cells are adherent to a tissue culture surface, *e*.*g*., tissue culture plastic. The adherent placental cells useful in the compositions and methods disclosed herein are generally not trophoblasts, embryonic germ cells or embryonic stem cells.

The NK cells and/or ILC3 cells produced using the methods described herein, e.g., NK cell and/or II,C3 cell populations, either alone or in combination with placental perfusate or placental perfusate cells can be supplemented with, *e*.*g*., 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸ or more adherent placental cells per milliliter, or 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸, 1 × 10⁹, 5 × 10⁹, 1 × 10¹⁰, 5 × 10¹⁰, 1 × 10¹¹ or more adherent placental cells. The adherent placental cells in the combinations can be, *e.g.,* adherent placental cells that have been cultured for, *e.g.,* 1, 2, 3, 4, 5, 6, 7, 8, 9, 10, 12, 14, 16, 18, 20, 22, 24, 26, 28, 30, 32, 34, 36, 38, or 40 population doublings, or more.

Isolated adherent placental cells, when cultured in primary cultures or expanded in cell culture, adhere to the tissue culture substrate, *e*.*g*., tissue culture container surface (*e.g*., tissue culture plastic). Adherent placental cells in culture assume a generally fibroblastoid, stellate appearance, with a number of cytoplasmic processes extending from the central cell body. Adherent placental cells are, however, morphologically distinguishable from fibroblasts cultured under the same conditions, as the adherent placental cells exhibit a greater number of such processes than do fibroblasts. Morphologically, adherent placental cells are also distinguishable from hematopoietic stem cells, which generally assume a more rounded, or cobblestone, morphology in culture.

The isolated adherent placental cells, and populations of adherent placental cells, useful in the compositions and methods provided herein, express a plurality of markers that can be used to identify and/or isolate the cells, or populations of cells that comprise the adherent placental cells. The adherent placental cells, and adherent placental cell populations useful in the compositions and methods provided herein include adherent placental cells and adherent placental cell-containing cell populations obtained directly from the placenta, or any part thereof (*e*.*g*., amnion, chorion, amnion-chorion plate, placental cotyledons, umbilical cord, and the like). The adherent placental stem cell population, in one embodiment, is a population (that is, two or more) of adherent placental stem cells in culture, *e.g.,* a population in a container, *e*.*g*., a bag.

The adherent placental cells generally express the markers CD73, CD105, and CD200, and/or OCT-4, and do not express CD34, CD38, or CD45. Adherent placental stem cells can also express HLA-ABC (MHC-1) and HLA-DR. These markers can be used to identify adherent placental cells, and to distinguish the adherent placental cells from other cell types. Because the adherent placental cells can express CD73 and CD105, they can have mesenchymal stem cell-like characteristics. Lack of expression of CD34, CD38 and/or CD45 identifies the adherent placental stem cells as non-hematopoietic stem cells.

In certain examples, the isolated adherent placental cells described herein detectably suppress cancer cell proliferation or tumor growth.

In certain examples, the isolated adherent placental cells are isolated placental stem cells. In certain other examples, the isolated adherent placental cells are isolated placental multipotent cells. In a specific example, the isolated adherent placental cells are CD34⁻, CD10⁺ and CD105⁺ as detected by flow cytometry. In a more specific embodiment, the isolated CD34⁻, CD10⁺, CD105⁺ adherent placental cells are placental stem cells. In another more specific example, the isolated CD34⁻, CD10⁺, CD105⁺ placental cells are multipotent adherent placental cells. In another specific example, the isolated CD34⁻, CD10⁺, CD105⁺ placental cells have the potential to differentiate into cells of a neural phenotype, cells of an osteogenic phenotype, or cells of a chondrogenic phenotype. In a more specific example, the isolated CD34⁻, CD10⁺, CD105⁺ adherent placental cells are additionally CD200⁺. In another more specific example, the isolated CD34⁻, CD10⁺, CD105⁺ adherent placental cells are additionally CD90⁺ or CD45⁻, as detected by flow cytometry. In another more specific example, the isolated CD34⁻, CD10⁺, CD105⁺ adherent placental cells are additionally CD90⁺ or CD45⁻, as detected by flow cytometry. In a more specific example, the CD34⁻, CD10⁺, CD105⁺, CD200⁺ adherent placental cells are additionally CD90⁺ or CD45⁻, as detected by flow cytometry. In another more specific example, the CD34⁻, CD10⁺, CD105⁺, CD200⁺ adherent placental cells are additionally CD90⁺ and CD45⁻, as detected by flow cytometry. In another more specific example, the CD34⁻, CD10⁺, CD105⁺, CD200⁺, CD90⁺, CD45⁻ adherent placental cells are additionally CD80⁻ and CD86⁻, as detected by flow cytometry.

In one example, the isolated adherent placental cells are CD200⁺, HLA-G⁺. In a specific example, said isolated adherent placental cells are also CD73⁺ and CD105⁺. In another specific example, said isolated adherent placental cells are also CD34⁻, CD38⁻ or CD45⁻. In a more specific example, said isolated adherent placental cells are also CD34⁻, CD38⁻, CD45⁻, CD73⁺ and CD105⁺. In another example, said isolated adherent placental cells produce one or more embryoid-like bodies when cultured under conditions that allow the formation of embryoid-like bodies.

In another example, the isolated adherent placental cells are CD73⁺, CD105⁺, CD200⁺. In a specific examples of said populations, said isolated adherent placental cells are also HLA-G⁺. In another specific example, said isolated adherent placental cells are also CD34⁻, CD38⁻ or CD45⁻. In another specific example, said isolated adherent placental cells are also CD34⁻, CD38⁻ and CD45⁻. In a more specific example, said isolated adherent placental cells are also CD34⁻, CD38⁻, CD45⁻, and HLA-G⁺. In another specific example, said isolated adherent placental cells produce one or more embryoid-like bodies when cultured under conditions that allow the formation of embryoid-like bodies.

In another example, the isolated adherent placental cells are CD200⁺, OCT-4⁺. In a specific example, said isolated adherent placental cells are also CD73⁺ and CD105⁺. In another specific example, said isolated adherent placental cells are also HLA-G⁺. In another specific example, said isolated adherent placental cells are also CD34⁻, CD38⁻ and CD45⁻. In a more specific example, said isolated adherent placental cells are also CD34⁻, CD38⁻, CD45⁻, CD73⁺, CD105⁺ and HLA-G⁺. In another specific example, the isolated adherent placental cells also produce one or more embryoid-like bodies when cultured under conditions that allow the formation of embryoid-like bodies.

In another example, the isolated adherent placental cells are CD73⁺, CD105⁺ and HLA-G⁺. In a specific example, said isolated adherent placental cells are also CD34⁻, CD38⁻ or CD45⁻. In another specific example, said isolated adherent placental cells also CD34⁻, CD38⁻ and CD45⁻. In another specific example, said adherent stem cells are also OCT-4. In another specific example, said adherent stem cells are also CD200⁺. In a more specific embodiment, said adherent stem cells are also CD34⁻, CD38⁻, CD45⁻, OCT-4⁺ and CD200⁺.

In another example, the isolated adherent placental cells are CD73⁺, CD105⁺ stem cells, wherein said cells produce one or more embryoid-like bodies under conditions that allow formation of embryoid-like bodies. In a specific example, said isolated adherent placental cells are also CD34⁻, CD38⁻ or CD45⁻. In another specific example, isolated adherent placental cells are also CD34⁻, CD38⁻ and CD45⁻. In another specific example, isolated adherent placental cells are also OCT-4⁺. In a more specific example, said isolated adherent placental cells are also OCT-4⁺, CD34⁻, CD38⁻ and CD45⁻.

In another example, the adherent placental stem cells are OCT-4⁺ stem cells, wherein said adherent placental stem cells produce one or more embryoid-like bodies when cultured under conditions that allow the formation of embryoid-like bodies, and wherein said stem cells have been identified as detectably suppressing cancer cell proliferation or tumor growth.

In various examples, at least 10%, at least 20%, at least 30%, at least 40%, at least 50% at least 60%, at least 70%, at least 80%, at least 90%, or at least 95% of said isolated adherent placental cells are OCT-4⁺. In a specific example, of the above populations, said isolated adherent placental cells are also CD73⁺ and CD105⁺. In another specific example, said isolated adherent placental cells are also CD34⁻, CD38⁻, or CD45⁻. In another specific example, said stem cells are CD200⁺. In a more specific example, said isolated adherent placental cells are also CD73⁺, CD105⁺, CD200⁺, CD34⁻, CD38⁻, and CD45⁻. In another specific example, said isolated adherent placental cells have been expanded, for example, passaged at least once, at least three times, at least five times, at least 10 times, at least 15 times, or at least 20 times.

In a more specific example, of any of the above embodiments, the isolated adherent placental cells express ABC-p (a placenta-specific ABC transporter protein; *see, e.g.,* Allikmets et al., Cancer Res. 58(23):5337-9 (1998)).

In another example, the isolated adherent placental cells CD29⁺, CD44⁺, CD73⁺, CD90⁺, CD105⁺, CD200⁺, CD34⁺ and CD133⁻. In another example, the isolated adherent placental cells constitutively secrete IL-6, IL-8 and monocyte chemoattractant protein (MCP-1).

Each of the above-referenced isolated adherent placental cells can comprise cells obtained and isolated directly from a mammalian placenta, or cells that have been cultured and passaged at least 1, 2, 3, 4, 5, 6, 7, 8, 9, 10, 12, 14, 16, 18, 20, 25, 30 or more times, or a combination thereof. Tumor cell suppressive pluralities of the isolated adherent placental cells described above can comprise about, at least, or no more than, 1 × 10⁵, 5 × 10⁵, 1 × 10⁶, 5 × 10⁶, 1 × 10⁷, 5 × 10⁷, 1 × 10⁸, 5 × 10⁸, 1 × 10⁹, 5 × 10⁹, 1 × 10¹⁰, 5 × 10¹⁰, 1 × 10¹¹ or more isolated adherent placental cells.

### 5.8.3. Compositions Comprising Adherent Placental Cell Conditioned Media

Disclosed herein is the use of a composition comprising NK cells and/or ILC3 cells produced using the methods described herein, *e.g.,* NK cell and/or ILC3 cell populations produced using the three-stage method described herein, and additionally conditioned medium, wherein said composition is tumor suppressive, or is effective in the treatment of cancer or viral infection. In specific examples, the NK cells and ILC3 cells are present in ratios as described herein. Adherent placental cells as described herein can be used to produce conditioned medium that is tumor cell suppressive, anti-cancer or anti-viral that is, medium comprising one or more biomolecules secreted or excreted by the cells that have a detectable tumor cell suppressive effect, anti-cancer effect or antiviral effect. In various examples, the conditioned medium comprises medium in which the cells have proliferated (that is, have been cultured) for at least 1, 2, 3, 4, 5, 6, 7, 8, 9, 10, 11, 12, 13, 14 or more days. In other examples, the conditioned medium comprises medium in which such cells have grown to at least 30%, 40%, 50%, 60%, 70%, 80%, 90% confluence, or up to 100% confluence. Such conditioned medium can be used to support the culture of a separate population of cells, e.g., example, placental cells, or cells of another kind. In another example, the conditioned medium provided herein comprises medium in which isolated adherent placental cells, e.g., isolated adherent placental stem cells or isolated adherent placental multipotent cells, and cells other than isolated adherent placental cells, e.g., non-placental stem cells or multipotent cells, have been cultured.

Such conditioned medium can be combined with any of, or any combination of NK cells and/or ILC3 cells produced using the methods described herein, placental perfusate, or placental perfusate cells to form a composition that is tumor cell suppressive, anticancer or antiviral. In certain examples, the composition comprises less than half conditioned medium by volume, e.g., about, or less than about, 50%, 45%, 40%, 35%, 30%, 25%, 20%, 15%, 10%, 5%, 4%, 3%, 2%, or 1% by volume.

Thus, in one example, provided herein is a composition comprising NK cells and/or ILC3 cells produced using the methods described herein and culture medium from a culture of isolated adherent placental cells, wherein said isolated adherent placental cells (a) adhere to a substrate; and (b) are CD34⁻, CD10⁺ and CD105⁺; wherein said composition detectably suppresses the growth or proliferation of tumor cells, or is anti-cancer or antiviral. In a specific example, the isolated adherent placental cells are CD34⁻, CD10⁺ and CD105⁺ as detected by flow cytometry. In a more specific example, the isolated CD34⁻, CD10⁺, CD105⁺ adherent placental cells are placental stem cells. In another more specific example, the isolated CD34⁻, CD10⁺, CD105⁺ placental cells are multipotent adherent placental cells. In another specific example, the isolated CD34⁻, CD10⁺, CD105⁺ placental cells have the potential to differentiate into cells of a neural phenotype, cells of an osteogenic phenotype, or cells of a chondrogenic phenotype. In a more specific example, the isolated CD34⁻, CD10⁺, CD105⁺ adherent placental cells are additionally CD200⁺. In another more specific example, the isolated CD34⁻, CD10⁺, CD105⁺ adherent placental cells are additionally CD90⁺ or CD45⁻, as detected by flow cytometry. In another more specific example, the isolated CD34⁻, CD10⁺, CD105⁺ adherent placental cells are additionally CD90⁺ or CD45⁻, as detected by flow cytometry. In a more specific example, the CD34⁻, CD10⁺, CD105⁺, CD200⁺ adherent placental cells are additionally CD90⁺ or CD45⁻, as detected by flow cytometry. In another more specific example, the CD34⁻, CD10⁺, CD105⁺, CD200⁺ adherent placental cells are additionally CD90⁺ and CD45⁻, as detected by flow cytometry. In another more specific example, the CD34⁻, CD10⁺, CD105⁺, CD200⁺, CD90⁺, CD45⁻ adherent placental cells are additionally CD80⁻ and CD86⁻, as detected by flow cytometry.

In another example, provided herein is a composition comprising NK cells and/or ILC3 cells produced using the methods described herein, and culture medium from a culture of isolated adherent placental cells, wherein said isolated adherent placental cells (a) adhere to a substrate; and (b) express CD200 and HLA-G, or express CD73, CD105, and CD200, or express CD200 and OCT-4, or express CD73, CD105, and HLA-G, or express CD73 and CD105 and facilitate the formation of one or more embryoid-like bodies in a population of placental cells that comprise the placental stem cells when said population is cultured under conditions that allow formation of embryoid-like bodies, or express OCT-4 and facilitate the formation of one or more embryoid-like bodies in a population of placental cells that comprise the placental stem cells when said population is cultured under conditions that allow formation of embryoid-like bodies; wherein said composition detectably suppresses the growth or proliferation of tumor cells, or is anti-cancer or antiviral. In a specific example, the composition further comprises a plurality of said isolated placental adherent cells. In another specific example, the composition comprises a plurality of non-placental cells. In a more specific example, said non-placental cells comprise CD34⁺ cells, e.g., hematopoietic progenitor cells, such as peripheral blood hematopoietic progenitor cells, cord blood hematopoietic progenitor cells, or placental blood hematopoietic progenitor cells. The non-placental cells can also comprise stem cells, such as mesenchymal stem cells, e.g., bone marrow-derived mesenchymal stem cells. The non-placental cells can also be one or more types of adult cells or cell lines. In another specific example, the composition comprises an antiproliferative agent, e.g., an anti-MIP-1α or anti-MIP-1β antibody.

In a specific example, culture medium conditioned by one of the cells or cell combinations described above is obtained from a plurality of isolated adherent placental cells co-cultured with a plurality of tumor cells at a ratio of about 1:1, about 2:1, about 3:1, about 4:1, or about 5:1 isolated adherent placental cells to tumor cells. For example, the conditioned culture medium or supernatant can be obtained from a culture comprising about 1 × 10⁵ isolated adherent placental cells, about 1 × 10⁶ isolated adherent placental cells, about 1 × 10⁷ isolated adherent placental cells, or about 1 × 10⁸ isolated adherent placental cells, or more. In another specific example, the conditioned culture medium or supernatant is obtained from a co-culture comprising about 1 × 10⁵ to about 5 × 10⁵ isolated adherent placental cells and about 1 × 10⁵ tumor cells; about 1 × 10⁶ to about 5 × 10⁶ isolated adherent placental cells and about 1 × 10⁶ tumor cells; about 1 × 10⁷ to about 5 × 10⁷ isolated adherent placental cells and about 1 × 10⁷ tumor cells; or about 1 × 10⁸ to about 5 × 10⁸ isolated adherent placental cells and about 1 × 10⁸ tumor cells.

### 5.9. Preservation of Cells

Cells, *e*.*g*., NK cells and/or ILC3 cells produced using the methods described herein, *e*.*g*., NK cell and/or ILC3 cell populations produced using the three-stage method described herein, or placental perfusate cells comprising hematopoietic stem cells or progenitor cells, can be preserved, that is, placed under conditions that allow for long-term storage, or under conditions that inhibit cell death by, *e*.*g*., apoptosis or necrosis.

Placental perfusate can be produced by passage of a cell collection composition through at least a part of the placenta, *e*.*g*., through the placental vasculature. The cell collection composition comprises one or more compounds that act to preserve cells contained within the perfusate. Such a placental cell collection composition can comprise an apoptosis inhibitor, necrosis inhibitor and/or an oxygen-carrying perfluorocarbon, as described in related U.S. Application Publication No. 20070190042,

In one embodiment, perfusate or a population of placental cells are collected from a mammalian, *e*.*g*., human, post-partum placenta by bringing the perfusate or population of cells into proximity with a cell collection composition comprising an inhibitor of apoptosis and an oxygen-carrying perfluorocarbon, wherein said inhibitor of apoptosis is present in an amount and for a time sufficient to reduce or prevent apoptosis in the population of placental cells, *e*.*g*., adherent placental cells, for example, placental stem cells or placental multipotent cells, as compared to a population of cells not contacted or brought into proximity with the inhibitor of apoptosis. For example, the placenta can be perfused with the cell collection composition, and placental cells, *e*.*g*., total nucleated placental cells, are isolated therefrom. In a specific embodiment, the inhibitor of apoptosis is a caspase inhibitor. In another specific embodiment, said inhibitor of apoptosis is a JNK inhibitor. In a more specific embodiment, said JNK inhibitor does not modulate differentiation or proliferation of adherent placental cells, *e*.*g*., adherent placental stem cells or adherent placental multipotent cells. In another embodiment, the cell collection composition comprises said inhibitor of apoptosis and said oxygen-carrying perfluorocarbon in separate phases. In another embodiment, the cell collection composition comprises said inhibitor of apoptosis and said oxygen-carrying perfluorocarbon in an emulsion. In another embodiment, the cell collection composition additionally comprises an emulsifier, *e*.*g*., lecithin. In another embodiment, said apoptosis inhibitor and said perfluorocarbon are between about 0 °C and about 25 °C at the time of bringing the placental cells into proximity with the cell collection composition. In another more specific embodiment, said apoptosis inhibitor and said perfluorocarbon are between about 2 °C and 10 °C, or between about 2 °C and about 5 °C, at the time of bringing the placental cells into proximity with the cell collection composition. In another more specific embodiment, said bringing into proximity is performed during transport of said population of cells. In another more specific embodiment, said bringing into proximity is performed during freezing and thawing of said population of cells.

In another embodiment, placental perfusate and/or placental cells can be collected and preserved by bringing the perfusate and/or cells into proximity with an inhibitor of apoptosis and an organ-preserving compound, wherein said inhibitor of apoptosis is present in an amount and for a time sufficient to reduce or prevent apoptosis of the cells, as compared to perfusate or placental cells not contacted or brought into proximity with the inhibitor of apoptosis. In a specific embodiment, the organ-preserving compound is UW solution (described in U.S. Patent No. 4,798,824; also known as VIASPAN^{™}; *see also* Southard et al., Transplantation 49(2):251-257 (1990) or a solution described in Stern et al., U.S. Patent No. 5,552,267 In another embodiment, said organ-preserving composition is hydroxyethyl starch, lactobionic acid, raffinose, or a combination thereof. In another embodiment, the placental cell collection composition additionally comprises an oxygen-carrying perfluorocarbon, either in two phases or as an emulsion.

In another embodiment of the method, placental cells are brought into proximity with a cell collection composition comprising an apoptosis inhibitor and oxygen-carrying perfluorocarbon, organ-preserving compound, or combination thereof, during perfusion. In another embodiment, placental cells are brought into proximity with said cell collection compound after collection by perfusion.

Typically, during placental cell collection, enrichment and isolation, it is preferable to minimize or eliminate cell stress due to hypoxia and mechanical stress. In another embodiment of the method, therefore, placental perfusate or a population of placental cells is exposed to a hypoxic condition during collection, enrichment or isolation for less than six hours during said preservation, wherein a hypoxic condition is a concentration of oxygen that is less than normal blood oxygen concentration. In a more specific embodiment, said perfusate or population of placental cells is exposed to said hypoxic condition for less than two hours during said preservation. In another more specific embodiment, said population of placental cells is exposed to said hypoxic condition for less than one hour, or less than thirty minutes, or is not exposed to a hypoxic condition, during collection, enrichment or isolation. In another specific embodiment, said population of placental cells is not exposed to shear stress during collection, enrichment or isolation.

Cells, *e.g.,* placental perfusate cells, hematopoietic cells, *e.g.,* CD34⁺ hematopoietic stem cells; NK cells and/or ILC3 cells produced using the methods described herein; isolated adherent placental cells provided herein can be cryopreserved, *e*.*g*., in cryopreservation medium in small containers, *e*.*g*., ampoules or septum vials. In certain embodiments, cells provided herein are cryopreserved at a concentration of about 1 × 10⁴ - 5 × 10⁸ cells per mL. In specific embodiments, cells provided herein are cryopreserved at a concentration of about 1 × 10⁶ - 1.5 × 10⁷ cells per mL. In more specific embodiments, cells provided herein are cryopreserved at a concentration of about 1 × 10⁴, 5 × 10⁴, 1 × 10⁵, 5 × 10⁵, 1 × 10⁶, 5 × 10⁶, 1 × 10⁷, 1.5 × 10⁷ cells per mL.

Suitable cryopreservation medium includes, but is not limited to, normal saline, culture medium including, *e*.*g*., growth medium, or cell freezing medium, for example commercially available cell freezing medium, *e*.*g*., C2695, C2639 or C6039 (Sigma); CryoStor^{®} CS2, CryoStor^{®} CS5 or CryoStor^{®}CS10 (BioLife Solutions). In one embodiment, cryopreservation medium comprises DMSO (dimethylsulfoxide), at a concentration of, *e.g.,* about 1, 2, 3, 4, 5, 6, 7, 8, 9 or 10% (v/v). Cryopreservation medium may comprise additional agents, for example, methylcellulose, dextran, albumin (*e*.*g*., human serum albumin), trehalose, and/or glycerol. In certain embodiments, the cryopreservation medium comprises about 1%-10% DMSO, about 25%-75% dextran and/or about 20-60% human serum albumin (HSA). In certain embodiments, the cryopreservation medium comprises about 1%-10% DMSO, about 25%-75% trehalose and/or about 20-60% human HSA. In a specific embodiment, the cryopreservation medium comprises 5% DMSO, 55% dextran and 40% HSA. In a more specific embodiment, the cryopreservation medium comprises 5% DMSO, 55% dextran (10% w/v in normal saline) and 40% HSA. In another specific embodiment, the cryopreservation medium comprises 5% DMSO, 55% trehalose and 40% HSA. In a more specific embodiment, the cryopreservation medium comprises 5% DMSO, 55% trehalose (10% w/v in normal saline) and 40% HSA. In another specific embodiment, the cryopreservation medium comprises CryoStor^{®} CS5. In another specific embodiment, the cryopreservation medium comprises CryoStor^{®}CS10.

Cells provided herein can be cryopreserved by any of a variety of methods, and at any stage of cell culturing, expansion or differentiation. For example, cells provided herein can be cryopreserved right after isolation from the origin tissues or organs, *e*.*g*., placental perfusate or umbilical cord blood, or during, or after either the first, second, or third step of the methods outlined above. In certain embodiments, the hematopoietic cells, *e*.*g*., hematopoietic stem or progenitor cells are cryopreserved within about 1, 5, 10, 15, 20, 30, 45 minutes or within about 1, 2, 4, 6, 10, 12, 18, 20 or 24 hours after isolation from the origin tissues or organs. In certain embodiments, said cells are cryopreserved within 1, 2 or 3 days after isolation from the origin tissues or organs. In certain embodiments, said cells are cryopreserved after being cultured in a first medium as described above, for about 1, 2, 3, 4, 5, 6, 7, 8, 9, 10, 11, 12, 13, 14, 15, 16, 17, 18, 19, 20, 21, 22, 23, 24, 25, 26, 27 or 28 days. In some embodiments, said cells are cryopreserved after being cultured in a first medium as described above, for about 1, 2, 3, 4, 5, 6, 7, 8, 9, 10, 11, 12, 13, 14, 15, 16, 17, 18, 19, 20, 21, 22, 23, 24, 25, 26, 27 or 28 days, and in a second medium for about 1, 2, 3, 4, 5, 6, 7, 8, 9, 10, 11, 12, 13, 14, 15, 16, 17, 18, 19, 20, 21, 22, 23, 24, 25, 26, 27 or 28 days as described above. In some embodiments, when NK cells are made using a three-stage method according to claims, said cells are cryopreserved after being cultured in a first medium about 1, 2, 3, 4, 5, 6, 7, 8, 9, 10, 11, 12, 13, 14, 15, 16, 17, 18, 19, 20, 21, 22, 23, 24, or 25 days; and/or after being cultured in a second medium about 1, 2, 3, 4, 5, 6, 7, 8, 9, 10, 11, 12, 13, 14, 15, 16, 17, 18, 19, 20, 21, 22, 23, 24, or 25 days; and/or after being cultured in a third medium about 1, 2, 3, 4, 5, 6, 7, 8, 9, 10, 11, 12, 13, 14, 15, 16, 17, 18, 19, 20, 21, 22, 23, 24, or 25 days. In a specific embodiment, NK cells and/or ILC3 cells are made using a three-stage method according to the claims, and said cells are cryopreserved after being cultured in a first medium for 10 days; after being cultured in a second medium for 4 days; and after being cultured in a third medium for 21 days.

In one aspect, provided herein is a method of cryopreserving a population of NK cells and/or ILC3 cells, e.g., NK cells and/or ILC3 cells produced by a three-stage method according to the claims. In one embodiment, said method comprises: culturing hematopoietic stem cells or progenitor cells, e.g., CD34⁺ stem cells or progenitor cells, in a first medium comprising a stem cell mobilizing agent and thrombopoietin (Tpo) to produce a first population of cells, subsequently culturing said first population of cells in a second medium comprising a stem cell mobilizing agent and interleukin-15 (IL-15), and lacking Tpo, to produce a second population of cells, and subsequently culturing said second population of cells in a third medium comprising IL-2 and IL-15, and lacking a stem cell mobilizing agent and LMWH, to produce a third population of cells, wherein the third population of cells comprises natural killer cells that are CD56+, CD3-, CD16- or CD16+, and CD94+ or CD94-, and wherein at least 80%, of the natural killer cells are viable, and next, cryopreserving the NK cells in a cryopreservation medium. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, e.g., low-molecular weight heparin. In a specific embodiment, said cryopreservation step further comprises (1) preparing a cell suspension solution; (2) adding cryopreservation medium to the cell suspension solution from step (1) to obtain cryopreserved cell suspension; (3) cooling the cryopreserved cell suspension from step (3) to obtain a cryopreserved sample; and (4) storing the cryopreserved sample below -80 °C. In certain embodiments, the method includes no intermediary steps.

In one example, said method comprises: (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising IL-2 and IL-15, and lacking LMWH, to produce a third population of cells; wherein the third population of cells comprises natural killer cells that are CD56+, CD3-, and CD11a+ and next, cryopreserving the NK cells in a cryopreservation medium. In certain examples, said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, e.g., low-molecular weight heparin. In a specific example, said cryopreservation step further comprises (1) preparing a cell suspension solution; (2) adding cryopreservation medium to the cell suspension solution from step (1) to obtain cryopreserved cell suspension; (3) cooling the cryopreserved cell suspension from step (3) to obtain a cryopreserved sample; and (4) storing the cryopreserved sample below - 80 °C. In certain examples, the method includes no intermediary steps.

In one embodiment, said method comprises: (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising IL-2 and IL-15, and lacking each of stem cell factor (SCF) and LMWH, to produce a third population of cells; wherein the third population of cells comprises natural killer cells that are CD56+, CD3-, and CD11a+ and next, cryopreserving the NK cells in a cryopreservation medium. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, *e*.*g*., low-molecular weight heparin. In a specific embodiment, said cryopreservation step further comprises (1) preparing a cell suspension solution; (2) adding cryopreservation medium to the cell suspension solution from step (1) to obtain cryopreserved cell suspension; (3) cooling the cryopreserved cell suspension from step (3) to obtain a cryopreserved sample; and (4) storing the cryopreserved sample below -80 °C. In certain embodiments, the method includes no intermediary steps.

In one embodiment, said method comprises: (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising IL-2 and IL-15, and lacking each of SCF, a stem cell mobilizing agent, and LMWH, to produce a third population of cells; wherein the third population of cells comprises natural killer cells that are CD56+, CD3-, and CD11a+ and next, cryopreserving the NK cells in a cryopreservation medium. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, e.g., low-molecular weight heparin. In a specific embodiment, said cryopreservation step further comprises (1) preparing a cell suspension solution; (2) adding cryopreservation medium to the cell suspension solution from step (1) to obtain cryopreserved cell suspension; (3) cooling the cryopreserved cell suspension from step (3) to obtain a cryopreserved sample; and (4) storing the cryopreserved sample below -80 °C. In certain embodiments, the method includes no intermediary steps.

In one embodiment, said method comprises: (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; (c) culturing the second population of cells in a third medium comprising IL-2 and IL-15, and lacking each of a stem cell mobilizing agent and LMWH, to produce a third population of cells; and (d) isolating CD11a+ cells from the third population of cells to produce a fourth population of cells; wherein the fourth population of cells comprises natural killer cells that are CD56+, CD3-, and CD11a+ and next, cryopreserving the NK cells in a cryopreservation medium. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, e.g., low-molecular weight heparin. In a specific embodiment, said cryopreservation step further comprises (1) preparing a cell suspension solution; (2) adding cryopreservation medium to the cell suspension solution from step (1) to obtain cryopreserved cell suspension; (3) cooling the cryopreserved cell suspension from step (3) to obtain a cryopreserved sample; and (4) storing the cryopreserved sample below -80 °C. In certain embodiments, the method includes no intermediary steps.

In one example, said method comprises: (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising IL-2 and IL-15, and lacking LMWH, to produce a third population of cells; wherein the third population of cells comprises ILC3 cells that are CD56+, CD3-, and CD11a- and next, cryopreserving the ILC3 cells in a cryopreservation medium. In certain examples, said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, e.g., low-molecular weight heparin. In a specific example, said cryopreservation step further comprises (1) preparing a cell suspension solution; (2) adding cryopreservation medium to the cell suspension solution from step (1) to obtain cryopreserved cell suspension; (3) cooling the cryopreserved cell suspension from step (3) to obtain a cryopreserved sample; and (4) storing the cryopreserved sample below -80 °C. In certain examples, the method includes no intermediary steps.

In one example, said method comprises: (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising a stem cell mobilizing agent, IL-2 and IL-15, and lacking LMWH, to produce a third population of cells; wherein the third population of cells comprises ILC3 cells that are CD56+, CD3-, and CD11a- and next, cryopreserving the ILC3 cells in a cryopreservation medium. In certain examples, said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, e.g., low-molecular weight heparin. In a specific example, said cryopreservation step further comprises (1) preparing a cell suspension solution; (2) adding cryopreservation medium to the cell suspension solution from step (1) to obtain cryopreserved cell suspension; (3) cooling the cryopreserved cell suspension from step (3) to obtain a cryopreserved sample; and (4) storing the cryopreserved sample below -80 °C. In certain examples, the method includes no intermediary steps.

In one example, said method comprises: (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising SCF, IL-2 and IL-15, and lacking LMWH, to produce a third population of cells; wherein the third population of cells comprises ILC3 cells that are CD56+, CD3-, and CD11a- and next, cryopreserving the ILC3 cells in a cryopreservation medium. In certain examples, said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, e.g., low-molecular weight heparin. In a specific example, said cryopreservation step further comprises (1) preparing a cell suspension solution; (2) adding cryopreservation medium to the cell suspension solution from step (1) to obtain cryopreserved cell suspension; (3) cooling the cryopreserved cell suspension from step (3) to obtain a cryopreserved sample; and (4) storing the cryopreserved sample below-80 °C. In certain examples, the method includes no intermediary steps.

In one example, said method comprises: (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; and (c) culturing the second population of cells in a third medium comprising a stem cell mobilizing agent, SCF, IL-2 and IL-15, and lacking LMWH, to produce a third population of cells; wherein the third population of cells comprises ILC3 cells that are CD56+, CD3-, and CD11a- and next, cryopreserving the ILC3 cells in a cryopreservation medium. In certain examples, said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, e.g., low-molecular weight heparin. In a specific example, said cryopreservation step further comprises (1) preparing a cell suspension solution; (2) adding cryopreservation medium to the cell suspension solution from step (1) to obtain cryopreserved cell suspension; (3) cooling the cryopreserved cell suspension from step (3) to obtain a cryopreserved sample; and (4) storing the cryopreserved sample below -80 °C. In certain examples, the method includes no intermediary steps.

In one embodiment, said method comprises: (a) culturing hematopoietic stem or progenitor cells in a first medium comprising a stem cell mobilizing agent and thrombopoietin (Tpo) to produce a first population of cells; (b) culturing the first population of cells in a second medium comprising a stem cell mobilizing agent and interleukin-15 (IL-15), and lacking Tpo, to produce a second population of cells; (c) culturing the second population of cells in a third medium comprising IL-2 and IL-15, and lacking each of a stem cell mobilizing agent and LMWH, to produce a third population of cells; and (d) isolating CD11a- cells from the third population of cells to produce a fourth population of cells; wherein the fourth population of cells comprises ILC3 cells that are CD56+, CD3-, and CD11a- and next, cryopreserving the ILC3 cells in a cryopreservation medium. In certain embodiments, said first medium and/or said second medium lack leukemia inhibiting factor (LIF) and/or macrophage inflammatory protein-1 alpha (MIP-1α). In certain embodiments, said third medium lacks LIF, MIP-1α, and FMS-like tyrosine kinase-3 ligand (Flt-3L). In specific embodiments, said first medium and said second medium lack LIF and MIP-1α, and said third medium lacks LIF, MIP-1α, and Flt3L. In certain embodiments, none of the first medium, second medium or third medium comprises heparin, e.g., low-molecular weight heparin. In a specific embodiment, said cryopreservation step further comprises (1) preparing a cell suspension solution; (2) adding cryopreservation medium to the cell suspension solution from step (1) to obtain cryopreserved cell suspension; (3) cooling the cryopreserved cell suspension from step (3) to obtain a cryopreserved sample; and (4) storing the cryopreserved sample below -80 °C. In certain embodiments, the method includes no intermediary steps.

Cells provided herein can be cooled in a controlled-rate freezer, e.g., at about 0.1, 0.3, 0.5, 1, or 2 °C/min during cryopreservation. In one embodiment, the cryopreservation temperature is about -80 °C to about -180 °C, or about -125 °C to about -140 °C. Cryopreserved cells can be transferred to liquid nitrogen prior to thawing for use. In some embodiments, for example, once the ampoules have reached about -90 °C, they are transferred to a liquid nitrogen storage area. Cryopreserved cells can be thawed at a temperature of about 25 °C to about 40 °C, more specifically can be thawed to a temperature of about 37 °C. In certain embodiments, the cryopreserved cells are thawed after being cryopreserved for about 1, 2, 4, 6, 10, 12, 18, 20 or 24 hours, or for about 1, 2, 3, 4, 5, 6, 7, 8, 9, 10, 11, 12, 13, 14, 15, 16, 17, 18, 19, 20, 21, 22, 23, 24, 25, 26, 27 or 28 days. In certain embodiments, the cryopreserved cells are thawed after being cryopreserved for about 1, 2, 3, 4, 5, 6, 7, 8, 9, 10, 11, 12, 13, 14, 15, 16, 17, 18, 19, 20, 21, 22, 23, 24, 25, 26, 27 or 28 months. In certain embodiments, the cryopreserved cells are thawed after being cryopreserved for about 1, 2, 3, 4, 5, 6, 7, 8, 9 or 10 years.

Suitable thawing medium includes, but is not limited to, normal saline, plasmalyte culture medium including, for example, growth medium, e.g., RPMI medium. In certain embodiments, the thawing medium comprises one or more of medium supplements (e.g., nutrients, cytokines and/or factors). Medium supplements suitable for thawing cells provided herein include, for example without limitation, serum such as human serum AB, fetal bovine serum (FBS) or fetal calf serum (FCS), vitamins, human serum albumin (HSA), bovine serum albumin (BSA), amino acids *(e.g.,* L-glutamine), fatty acids *(e.g.,* oleic acid, linoleic acid or palmitic acid), insulin (e.g., recombinant human insulin), transferrin (iron saturated human transferrin), β-mercaptoethanol, stem cell factor (SCF), Fms-like-tyrosine kinase 3 ligand (Flt3-L), cytokines such as interleukin-2 (IL-2), interleukin-7 (IL-7), interleukin-15 (IL-15), thrombopoietin (Tpo) or heparin. In a specific embodiment, the thawing medium useful in the methods provided herein comprises RPMI. In another specific embodiment, said thawing medium comprises plasmalyte. In another specific embodiment, said thawing medium comprises about 0.5-20% FBS. In another specific embodiment, said thawing medium comprises about 1, 2, 5, 10, 15 or 20% FBS. In another specific embodiment, said thawing medium comprises about 0.5%-20% HSA. In another specific embodiment, said thawing medium comprises about 1, 2.5, 5, 10, 15, or 20% HSA. In a more specific embodiment, said thawing medium comprises RPMI and about 10% FBS. In another more specific embodiment, said thawing medium comprises plasmalyte and about 5% HSA.

The cryopreservation methods provided herein can be optimized to allow for long-term storage, or under conditions that inhibit cell death by, *e*.*g*., apoptosis or necrosis. In one embodiments, the post-thaw cells comprise greater than 60%, 65%, 70%, 75%, 80%, 85%, 90%, 95% or 98% of viable cells, as determined by, *e*.*g*., automatic cell counter or trypan blue method. In another embodiment, the post-thaw cells comprise about 0.5, 1, 5, 10, 15, 20 or 25% of dead cells. In another embodiment, the post-thaw cells comprise about 0.5, 1, 5, 10, 15, 20 or 25% of early apoptotic cells. In another embodiment, about 0.5, 1, 5, 10, 15 or 20% of post-thaw cells undergo apoptosis after 1, 2, 3, 4, 5, 6, 7, 8, 9, 10, 11, 12, 13, 14, 15, 16, 17, 18, 19, 20, 21, 22, 23, 24, 25, 26, 27 or 28 days after being thawed, *e*.*g*., as determined by an apoptosis assay *(e.g.,* TO-PRO3 or AnnV/PI Apoptosis assay kit). In certain embodiments, the post-thaw cells are re-cryopreserved after being cultured, expanded or differentiated using methods provided herein.

### 5.10. Compositions Comprising NK Cells and/or ILC3 Cells

### 5.10.1. NK Cells and/or ILC3 Cells Produced Using The Three-Stage Method

Disclosed herein is a composition, *e*.*g*., a pharmaceutical composition, comprising an isolated NK cell and/or ILC3 cell population produced using the three-stage method described herein. In a specific example, said isolated NK cell and/or ILC3 cell population is produced from hematopoietic cells, *e*.*g*., hematopoietic stem or progenitor cells isolated from placental perfusate, umbilical cord blood, and/or peripheral blood. In another specific example, said isolated NK cell and/or ILC3 cell population comprises at least 50% of cells in the composition. In another specific example, said isolated NK cell and/or ILC3 cell population, *e*.*g*., CD3⁻CD56⁺ cells, comprises at least 80%, 85%, 90%. 95%, 98% or 99% of cells in the composition. In certain examples, no more than 5%, 10%, 15%, 20%, 25%, 30%, 35%, or 40% of the cells in said isolated NK cell and/or ILC3 cell population are CD3⁻CD56⁺ cells. In certain examples, said CD3⁻CD56⁺ cells are CD16⁻.

NK cell and/or ILC3 cell populations produced using the three-stage method described herein, can be formulated into pharmaceutical compositions for use *in vivo.* Such pharmaceutical compositions comprise a population of NK cells and/or ILC3 cells in a pharmaceutically-acceptable carrier, *e.g.,* a saline solution or other accepted physiologically-acceptable solution for *in vivo* administration. Pharmaceutical compositions can comprise any of the NK cell and/or ILC3 cell populations described elsewhere herein.

The pharmaceutical compositions comprise populations of cells that comprise 50% viable cells or more (that is, at least 50% of the cells *in* the population are functional or living). Preferably, at least 60% of the cells in the population are viable. More preferably, at least 70%, 80%, 90%, 95%, or 99% of the cells in the population in the pharmaceutical composition are viable.

The pharmaceutical compositions can comprise one or more compounds that, *e*.*g*., facilitate engraftment; stabilizers such as albumin, dextran 40, gelatin, hydroxyethyl starch, and the like.

When formulated as an injectable solution, in one example, the pharmaceutical composition of the invention comprises about 1.25% HSA and about 2.5% dextran. Other injectable formulations, suitable for the administration of cellular products, may be used.

In one example, the compositions, *e*.*g*., pharmaceutical compositions, provided herein are suitable for systemic or local administration. In specific examples, the compositions, *e*.*g*., pharmaceutical compositions, provided herein are suitable for parenteral administration. In specific examples, the compositions, *e*.*g*., pharmaceutical compositions, provided herein are suitable for injection, infusion, intravenous (IV) administration, intrafemoral administration, or intratumor administration. In specific examples, the compositions, *e*.*g*., pharmaceutical compositions, provided herein are suitable for administration via a device, a matrix, or a scaffold. In specific examples, the compositions, *e*.*g*., pharmaceutical compositions provided herein are suitable for injection. In specific examples, the compositions, *e*.*g*., pharmaceutical compositions, provided herein are suitable for administration via a catheter. In specific examples, the compositions, *e*.*g*., pharmaceutical compositions, provided herein are suitable for local injection. In more specific examples, the compositions, *e*.*g*., pharmaceutical compositions, provided herein are suitable for local injection directly into a solid tumor *(e.g.,* a sarcoma). In specific examples, the compositions, *e*.*g*., pharmaceutical compositions, provided herein are suitable for injection by syringe. In specific examples, the compositions, *e*.*g*., pharmaceutical compositions, provided herein are suitable for administration via guided delivery. In specific examples, the compositions, *e*.*g*., pharmaceutical compositions, provided herein are suitable for injection aided by laparoscopy, endoscopy, ultrasound, computed tomography, magnetic resonance, or radiology.

In certain examples, the compositions, *e*.*g*., pharmaceutical compositions provided herein, comprising NK cells and/or ILC3 cells produced using the methods described herein, are provided as pharmaceutical grade administrable units. Such units can be provided in discrete volumes, e.g., 15 mL, 20 mL, 25 mL, 30 nL. 35 mL, 40 mL, 45 mL, 50 mL, 55 mL, 60 mL, 65 mL, 70 mL, 75 mL, 80 mL, 85 mL, 90 mL, 95 mL, 100 mL, 150 mL, 200 mL, 250 mL, 300 mL, 350 mL, 400 mL, 450 mL, 500 mL, or the like. Such units can be provided so as to contain a specified number of cells, *e.g.,* NK cells and/or ILC3 cells, *e.g.,* 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸ or more cells per milliliter, or 1 × 10⁴, 5 × 10⁴, 1 × 10⁵ 5 × 10⁵ 1 × 10⁶, 5 × 10⁶, 1 × 10⁷, 5 × 10⁷, 1 × 10⁸, 5 × 10⁸, 1 × 10⁹, 5 × 10⁹, 1 × 10¹⁰, 5 × 10¹⁰, 1 × 10¹¹ or more cells per unit. In specific examples, the units can comprise about, at least about, or at most about 1 × 10⁴, 5 × 10⁴, 1 × 10⁵, 5 × 10⁵, 1 × 10⁶, 5 × 10⁶ or more NK cells and/or ILC3 cells per milliliter, or 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸, 1 × 10⁹, 5 × 10⁹, 1 × 10¹⁰, 5 × 10¹⁰, 1 × 10¹¹ or more cells per unit. Such units can be provided to contain specified numbers of NK cells and/or ILC3 cells or NK cell and/or ILC3 cell populations and/or any of the other cells. In specific examples, the NK cells and II,C3 cells are present in ratios provided herein.

In another specific example, said isolated NK cells and/or ILC3 cells in said composition are from a single individual. In a more specific example, said isolated NK cells and/or ILC3 cells comprise NK cells and/or ILC3 cells from at least two different individuals. In another specific example, said isolated NK cells and/or ILC3 cells in said composition are from a different individual than the individual for whom treatment with the NK cells and/or ILC3 cells is intended. In another specific example, said NK cells have been contacted or brought into proximity with an immunomodulatory compound or thalidomide in an amount and for a time sufficient for said NK cells to express detectably more granzyme B or perforin than an equivalent number of natural killer cells, *i.e.* NK cells not contacted or brought into proximity with said immunomodulatory compound or thalidomide. In another specific example, said composition additionally comprises an immunomodulatory compound or thalidomide. In certain examples, the immunomodulatory compound is a compound described below. *See, e.g.,* U.S. Patent No. 7,498,171, In certain examples, the immunomodulatory compound is an amino-substituted isoindoline. In one examples, the immunomodulatory compound is 3-(4-amino-1-oxo-1,3-dihydroisoindol-2-yl)-piperidine-2,6-dione; 3-(4'aminoisolindoline-1'-one)-1-piperidine-2,6-dione; 4-(amino)-2-(2,6-dioxo(3-piperidyl))-isoindoline-1,3-dione; or 4-Amino-2-(2,6-dioxopiperidin-3-yl)isoindole-1,3-dione. In another example, the immunomodulatory compound is pomalidomide, or lenalidomide. In another example, said immunomodulatory compound is a compound having the structure 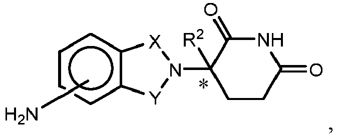
wherein one of X and Y is C=O, the other of X and Y is C=O or CH₂, and R² is hydrogen or lower alkyl, or a pharmaceutically acceptable salt, hydrate, solvate, clathrate, enantiomer, diastereomer, racemate, or mixture of stereoisomers thereof. In another example, said immunomodulatory compound is a compound having the structure 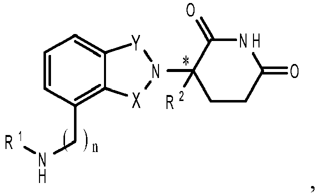
   wherein one of X and Y is C=O and the other is CH₂ or C=O;
   R¹ is H, (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, C(O)R³, C(S)R³, C(O)OR⁴, (C₁-C₈)alkyl-N(R⁶)₂, (C₁-C₈)alkyl-OR⁵, (C₁-C₈)alkyl-C(O)OR⁵, C(O)NHR³, C(S)NHR³, C(O)NR³R^{3'}, C(S)NR³R^{3'} or (C₁-C₈)alkyl-O(CO)R⁵;
   R² is H, F, benzyl, (C₁-C₈)alkyl, (C₂-C₈)alkenyl, or (C₂-C₈)alkynyl;
   R³ and R^{3'} are independently (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, (C₀-C₈)alkyl-N(R⁶)₂, (C₁-C₈)alkyl-OR⁵, (C₁-C₈)alkyl-C(O)OR⁵, (C₁-C₈)alkyl-O(CO)R⁵, or C(O)OR⁵;
   R⁴ is (C₁-C₈)alkyl, (C₂-C₈)alkenyl, (C₂-C₈)alkynyl, (C₁-C₄)alkyl-OR⁵, benzyl, aryl, (C₀-C₄)alkyl-(C₁-C₆)heterocycloalkyl, or (C₀-C₄)alkyl-(C₂-C₅)heteroaryl;
   R⁵ is (C₁-C₈)alkyl, (C₂-C₈)alkenyl, (C₂-C₈)alkynyl, benzyl, aryl, or (C₂-C₅)heteroaryl;
   each occurrence of R⁶ is independently H, (C₁-C₈)alkyl, (C₂-C₈)alkenyl, (C₂-C₈)alkynyl, benzyl, aryl, (C₂-C₅)heteroaryl, or (C₀-C₈)alkyl-C(O)O-R⁵ or the R⁶ groups can join to form a heterocycloalkyl group;
   n is 0 or 1; and
   * represents a chiral-carbon center;
   or a pharmaceutically acceptable salt, hydrate, solvate, clathrate, enantiomer, diastereomer, racemate, or mixture of stereoisomers thereof. In another example, said immunomodulatory compound is a compound having the structure 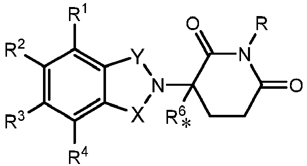
   wherein:
      one of X and Y is C=O and the other is CH₂ or C=O;
      R isH or CH₂OCOR';
      (i) each of R¹, R², R³, or R⁴, independently of the others, is halo, alkyl of 1 to 4 carbon atoms, or alkoxy of 1 to 4 carbon atoms or (ii) one of R¹, R², R³, or R⁴ is nitro or -NHR⁵ and the remaining of R¹, R², R³, or R⁴ are hydrogen;
      R⁵ is hydrogen or alkyl of 1 to 8 carbons
      R⁶ hydrogen, alkyl of 1 to 8 carbon atoms, benzo, chloro, or fluoro;
      R' is R⁷-CHR¹⁰-N(R⁸R⁹);
      R⁷ is m-phenylene or p-phenylene or -(CₙH₂ₙ)- in which n has a value of 0 to 4;
      each of R⁸ and R⁹ taken independently of the other is hydrogen or alkyl of 1 to 8 carbon atoms, or R⁸ and R⁹ taken together are tetramethylene, pentamethylene, hexamethylene, or -CH₂CH₂X₁CH₂CH₂- in which X₁ is -O-, -S-, or -NH-;
      R¹⁰ is hydrogen, alkyl of to 8 carbon atoms, or phenyl; and
      * represents a chiral-carbon center;
or a pharmaceutically acceptable salt, hydrate, solvate, clathrate, enantiomer, diastereomer, racemate, or mixture of stereoisomers thereof.

In another specific example, the composition additionally comprises one or more anticancer compounds, *e*.*g*., one or more of the anticancer compounds described below.

In a more specific example, the composition comprises NK cells and/or ILC3 cells from another source, or made by another method. In a specific example, said other source is placental blood and/or umbilical cord blood. In another specific example, said other source is peripheral blood. In more specific examples, the NK cell and/or ILC3 cell population in said composition is combined with NK cells and/or ILC3 cells from another source, or made by another method in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

In another specific example, the composition comprises an NK cell and/or ILC3 cell population produced using the three-stage method described herein and either isolated placental perfusate or isolated placental perfusate cells. In a more specific example, said placental perfusate is from the same individual as said NK cell and/or ILC3 cell population. In another more specific example, said placental perfusate comprises placental perfusate from a different individual than said NK cell and/or ILC3 cell population. In another specific example, all, or substantially all (*e*.*g*., greater than 90%, 95%, 98% or 99%) of cells in said placental perfusate are fetal cells. In another specific example, the placental perfusate or placental perfusate cells, comprise fetal and maternal cells. In a more specific example, the fetal cells in said placental perfusate comprise less than about 90%, 80%, 70%, 60% or 50% of the cells in said perfusate. In another specific example, said perfusate is obtained by passage of a 0.9% NaCl solution through the placental vasculature. In another specific example, said perfusate comprises a culture medium. In another specific example, said perfusate has been treated to remove erythrocytes. In another specific example, said composition comprises an immunomodulatory compound, *e*.*g*., an immunomodulatory compound described below, *e.g.,* an amino-substituted isoindoline compound. In another specific example, the composition additionally comprises one or more anticancer compounds, *e*.*g*., one or more of the anticancer compounds described below.

In another specific example, the composition comprises an NK cell and/or ILC3 cell population and placental perfusate cells. In a more specific example, said placental perfusate cells are from the same individual as said NK cell and/or ILC3 cell population. In another more specific example, said placental perfusate cells are from a different individual than said NK cell and/or ILC3 cell population. In another specific example, the composition comprises isolated placental perfusate and isolated placental perfusate cells, wherein said isolated perfusate and said isolated placental perfusate cells are from different individuals. In another more specific example, of any of the above examples comprising placental perfusate, said placental perfusate comprises placental perfusate from at least two individuals. In another more specific example of any of the above examples comprising placental perfusate cells, said isolated placental perfusate cells are from at least two individuals. In another specific example, said composition comprises an immunomodulatory compound. In another specific example, the composition additionally comprises one or more anticancer compounds, e.g., one or more of the anticancer compounds described below.

### 5.11. Uses of NK Cells and/or ILC3 Cells Produced Using the Three-Stage Method

The NK cells and/or ILC3 cells produced using the methods described herein, *e*.*g*., NK cell and/or ILC3 cell produced according to the three-stage method described herein, provided herein can be used in methods of treating individuals having cancer, *e*.*g*., individuals having solid tumor cells and/or blood cancer cells, or persons having a viral infection. In some such examples, an effective dosage of NK cells and/or ILC3 cells produced using the methods described herein ranges from 1 × 10⁴ to 5 × 10⁴, 5 × 10⁴ to 1 × 10⁵, 1 × 10⁵ to 5 × 10⁵, 5 × 10⁵ to 1 × 10⁶, 1 × 10⁶ to 5 × 10⁶, 5 × 10⁶ to 1 × 10⁷, or more cells/kilogram body weight. The NK cells and/or ILC3 cells produced using the methods described herein, can also be used in methods of suppressing proliferation of tumor cells.

### 5.11.1. Treatment of Individuals Having Cancer

Disclosed herein is a method of treating an individual having a cancer, for example, a blood cancer or a solid tumor, comprising administering to said individual a therapeutically effective amount of NK cells produced using the methods described herein, e.g., NK cell populations produced using the three-stage method described herein.

Disclosed herein is a method of treating an individual having a cancer, for example, a blood cancer or a solid tumor, comprising administering to said individual a therapeutically effective amount of ILC3 cells produced using the methods described herein, e.g., ILC3 cell populations produced using the three-stage method described herein. In certain examples the individual has a deficiency of natural killer cells, *e*.*g*., a deficiency of NK cells active against the individual's cancer. In a specific example, the method additionally comprises administering to said individual isolated placental perfusate or isolated placental perfusate cells, *e.g.,* a therapeutically effective amount of placental perfusate or isolated placental perfusate cells. In another specific example, the method comprises additionally administering to said individual an effective amount of an immunomodulatory compound, *e*.*g*., an immunomodulatory compound described above, or thalidomide. As used herein, an "effective amount" is an amount that, *e*.*g*., results in a detectable improvement of, lessening of the progression of, or elimination of, one or more symptoms of a cancer from which the individual suffers.

Administration of an isolated population of NK cells and/or ILC3 cells or a pharmaceutical composition thereof may be systemic or local. In specific administration is parenteral. In specific examples, administration of an isolated population of NK cells and/or ILC3 cells or a pharmaceutical composition thereof to a subject is by injection, infusion, intravenous (IV) administration, intrafemoral administration, or intratumor administration. In specific examples, administration of an isolated population of NK cells and/or ILC3 cells or a pharmaceutical composition thereof to a subject is performed with a device, a matrix, or a scaffold. In specific examples, administration an isolated population of NK cells and/or ILC3 cells or a pharmaceutical composition thereof to a subject is by injection. In specific examples, administration an isolated population of NK cells and/or ILC3 cells or a pharmaceutical composition thereof to a subject is via a catheter. In specific examples, the injection of NK cells and/or ILC3 cells is local injection. In more specific examples, the local injection is directly into a solid tumor *(e.g.,* a sarcoma). In specific examples, administration of an isolated population of NK cells and/or ILC3 cells or a pharmaceutical composition thereof to a subject is by injection by syringe. In specific examples, administration of an isolated population of NK cells and/or ILC3 cells or a pharmaceutical composition thereof to a subject is via guided delivery. In specific examples, administration of an isolated population of NK cells and/or ILC3 cells or a pharmaceutical composition thereof to a subject by injection is aided by laparoscopy, endoscopy, ultrasound, computed tomography, magnetic resonance, or radiology.

In a specific example, the cancer is a blood cancer, *e.g.,* a leukemia or a examples, lymphoma. In more specific examples, the cancer is an acute leukemia, e.g., acute T cell leukemia, acute myelogenous leukemia (AML), acute promyelocytic leukemia, acute myeloblastic leukemia, acute megakaryoblastic leukemia, precursor B acute lymphoblastic leukemia, precursor T acute lymphoblastic leukemia, Burkitt's leukemia (Burkitt's lymphoma), or acute biphenotypic leukemia; a chronic leukemia, *e*.*g*., chronic myeloid lymphoma, chronic myelogenous leukemia (CML), chronic monocytic leukemia, chronic lymphocytic leukemia (CLL)/Small lymphocytic lymphoma, or B-cell prolymphocytic leukemia; hairy cell lymphoma; T-cell prolymphocytic leukemia; or a lymphoma, *e*.*g*., histiocytic lymphoma, lymphoplasmacytic lymphoma (*e*.*g*., Waldenström macroglobulinemia), splenic marginal zone lymphoma, plasma cell neoplasm (*e*.*g*., plasma cell myeloma, plasmacytoma, a monoclonal immunoglobulin deposition disease, or a heavy chain disease), extranodal marginal zone B cell lymphoma (MALT lymphoma), nodal marginal zone B cell lymphoma (NMZL), follicular lymphoma, mantle cell lymphoma, diffuse large B cell lymphoma, mediastinal (thymic) large B cell lymphoma, intravascular large B cell lymphoma, primary effusion lymphoma, T cell large granular lymphocytic leukemia, aggressive NK cell leukemia, adult T cell leukemia/lymphoma, extranodal NK/T cell lymphoma, nasal type, enteropathy-type T cell lymphoma, hepatosplenic T cell lymphoma, blastic NK cell lymphoma, mycosis fungoides (Sezary syndrome), a primary cutaneous CD30-positive T cell lymphoproliferative disorder (*e*.*g*., primary cutaneous anaplastic large cell lymphoma or lymphomatoid papulosis), angioimmunoblastic T cell lymphoma, peripheral T cell lymphoma, unspecified, anaplastic large cell lymphoma, a Hodgkin's lymphoma or a nodular lymphocyte-predominant Hodgkin's lymphoma. In another specific example, example, the cancer is multiple myeloma or myelodysplastic syndrome.

In certain other specific examples, the cancer is a solid tumor, *e*.*g*., a carcinoma, such as an adenocarcinoma, an adrenocortical carcinoma, a colon adenocarcinoma, a colorectal adenocarcinoma, a colorectal carcinoma, a ductal cell carcinoma, a lung carcinoma, a thyroid carcinoma, a nasopharyngeal carcinoma, a melanoma (*e*.*g*., a malignant melanoma), a non-melanoma skin carcinoma, or an unspecified carcinoma; a desmoid tumor; a desmoplastic small round cell tumor; an endocrine tumor; an Ewing sarcoma; a germ cell tumor (*e*.*g*., testicular cancer, ovarian cancer, choriocarcinoma, endodermal sinus tumor, germinoma, *etc.*); a hepatosblastoma; a hepatocellular carcinoma; a neuroblastoma; a non-rhabdomyosarcoma soft tissue sarcoma; an osteosarcoma; a retinoblastoma; a rhabdomyosarcoma; or a Wilms tumor. In another embodiment, the solid tumor is pancreatic cancer or breast cancer. In other examples, the solid tumor is an acoustic neuroma; an astrocytoma (*e*.*g*., a grade I pilocytic astrocytoma, a grade II low-grade astrocytoma; a grade III anaplastic astrocytoma; or a grade IV glioblastoma multiforme); a chordoma; a craniopharyngioma; a glioma (*e*.*g*., a brain stem glioma; an ependymoma; a mixed glioma; an optic nerve glioma; or a subependymoma); a glioblastoma; a medulloblastoma; a meningioma; a metastatic brain tumor; an oligodendroglioma; a pineoblastoma; a pituitary tumor; a primitive neuroectodermal tumor; or a schwannoma. In another example, the cancer is prostate cancer. In another example, the cancer is liver cancer. In another example, the cancer is lung cancer. In another example, the cancer is renal cancer.

In certain examples, the individual having a cancer, for example, a blood cancer or a solid tumor, e.g., an individual having a deficiency of natural killer cells, is an individual that has received a bone marrow transplant before said administering. In certain examples, the bone marrow transplant was in treatment of said cancer. In certain other examples, the bone marrow transplant was in treatment of a condition other than said cancer. In certain examples, the individual received an immunosuppressant in addition to said bone marrow transplant. In certain examples, the individual who has had a bone marrow transplant exhibits one or more symptoms of graft-versus-host disease (GVHD) at the time of said administration. In certain other examples, the individual who has had a bone marrow transplant is administered said cells before a symptom of GVHD has manifested.

In certain specific examples, the individual having a cancer, for example, a blood cancer, has received at least one dose of a TNFα inhibitor, e.g., ETANERCEPT^{®} (Enbrel), prior to said administering. In specific examples, said individual received said dose of a TNFα inhibitor within 1, 2, 3, 4, 5, 6, 7, 8, 9, 10, 11 or 12 months of diagnosis of said cancer. In a specific example, the individual who has received a dose of a TNFα inhibitor exhibits acute myeloid leukemia. In a more specific example, the individual who has received a dose of a TNFα inhibitor and exhibits acute myeloid leukemia further exhibits deletion of the long arm of chromosome 5 in blood cells. In another example, the individual having a cancer, for example, a blood cancer, exhibits a Philadelphia chromosome.

In certain other examples, the cancer, for example, a blood cancer or a solid tumor, in said individual is refractory to one or more anticancer drugs. In a specific example, the cancer is refractory to GLEEVEC^{®} (imatinib mesylate).

In certain examples, the cancer, for example, a blood cancer, in said individual responds to at least one anticancer drug; in this example, placental perfusate, isolated placental perfusate cells, isolated natural killer cells, *e*.*g*., placental natural killer cells, e.g., placenta-derived intermediate natural killer cells, isolated combined natural killer cells, or NK cells described herein, and/or combinations thereof, and optionally an immunomodulatory compound, are added as adjunct treatments or as a combination therapy with said anticancer drug. In certain other examples, the individual having a cancer, for example, a blood cancer, has been treated with at least one anticancer drug, and has relapsed, prior to said administering. In certain examples, the individual to be treated has a refractory cancer. In one example, the cancer treatment method with the cells described herein protects against (*e*.*g*., prevents or delays) relapse of cancer. In one example, the cancer treatment method described herein results in remission of the cancer for 1 month or more, 2, 3, 4, 5, 6, 7, 8, 9, 10, 11, or 12 months or more, 1 year or more, 2 years or more, 3 years or more, or 4 years or more.

Disclosed herein is a method of treating an individual having multiple myeloma, comprising administering to the individual (1) lenalidomide; (2) melphalan; and (3) NK cells, wherein said NK cells are effective to treat multiple myeloma in said individual. In a specific example, said NK cells are cord blood NK cells, or NK cells produced from cord blood hematopoietic cells, e.g., hematopoietic stem cells. In another example, said NK cells have been produced by a three-stage method described herein for producing NK cells. In another example, said lenalidomide, melphalan, and/or NK cells are administered separately from each other. In certain specific examples, of the method of treating an individual with multiple myeloma, said NK cells are produced by a method comprising: culturing hematopoietic stem cells or progenitor cells, *e.g.,* CD34⁺ stem cells or progenitor cells, in a first medium comprising a stem cell mobilizing agent and thrombopoietin (Tpo) to produce a first population of cells, subsequently culturing said first population of cells in a second medium comprising a stem cell mobilizing agent and interleukin-15 (IL-15), and lacking Tpo, to produce a second population of cells, and subsequently culturing said second population of cells in a third medium comprising IL-2 and IL-15, and lacking a stem cell mobilizing agent and LMWH, to produce a third population of cells, wherein the third population of cells comprises natural killer cells that are CD56+, CD3-, CD16- or CD16+, and CD94+ or CD94-, and wherein at least 70%, or at least 80%, of the natural killer cells are viable. In certain examples, said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, e.g., low-molecular weight heparin.

Disclosed herein is a method of treating an individual having acute myelogenous leukemia (AML), comprising administering to the individual NK cells (optionally activated by pretreatment with IL2 alone, or IL-15 alone, IL2 and IL12 and IL18, IL12 and IL15, IL12 and IL18, IL2 and IL12 and IL15 and IL18, or IL2 and IL15 and IL18), wherein said NK cells are effective to treat AML in said individual. In a specific example, the isolated NK cell population produced using the three-stage methods described herein has been pretreated with one or more of IL2, IL12, IL18, or IL15 prior to said administering. In a specific example, said NK cells are cord blood NK cells, or NK cells produced from cord blood hematopoietic cells, e.g., hematopoietic stem cells. In another example, said NK cells have been produced by a three-stage method described herein for producing NK cells. In certain specific examples, of the method of treating an individual with AML, said NK cells are produced by a three-stage method, as described herein. In a particular example, the AML to be treated by the foregoing methods comprises refractory AML, poor-prognosis AML, or childhood AML. Methods known in the art for administering NK cells for the treatment of refractory AML, poor-prognosis AML, or childhood AML may be adapted for this purpose; see, e.g., Miller et al., 2005, Blood 105:3051-3057; Rubnitz et al., 2010, J Clin Oncol. 28:955-959. In certain examples, said individual has AML that has failed at least one non-natural killer cell therapeutic against AML. In specific examples, said individual is 65 years old or greater, and is in first remission. In specific examples, said individual has been conditioned with fludarabine, cytarabine, or both prior to administering said natural killer cells.

Disclosed herein is a method of treating an individual having multiple myeloma, comprising administering to the individual (1) lenalidomide; (2) melphalan; and (3) II,C3 cells, wherein said ILC3 cells are effective to treat multiple myeloma in said individual. In a specific example, said ILC3 cells are cord blood ILC3 cells, or ILC3 cells produced from cord blood hematopoietic cells, e.g., hematopoietic stem cells. In another example, said ILC3 cells have been produced by a three-stage method described herein for producing ILC3 cells. In another example, said lenalidomide, melphalan, and/or ILC3 cells are administered separately from each other. In certain specific examples, of the method of treating an individual with multiple myeloma, said II,C3 cells are produced by a method comprising: culturing hematopoietic stem cells or progenitor cells, e.g., CD34⁺ stem cells or progenitor cells, in a first medium comprising a stem cell mobilizing agent and thrombopoietin (Tpo) to produce a first population of cells, subsequently culturing said first population of cells in a second medium comprising a stem cell mobilizing agent and interleukin-15 (IL-15), and lacking Tpo, to produce a second population of cells, and subsequently culturing said second population of cells in a third medium comprising IL-2 and IL-15, and lacking a stem cell mobilizing agent and LMWH, to produce a third population of cells, wherein the third population of cells comprises natural killer cells that are CD56+, CD3-, CD16- or CD16+, and CD94+ or CD94-, and wherein at least 70%, or at least 80%, of the natural killer cells are viable. In certain examples, said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, e.g., low-molecular weight heparin.

Disclosed herein is a method of treating an individual having acute myelogenous leukemia (AML), comprising administering to the individual ILC3 cells (optionally activated by pretreatment with IL2 and IL12 and IL18, IL12 and IL15, IL12 and IL18, IL2 and IL12 and IL15 and IL18, or IL2 and IL15 and IL18), wherein said ILC3 cells are effective to treat AML in said individual. In a specific example, the ILC3 cell population produced using the three-stage methods described herein has been pretreated with one or more of IL2, IL12, IL18, or IL15 prior to said administering. In a specific example, said ILC3 cells are cord blood ILC3 cells, or ILC3 cells produced from cord blood hematopoietic cells, *e*.*g*., hematopoietic stem cells. In another example, said ILC3 cells have been produced by a three-stage method described herein for producing II,C3 cells. In certain specific examples, of the method of treating an individual with AML, said ILC3 cells are produced by a three-stage method, as described herein. In a particular example, the AML to be treated by the foregoing methods comprises refractory AML, poor-prognosis AML, or childhood AML. Methods known in the art for administering ILC3 cells for the treatment of refractory AML, poor-prognosis AML, or childhood AML may be adapted for this purpose; see, *e*.*g*., Miller et al., 2005, Blood 105:3051-3057; Rubnitz et al., 2010, J Clin Oncol. 28:955-959. In certain examples, said individual has AML that has failed at least one non-natural killer cell therapeutic against AML. In specific examples, said individual is 65 years old or greater, and is in first remission. In specific examples, said individual has been conditioned with fludarabine, cytarabine, or both prior to administering said natural killer cells.

In other specific examples, of the method of treating an individual with AML, said NK cells are produced by a method comprising: culturing hematopoietic stem cells or progenitor cells, *e*.*g*., CD34⁺ stem cells or progenitor cells, in a first medium comprising a stem cell mobilizing agent and thrombopoietin (Tpo) to produce a first population of cells, subsequently culturing said first population of cells in a second medium comprising a stem cell mobilizing agent and interleukin-15 (IL-15), and lacking Tpo, to produce a second population of cells, and subsequently culturing said second population of cells in a third medium comprising IL-2 and IL-15, and lacking a stem cell mobilizing agent and LMWH, to produce a third population of cells, wherein the third population of cells comprises natural killer cells that are CD56+, CD3-, CD16- or CD16+, and CD94+ or CD94-, and wherein at least 70%, or at least 80%, of the natural killer cells are viable. In certain examples, said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, *e*.*g*., low-molecular weight heparin.

Disclosed herein is a method of treating an individual having chronic lymphocytic leukemia (CLL), comprising administering to the individual a therapeutically effective dose of (1) lenalidomide; (2) melphalan; (3) fludarabine; and (4) NK cells, e.g., NK cells produced by a three-stage method described herein, wherein said NK cells are effective to treat said CLL in said individual. In a specific example, said NK cells are cord blood NK cells, or NK cells produced from cord blood hematopoietic stem cells. In another example, said NK cells have been produced by a three-stage method described herein for producing NK cells. In a specific example, of any of the above methods, said lenalidomide, melphalan, fludarabine, and expanded NK cells are administered to said individual separately. In certain specific examples, of the method of treating an individual with CLL, said NK cells are produced by a method comprising: culturing hematopoietic stem cells or progenitor cells, e.g., CD34⁺ stem cells or progenitor cells, in a first medium comprising a stem cell mobilizing agent and thrombopoietin (Tpo) to produce a first population of cells, subsequently culturing said first population of cells in a second medium comprising a stem cell mobilizing agent and interleukin-15 (IL-15), and lacking Tpo, to produce a second population of cells, and subsequently culturing said second population of cells in a third medium comprising IL-2 and IL-15, and lacking a stem cell mobilizing agent and LMWH, to produce a third population of cells, wherein the third population of cells comprises natural killer cells that are CD56+, CD3-, CD16- or CD16+, and CD94+ or CD94-, and wherein at least 70%, or at least 80%, of the natural killer cells are viable. In certain examples, said first medium and/or said second medium lack leukemia inhibiting factor (LIF) and/or macrophage inflammatory protein-1 alpha (MIP-1α). In certain examples, said third medium lacks LIF, MIP-1α, and FMS-like tyrosine kinase-3 ligand (Flt-3L). In specific examples, said first medium and said second medium lack LIF and MIP-1α, and said third medium lacks LIF, MIP-1α, and Flt3L. In certain examples, none of the first medium, second medium or third medium comprises heparin, e.g., low-molecular weight heparin.

### 5.11.2. Suppression of Tumor Cell Proliferation

Disclosed herein is a method of suppressing the proliferation of tumor cells, comprising bringing NK cells produced using the methods described herein, e.g., NK cell populations produced using the three-stage method described herein, into proximity with the tumor cells, *e.g.,* contacting the tumor cells with NK cells produced using the methods described herein. A plurality of the NK cells can thus be used in the method of suppressing the proliferation of the tumor cells comprising bringing a therapeutically effective amount of the NK cell population into proximity with the tumor cells, *e.g.,* contacting the tumor cells with the cells in the NK cell population. Optionally, isolated placental perfusate or isolated placental perfusate cells is brought into proximity with the tumor cells and/or NK cells produced using the methods described herein. In another specific example, an immunomodulatory compound, *e.g.,* an immunomodulatory compound described above, or thalidomide is additionally brought into proximity with the tumor cells and/or NK cells produced using the methods described herein, such that proliferation of the tumor cells is detectably reduced compared to tumor cells of the same type not brought into proximity with NK cells produced using the methods described herein. Optionally, isolated placental perfusate or isolated placental perfusate cells are brought into proximity with the tumor cells and/or NK cells produced using the methods described herein that have been contacted or brought into proximity with an immunomodulatory compound.

Disclosed herein herein is a method of suppressing the proliferation of tumor cells, comprising bringing ILC3 cells produced using the methods described herein, *e.g.,* ILC3 cell populations produced using the three-stage method described herein, into proximity with the tumor cells, *e.g.,* contacting the tumor cells with ILC3 cells produced using the methods described herein. A plurality of the ILC3 cells can thus be used in the method of suppressing the proliferation of the tumor cells comprising bringing a therapeutically effective amount of the ILC3 cell population into proximity with the tumor cells, *e.g.,* contacting the tumor cells with the cells in the ILC3 cell population. Optionally, isolated placental perfusate or isolated placental perfusate cells is brought into proximity with the tumor cells and/or ILC3 cells produced using the methods described herein. In another specific example, an immunomodulatory compound, *e.g.,* an immunomodulatory compound described above, or thalidomide is additionally brought into proximity with the tumor cells and/or ILC3 cells produced using the methods described herein, such that proliferation of the tumor cells is detectably reduced compared to tumor cells of the same type not brought into proximity with ILC3 cells produced using the methods described herein. Optionally, isolated placental perfusate or isolated placental perfusate cells are brought into proximity with the tumor cells and/or ILC3 cells produced using the methods described herein that have been contacted or brought into proximity with an immunomodulatory compound.

As used herein, in certain examples, "contacting," or "bringing into proximity," with respect to cells, in one example, encompasses direct physical, *e.g*., cell-cell, contact between placental perfusate, placental perfusate cells, natural killer cells, *e.g.,* NK cell populations produced according to the three-stage method described herein, ILC3 cells, *e.g.,* ILC3 cell populations produced according to the three-stage method described herein, and/or isolated combined natural killer cells and the tumor cells. In another example, "contacting" encompasses presence in the same physical space, *e.g.,* placental perfusate, placental perfusate cells, natural killer cells, *e.g.,* placental intermediate natural killer cells, natural killer cells described herein, *e.g.,* NK cell populations produced according to the three-stage method described herein, ILC3 cells described herein, *e.g.,* ILC3 cell populations produced according to the three-stage method described herein, and/or isolated combined natural killer cells are placed in the same container (*e.g.,* culture dish, multiwell plate) as tumor cells. In another example, "contacting" placental perfusate, placental perfusate cells, combined natural killer cells, placental intermediate natural killer cells, or natural killer cells described herein, *e.g.,* NK cell populations produced according to the three-stage method described herein or ILC3 cells described herein, *e.g.,* ILC3 cell populations produced according to the three-stage method described herein, and tumor cells is accomplished, *e.g.,* by injecting or infusing the placental perfusate or cells, *e.g.,* placental perfusate cells, combined natural killer cells, natural killer cells, *e.g.,* placental intermediate natural killer cells, or ILC3 cells, into an individual, *e.g.,* a human comprising tumor cells, *e.g.,* a cancer patient. "Contacting," in the context of immunomodulatory compounds and/or thalidomide, means, *e.g.,* that the cells and the immunomodulatory compound and/or thalidomide are directly physically contacted with each other, or are placed within the same physical volume (*e.g*., a cell culture container or an individual).

In a specific example, the tumor cells are blood cancer cells, *e.g*., leukemia cells or lymphoma cells. In more specific examples, the cancer is an acute leukemia, *e.g*., acute T cell leukemia cells, acute myelogenous leukemia (AML) cells, acute promyelocytic leukemia cells, acute myeloblastic leukemia cells, acute megakaryoblastic leukemia cells, precursor B acute lymphoblastic leukemia cells, precursor T acute lymphoblastic leukemia cells, Burkitt's leukemia (Burkitt's lymphoma) cells, or acute biphenotypic leukemia cells; chronic leukemia cells, *e.g.,* chronic myeloid lymphoma cells, chronic myelogenous leukemia (CML) cells, chronic monocytic leukemia cells, chronic lymphocytic leukemia (CLL)/Small lymphocytic lymphoma cells, or B-cell prolymphocytic leukemia cells; hairy cell lymphoma cells; T-cell prolymphocytic leukemia cells; or lymphoma cells, *e.g.,* histiocytic lymphoma cells, lymphoplasmacytic lymphoma cells (*e.g*., Waldenström macroglobulinemia cells), splenic marginal zone lymphoma cells, plasma cell neoplasm cells (*e.g*., plasma cell myeloma cells, plasmacytoma cells, monoclonal immunoglobulin deposition disease, or a heavy chain disease), extranodal marginal zone B cell lymphoma (MALT lymphoma) cells, nodal marginal zone B cell lymphoma (NMZL) cells, follicular lymphoma cells, mantle cell lymphoma cells, diffuse large B cell lymphoma cells, mediastinal (thymic) large B cell lymphoma cells, intravascular large B cell lymphoma cells, primary effusion lymphoma cells, T cell large granular lymphocytic leukemia cells, aggressive NK cell leukemia cells, adult T cell leukemia/lymphoma cells, extranodal NK/T cell lymphoma - nasal type cells, enteropathy-type T cell lymphoma cells, hepatosplenic T cell lymphoma cells, blastic NK cell lymphoma cells, mycosis fungoides (Sezary syndrome), primary cutaneous CD30-positive T cell lymphoproliferative disorder (*e.g.,* primary cutaneous anaplastic large cell lymphoma or lymphomatoid papulosis) cells, angioimmunoblastic T cell lymphoma cells, peripheral T cell lymphoma - unspecified cells, anaplastic large cell lymphoma cells, Hodgkin lymphoma cells or nodular lymphocyte-predominant Hodgkin lymphoma cells. In another specific example, the tumor cells are multiple myeloma cells or myelodysplastic syndrome cells.

In specific examples, the tumor cells are solid tumor cells, *e.g.,* carcinoma cells, for example, adenocarcinoma cells, adrenocortical carcinoma cells, colon adenocarcinoma cells, colorectal adenocarcinoma cells, colorectal carcinoma cells, ductal cell carcinoma cells, lung carcinoma cells, thyroid carcinoma cells, nasopharyngeal carcinoma cells, melanoma cells (*e.g.,* malignant melanoma cells), non-melanoma skin carcinoma cells, or unspecified carcinoma cells; desmoid tumor cells; desmoplastic small round cell tumor cells; endocrine tumor cells; Ewing sarcoma cells; germ cell tumor cells (*e.g.,* testicular cancer cells, ovarian cancer cells, choriocarcinoma cells, endodermal sinus tumor cells, germinoma cells, *etc.*); hepatosblastoma cells; hepatocellular carcinoma cells; neuroblastoma cells; non-rhabdomyosarcoma soft tissue sarcoma cells; osteosarcoma cells; retinoblastoma cells; rhabdomyosarcoma cells; or Wilms tumor cells. In another example, the tumor cells are pancreatic cancer cells or breast cancer cells. In other examples, the solid tumor cells are acoustic neuroma cells; astrocytoma cells (*e.g.,* grade I pilocytic astrocytoma cells, grade II low-grade astrocytoma cells; grade III anaplastic astrocytoma cells; or grade IV glioblastoma multiforme cells); chordoma cells; craniopharyngioma cells; glioma cells (*e.g.,* brain stem glioma cells; ependymoma cells; mixed glioma cells; optic nerve glioma cells; or subependymoma cells); glioblastoma cells; medulloblastoma cells; meningioma cells; metastatic brain tumor cells; oligodendroglioma cells; pineoblastoma cells; pituitary tumor cells; primitive neuroectodermal tumor cells; or schwannoma cells. In another example, the tumor cells are prostate cancer cells.

As used herein, "therapeutically beneficial" and "therapeutic benefits" include, but are not limited to, *e.g.,* reduction in the size of a tumor; lessening or cessation of expansion of a tumor; reducing or preventing metastatic disease; reduction in the number of cancer cells in a tissue sample, *e.g.,* a blood sample, per unit volume; the clinical improvement in any symptom of the particular cancer or tumor said individual has, the lessening or cessation of worsening of any symptom of the particular cancer the individual has, *etc.*

### 5.11.3. Treatment of cancers using NK cells and/or ILC3 cells and other anticancer agents

Treatment of an individual having cancer using the NK cells produced using the methods described herein, *e.g.,* NK cell populations produced using the three-stage method described herein, can be part of an anticancer therapy regimen that includes one or more other anticancer agents. Likewise, treatment of an individual having cancer using the ILC3 cells produced using the methods described herein, *e.g.,* ILC3 cell populations produced using the three-stage method described herein, can be part of an anticancer therapy regimen that includes one or more other anticancer agents. In addition or alternatively, treatment of an individual having cancer using the NK cells and/or ILC3 cells produced using the methods described herein can be used to supplement an anticancer therapy that includes one or more other anticancer agents. Such anticancer agents are well-known in the art and include anti-inflammatory agents, immumodulatory agents, cytotoxic agents, cancer vaccines, chemotherapeutics, HDAC inhibitors (*e.g.,* HDAC6i (ACY-241)), and siRNAs. Specific anticancer agents that may be administered to an individual having cancer, *e.g.,* an individual having tumor cells, in addition to the NK cells produced using the methods described herein and optionally perfusate, perfusate cells, natural killer cells other than NK cells produced using the methods described herein include, but are not limited to: acivicin; aclarubicin; acodazole hydrochloride; acronine; adozelesin; adriamycin; adrucil; aldesleukin; altretamine; ambomycin; ametantrone acetate; amsacrine; anastrozole; anthramycin; asparaginase (*e.g.,* from Erwinia chrysan; Erwinaze); asperlin; avastin (bevacizumab); azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; celecoxib (COX-2 inhibitor); Cerubidine; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflomithine hydrochloride; elsamitrucin; Elspar; enloplatin; enpromate; epipropidine; epirubicin hydrochloride; erbulozole; esorubicin hydrochloride; estramustine; estramustine phosphate sodium; etanidazole; etoposide; etoposide phosphate; Etopophos; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; Idamycin; idarubicin hydrochloride; ifosfamide; ilmofosine; iproplatin; irinotecan; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; Proleukin; Purinethol; puromycin; puromycin hydrochloride; pyrazofurin; Rheumatrex; riboprine; safingol; safingol hydrochloride; semustine; simtrazene; sparfosate sodium; sparsomycin; spirogermanium hydrochloride; spiromustine; spiroplatin; streptonigrin; streptozocin; sulofenur; Tabloid; talisomycin; tecogalan sodium; taxotere; tegafur; teloxantrone hydrochloride; temoporfin; teniposide; teroxirone; testolactone; thiamiprine; thioguanine; thiotepa; tiazofurin; tirapazamine; Toposar; toremifene citrate; trestolone acetate; Trexall; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; and zorubicin hydrochloride.

Other anti-cancer drugs include, but are not limited to: 20-epi-1,25 dihydroxyvitamin D3; 5-azacytidine; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptosar (also called Campto; irinotecan) camptothecin derivatives; capecitabine; carboxamide-amino-triazole; carboxyamidotriazole; CaRest M3; CARN 700; cartilage derived inhibitor; carzelesin; casein kinase inhibitors (ICOS); castanospermine; CC-122; CC-220; CC-486; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidenmin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; doxorubicin; droloxifene; dronabinol; duocarmycin SA; ebselen; ecomustine; edelfosine; edrecolomab; eflomithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine (*e.g.,* Fludara); fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; homoharringtonine (HHT, omacetaxine mepesuccinate); hypericin; ibandronic acid; idarubicin; idoxifene; idramantone; ilmofosine; ilomastat; imatinib (*e.g.,* GLEEVEC^{®}), imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide + estrogen + progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mitoguazone; mitolactol; mitomycin analogues; mitonafide; mitotoxin fibroblast growth factor-saporin; mitoxantrone; mofarotene; molgramostim; anti-EGFR antibody (*e.g.,* Erbitux (cetuximab)); anti-CD19 antibody; anti-CD20 antibody (*e.g.,* rituximab); anti-CS-1 antibody (*e.g.,* elotuzumab (BMS/AbbVie)); anti-CD38 antiobdy (*e.g.,* daratumumab (Genmab/Janssen Biotech); anti-CD138 antibody (*e.g.,* indatuximab (Biotest AG Dreieich)); anti-PD-1 antibody; anti- PD-L1 antibody (*e.g.,* durvalumab (AstraZeneca)); anti-NKG2A antibody (*e.g.,* monalizumab (IPH2201; Innate Pharma)); anti-DLL4 antibody (e.g., demcizumab (Oncomed/Celgene)); anti-DLL4 and anti-VEGF bispecific antibody; anti-RSPO3 antibody; anti-TIGIT antibody; ICOS agonist antibody; anti-disialoganglioside (GD2) antibody (*e.g.,* monoclonal antibody 3F8 or ch14.18); anti-ErbB2 antibody (*e.g.,* herceptin); human chorionic gonadotrophin; monophosphoryl lipid A+myobacterium cell wall sk; mopidamol; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; nilutamide; nisamycin; nitric oxide modulators; nitroxide antioxidant; nitrullyn; oblimersen (GENASENSE^{®}); O⁶-benzylguanine; octreotide; okicenone; oligonucleotides; onapristone; ondansetron; ondansetron; oracin; oral cytokine inducer; ormaplatin; osaterone; oxaliplatin (*e.g.,* Floxatin); oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stipiamide; stromelysin inhibitors; sulfinosine; superactive vasoactive intestinal peptide antagonist; suradista; suramin; swainsonine; tallimustine; tamoxifen methiodide; tauromustine; tazarotene; tecogalan sodium; tegafur; tellurapyrylium; telomerase inhibitors; temoporfin; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; Vectibix (panitumumab)velaresol; veramine; verdins; verteporfin; vinorelbine; vinxaltine; vitaxin; vorozole; Welcovorin (leucovorin); Xeloda (capecitabine); zanoterone; zeniplatin; zilascorb; and zinostatin stimalamer.

Treatment of an individual having cancer using the NK cells produced using the methods described herein, e.g., NK cell populations produced using the three-stage method described herein, can be part of an anticancer therapy regimen that includes one or more immune checkpoint modulator. In certain examples, the immune checkpoint modulator modulates an immune checkpoint molecule such as CD28, OX40, Glucocorticoid-Induced Tumour-necrosis factor Receptor-related protein (GITR), CD137 (4-1BB), CD27, Herpes Virus Entry Mediator (HVEM), T cell Immunoglobulin and Mucin-domain containing-3 (TIM-3), Lymphocyte-Activation Gene 3 (LAG-3), Cytotoxic T-Lymphocyte-associated Antigen-4 (CTLA-4), V-domain Immunoglobulin Suppressor of T cell Activation (VISTA), B and T Lymphocyte Attenuator (BTLA), PD-1, and/or PD-L1. In certain examples, the immune checkpoint molecule is an antibody or antigen-binding fragment thereof.

In certain examples, the immune checkpoint modulator is an agonist of an immune checkpoint molecule. In certain examples, the immune checkpoint molecule is CD28, OX40, Glucocorticoid-Induced Tumour-necrosis factor Receptor-related protein (GITR), CD137 (4-1BB), CD27, ICOS (CD278); Inducible T-cell Costimulator) and/or Herpes Virus Entry Mediator (HVEM). In certain examples, the immune checkpoint modulator is an antibody or antigen-binding fragment thereof.

In certain examples, the immune checkpoint modulator is an antagonist of an immune checkpoint molecule. In certain examples, the immune checkpoint molecule is T cell Immunoglobulin and Mucin-domain containing-3 (TIM-3), Lymphocyte-Activation Gene 3 (LAG-3), Cytotoxic T-Lymphocyte-associated Antigen-4 (CTLA-4), V-domain Immunoglobulin Suppressor of T cell Activation (VISTA), B and T Lymphocyte Attenuator (BTLA), PD-1, and/or PD-L1. In certain examples, the immune checkpoint modulator is an antibody or antigen-binding fragment thereof.

In certain examples, the immune checkpoint modulator is an antibody or antigen-binding fragment thereof. In certain examples, the antibody or antibody-binding fragment thereof binds PD-1. In certain examples, the antibody or antibody-binding fragment thereof that binds PD-1 is nivolumab (OPDIVO^{®,} BMS-936558, MDX-1106, ONO-4538; Bristol-Myers Squibb, Ono Pharmaceuticals, Inc.), pembrolizumab (KEYTRUDA^{®}, lambrolizumab, MK-3475; Merck), pidilizumab (CT-011; Curetech, Medivation); MEDI0680 (AMP-514; MedImmune, AstraZeneca); PDR-001 (Novartis), SHR1210, or INCSHR1210; Incyte, Jiangsu Hengrui). In certain examples, the antibody or antigen-binding fragment thereof binds PD-L1. In certain examples, the antibody or antigen-binding fragment thereof that binds PD-L1 is durvalumab (MEDI4736; MedImmune, AstraZeneca), BMS-936559 (MDX-1105; Bristol-Myers Squibb), avelumab (MSB0010718C; Merck Serono, Pfizer), or atezolizumab (MPDL-3280A; Genentech, Roche). In certain examples, the antibody or antibody-binding fragment thereof binds LAG-3. In certain examples, the antibody or antibody-binding fragment thereof that binds LAG-3 is BMS-986016 (Bristol-Myers Squibb), GSK2831781 (GlaxoSmithKline), or LAG525 (Novartis). In certain examples, the antibody or antibody-binding fragment thereof binds CTLA-4. In certain examples, the antibody or antibody-binding fragment thereof that binds CTLA-4 is ipilimumab (YERVOY^{™}, BMS-734016, MDX010, MDX-101; Bristol-Myers Squibb), or tremelimumab (CP-675,206; MedImmune, AstraZeneca). In certain examples, the antibody or antibody-binding fragment thereof binds OX40. In certain examples, the antibody or antibody-binding fragment thereof that binds OX40 is MEDI6469 (MedImmune, AstraZeneca), MEDI0562 (MedImmune, AstraZeneca), or KHK4083 (Kyowa Hakko Kirin). In certain examples, the antibody or antibody-binding fragment thereof binds GITR. In certain examples, the antibody or antibody-binding fragment thereof that binds GITR is TRX518 (Leap Therapeutics) or MEDI1873 (MedImmune, AstraZeneca). In certain examples, the antibody or antibodybinding fragment thereof binds CD137 (4-1BB). In certain examples, the antibody or antibody-binding fragment thereof that binds CD137 (4-1BB) is PF-2566 (PF-05082566; Pfizer), or urelumab (BMS-663513; Bristol-Myers Squibb). In certain examples, the antibody or antibody-binding fragment thereof binds CD27. In certain examples, the antibody or antibody-binding fragment thereof that binds CD27 is varilumab (CDX-1127; Celldex Therapies).

In certain examples, treatment of an individual having cancer using the NK cells produced using the methods described herein, e.g., NK cell populations produced using the three-stage method described herein, is part of an anticancer therapy regimen that includes lenalidomide or pomalidomide. In certain examples, treatment of an individual having cancer using the NK cells produced using the methods described herein, e.g., NK cell populations produced using the three-stage method described herein, is part of an anticancer therapy regimen that includes an HDAC inhibitor. In certain examples, treatment of an individual having cancer using the NK cells produced using the methods described herein, e.g., NK cell populations produced using the three-stage method described herein, is part of an anticancer therapy regimen that includes an anti-CS-1 antibody. In certain examples, treatment of an individual having cancer using the NK cells produced using the methods described herein, *e.g.,* NK cell populations produced using the three-stage method described herein, is part of an anticancer therapy regimen that includes an anti-CD38 antibody. In certain examples, treatment of an individual having cancer using the NK cells produced using the methods described herein, *e.g.,* NK cell populations produced using the three-stage method described herein, is part of an anticancer therapy regimen that includes an anti-CD138 antibody. In certain examples, treatment of an individual having cancer using the NK cells produced using the methods described herein, *e.g.,* NK cell populations produced using the three-stage method described herein, is part of an anticancer therapy regimen that includes an anti-PD-1 antibody. In certain examples, treatment of an individual having cancer using the NK cells produced using the methods described herein, *e.g.,* NK cell populations produced using the three-stage method described herein, is part of an anticancer therapy regimen that includes an anti-PD-L1 antibody. In certain examples, treatment of an individual having cancer using the NK cells produced using the methods described herein, *e.g.,* NK cell populations produced using the three-stage method described herein, is part of an anticancer therapy regimen that includes an anti-NKG2A antibody. In certain examples, treatment of an individual having cancer using the NK cells produced using the methods described herein, *e.g.,* NK cell populations produced using the three-stage method described herein, is part of an anticancer therapy regimen that includes an anti-CD20 antibody (*e.g.,* rituximab; RITUXAN^{®}). In certain examples, treatment of an individual having cancer using the NK cells produced using the methods described herein, *e.g.,* NK cell populations produced using the three-stage method described herein, is part of an anticancer therapy regimen that includes CC-122. In certain examples, treatment of an individual having cancer using the NK cells produced using the methods described herein, *e.g.,* NK cell populations produced using the three-stage method described herein, is part of an anticancer therapy regimen that includes CC-220. In certain examples, treatment of an individual having cancer using the NK cells produced using the methods described herein, *e.g.,* NK cell populations produced using the three-stage method described herein, is part of an anticancer therapy regimen that includes an anti-DLL4 antibody (*e.g.,* demcizumab). In certain examples, treatment of an individual having cancer using the NK cells produced using the methods described herein, *e.g.,* NK cell populations produced using the three-stage method described herein, is part of an anticancer therapy regimen that includes an anti-DLL4 and anti-VEGF bispecific antibody. In certain examples, treatment of an individual having cancer using the NK cells produced using the methods described herein, *e.g.,* NK cell populations produced using the three-stage method described herein, is part of an anticancer therapy regimen that includes an anti-RSPO3 antibody. In certain examples, treatment of an individual having cancer using the NK cells produced using the methods described herein, *e.g.,* NK cell populations produced using the three-stage method described herein, is part of an anticancer therapy regimen that includes an anti-TIGIT antibody. In certain examples, treatment of an individual having cancer using the NK cells produced using the methods described herein, *e.g.,* NK cell populations produced using the three-stage method described herein, is part of an anticancer therapy regimen that includes an ICOS agonist antibody. In certain examples, treatment of an individual having cancer using the NK cells produced using the methods described herein, *e.g.,* NK cell populations produced using the three-stage method described herein, is part of an anticancer therapy regimen that includes homoharringtonine (*e.g.,* omacetaxine mepesuccinate).

In some examples, treatment of an individual having cancer using the NK cells produced using the methods described herein is part of an anticancer therapy regimen for antibody-dependent cell-mediated cytotoxicity (ADCC). In some examples, treatment of an individual having cancer using the ILC3 cells produced using the methods described herein is part of an anticancer therapy regimen for antibody-dependent cell-mediated cytotoxicity (ADCC). In one example, the ADCC regimen comprises administration of one or more antibodies (e.g., an antibody described in the foregoing paragraph) in combination with NK cells and/or ILC3 cells produced using the methods described herein. Several types of cancer can be treated using such ADCC methods, including but not limited to acute lymphoblastic leukemia (ALL) or other B-cell malignancies (lymphomas and leukemias), neuroblastoma, melanoma, breast cancers, and head and neck cancers. In specific examples, the ADCC therapy comprises administration of one or more of the following antibodies anti-EGFR antibody (*e.g.,* Erbitux (cetuximab)), anti-CD19 antibody, anti-CD20 antibody (e.g., rituximab), anti-disialoganglioside (GD2) antibody (*e.g.,* monoclonal antibody 3F8 or ch14.18), or anti-ErbB2 antibody (*e.g.,* herceptin), in combination with NK cells and/or ILC3 cells produced using the methods described herein. In one example, the ADCC regimen comprises administration of an anti-CD33 antibody in combination with NK cells and/or ILC3 cells produced using the methods described herein. In one example, the ADCC regimen comprises administration of an anti-CD20 antibody in combination with NK cells and/or ILC3 cells produced using the methods described herein. In one example, the ADCC regimen comprises administration of an anti-CD138 antibody in combination with NK cells and/or ILC3 cells produced using the methods described herein. In one example, the ADCC regimen comprises administration of an anti-CD32 antibody in combination with NK cells and/or ILC3 cells produced using the methods described herein.

### 5.11.4. Treatment of Viral Infection

Disclosed herein is a method of treating an individual having a viral infection, comprising administering to said individual a therapeutically effective amount of NK cells produced using the methods described herein, *e.g.,* NK cell populations produced using the three-stage method described herein. Disclosed herein is a method of treating an individual having a viral infection, comprising administering to said individual a therapeutically effective amount of II,C3 cells produced using the methods described herein, *e.g.,* ILC3 cell populations produced using the three-stage method described herein. In certain examples, the individual has a deficiency of natural killer cells, *e.g.,* a deficiency of NK cells or other innate lymphoid cells active against the individual's viral infection. In certain specific examples, said administering additionally comprises administering to the individual one or more of isolated placental perfusate, isolated placental perfusate cells, isolated natural killer cells, *e.g.,* placental natural killer cells, *e.g.,* placentaderived intermediate natural killer cells, isolated combined natural killer cells, and/or combinations thereof. In certain specific examples, the NK cells and/or ILC3 cells produced using the methods described herein are contacted or brought into proximity with an immunomodulatory compound, *e.g.,* an immunomodulatory compound above, or thalidomide, prior to said administration. In certain other specific examples, said administering comprises administering an immunomodulatory compound, *e.g.,* an immunomodulatory compound described above, or thalidomide, to said individual in addition to said NK cells and/or ILC3 cells produced using the methods described herein, wherein said amount is an amount that, *e.g.,* results in a detectable improvement of, lessening of the progression of, or elimination of, one or more symptoms of said viral infection. In specific examples, the viral infection is an infection by a virus of the Adenoviridae, Picornaviridae, Herpesviridae, Hepadnaviridae, Flaviviridae, Retroviridae, Orthomyxoviridae, Paramyxoviridae, Papilommaviridae, Rhabdoviridae, or Togaviridae family. In more specific examples, said virus is human immunodeficiency virus (HIV).coxsackievirus, hepatitis A virus (HAV), poliovirus, Epstein-Barr virus (EBV), herpes simplex type 1 (HSV1), herpes simplex type 2 (HSV2), human cytomegalovirus (CMV), human herpesvirus type 8 (HHV8), herpes zoster virus (varicella zoster virus (VZV) or shingles virus), hepatitis B virus (HBV), hepatitis C virus (HCV), hepatitis D virus (HDV), hepatitis E virus (HEV), influenza virus (*e.g.,* influenza A virus, influenza B virus, influenza C virus, or thogotovirus), measles virus, mumps virus, parainfluenza virus, papillomavirus, rabies virus, or rubella virus.

In other more specific examples, said virus is adenovirus species A, serotype 12, 18, or 31; adenovirus species B, serotype 3, 7, 11, 14, 16, 34, 35, or 50; adenovirus species C, serotype 1, 2, 5, or 6; species D, serotype 8, 9, 10, 13, 15, 17, 19, 20, 22, 23, 24, 25, 26, 27, 28, 29, 30, 32, 33, 36, 37, 38, 39, 42, 43, 44, 45, 46, 47, 48, 49, or 51; species E, serotype 4; or species F, serotype 40 or 41.

In certain other more specific examples, the virus is Apoi virus (APOIV), Aroa virus (AROAV), bagaza virus (BAGV), Banzi virus (BANV), Bouboui virus (BOUV), Cacipacore virus (CPCV), Carey Island virus (CIV), Cowbone Ridge virus (CRV), Dengue virus (DENV), Edge Hill virus (EHV), Gadgets Gully virus (GGYV), Ilheus virus (ILHV), Israel turkey meningoencephalomyelitis virus (ITV), Japanese encephalitis virus (JEV), Jugra virus (JUGV), Jutiapa virus (JUTV), kadam virus (KADV), Kedougou virus (KEDV), Kokobera virus (KOKV), Koutango virus (KOUV), Kyasanur Forest disease virus (KFDV), Langat virus (LGTV), Meaban virus (MEAV), Modoc virus (MODV), Montana myotis leukoencephalitis virus (MMI,V), Murray Valley encephalitis virus (MVEV), Ntaya virus (NTAV), Omsk hemorrhagic fever virus (OHFV), Powassan virus (POWV), Rio Bravo virus (RBV), Royal Farm virus (RFV), Saboya virus (SABV), St. Louis encephalitis virus (SLEV), Sal Vieja virus (SVV), San Perlita virus (SPV), Saumarez Reef virus (SREV), Sepik virus (SEPV), Tembusu virus (TMUV), tick-borne encephalitis virus (TBEV), Tyuleniy virus (TYUV), Uganda S virus (UGSV), Usutu virus (USUV), Wesselsbron virus (WESSV), West Nile virus (WNV), Yaounde virus (YAOV), Yellow fever virus (YFV), Yokose virus (YOKV), or Zika virus (ZIKV).

In other examples, the NK cells produced using the methods described herein, and optionally placental perfusate and/or perfusate cells, are administered to an individual having a viral infection as part of an antiviral therapy regimen that includes one or more other antiviral agents. Specific antiviral agents that may be administered to an individual having a viral infection include, but are not limited to: imiquimod, podofilox, podophyllin, interferon alpha (IFNα), reticolos, nonoxynol-9, acyclovir, famciclovir, valaciclovir, ganciclovir, cidofovir; amantadine, rimantadine; ribavirin; zanamavir and oseltaumavir; protease inhibitors such as indinavir, nelfinavir, ritonavir, or saquinavir; nucleoside reverse transcriptase inhibitors such as didanosine, lamivudine, stavudine, zalcitabine, or zidovudine; and non-nucleoside reverse transcriptase inhibitors such as nevirapine, or efavirenz.

### 5.11.5. Other Treatment Uses for ILC3 cells

Disclosed herein are ILC3 cells that can be used in all the methods as providedherein. Exemplary methods in which ILC3 cells can be used are disclosed in the following aspects.

Disclosed herein is a method of repairing the gastrointestinal tract after chemotherapy comprising administering to an individual a plurality of ILC3 cells, wherein the ILC3 cells are by a three-stage method described herein.

Disclosed herein is a method of protecting an individual against radiation comprising administering to an individual a plurality of ILC3 cells, wherein the ILC3 cells are produced by a three-stage method described herein. In certain aspects, said ILC3 cells are used as an adjunct to bone marrow transplantation.

Disclosed herein is a method of reconstituting the thymus of an individual comprising administering to an individual a plurality of ILC3 cells, wherein the ILC3 cells are produced by a three-stage method described herein.

Disclosed herein is a method of promoting protective immunity to pathogens in an individual comprising administering to an individual a plurality of ILC3 cells, wherein the ILC3 cells are produced by a three-stage method described herein. In certain aspects, promoting protective immunity to pathogens is performed to treat intestinal infection. In certain aspects, promoting protective immunity to pathogens is performed to prevent intestinal infection. In certain aspects, the intestinal infection is *Citrobacter rodentium.*

Disclosed herein is a method of tumor rejection comprising administering to an individual a plurality of ILC3 cells, wherein the ILC3 cells have been produced by a three-stage method described herein.

Disclosed herein is a method of maintaining tissue integrity during organogenesis comprising administering to an individual a plurality of ILC3 cells, wherein the ILC3 cells have been produced by a three-stage method described herein.

Disclosed herein is a method of tissue repair comprising administering to an individual a plurality of ILC3 cells, wherein the ILC3 cells have been produced by a three-stage method described herein.

Disclosed herein is a method of regulation of inflammation comprising administering to an individual a plurality of ILC3 cells, wherein the ILC3 cells have been produced by a three-stage method described herein.

### 5.11.6. Administration

Determination of the number of cells, *e.g.,* placental perfusate cells, *e.g.,* nucleated cells from placental perfusate, combined natural killer cells, ILC3 cells, and/or isolated natural killer cells, *e.g.,* NK cell populations produced using the three-stage method described herein, and determination of the amount of an immunomodulatory compound, *e.g.,* an immunomodulatory compound, or thalidomide, can be performed independently of each other.

Administration of an isolated population of NK cells and/or ILC3 cells or a pharmaceutical composition thereof may be systemic or local. In specific examples, administration is parenteral. In specific examples, administration of an isolated population of NK cells and/or ILC3 cells or a pharmaceutical composition thereof to a subject is by injection, infusion, intravenous (IV) administration, intrafemoral administration, or intratumor administration. In specific examples, administration of an isolated population of NK cells and/or ILC3 cells or a pharmaceutical composition thereof to a subject is performed with a device, a matrix, or a scaffold. In specific examples, administration an isolated population of NK cells and/or ILC3 cells or a pharmaceutical composition thereof to a subject is by injection. In specific examples, administration an isolated population of NK cells and/or ILC3 cells or a pharmaceutical composition thereof to a subject is via a catheter. In specific examples, the injection of NK cells and/or ILC3 cells is local injection. In more specific examples, the local injection is directly into a solid tumor (*e.g.,* a sarcoma). In specific examples, administration of an isolated population of NK cells and/or ILC3 cells or a pharmaceutical composition thereof to a subject is by injection by syringe. In specific examples, administration of an isolated population of NK cells and/or ILC3 cells or a pharmaceutical composition thereof to a subject is via guided delivery. In specific examples, administration of an isolated population of NK cells and/or ILC3 cells or a pharmaceutical composition thereof to a subject by injection is aided by laparoscopy, endoscopy, ultrasound, computed tomography, magnetic resonance, or radiology.

### 5.11.6.1.Administration of Cells

In certain examples, NK cells and/or ILC3 cells produced using the methods described herein, *e.g.,* NK cell and/or ILC3 cell populations produced using the three-stage method described herein, are used, *e.g.,* administered to an individual, in any amount or number that results in a detectable therapeutic benefit to the individual, *e.g.,* an effective amount, wherein the individual has a viral infection, cancer, or tumor cells, for example, an individual having tumor cells, a solid tumor or a blood cancer, *e.g.,* a cancer patient. Such cells can be administered to such an individual by absolute numbers of cells, *e.g.,* said individual can be administered at about, at least about, or at most about, 1 × 10⁵, 5 x 10⁵, 1 × 10⁶, 5 × 10⁶, 1 × 10⁷, 5 × 10⁷, 1 × 10⁸, 5 × 10⁸, 1 × 10⁹, 5 × 10⁹, 1 × 10¹⁰, 5 × 10¹⁰, or 1 × 10¹¹ NK cells and/or ILC3 cells produced using the methods described herein. In other examples, NK cells and/or ILC3 cells produced using the methods described herein can be administered to such an individual by relative numbers of cells, *e.g.,* said individual can be administered at about, at least about, or at most about, 1 × 10⁵, 5 × 10⁵, 1 × 10⁶, 5 × 10⁶, 1 × 10⁷, 5 × 10⁷, 1 × 10⁸, 5 × 10⁸, 1 × 10⁹, 5 × 10⁹, 1 × 10¹⁰, 5 × 10¹⁰, or 1 × 10¹¹ NK cells and/or ILC3 cells produced using the methods described herein per kilogram of the individual. In other examples, NK cells and/or ILC3 cells produced using the methods described herein can be administered to such an individual by relative numbers of cells, *e.g.,* said individual can be administered at about, at least about, or at most about, 1 × 10⁵, 5 × 10⁵, 1 × 10⁶, 5 × 10⁶, 1 × 10⁷, 5 × 10⁷, 1 × 10⁸, or 5 × 10⁸ NK cells and/or ILC3 cells produced using the methods described herein per kilogram of the individual. NK cells and/or ILC3 cells produced using the methods described herein can be administered to such an individual according to an approximate ratio between a number of NK cells and/or ILC3 cells produced using the methods described herein, and optionally placental perfusate cells and/or natural killer cells other than NK cells and/or ILC3 cells produced using the methods described herein, and a number of tumor cells in said individual (*e.g.,* an estimated number). For example, NK cells and/or ILC3 cells produced using the methods described herein can be administered to said individual in a ratio of about, at least about or at most about 1:1, 1:1, 3:1, 4:1, 5:1, 6:1, 7:1, 8:1, 9:1, 10:1, 15:1, 20:1, 25:1, 30:1, 35:1, 40:1, 45:1, 50:1, 55:1, 60:1, 65:1, 70:1, 75:1, 80:1, 85:1, 90:1, 95:1 or 100:1 to the number of tumor cells in the individual. The number of tumor cells in such an individual can be estimated, *e.g.,* by counting the number of tumor cells in a sample of tissue from the individual, *e.g.,* blood sample, biopsy, or the like. In specific embodiments, *e.g.,* for solid tumors, said counting is performed in combination with imaging of the tumor or tumors to obtain an approximate tumor volume. In a specific example, an immunomodulatory compound or thalidomide, *e.g.,* an effective amount of an immunomodulatory compound or thalidomide, are administered to the individual in addition to the NK cells and/or ILC3 cells produced using the methods described herein, optionally placental perfusate cells and/or natural killer cells other than NK cells and/or ILC3 cells produced using the methods described herein.

In certain examples, the method of suppressing the proliferation of tumor cells, *e.g.,* in an individual; treatment of an individual having a deficiency in the individual's natural killer cells; or treatment of an individual having a viral infection; or treatment of an individual having cancer, *e.g.,* an individual having tumor cells, a blood cancer or a solid tumor, comprises bringing the tumor cells into proximity with, or administering to said individual, a combination of NK cells and/or ILC3 cells produced using the methods described herein and one or more of placental perfusate and/or placental perfusate cells. In specific examples, the method additionally comprises bringing the tumor cells into proximity with, or administering to the individual, an immunomodulatory compound or thalidomide.

In a specific example, for example, treatment of an individual having a deficiency in the individual's natural killer cells (*e.g.,* a deficiency in the number of NK cells or in the NK cells' reactivity to a cancer, tumor or virally-infected cells); or treatment of an individual having a cancer or a viral infection, or suppression of tumor cell proliferation, comprises bringing said tumor cells into proximity with, or administering to said individual, NK cells and/or ILC3 cells produced using the methods described herein supplemented with isolated placental perfusate cells or placental perfusate. In specific examples, about 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸ or more NK cells produced using the methods described herein per milliliter, or 1 x 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸, 1 × 10⁹, 5 × 10⁹, 1 × 10¹⁰, 5 × 10¹⁰, 1 × 10¹¹ or more NK cells produced using the methods described herein are supplemented with about, or at least about, 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸ or more isolated placental perfusate cells per milliliter, or 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸, 1 × 10⁹, 5 × 10⁹, 1 × 10¹⁰, 5 × 10¹⁰, 1 × 10¹¹ or more isolated placental perfusate cells. In other more specific examples, about 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸ or more NK cells produced using the methods described herein or 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸, 1 × 10⁹, 5 × 10⁹, 1 × 10¹⁰, 5 × 10¹⁰, 1 × 10¹¹ or more NK cells produced using the methods described herein are supplemented with about, or at least about, 1, 2, 3, 4, 5, 6, 7, 8, 9, 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900, 950 or 1000 mL of perfusate, or about 1 unit of perfusate.

In another specific example, treatment of an individual having a deficiency in the individual's natural killer cells; treatment of an individual having cancer; treatment of an individual having a viral infection; or suppression of tumor cell proliferation, comprises bringing the tumor cells into proximity with, or administering to the individual, NK cells and/or ILC3 cells produced using the methods described herein, wherein said cells are supplemented with adherent placental cells, *e.g.,* adherent placental stem cells or multipotent cells, *e.g.,* CD34⁻, CD10⁺, CD105⁺, CD200⁺ tissue culture plastic-adherent placental cells. In specific examples, the NK cells and/or ILC3 cells produced using the methods described herein are supplemented with about 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸ or more adherent placental stem cells per milliliter, or 1 × 10⁴, 5 × 10⁴, 1 × 10⁵, 5 × 10⁵, 1 × 10⁶, 5 × 10⁶, 1 × 10⁷, 5 × 10⁷, 1 × 10⁸, 5 × 10⁸, 1 × 10⁹, 5 × 10⁹, 1 × 10¹⁰, 5 × 10¹⁰, 1 × 10¹¹ or more adherent placental cells, *e.g.,* adherent placental stem cells or multipotent cells.

In another specific example, treatment of an individual having a deficiency in the individual's natural killer cells; treatment of an individual having cancer; treatment of an individual having a viral infection; or suppression of tumor cell proliferation, is performed using an immunomodulatory compound or thalidomide in combination with NK cells and/or ILC3 cells produced using the methods described herein, wherein said cells are supplemented with conditioned medium, *e.g.,* medium conditioned by CD34⁻, CD10⁺, CD105⁺, CD200⁺ tissue culture plastic-adherent placental cells, *e.g.,* 0.1, 0.2, 0.3, 0.4, 0.5, 0.6, 0.1, 0.8, 0.9, 1, 2, 3, 4, 5, 6, 7, 8, 9, 10 mL of stem cell-conditioned culture medium per unit of perfusate, or per 10⁴, 10⁵, 10⁶, 10⁷, 10⁸, 10⁹, 10¹⁰, or 10¹¹ NK cells and/or ILC3 cells produced using the methods described herein. In certain examples, the tissue culture plastic-adherent placental cells are the multipotent adherent placental cells described in U.S. Patent Nos. 7,468,276 and8,057,788, . In another specific example, the method additionally comprises bringing the tumor cells into proximity with, or administering to the individual, an immunomodulatory compound or thalidomide.

In another specific example, treatment of an individual having a deficiency in the individual's natural killer cells; treatment of an individual having cancer; treatment of an individual having a viral infection; or suppression of tumor cell proliferation, in which said NK cells and/or ILC3 cells produced using the methods described herein are supplemented with placental perfusate cells, the perfusate cells are brought into proximity with interleukin-2 (IL-2) for a period of time prior to said bringing into proximity. In certain examples, said period of time is about, at least, or at most 1, 2, 3, 4, 5, 6, 7, 8, 9, 10, 12, 14, 16, 18, 20, 22, 24, 26, 28, 30, 32, 34, 36, 38, 40, 42, 44, 46 or 48 hours prior to said bringing into proximity.

The NK cells and/or ILC3 cells produced using the methods described herein and optionally perfusate or perfusate cells, can be administered once to an individual having a viral infection, an individual having cancer, or an individual having tumor cells, during a course of anticancer therapy; or can be administered multiple times, *e.g.,* once every 1, 2, 3, 4, 5, 6, 7, 8, 9, 10, 11, 12, 13, 14, 15, 16, 17, 18, 19, 20, 21, 22 or 23 hours, or once every 1, 2, 3, 4, 5, 6 or 7 days, or once every 1, 2, 3, 4, 5, 6, 7, 8, 9, 10, 24, 36 or more weeks during therapy. In examples, in which cells and an immunomodulatory compound or thalidomide are used, the immunomodulatory compound or thalidomide, and cells or perfusate, can be administered to the individual together, *e.g.,* in the same formulation; separately, *e.g.,* in separate formulations, at approximately the same time; or can be administered separately, *e.g.,* on different dosing schedules or at different times of the day. Similarly, in examples, in which cells and an antiviral compound or anticancer compound are used, the antiviral compound or anticancer compound, and cells or perfusate, can be administered to the individual together, *e.g.,* in the same formulation; separately, *e.g.,* in separate formulations, at approximately the same time; or can be administered separately, *e.g.,* on different dosing schedules or at different times of the day. The NK cells and/or ILC3 cells produced using the methods described herein and perfusate or perfusate cells, can be administered without regard to whether NK cells and/or ILC3 cells produced using the methods described herein, perfusate, or perfusate cells have been administered to the individual in the past.

### 6. KITS

Disclosed herein is a pharmaceutical pack or kit comprising one or more containers filled with one or more of the compositions described herein, e.g., a composition comprising NK cells and/or ILC3 cells produced by a method described herein, e.g., NK cell and/or ILC3 cell populations produced using the three-stage method described herein. Optionally associated with such container(s) can be a notice in the form prescribed by a governmental agency regulating the manufacture, use or sale of pharmaceuticals or biological products, which notice reflects approval by the agency of manufacture, use or sale for human administration.

The kits encompassed herein can be used in accordance with the methods described herein, *e.g.,* methods of suppressing the growth of tumor cells and/or methods of treating cancer, *e.g.,* hematologic cancer, and/or methods of treating viral infection. In one example, a kit comprises NK cells and/or ILC3 cells produced by a method described herein or a composition thereof, in one or more containers. In a specific example, provided herein is a kit comprising an NK cell and/or ILC3 cell population produced by a three-stage method described herein, or a composition thereof.

### 7. EXAMPLES

### 7.1. Example 1: Three-stage method of producing natural killer cells from hematopoietic stem or progenitor cells

CD34⁺ cells are cultured in the following medium formulations for the indicated number of days, and aliquots of cells are taken for assessment of cell count, cell viability, characterization of natural killer cell differentiation and functional evaluation.

Stage 1 medium: 90% Stem Cell Growth Medium (SCGM) (CellGro^{®}), 10% Human Serum-AB, supplemented with 4.5 U/mL low molecular weight heparin (LMWH), 25 ng/mL recombinant human thrombopoietin (TPO), 25 ng/mL recombinant human Flt3L, 27 ng/mL recombinant human stem cell factor (SCF), 25 ng/mL recombinant human IL-7, 0.05 ng/mL recombinant human IL-6 (500-fold), 0.25 ng/mL recombinant human granulocyte colony-stimulating factor (G-CSF) (50-fold), 0.01 ng/mL recombinant human granulocyte-macrophage colony-stimulating factor (GM-CSF) (500-fold), 0.10% gentamicin, and 1 to 10µm StemRegenin-1 (SR-1).

Stage 2 medium: 90% SCGM, 10% Human Serum-AB, supplemented with 4.5 U/mL low molecular weight heparin (LMWH), 25 ng/mL recombinant human Flt3L, 27 ng/mL recombinant human SCF, 25 ng/mL recombinant human IL-7, 20 ng/mL recombinant human IL-15, 0.05 ng/mL recombinant human IL-6 (500-fold), 0.25 ng/mL recombinant human G-CSF (50-fold), 0.01 ng/mL recombinant human GM-CSF (500-fold), 0.10% gentamicin, and 1 to 10µm SR1.

Stage 3 medium: 90% STEMMACS^{™}, 10% Human Serum-AB, 0.025 mM 2-mercaptoethanol (55 mM), supplemented with 22 ng/mL recombinant human SCF, 1000 U/mL recombinant human IL-2, 20 ng/mL recombinant human IL-7, 20 ng/mL recombinant human IL-15, 0.05 ng/mL recombinant human IL-6 (500-fold), 0.25 ng/mL recombinant human G-CSF (50-fold), 0.01 ng/mL recombinant human GM-CSF (500-fold), and 0.10% gentamicin.

Cells are seeded at Day 0 at 3 × 10⁴ cells/mL in Stage 1 media, and cells are tested for purity by a CD34+ and CD45+ count and viability by 7AAD staining. At Day 5 cells are counted and seeded to a concentration of 1 × 10⁵ cells/mL with Stage 1 medium. At Day 7 cells are counted and seeded to a concentration of 1 × 10⁵ cells/mL with Stage 1 medium.

At Day 10, cells are counted and seeded to a concentration of 1 × 10⁵ cells/mL in Stage 2 medium. At Day 12, cells are counted and seeded to a concentration of 3 × 10⁵ cells/mL in Stage 2 medium.

Alternatively, the following protocol is used through Day 14: Cells seeded at Day 0 at 7.5×10³ cells/mL in Stage 1 media, and cells are tested for purity by a CD34+ and CD45+ count and viability by 7AAD staining. At Day 7 cells are counted and seeded to a concentration of 3×10⁵ cells/mL with Stage 1 medium. At Day 9 cells are counted and seeded to a concentration of 3×10⁵ cells/mL with Stage 2 medium. At Day 12, cells are counted and seeded to a concentration of 3×10⁵ cells/mL in Stage 2 medium.

For dynamic differentiation in spinner flasks, at Day 14, cells are centrifuged to concentrate, counted and seeded to a concentration of 5×10⁵ cells/mL in Stage 3 medium. At Day 17, cells are centrifuged, counted and seeded to a concentration of 7.5×10⁵ cells/mL in Stage 3 medium. At Day 21, cells are centrifuged, counted, phenotyped for CD56, CD3, CD16, and CD94, assayed for viability by 7AAD staining, and seeded to a concentration of 1×10⁶ cells/mL in Stage 3 medium. At Day 24, cells are counted and seeded to a concentration of 1×10⁶ cells/mL in Stage 3 medium. From Days 25 to 27, volume is added at 5 mL per day of Stage 3 medium. At Day 28, cells are counted and seeded to a concentration of 1×10⁶ cells/mL in Stage 3 medium. At Day 31, cells are counted and seeded to a concentration of 1×10⁶ cells/mL in Stage 3 medium. From Days 32 to 34, volume is added at 5 mL per day of Stage 3 medium. At Day 35, cells are harvested, counted, phenotyped, and assayed for cytotoxicity.

For static differentiation, at Day 14, cells are centrifuged to concentrate, counted and seeded to a concentration of 3×10⁵ cells/mL in Stage 3 medium. At Day 17, cells are counted and seeded to a concentration of 3×10⁵ cells/mL in Stage 3 medium. At Day 19, cells are counted and seeded to a concentration of 3×10⁵ cells/mL in Stage 3 medium. At Day 21, cells are centrifuged, counted, phenotyped for CD56, CD3, CD16, and CD94, assayed for viability by 7AAD staining, and seeded to a concentration of 5×10⁶ cells/mL in Stage 3 medium. At Day 24, cells are centrifuged, counted and seeded to a concentration of 7.5×10⁶ cells/mL in Stage 3 medium. At Day 26, cells are counted and seeded to a concentration of 7.5×10⁶ cells/mL in Stage 3 medium. At Day 28, cells are counted and seeded to a concentration of 1×10⁶ cells/mL in Stage 3 medium. At Day 31, cells are centrifuged, counted and seeded to a concentration of 1×10⁶ cells/mL in Stage 3 medium. At Day 33, cells are centrifuged, counted and seeded to a concentration of 1×10⁶ cells/mL in Stage 3 medium. At Day 35, cells are harvested, counted, phenotyped, and assayed for cytotoxicity.

For harvest, cells are spun at 400×g for seven minutes, followed by suspension of the pellet in an equal volume of Plasmalyte A. The suspension is spun at 400×g for seven minutes, and the resulting pellet is suspended in 10% HSA (w/v), 60% Plasmalyte A (v/v) at the target cell concentration. The cells are then strained through a 70 µm mesh, the final container is filled, an aliquot of the cells are tested for viability, cytotoxicity, purity, and cell count, and the remainder is packaged.

### 7.2. Example 2: Evaluation of concentration of SR-1 and CH223191 in three-stage method

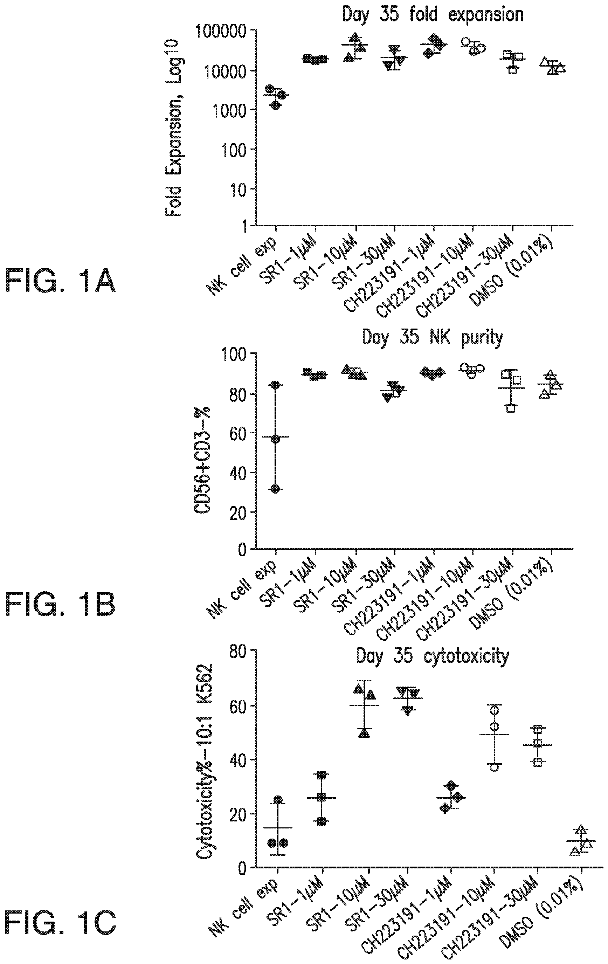

Stemregenin-1 (SR-1) was evaluated as a component of Stage 1 and Stage 2 media using the three-stage method outlined in Example 1, above, at concentrations of 1 µM, 10 µM, and 30 µM. The same concentrations of CH223191 in the three-stage method were also evaluated. SR-1 at 10 µM resulted in a higher cytotoxicity than the other two concentrations tested. Comparable effects on fold expansion, cell purity (CD56+CD3-), and cytotoxicity of K562 cells at a 10:1 (E:T) ratio were observed for SR-1 and CH223 191 at both 10 µM and 1 µM concentrations (Figures 1A-C). Both SR-1 and CH223191 also showed similar effects and trends regarding Day 7 and Day 14 expression of CD34.

### 7.3. Example 3: Characterization of three-stage NK cells

### METHODS

UCB CD34+ cells were cultivated in presence of cytokines including thrombopoietin, SCF, Flt3 ligand, IL-7, IL-15 and IL-2 for 35 days to produce three-stage NK cells, as described in Example 1. Multi-color flow cytometry was used to determine the phenotypic characteristics of three-stage NK cells. Eleven 6-marker panels utilizing 35 NK subtype and other surface markers (*see* Table 1) were evaluated.

**Table 1. Surface markers, including 35 NK subtype surface markers, used to evaluate phenotypic characteristics of three-stage NK cells.**

| **SURFACE MARKERS** | | | |
|---|---|---|---|
| CD16 | NKB1 (KIR3DL1) | NKG2C | CD7 |
| CD56 | KIR2DL3 | CCR5 (CD195) | NKp80 |
| 7AAD | CD11a | CXCR3 (CD183) | CD44 |
| CD94 | CD122 (IL-2Rb) | NKp30 | CD85j (LIR1, ILT2) |
| NKp46 | CD62L | NKG2A | CRACC |
| CD3 | CD117 | CD2 | CD14 |
| KIR2DL4 (CD158d) | CCL3 (MIP1a) | CD27 | CD45 |
| CD25 | CD226 | CD57 | HLA-ABC |
| NKG2D | CD161 | CD96 | CD19 |
| CD69 | CD11b | CXCR4 (CD184) | |
| KIR2DL1 | CD132 | 2B4 (CD244) | |
| | NKp44 | | |

Cytotoxicity assays were performed by co-culturing three-stage NK cells with tumor cell lines for 4 hours. Furthermore, supernatants were collected to analyze secreted perforin, granzymes and cytokines.

To further investigate cytolytic activity, immune synapse formation was monitored. NK-sensitive target cells (K562, chronic myelogenous leukemia cells) were labeled with CellTracker Violet (Life Techology). NK cell/target cell conjugates were formed by suspending equal volumes and cell numbers of NK effector cells (1 × 10⁶/ml) and target cells in culture medium on coverslip for 15 min at 37°C. Cells were then fixed with 3% methanol-free formaldehyde and permeabilized. F-Actin was stained with Alexa-488 conjugated phalloidin (Life Techology). For perforin, CD2, or LFA-1 antibody co-staining with F-actin, slides were incubated for 1 h with primary antibodies followed by the addition of the Alexa Fluor 555 dyeconjugated goat anti-rabbit secondary antibody (Life Technology). Confocal imaging was performed using a Leica SP8 LIAchroics Compact Unit with Inverted DMI 6000 microscope outfitted with 2 HyD detectors.

### RESULTS

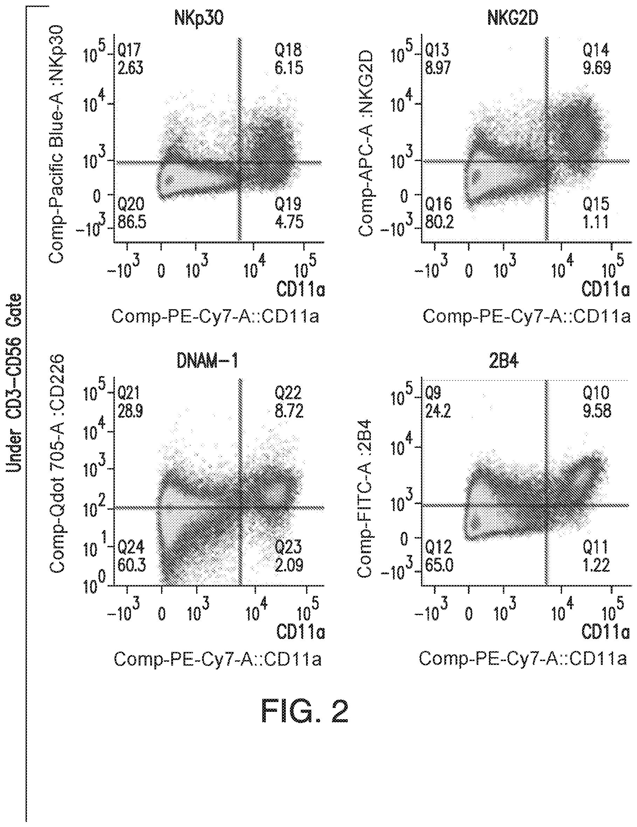

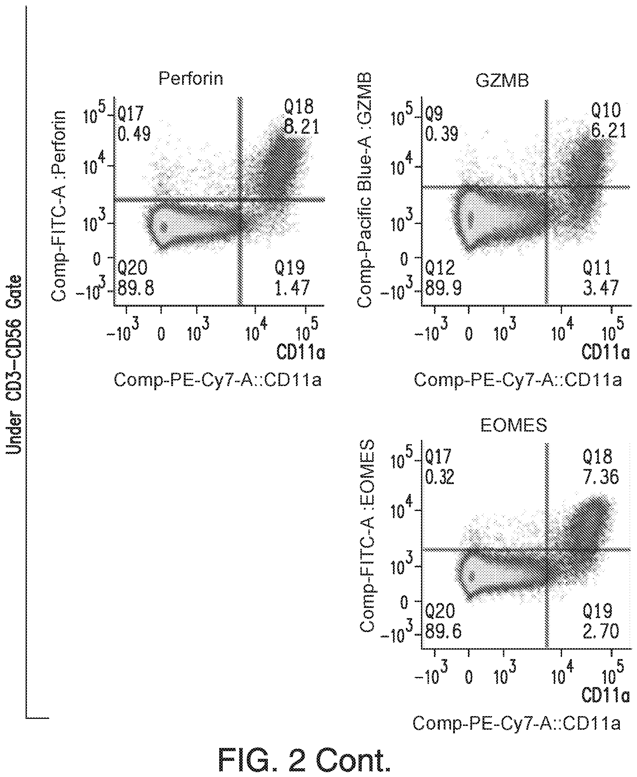

Using the cultivation process described in Example 1, a highly pure population (88.3% ± 6.3%) of CD3-CD56+ NK cells was routinely achieved. Three-stage NK cells displayed a developmentally intermediate immunophenotype, evidenced by low / negative expression of CD16 and KIRs. Three-stage NK cells expressed the natural cytotoxicity receptors (NKp30, NKp46 and NKp44), the c-lectin receptors (CD94, NKG2D and CD161), DNAM-1, 2B4, CD117, and CD11a (Figure 2). Cytolytic mediators (perforin and granzymes) and EOMES, the regulator of NK cell maturation and cytolytic function, were also detected in three-stage NK cells (Figure 2).

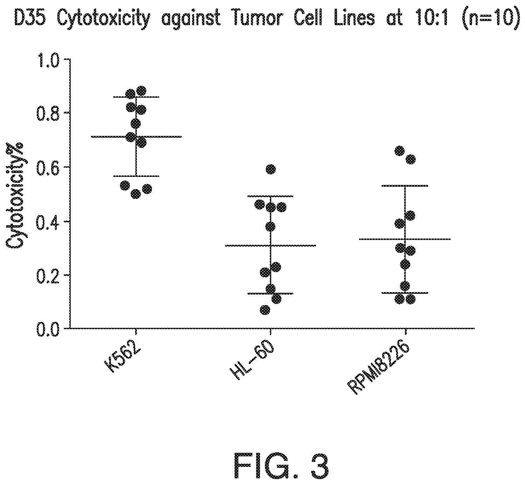

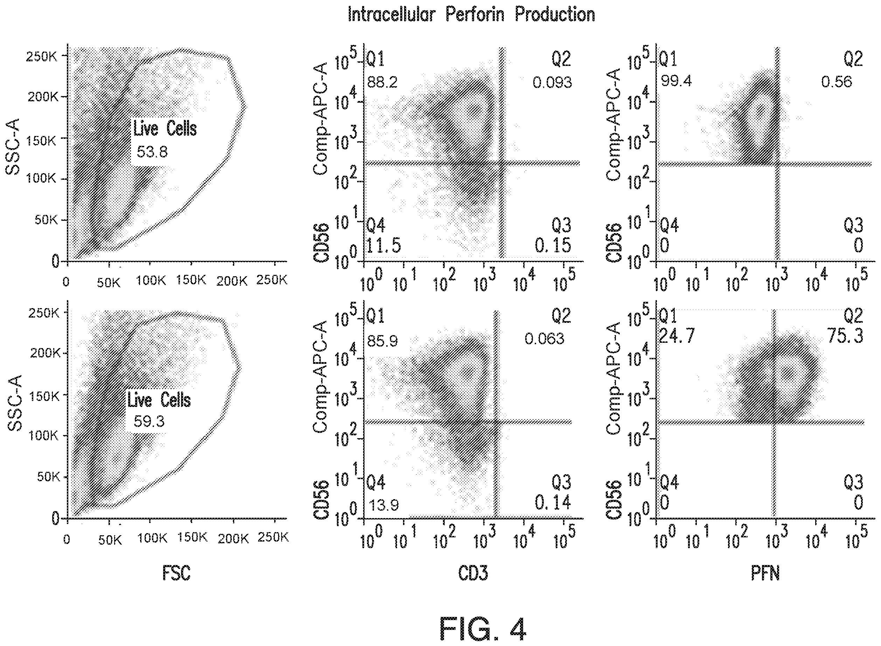

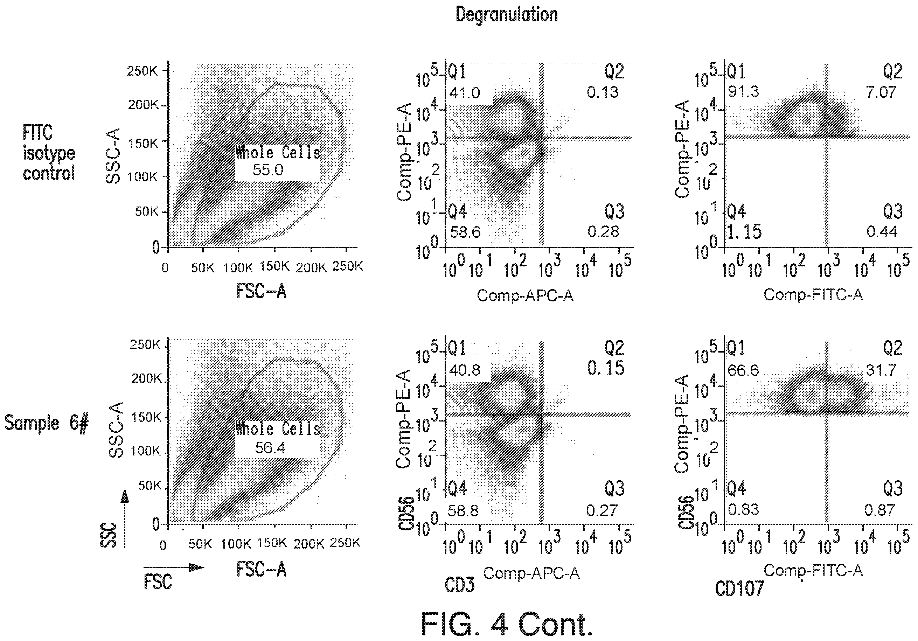

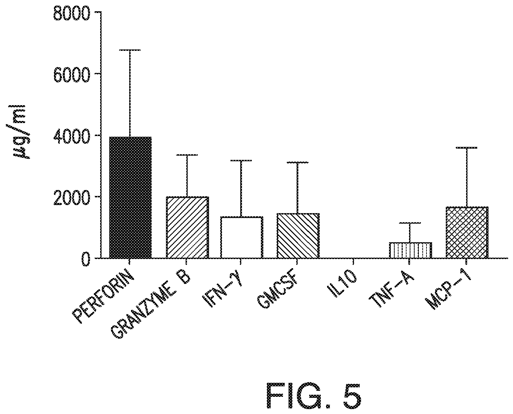

Three-stage NK cells exhibited cytotoxicity against hematological tumor cell lines *in vitro.* At an effector-to-target ratio of 10:1, three-stage NK cells exerted lysis towards cell lines, including CML (K562, 70.3% ± 14.8%), AML (HL-60, 31.0% ± 17.8%) and multiple myeloma (RPMI8266, 32.4% ± 19.5%) (Figure 3). The three-stage NK cells also demonstrated high perforin production and a high degree of granulation (Figure 4). When co-cultured with K562 cells at a 1:1 ratio for 24 hours, three-stage NK cells produced functional cytokines including IFNy, TNFα and GM-CSF (Figure 5 and Table 2).

**Table 2. Three-stage NK cells produce functional cytokines when co-cultured with K562 at 1:1 for 24 hours.**

| | Average (pg/mL) @ E:T of 1:1 (n=11) against K562 | Range (Min, Max) (n=11) |
|---|---|---|
| PERFORIN | 3933.09 | 423, 11058 |
| GRANZYME B | 1976.35 | 46.80, 4281 |
| IFN-γ | 1323.20 | 12.55, 4251.06 |
| GMCSF | 1471.64 | 116.00, 4362.00 |
| IL10 | 3.95 | 2.6, 6.33 |
| GRANZYME-A | 32065.73 | 820, 74697 |
| TNF-A | 482.90 | 16.62, 1841.00 |
| MCP-1 | 1671.63 | 2.96, 6042 |
| Cytotoxicity (10:1) | 66.99% | 52.79%, 79.76% |
| Cytotoxicity (2.5:1) | 43.63% | 18.46%, 76.94% |

At an effector-to-target (E:T) ratio of 1:1, confocal imaging revealed that three-stage NK cells, when in contact with tumor cells, formed an F-actin immunological synapse with polarization of perforin (Figure 6A-B), demonstrating high cytolytic activity.

Furthermore, in the presence of anti-CD20 (rituximab, 10 µg/mL), the cytotoxicity of three-stage NK cells against Daudi cells (Burkitt's lymphoma, a lymphoblastoid cell line resistant to NK killing) increased from 7.3% ± 8.0% to 35.1% ± 5.7%, demonstrating potent antibody-dependent cell-medicated cytotoxicity (ADCC).

### 7.4. Example 4: Further Characterization of Three-Stage NK Cells

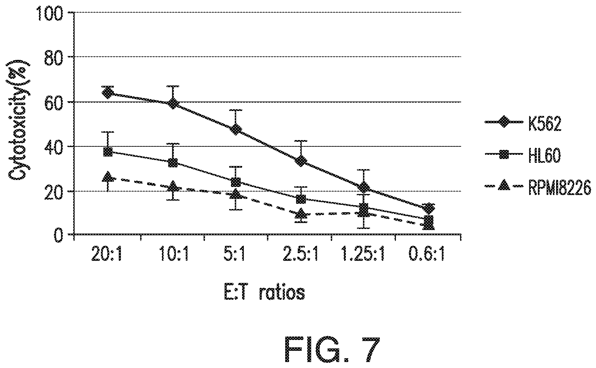

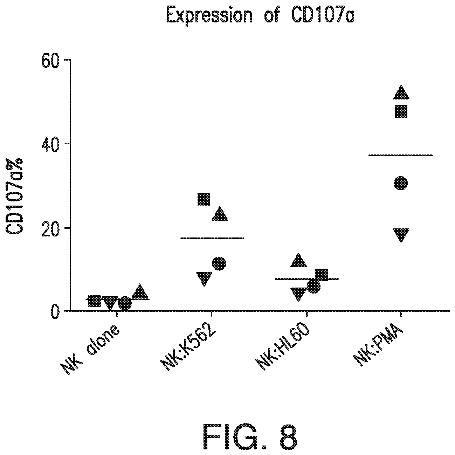

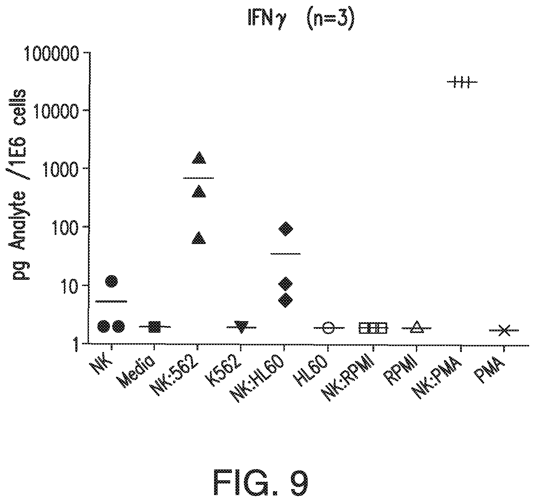

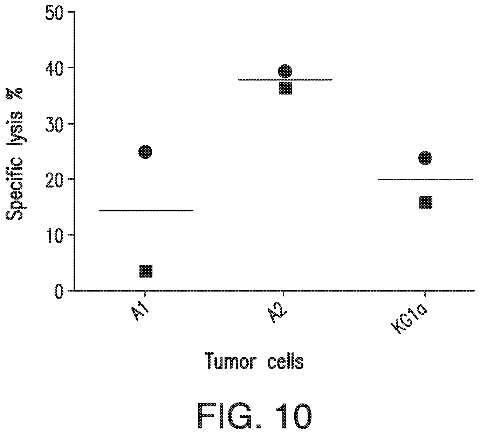

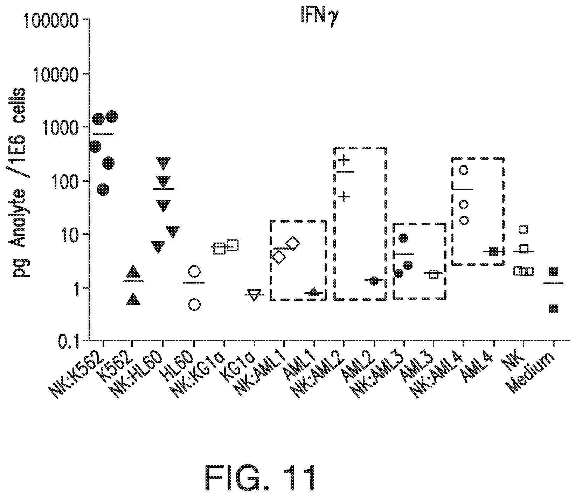

The cytotoxicity of three-stage NK cells against CML, AML, and multiple myeloma cells (K562, HL-60, and RPMI8226, respectively) at various effector to target ratios was examined, as shown in Figure 7. In the presence of K562, HL60, or PMA (phorbol 12-myristate 13-acetate), different levels of CD107a expression were observed, an indicator of degranulation (Figure 8). Likewise, an increase in IFNγ production by three-stage NK cells was observed when cocultured with K562 and HL60 cells lines, or upon PMA stimulation (Figure 9). Up to 40% specific lysis was observed in the presence of primary AML targets at an effector-to-target ratio of 3:1 after 24 hours of incubation, and a differential susceptibility of AML targets to NK killing was observed (Figure 10). A wide range of IFNγ production levels were observed across tumor cell lines and primary targets, as well as donor variation for both the three-stage NK cells and primary AML targets (Figure 11).

Three-stage NK cells were shown to produce various cytolytic enzymes and cytokines in the presence of various tumor cells lines or primary AML targets (AML1-4), as shown in Table 3.

**Table 3. The average cytokine secretion (pg/1 × 10⁶ cells) is shown at an effector-to-target ration of 1:1 against various primary and tumor cells.**

| pg/1E6 | NK + K562 | NK + HL-60 | NK + KG1a | NK + RPMI | NK + AML1 | NK + AML2 | NK + AML3 | NK + AML4 | NK + PMA |
|---|---|---|---|---|---|---|---|---|---|
| N= | 5 | 5 | 2 | 3 | 2 | 2 | 3 | 3 | 5 |
| PERFORIN | 4292 | 3430 | 2787 | 30 | 419 | 596 | 1462 | 1662 | 8466 |
| IFNG | 750 | 71 | 6 | 2 | 5 | 148 | 4 | 70 | 26601 |
| GRANZYME-A | 49192 | 49192 | 36560 | 274 | 10241 | 12003 | 51316 | 71886 | 147792 |
| GRANZYME-B | 8858 | 8858 6638 | 1276 | 2 | 1015 | 1699 | 1071 | 2123 | 22606 |
| GMCSF | 1920 | 1920 434 | 46 | 4 | 16 | 646 | 50 | 1311 | 70340 |
| TNF-A | 5272 | 5272 2110 | 30 | 17 | 46 | 306 | 138 | 554 | 7564 |
| MCP-1 | 1739 | 37004 | 146 | 153 | 131 | 1173 | 173 | 466 | 3811 |

In summary, three-stage NK cells showed cytolytic activity across various tumor cell lines, exhibited a degranulation capacity when in contact with tumor cells and upon activation by PMA, and secreted IFNy, perforin, granzyme A, and granzyme B when cocultured with tumor cells or upon activation by PMA. Furthermore, the three-stage NK cells exhibited a 24 hour cytolytic activity against primary AML targets at an effector to target ratio of 3:1 and showed the capacity to secrete IFNγ against primary AML cells.

### 7.5. Example 5: Two populations of three-stage NK cells

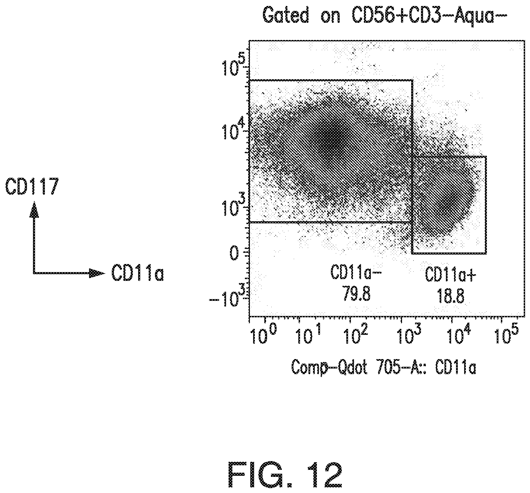

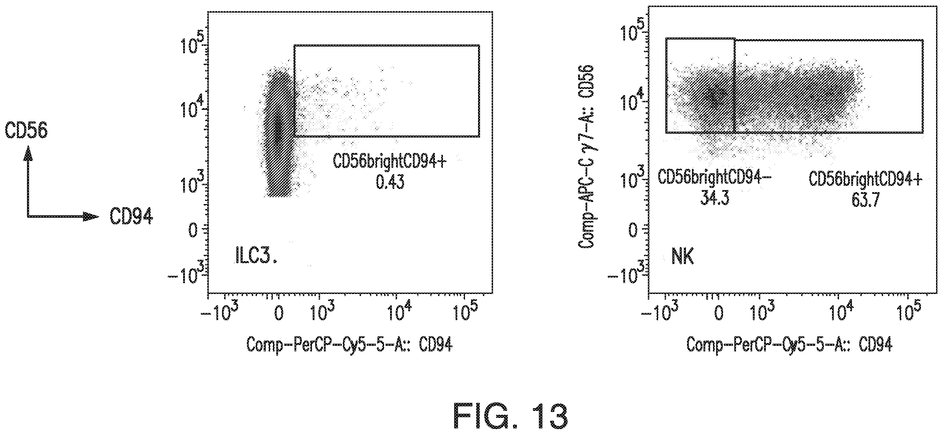

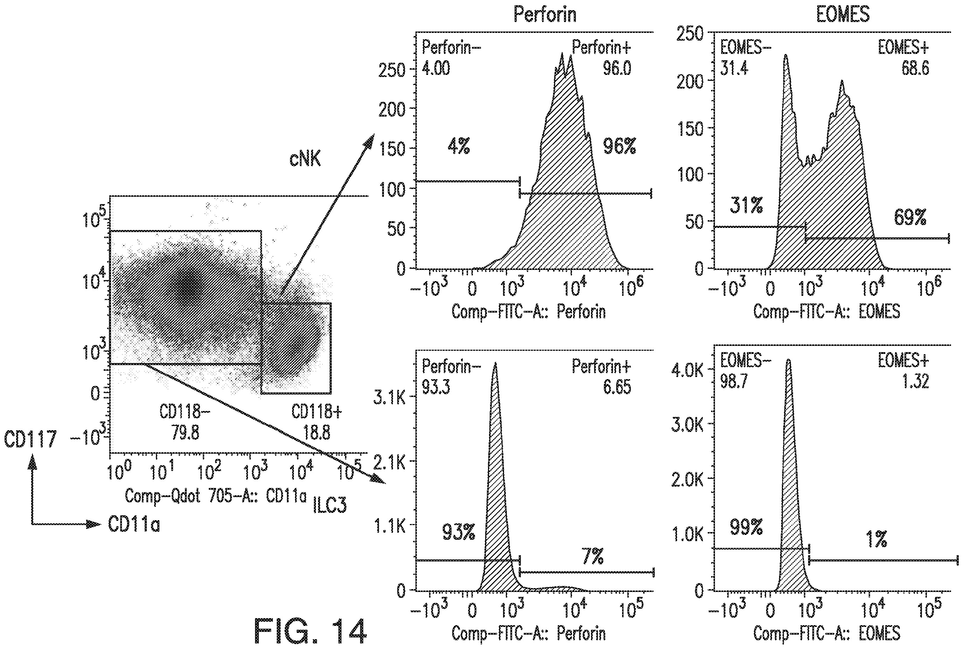

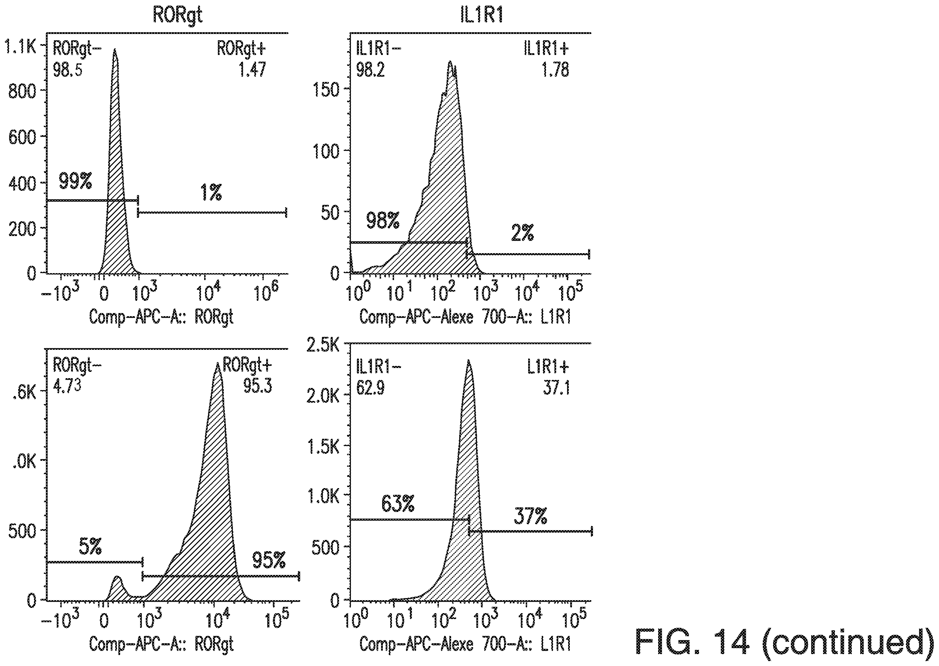

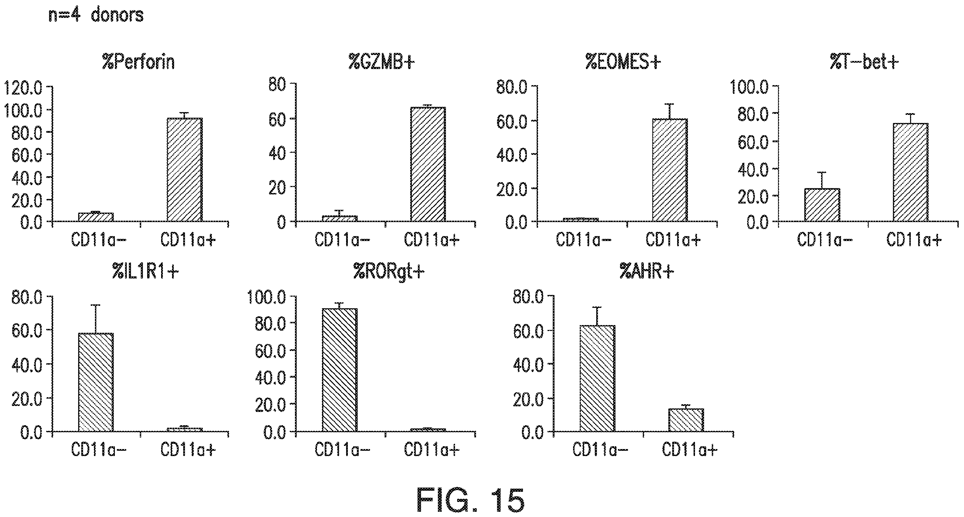

Using FACS, the CD56+CD3- population of three-stage NK cells was evaluated on a CD11a vs. CD117 plot, showing two populations of cells (Figure 12). The CD11a- fraction (ILC3) was shown to be CD94-, and the CD11a+ fraction (NK) was shown to contain both CD94- and CD94+ cells (Figure 13). Furthermore, the CD11a⁻ and CD11a+ cells could be distinguished based on their expression of perforin, eomesodermin (EOMES), RAR-related orphan receptor gamma t (RORyt) and interleukin-1 receptor 1 (IL1R1), with CD11a+ cells expressing perforin and EOMES but not RORyt and IL1R1, and CD11a- cells expressing RORyt and II,1R1, but not perforin and EOMES (Figure 14). Consistent expression patterns of perforin, granzyme B, EOMES, T-bet, IL1R1, RORyt and aryl hydrocarbon receptor (AHR) were shown across donors, with perforin, granzyme B, EOMES, and T-bet primarily expressed by CD11a+ cells, and II,1R1, RORyt and AHR by CD11a- cells. (Figure 15). The CD11a+ population were hypothesized to be traditional NK cells, whereas the CD11a- population were hypothesized to be ILC3 cells.

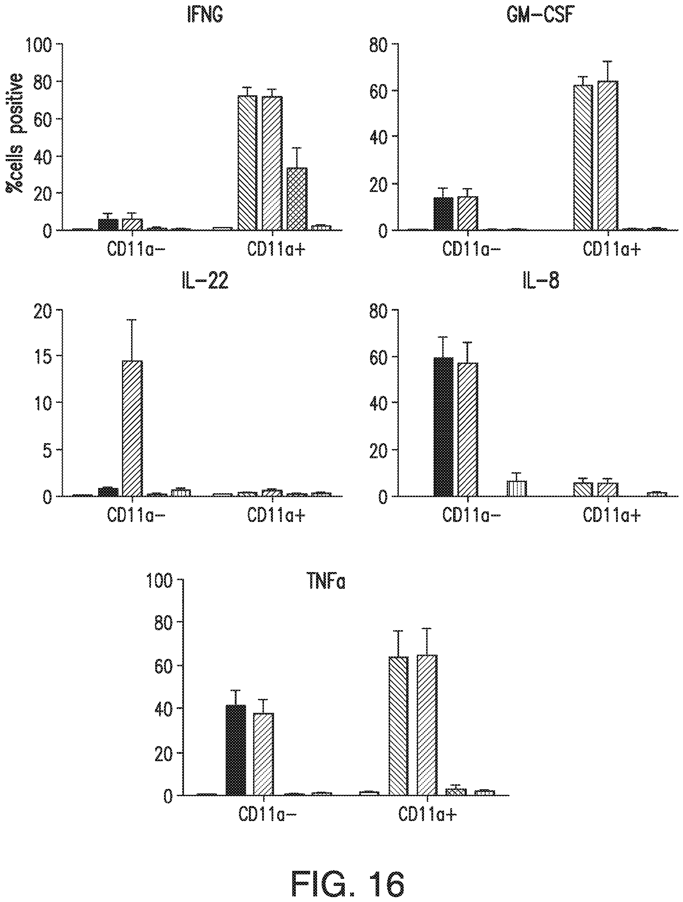

To perform a functional analysis of the two populations, an ICCS (intracellular cytokine staining) assay was used to detect cytokine expression in the three-stage NK cells. Stimulation was performed for 6 hours with 1) PMA + ionomycin, a trigger for IFNy, 2) PMA + ionomycin and IL-23 (to detect IL-22), and 3) cytokines IL-12+IL-18 and IL1b/IL-23 to trigger CD11a+ and CD11a- cells, respectively. The following cytokines were then analyzed in the context of CD56/CD3/ CD117/CD11a/CD94 expression: IFNy, GM-CSF, TNFα, which are NKspecific, and IL-22 an IL-8, which are ILC3-specific. It was determined that CD11a+ cells expressed IFNγ and GM-CSF, wherein CD11a- cells expressed IL-22 and IL-8 (Figure 16). Both CD11a+ cells and CD11a- cells expressed TNFα (Figure 16). Furthermore, it was shown that GM-CSF and IL-22/IL-8 expression were mutually exclusive, and that TNFα can be coexpressed with IL-22 and IL-8 (Figure 16).

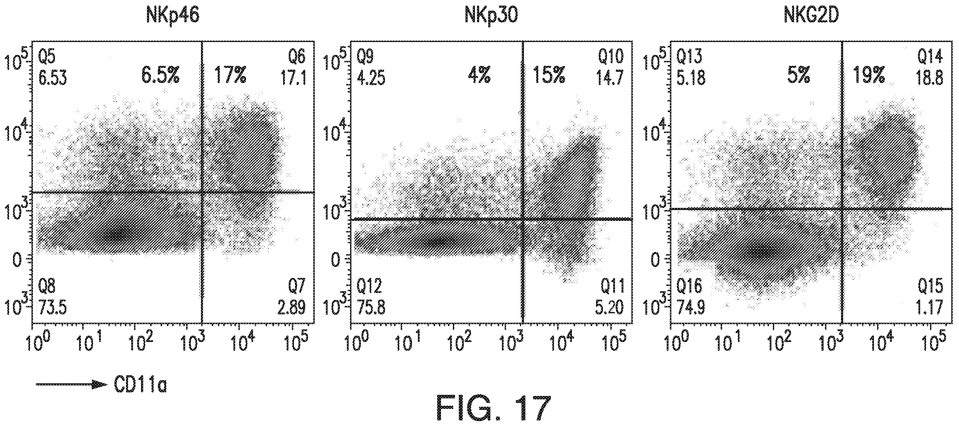

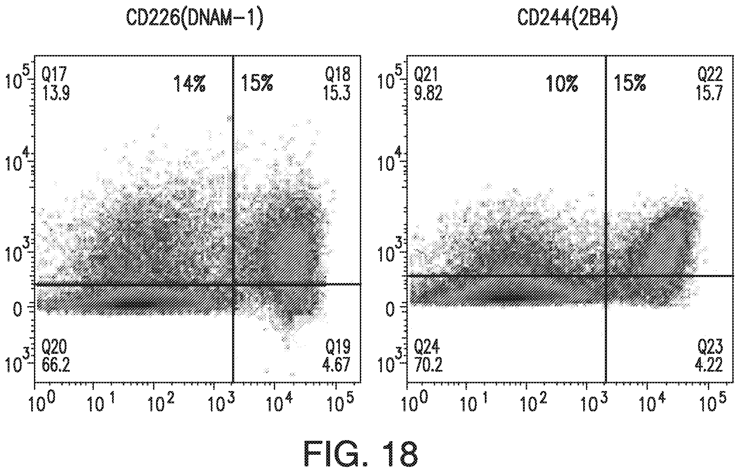

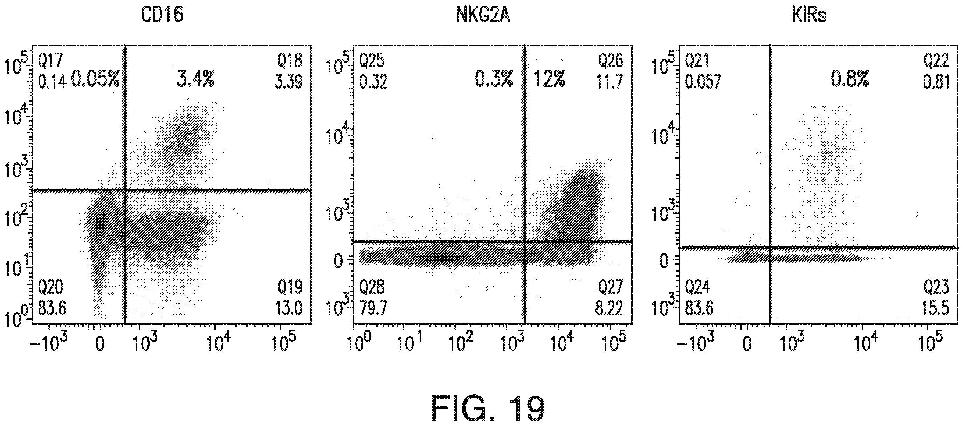

Expression of activating receptors in the three-stage NK subset populations was also determined. It was found that major NK activating receptors NKp46, NKp30, and NKG2D were only expressed in CD11a+ cells (Figure 17). CD226 (DNAM-1) and 2B4 (CD244) were capable of being expressed on CD11a- cells (Figure 18). Expression of CD 16 and KIR was low, and was restricted to CD94+ cells (Figure 19). The total expression of these activating receptors in the three-stage NK cells is summarized in Table 4 for several donors.

**Table 4. Total expression of activating receptors in three-stage NK cells from various donors**

| **% of Total Cells** | **NKp30+** | **NKp46+** | **NKG2D+** | **DNAM-1+** | **2B4+** | **NKG2A+** | **CD16+** |
|---|---|---|---|---|---|---|---|
| Donor 1 | 12.5 | 7.5 | 8.8 | 14.1 | 33.8 | 6.9 | 2.6 |
| Donor 2 | 24.2 | 19.2 | 23.2 | 36.2 | | 12 | 2.2 |
| Donor 3 | 13.8 | 10.6 | 12.2 | 30.3 | | 9.6 | 2.3 |
| Donor 4 | 23.6 | 19.0 | 24.0 | 29.2 | 25.5 | 12.0 | 3.4 |
| Donor 5 | 35.5 | 27.6 | 33.9 | 47.5 | 33.2 | 16.3 | 8.8 |

The CD11a- cells present in the three-stage NK cells recapitulate expression patterns previously described for ILC3 cells, respectively, for example, with respect to RORyt and AHR, and the secretion of IL-22 upon stimulation. However, the CD11a- ("ILC3") cell population found in the three-stage cells has characteristics not previously described for II,C3 cells, for example, the expression of T-bet, TNFα, DNAM-1, and 2B4.

### 7.6. Example 6: Functional Assessment of Two Populations of Three-Stage NK cells

### METHODS

Three-stage NK cells (CD3-CD56+) were subj ected to enrichment of either CD11a+ or CD94+ cells by FITC selection Kit (Stem Cell, Cat#18552) following the protocol provided by the manufacturer. After enrichment, purity of the selected population was evaluated by CD56-PerCP (BD Pharmingen, Cat#560842), CD3-APC-H7 (BD Pharmingen, Cat#560176), CD11a- FITC (BD Pharmingen, Cat#555383), or CD94-FITC (BD Pharmingen, Cat#555388). The cytotoxic activity of selected population was tested by 4h cytotoxicity assay against K562 at different E (effector):T (target) ratio as indicated. In brief, K562 cells were labeled with PKH26 (Sigma-Aldrich, Cat# PKH26-GL), placed in 96-well U-bottom tissue culture plates and incubated with NK cells at different E:T ratio as indicated in 200 µL RPMI1640 supplemented with 10% FBS. After 4h incubation at 37°C in 5% CO₂, cells were harvested and TO-PRO-3 (Invitrogen, Cat# T3605) was added to cultures at 1 µM final concentration followed by FACS analysis using BD FACSCanto II. Cytotoxicity was expressed as the percentage of dead cells (PKH26+TO-PRO-3+) within the total PKH26+ target tumor cells.

### RESULTS AND DISCUSSION

As shown in Table 5, 98% purity of CD11a+, or 91% CD94+ cells was achieved with the enrichment procedure. The CD11a+ population showed the highest cytotoxicity (67.4%) against K562 at an E:T ratio of 2.5:1 while the unfractionated three-stage NK cells exhibited 61.3% cytotoxicity and CD11a- cells exhibited 36.0% cytotoxicity.

**Table 5. Purity of CD11a/CD94 after enrichment**

| Cell Fractionation | CD94% | CD11a% |
|---|---|---|
| whole | 13.1 | 19 |
| CD94+ fraction | 91.4 | |
| CD 11a+ fraction | | 97.9 |
| CD11a- fraction | | 7.48 |

These results show that NK-enriched CD11a+CD3-CD56+ cells exhibit high cytotoxicity against K562 cells, and that ILC3-enriched CD11a-CD3-CD56+ cells exhibit cytolytic activity as well at a lower level when compared with unfractionated cells or NK-enriched cells. The cytotoxicity exhibited by the CD11a-CD3-CD56+ cell population is another unique property of the three-stage ILC3 cells compared to the ILC3 cells described previously.

### 7.7. Example 7: Effect of Varying Cytokine Concentrations on the Purity and Activity of the Three-Stage NK cells

A total of 15 different conditions were evaluated to determine the effect of varying components of the third medium of Example 1 on the two (CD11a+ and CD11a-) populations found in the three-stage NK cells. Table 6 summarizes the different conditions tested.

**Table 6. Fifteen conditions with varying third medium components.**

| | Condition # | IL-7 (ng/ml) | SCF (ng/ml) | IL-2 (ng/ml) | SR1 | IL-15 (ng/ml) |
|---|---|---|---|---|---|---|
| 1 | 1a | 100 | 22 | 1000 | 0uM | 20 |
| 2 | 1b | 100 | 22 | 1000 | 10uM | 20 |
| 3 | 2a | 100 | 0 | 1000 | 0uM | 20 |
| 4 | 2b | 100 | 0 | 1000 | 10uM | 20 |
| 5 | 3a | 20 | 22 | 1000 | 0uM | 20 |
| 6 | 3b | 20 | 22 | 1000 | 10uM | 20 |
| 7 | 4a | 20 | 0 | 1000 | 0uM | 20 |
| 8 | 4b | 20 | 0 | 1000 | 10uM | 20 |
| 9 | 5a | 0 | 22 | 1000 | 0uM | 20 |
| 10 | 6a | 0 | 0 | 1000 | 0uM | 20 |
| 11 | 7a | 20 | 22 | 0 | 0uM | 20 |
| 12 | 7b | 20 | 22 | 0 | 10uM | 20 |
| 13 | 8a | 20 | 0 | 0 | 0uM | 20 |
| 14 | 8b | 20 | 0 | 0 | 10uM | 20 |
| 15 | 9a | 20 | 22 | 1000 | 0uM | 0 |

A flow cytometry panel (Table 7) was utilized for evaluation on days 0, 7, 14, 21, 28, and 35, including cell surface markers CD56, CD3, CD117, CD94, IL1R1, CD11a, CD34, and CD14/CD15 and intracellular markers RORyt, AHR, and perforin.

**Table 7. Panel of antibodies used for flow cytometry evaluation.**

| **Fluorophore** | **Antibody** |
|---|---|
| APCCy7 | CD56 |
| AmCyan | Viability/CD3 |
| BV605 | CD117 |
| PerCPCy5.5 | CD94 |
| AF700 | IL1R1 |
| PECY7 | CD11a |
| BV421 | CD34 |
| BV711 | CD14+CD15 |
| APC | RORγt |
| PE | AHR |
| FITC | Perforin |

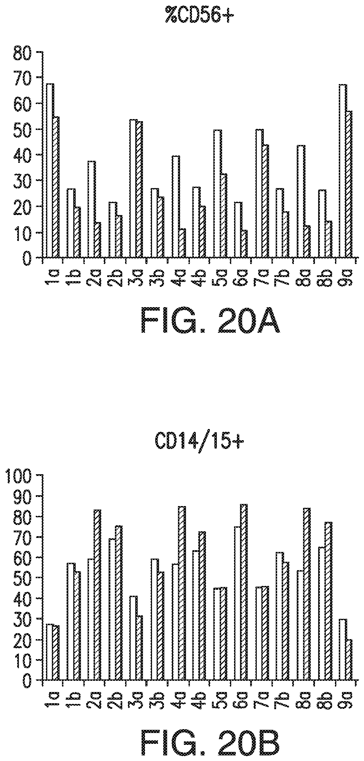

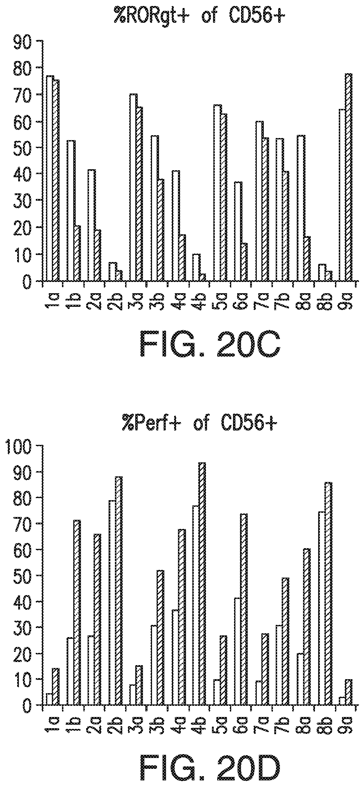

The expression data at day 28 is shown in Figures 20A-D. A summary of the results over the 15 conditions can be found in Table 8.

**Table 8. Effect of varying components of the third medium on the two (CD11a+ and CD1 1a-) populations found in the three-stage NK cells.**

| Conditions | | | | | | D28 results for Donor 1 | | | D28 results for Donor 2 | | |
|---|---|---|---|---|---|---|---|---|---|---|---|
| # | IL-7 (ng/ml) | SCF (ng/ml) | IL-2 (ng/ml) | IL-15 (ng/ml) | SR1 | %CD56+ | %RORgt+ of CD56+ | %Perforin+ of CD56+ | %CD56+ | %RORgt+ of CD56+ | %Perforin+ of CD56+ |
| 1a | 100 | 22 | 1000 | 20 | 0uM | 67 | 77 | 4 | 55 | 75 | 14 |
| 1b | 100 | 22 | 1000 | 20 | 10um | 27 | 53 | 26 | 19 | 21 | 71 |
| 2a | 100 | 0 | 1000 | 20 | 0uM | 37 | 42 | 27 | 14 | 19 | 66 |
| 2b | 100 | 0 | 1000 | 20 | 10um | 22 | 7 | 79 | 16 | 4 | 88 |
| 3a | 20 | 22 | 1000 | 20 | 0uM | 54 | 70 | 8 | 53 | 65 | 15 |
| 3b | 20 | 22 | 1000 | 20 | 10uM | 27 | 54 | 31 | 24 | 38 | 52 |
| 4a | 20 | 0 | 1000 | 20 | 0uM | 40 | 41 | 37 | 11 | 17 | 68 |
| 4b | 20 | 0 | 1000 | 20 | 10um | 27 | 10 | 77 | 20 | 3 | 93 |
| 5a | 0 | 22 | 1000 | 20 | 0uM | 50 | 66 | 10 | 32 | 63 | 27 |
| 6a | 0 | 0 | 1000 | 20 | 0uM | 22 | 37 | 41 | 11 | 14 | 74 |
| 7a | 20 | 22 | 0 | 20 | 0uM | 50 | 60 | 10 | 44 | 53 | 28 |
| 7b | 20 | 22 | 0 | 20 | 10um | 27 | 53 | 31 | 18 | 41 | 49 |
| 8a | 20 | 0 | 0 | 20 | 0uM | 43 | 55 | 20 | 13 | 16 | 60 |
| 8b | 20 | 0 | 0 | 20 | 10um | 26 | 7 | 75 | 14 | 3 | 86 |
| 9a | 20 | 22 | 1000 | 0 | 0uM | 67 | 64 | 3 | 57 | 78 | 10 |

In summary, low SCF in the third medium and the presence of SR1 in the third medium was found to favor NK (CD11a+) cell production, whereas the absence of SR1 in the third medium was found to favor ILC3 (CD11a-) cell production.

### 7.8. Example 8: Additional Characterization and Functional Assessment of Two Populations of Three-Stage NK cells

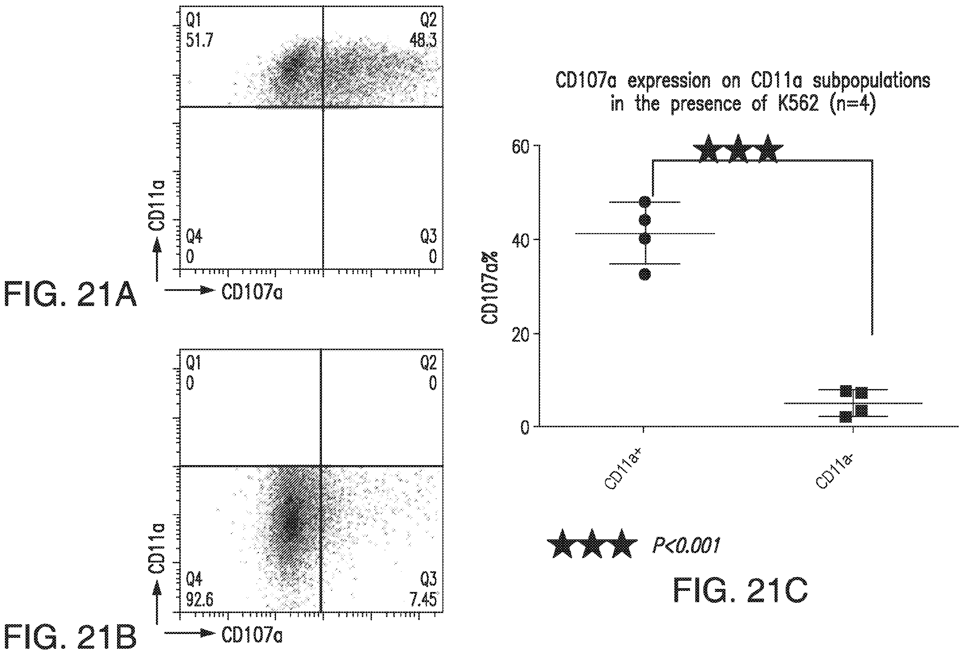

Using FACS, the CD56+CD3- population of three-stage NK cells was evaluated on a CD11a vs. CD117 plot, and the two populations of cells, the CD11a- fraction and the CD11a+ fraction, were shown to be distinguishable by CD107a expression in the presence of K562 cells (Fig. 21A-B). The CD1 1a+ fraction was shown to be capable of degranulation, as measured by a significantly higher CD107a+ fraction in the presence of K562 cells (Fig. 21C).

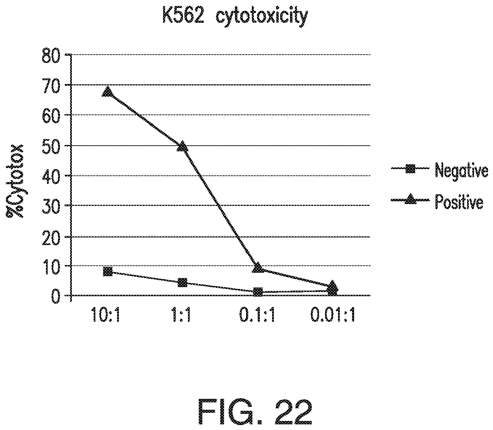

The CD11a+ cells, making up an 81% fraction of the CD56+CD3- population of three-stage NK cells, and the CD11a- cells, making up a 5% fraction of the CD56+CD3-population of three-stage NK cells were tested for cytotoxicity against K562 cells (Fig. 22). The CD11a+ enriched population exhibited a higher K562 cytotoxicity compared to the CD11a-enriched population, especially at higher effector:target cell ratios (Fig. 22).

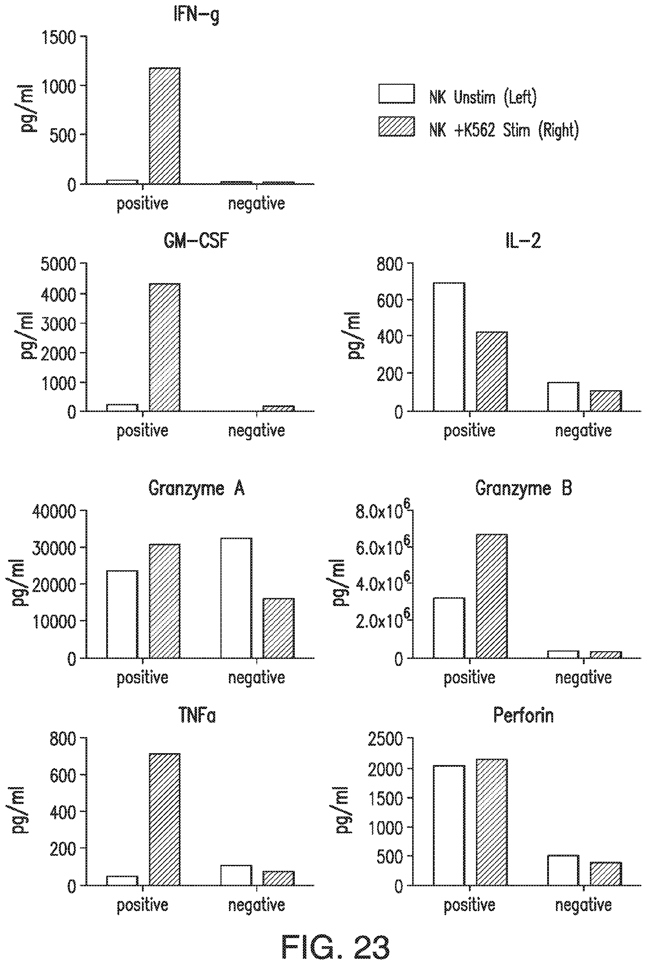

Cytokine secretion analysis was then conducted by overnight co-culture of with K562 target cells and CD11a positive and negative cells, respectively, as effector cells at a 1:1 ratio of effector: target. Supernatant was then collected and analyzed by multiplex. In the presence and absence of K562 cells, the CD11a- and CD11a+ cells could be distinguished in their expression of certain tested cytokines, including Granzyme A, Granzyme B, IFN-y, TNFα, perforin, GM-CSF, and IL-2 (Table 9 and Figure 23). Cytokines IL-17A, IL-22, TNFβ, and IL-10 were found at low levels or below detection (Table 9).

**Table 9. Cytokine secretion analysis of CD11a positive and negative cells in the presence and absence of K562 target cells.**

| | | GzA (pg/mL) | GzB (ng/mL) | IFN-γ (pg/mL) | IL-10 (pg/mL) | TNFα (pg/mL) | Perf (pg/mL) | GM-CSF (ng/mL) | IL-17A (pg/mL) | IL-22 (pg/mL) | IL-2 (pg/mL) | TNFβ (pg/mL) |
|---|---|---|---|---|---|---|---|---|---|---|---|---|
| CD11a- | NK alone | 32233 | 282.62 | 1.65 | below | 106.45 | 502.87 | below | below | below | 141.87 | 24.64 |
| CD11a+ | NK alone | 23322 | 3182 | 19.78 | 3 | 46.62 | 2021 | 0.21 | below | below | 681.98 | below |
| CD11a- | NK+ K562 1:1 | 16034 | 236.17 | 1.99 | below | 71.63 | 382.37 | 0.19 | below | below | 99.25 | 23.47 |
| CD11a+ | NK+ K562 1:1 | 30648 | 6632 | 1169 | 2.43 | 711.62 | 2133 | 4.31 | below | below | 417.45 | 25.47 |

Table 10 provides a summary of CD11a positive and negative cells, as characterized above.

**Table 10. Summary of characterization of CD11a positive and negative cells.**

| | **CD11a+** | **CD11a-** |
|---|---|---|
| **Phenotype** | | |
| Surface markers | CD56+CD3-CD94+ | CD56+CD3-CD117+ILR1+ |
| Intracellular | Perforin+ | |
| | Granzyme+ | |
| | IFN-γ+ | |
| Transcription Markers | T-bet+ | RORγt+ |
| | EOMES+ | AHR+ |

| **Function** | | |
|---|---|---|
| Cytotoxicity | High | Low |
| Degranulation | Yes | Low |
| Cytokine Secretion | Granzyme A | Granzyme A |
| | Granzyme B | Granzyme B |
| | IFN-γ | |
| | TNFα (low) | TNFα (low) |
| | Perforin | Perforin |
| | GM-CSF | |
| | IL-2 | IL-2 |

### Equivalents:

The present invention is not to be limited in scope by the specific embodiments described herein. Indeed, various modifications of the invention in addition to those described will become apparent to those skilled in the art from the foregoing description and accompanying figures. Such modifications are intended to fall within the scope of the appended claims.

## Claims

1. A method of producing a cell population comprising natural killer cells and/or ILC3 cells, comprising the steps of:
(a) culturing hematopoietic stem or progenitor cells in a first medium comprising a stem cell mobilizing agent and thrombopoietin (Tpo) to produce a first population of cells;
(b) culturing the first population of cells in a second medium comprising a stem cell mobilizing agent and interleukin-15 (IL-15), and lacking Tpo, to produce a second population of cells; and
(c) culturing the second population of cells in a third medium comprising IL-2 and IL-15, and lacking low molecular weight heparin (LMWH), to produce a third population of cells;
wherein said method comprises culturing the hematopoietic stem cells in the first medium for 7-13 days; culturing said first population of cells in said second medium for 2-6 days; and culturing said second population of cells in said third medium for 10-30 days;
wherein:
(i) the method is a method of producing a cell population comprising natural killer cells, the third medium lacks a stem cell mobilizing agent, and the third population of cells comprises natural killer cells that are CD56+, CD3-, and wherein at least 80% of the natural killer cells are viable; or
(ii) wherein the method is a method of producing a cell population comprising II,C3 cells, the third medium lacks a stem cell mobilizing agent, and the method further comprises the step of:
(d) isolating CD11a- cells from the third population of cells to produce a fourth population of cells
wherein the fourth population of cells comprises ILC3 cells that are CD56+, CD3-, and CD11a-; or
(iii) wherein the method is a method of producing a cell population comprising natural killer cells and ILC3 cells, the third medium lacks a stem cell mobilizing agent, and the method further comprises the steps of:
e) separating CD11a+ cells and CD11a- cells from the third population of cells; and
(f) combining the CD11a+ cells with the CD11a- cells in a ratio of 50:1, 40:1, 30:1, 20:1, 10:1, 5:1, 4:1, 3:1, 2:1, 1:1, 1:2, 1:3, 1:4, 1:5, 1:10, 1:20, 1:30, 1:40, or 1:50 to produce a fourth population of cells; wherein said stem cell mobilizing agent is an aryl hydrocarbon receptor inhibitor, wherein: said aryl hydrocarbon receptor inhibitor is StemRegenin-1 (SR-1) (4-(2-(2-(benzo[b]thiophen-3-yl)-9-isopropyl-9H-purin- 6-ylamino)ethyl)phenol), or the compound CH223191 (1-Methyl-N-[2-methyl-4-[2-(2-methylphenyl)diazenyl]phenyl-1H-pyrazole-5-carboxamide].

2. The method of claim 1(i), wherein said third population of cells comprises natural killer cells that are (i) CD94+ or CD16+, (ii)CD94- or CD16-, (iii) CD94+ and CD16+, or (iv) CD94- and CD16-.

3. The method of claim 1(ii), wherein: (i) said ILC3 cells express RORγt and IL1R1 and/or (ii) wherein said ILC3 cells do not express either perforin or EOMES.

4. The method of any one of claims 1-3, wherein said hematopoietic stem or progenitor cells are mammalian cells, preferably wherein said hematopoietic stem or progenitor cells are human cells.

5. The method of any one of claims 1-4, wherein said hematopoietic stem or progenitor cells are CD34+ hematopoietic stem cells.

6. The method of any one of claims 1-5, wherein said hematopoietic stem or progenitor cells are:
(i) placental cells, optionally wherein:
(a) said placental cells are obtained from, or obtainable from, human placental perfusate, or
(b) said placental cells are obtained from, or obtainable from, nucleated cells isolated from human placental perfusate; or
(ii) obtained from, or obtainable from, umbilical cord blood, or
(iii) fetal liver cells, or
(iv) mobilized peripheral blood cells, or
(v) bone marrow cells.

7. The method of any of claims 1-6, wherein said Tpo, IL-2, and IL-15 are not comprised within an undefined component of the first medium, second medium or third medium, or wherein said Tpo, IL-2, and IL-15 are not comprised within serum.

8. The method of any of claims 1-7, wherein said first or second medium additionally comprises one or more of LMWH, Flt-3 Ligand (Flt-3L), stem cell factor (SCF), IL-6, IL-7, granulocyte colony-stimulating factor (G-CSF), or granulocyte-macrophage-stimulating factor (GM-CSF).

9. The method of any of claims 1-8, wherein said first or second medium does not comprise LMWH, and/or wherein said first medium does not comprise a desulphated glycosaminoglycan.

10. The method of any of claims 1-9, wherein said third medium additionally comprises one or more of IL-6, IL-7, G-CSF, or GM-CSF.

11. The method of any of claims 1-10, wherein:
(a) said method comprises culturing the hematopoietic stem cells in the first medium for 8-12 days, in particular in particular for about 10 days; or
(b) said method comprises culturing said first population of cells in said second medium for 3-5 days, in particular for about 4 days; and/or
(c) said method comprises culturing said second population of cells in said third medium for 15-25 days, in particular for about 21 days; and/or
(d) said culturing in said first medium, second medium and third medium are all done under static culture conditions, or wherein said culturing in at least one of said first medium, second medium or third medium are done in a spinner flask, or wherein said culturing in said first medium and said second medium is done under static culture conditions, and said culturing in said third medium is done in a spinner flask; and/or
(e) said hematopoietic cells are initially inoculated into said first medium from 1 × 10⁴ to 1 × 10⁵ cells/mL, in particular wherein said hematopoietic cells are initially inoculated into said first medium at about 3 × 10⁴ cells/mL; and/or
(f) said first population of cells are initially inoculated into said second medium from 5 × 10⁴ to 5 × 10⁵ cells/mL, in particular at about 1 × 10⁵ cells/mL; and/or
(g) said second population of cells is initially inoculated into said third medium from 1 × 10⁵ to 5 × 10⁶ cells/mL, in particular from 1 × 10⁵ to 1 × 10⁶ cells/mL, in particular at about 5 × 10⁵ cells/mL or about 3 × 10⁵ cells/mL.

12. The method of claim 1(iii), wherein:
(a) said first medium and/or said second medium lack LIF and/or MIP-1α, and/or
(b) said third medium lacks LIF, MIP-1α, and Flt-3L, and/or
(c) said first medium and said second medium lack LIF and MIP-1α, and said third medium lacks LIF, MIP-1α, and Flt3L, and/or
(d) none of the first medium, second medium or third medium comprises a desulphated glycosaminoglycan, and/or
(e) the first medium, second medium or third medium comprises heparin, optionally wherein said heparin is low-molecular weight heparin (LMWH), and/or
(f) in the fourth population of cells, the CD11a+ cells and CD11a- cells are combined in a ratio of 50:1, 20:1, 10:1, 5:1, 1:1, 1:5, 1:10, 1:20, or 1:50.

## Patentansprüche

1. Verfahren zur Herstellung einer natürliche Killerzellen und/oder ILC3-Zellen umfassenden Zellpopulation, das die folgenden Schritte umfasst:
(a) das Kultivieren von hämatopoetischen Stamm- oder Vorläuferzellen in einem ersten Medium, das ein Stammzellen-Mobilisierungsmittel und Thrombopoetin (Tpo) umfasst, um eine erste Population von Zellen zu produzieren;
(b) das Kultivieren der ersten Population von Zellen in einem zweiten Medium, das ein Stammzellen-Mobilisierungsmittel und Interleukin-15 (IL-15) umfasst und kein Tpo umfasst, um eine zweite Population von Zellen zu produzieren; und
(c) das Kultivieren der zweiten Population von Zellen in einem dritten Medium, das IL-2 und IL-15 umfasst und kein niedermolekulares Heparin (LMWH) umfasst, um eine dritte Population von Zellen zu produzieren;
wobei das Verfahren das Kultivieren der hämatopoetischen Stammzellen im ersten Medium für 7-13 Tage, das Kultivieren der ersten Population von Zellen im zweiten Medium für 2-6 Tage und das Kultivieren der zweiten Population von Zellen im dritten Medium für 10-30 Tage umfasst;
wobei:
(i) das Verfahren ein Verfahren zur Produktion einer Zellpopulation ist, die natürliche Killerzellen umfasst, wobei das dritte Medium kein Stammzellen-Mobilisierungsmittel umfasst und die dritte Population von Zellen natürliche Killerzellen umfasst, die CD56+, CD3- sind, und wobei zumindest 80 % der natürlichen Killerzellen lebensfähig sind; oder
(ii) wobei das Verfahren ein Verfahren zur Produktion einer Zellpopulation ist, die ILC3-Zellen umfasst, wobei das dritte Medium kein Stammzellen-Mobilisierungsmittel umfasst und das Verfahren weiters den folgenden Schritt umfasst:
(d) das Isolieren von CD11a- Zellen aus der dritten Population von Zellen, um eine vierte Population von Zellen zu produzieren,
wobei die vierte Population von Zellen ILC3-Zellen umfasst, die CD56+, CD3- und CD11a-sind; oder
(iii) wobei das Verfahren ein Verfahren zur Produktion einer Zellpopulation ist, die natürliche Killerzellen und ILC3-Zellen umfasst, wobei das dritte Medium kein Stammzellen-Mobilisierungsmittel umfasst und das Verfahren weiters die folgenden Schritte umfasst:
(e) das Abtrennen der CD11a+ Zellen und CD11a- Zellen von der dritten Population von Zellen; und
(f) das Vereinigen der CD11a+ Zellen mit den CD11a- Zellen in einem Verhältnis von 50:1, 40:1, 30:1, 20:1, 10:1, 5:1, 4:1, 3:1, 2:1, 1:1, 1:2, 1:3, 1:4, 1:5, 1:10, 1:20, 1:30, 1:40 oder 1:50, um eine vierte Population von Zellen zu produzieren; wobei das Stammzellen-Mobilisierungsmittel ein Arylkohlenwasserstoff-Rezeptor-Inhibitor ist, wobei der Arylkohlenwasserstoff-Rezeptor-Inhibitor StemRegenin-1 (SR-1), (4-(2-(2-(Benzo[b]thiophen-3-yl)-9-isopropyl-9H-purin-6-ylamino)ethyl)phenol) oder die Verbindung CH223191 (1-Methyl-N-[2-methyl-4-[2-(2-methylphenyl)diazenyl]phenyl-1H-pyrazol-5-carbonsäureamid] ist.

2. Verfahren nach Anspruch 1(i), wobei die dritte Population von Zellen natürliche Killerzellen umfasst, die (i) CD94+ oder CD16+, (ii) CD94- oder CD16-, (iii) CD94+ und CD16+ oder (iv) CD94- und CD16- sind.

3. Verfahren nach Anspruch 1(ii), wobei (i) die ILC3-Zellen RORyt und IL1R1 exprimieren und/oder (ii) die ILC3-Zellen weder Perforin noch EOMES exprimieren.

4. Verfahren nach einem der Ansprüche 1 bis 3, wobei die hämatopoetischen Stammoder Vorläuferzellen Säugetierzellen sind, wobei die hämatopoetischen Stamm- oder Vorläuferzellen vorzugsweise menschliche Zellen sind.

5. Verfahren nach einem der Ansprüche 1 bis 4, wobei die hämatopoetischen Stammoder Vorläuferzellen hämatopoetische CD34+ Stammzellen sind.

6. Verfahren nach einem der Ansprüche 1 bis 5, wobei die hämatopoetischen Stammoder Vorläuferzellen:
(i) Plazentazellen sind, wobei gegebenenfalls
(a) die Plazentazellen von menschlichem Plazentaperfusat erhalten werden oder erhältlich sind oder
(b) die Plazentazellen von kernhaltigen Zellen erhalten werden oder erhältlich sind, die aus menschlichem Plazentaperfusat isoliert wurden; oder
(ii) von Nabelschnurblut oder
(iii) von fötalen Leberzellen oder
(iv) von mobilisierten peripheren Blutzellen oder
(v) von Knochenmarkszellen erhalten werden oder erhältlich sind.

7. Verfahren nach einem der Ansprüche 1 bis 6, wobei das Tpo, IL-2 und IL-15 nicht in einer undefinierten Komponente des ersten Mediums, des zweiten Mediums oder des dritten Mediums enthalten sind oder wobei das Tpo, IL-2 und IL-15 nicht in Serum enthalten sind.

8. Verfahren nach einem der Ansprüche 1 bis 7, wobei das erste oder das zweite Medium zusätzlich eines oder mehrere aus LMWH, Flt-3-Ligand (Flt-3L), Stammzellenfaktor (SCF), IL-6, IL-7, Granulozyten-Kolonie-stimulierendem Faktor (G-CSF) und Granulozyten-Makrophagen-stimulierendem Faktor (GM-CSF) umfasst.

9. Verfahren nach einem der Ansprüche 1 bis 8, wobei das erste oder das zweite Medium kein LMWH umfasst und/oder wobei das erste Medium kein desulfatiertes Glykosaminoglykan umfasst.

10. Verfahren nach einem der Ansprüche 1 bis 9, wobei das dritte Medium zusätzlich eines oder mehrere aus IL-6, IL-7, G-CSF und GM-CSF umfasst.

11. Verfahren nach einem der Ansprüche 1 bis 10, wobei:
(a) das Verfahren das Kultivieren der hämatopoetischen Stammzellen im ersten Medium für 8-12 Tage, insbesondere für etwa 10 Tage, umfasst; oder
(b) das Verfahren das Kultivieren der ersten Population von Zellen im zweiten Medium für 3-5 Tage, insbesondere für etwa 4 Tage, umfasst; und/oder
(c) das Verfahren das Kultivieren der zweiten Population von Zellen im dritten Medium für 15-25 Tage, insbesondere für etwa 21 Tage, umfasst; und/oder
(d) das Kultivieren im ersten Medium, im zweiten Medium und im dritten Medium alle unter statischen Kulturbedingungen erfolgen oder wobei das Kultivieren in zumindest einem aus dem ersten Medium, dem zweiten Medium und dem dritten Medium in einer Spinnerflasche erfolgt oder wobei das Kultivieren im ersten Medium und im zweiten Medium unter statischen Kulturbedingungen erfolgt und das Kultivieren im dritten Medium in einer Spinnerflasche erfolgt; und/oder
(e) die hämatopoetischen Zellen anfangs mit 1 × 10⁴ bis 1 × 10⁵ Zellen/ml in das erste Medium inokuliert werden, wobei die hämatopoetischen Zellen insbesondere anfangs mit 3 × 10⁴ Zellen/ml in das erste Medium inokuliert werden; und/oder
(f) die erste Population von Zellen anfangs mit 5 × 10⁴ bis 5 × 10⁵ Zellen/ml, insbesondere etwa 1 × 10⁵ Zellen/ml, in das zweite Medium inokuliert wird; und/oder
(g) die zweite Population von Zellen anfangs mit 1 × 10⁵ bis 5 × 10⁶ Zellen/ml, insbesondere 1 × 10⁵ bis 1 × 10⁶ Zellen/ml, insbesondere etwa 5 × 10⁵ Zellen/ml oder etwa 3 × 10⁵ Zellen/ml, in das dritte Medium inokuliert wird.

12. Verfahren nach Anspruch 1(iii), wobei:
(a) das erste Medium und/oder das zweite Medium kein LIF und/oder MIP-1α aufweisen und/oder
(b) das dritte Medium kein LIF, MIP-1α und Flt-3L aufweist und/oder
(c) das erste Medium und das zweite Medium kein LIF und MIP-1α aufweisen und das dritte Medium kein LIF, MIP-1α und Flt3L aufweist und/oder
(d) keines aus dem ersten Medium, dem zweiten Medium und dem dritten Medium ein desulfatiertes Glykosaminoglykan umfasst und/oder
(e) das erste Medium, das zweite Medium oder das dritte Medium Heparin umfasst, wobei das Heparin gegebenenfalls niedermolekulares Heparin (LMWH) ist, und/oder
(f) in der vierten Population von Zellen die CD11a+ Zellen und die CD11a- Zellen in einem Verhältnis von 50:1, 20:1, 10:1, 5:1, 1:1, 1:5, 1:10, 1:20 oder 1:50 vereinigt werden.

## Revendications

1. Procédé de production d'une population de cellules comprenant des cellules tueuses naturelles et/ou des cellules ILC3, comprenant les étapes consistant à :
a) cultiver des cellules souches ou progénitrices hématopoïétiques dans un premier milieu comprenant un agent de mobilisation de cellules souches et de la thrombopoïétine (Tpo) pour produire une première population de cellules ;
(b) cultiver la première population de cellules dans un deuxième milieu comprenant un agent de mobilisation de cellules souches et de l'interleukine-15 (IL-15), et dépourvu de Tpo, pour produire une deuxième population de cellules ; et
(c) cultiver la deuxième population de cellules dans un troisième milieu comprenant de l'IL-2 et de l'IL-15, et dépourvu d'héparine à faible poids moléculaire (LMWH), pour produire une troisième population de cellules ;
dans lequel ledit procédé comprend une culture des cellules souches hématopoïétiques dans le premier milieu pendant 7 à 13 jours ; une culture de ladite première population de cellules dans ledit deuxième milieu pendant 2 à 6 jours ; et une culture de ladite deuxième population de cellules dans ledit troisième milieu pendant 10 à 30 jours ;
dans lequel :
(i) le procédé est un procédé de production d'une population de cellules comprenant des cellules tueuses naturelles, le troisième milieu est dépourvu d'un agent de mobilisation de cellules souches, et la troisième population cellulaire comprenant des cellules tueuses naturelles qui sont CD56+, CD3-, et dans lequel au moins 80 % des cellules tueuses naturelles sont viables ; ou
(ii) dans lequel le procédé est un procédé de production d'une population de cellules comprenant des cellules ILC3, le troisième milieu est dépourvu d'un agent de mobilisation de cellules souches, et le procédé comprend en outre l'étape consistant à
(d) isoler des cellules CD11 a- de la troisième population de cellules afin de produire une quatrième population de cellules ;
dans lequel la quatrième population de cellules comprend des cellules ILC3 qui sont CD56+, CD3- et CD11a- ; ou
(iii) dans lequel le procédé est un procédé de production d'une population de cellules comprenant des cellules tueuses naturelles et des cellules ILC3, le troisième milieu est dépourvu d'un agent de mobilisation de cellules souches, et le procédé comprend en outre les étapes consistant à :
(e) séparer des cellules CD11a+ et des cellules CD11a-de la troisième population de cellules ; et
(f) combiner les cellules CD11a+ avec les cellules CD11a- à un rapport de 50:1, 40:1, 30:1, 20:1, 10:1, 5:1, 4:1, 3:1, 2:1, 1: 1, 1:2, 1:3, 1:4, 1:5, 1:10, 1:20, 1:30, 1:40 ou 1:50 pour produire une quatrième population de cellules ; dans lequel ledit agent de mobilisation de cellules souches est un inhibiteur de récepteur d'hydrocarbure aryle, dans lequel : ledit inhibiteur de récepteur d'hydrocarbure aryle est le StemRegenin-1 (SR-1) (4-(2-(2-(benzo[b]thiophèn-3-yl)-9-isopropyl-9H-purin-6-ylamino)éthyl)phénol), ou le composé CH223191 (1-Méthyl-N-[2-méthyl-4-[2[-(2-méthylphényl)diazényl]phényl-1H-pyrazole-5-carboxamide].

2. Procédé selon la revendication 1(i), dans lequel ladite troisième population de cellules comprend des cellules tueuses naturelles qui sont (i) CD94+ ou CD16+, (ii) CD94-ou CD16-, (iii) CD94+ et CD16+, ou (iv) CD94- et CD16-.

3. Procédé selon la revendication 1(ii), dans lequel :
(i) lesdites cellules ILC3 expriment RORyt et IL1R1 et/ou
(ii) dans lequel lesdites cellules ILC3 n'expriment ni perforine ni EOMES.

4. Procédé selon l'une quelconque des revendications 1 à 3, dans lequel lesdites cellules souches ou progénitrices hématopoïétiques sont des cellules de mammifère, de préférence dans lequel lesdites cellules souches ou progénitrices hématopoïétiques sont des cellules humaines.

5. Procédé selon l'une quelconque des revendications 1 à 4, dans lequel lesdites cellules souches ou progénitrices hématopoïétiques sont des cellules souches hématopoïétiques CD34+.

6. Procédé selon l'une quelconque des revendications 1 à 5, dans lequel lesdites cellules souches ou progénitrices hématopoïétiques sont :
(i) des cellules placentaires, facultativement dans lequel :
(a) lesdites cellules placentaires sont obtenues ou peuvent être obtenues à partir d'un perfusat placentaire humain, ou
(b) lesdites cellules placentaires sont obtenues ou peuvent être obtenues à partir de cellules nucléées isolées à partir de perfusat placentaire humain ; ou
(ii) obtenues ou peuvent être obtenues à partir de sang de cordon ombilical, ou
(iii) cellules hépatiques fœtales, ou
(iv) des cellules sanguines périphériques mobilisées, ou
(v) des cellules de moelle osseuse.

7. Procédé selon l'une quelconque des revendications 1 à 6, dans lequel lesdites Tpo, IL-2 et IL-15 ne sont pas comprises dans un composant indéfini du premier milieu, du deuxième milieu ou du troisième milieu, ou dans lequel lesdites Tpo, IL-2 et IL-15 ne sont pas comprise dans du sérum.

8. Procédé selon l'une quelconque des revendications 1 à 7, dans lequel ledit premier ou ledit deuxième milieu comprend en outre un ou plusieurs parmi LMWH, ligand Flt-3 (Flt-3L), facteur de cellule souche (SCF), IL-6, IL-7, facteur de stimulation de colonies de granulocytes (G-CSF) ou facteur de stimulation de macrophages de granulocytes (GM-CSF) .

9. Procédé selon l'une quelconque des revendications 1 à 8, dans lequel ledit premier milieu ou ledit deuxième milieu ne comprend pas de LMWH, et/ou dans lequel ledit premier milieu ne comprend pas de glycosaminoglycane désulfaté.

10. Procédé selon l'une quelconque des revendications 1 à 9, dans lequel ledit troisième milieu comprend en outre un ou plusieurs parmi IL-6, IL-7, G-CSF ou GM-CSF.

11. Procédé selon l'une quelconque des revendications 1 à 10, dans lequel :
(a) ledit procédé comprend une culture des cellules souches hématopoïétiques dans le premier milieu pendant 8 à 12 jours, en particulier pendant environ 10 jours ; ou
(b) ledit procédé comprend une culture de ladite première population de cellules dans ledit deuxième milieu pendant 3 à 5 jours, en particulier pendant environ 4 jours ; et/ou
(c) ledit procédé comprend une culture de ladite deuxième population de cellules dans ledit troisième milieu pendant 15 à 25 jours, en particulier pendant environ 21 jours ; et/ou
(d) ladite culture dans ledit premier milieu, ledit deuxième milieu et ledit troisième milieu sont toutes effectuées dans des conditions de culture statique, ou dans lequel ladite culture dans au moins l'un dudit premier milieu, dudit deuxième milieu ou dudit troisième milieu est effectuée dans un flacon rotatif, ou dans lequel ladite culture dans ledit premier milieu et ledit deuxième milieu est effectuée dans des conditions de culture statique, et ladite culture dans ledit troisième milieu est effectuée dans un flacon rotatif ; et/ou
(e) lesdites cellules hématopoïétiques sont initialement inoculées dans ledit premier milieu entre 1 × 10⁴ et 1 × 10⁵ cellules/ml, en particulier dans lequel lesdites cellules hématopoïétiques sont initialement inoculées dans ledit premier milieu à environ 3 × 10⁴ cellules/ml ; et/ou
(f) ladite première population de cellules est initialement inoculée dans ledit deuxième milieu entre 5 × 10⁴ et 5 × 10⁵ cellules/ml, en particulier à environ 1 × 10⁵ cellules/ml ; et/ou
(g) ladite deuxième population de cellules est initialement inoculée dans ledit troisième milieu entre 1 × 10⁵ et 5 × 10⁶ cellules/ml, en particulier entre 1 × 10⁵ et 1 × 10⁶ cellules/ml, en particulier à environ 5 × 10⁵ cellules/ml ou environ 3 × 10⁵ cellules/ml.

12. Procédé selon la revendication 1(iii), dans lequel :
(a) ledit premier milieu et/ou ledit deuxième milieu sont dépourvus de LIF et/ou de MIP-1α, et/ou
(b) ledit troisième milieu est dépourvu de LIF, MIP-1α et Flt-3L, et/ou
(c) ledit premier milieu et ledit deuxième milieu sont dépourvus de LIF et de MIP-1α, et ledit troisième milieu est dépourvu de LIF, MIP-1α et Flt3L, et/ou
(d) aucun des premier, deuxième et troisième milieux ne comprend de glycosaminoglycane désulfaté, et/ou
(e) le premier milieu, le deuxième milieu ou le troisième milieu comprend de l'héparine, facultativement dans lequel ladite héparine est de l'héparine à faible poids moléculaire (LMWH), et/ou
(f) dans la quatrième population de cellules, les cellules CD11a+ et les cellules CD11a- sont combinées à un rapport de 50:1, 20:1, 10:1, 5:1, 1:1, 1:5, 1:10, 1:20 ou 1:50.
